# EUROPÄISCHE PATENTSCHRIFT

(11) **EP 1 515 967 B1**
(45) Veröffentlichungstag und Bekanntmachung des Hinweises auf die Patenterteilung: **02.11.2006**
(21) Anmeldenummer: 03732517.2
(22) Anmeldetag: 02.06.2003
(51) Int. Cl.: C07D 453/02, A61K 31/439, A61P 25/00

(54) **2-HETEROARYLCARBONSÄUREAMIDE**
2-HETEROARYL CARBOXAMIDES
2-HETEROARYLCARBOXAMIDES

(30) Priorität: 10.06.2002 DE 10225536; 06.12.2002 DE 10257078; 10.12.2002 DE 10257537; 13.02.2003 DE 10305922
(43) Veröffentlichungstag der Anmeldung: 23.03.2005
(73) Patentinhaber: Bayer HealthCare AG, 51368 Leverkusen (DE)
(72) Erfinder: LUITHLE, Joachim, 42489 Wülfrath (DE); BÖSS, Frank-Gerhard, Berkshire SL57DF (GB); ERB, Christina, Kriftel 65830 (DE); HAFNER, Frank-Thorsten, 42115 Wuppertal (DE); SCHNIZLER, Katrin, 63517 Rodenbach (DE); FLESSNER, Timo, 42113 Wuppertal (DE); VAN KAMPEN, Marja, 40223 Düsseldorf (DE); VAN DER STAAY, Franz-Josef, NL 8251 BB Dronten (NL)
(86) Internationale Anmeldenummer: PCT/EP2003/005735
(87) Internationale Veröffentlichungsnummer: WO 2003/104227

(56) Entgegenhaltungen:
- WO-A-02/100857
- WO-A-03/029252
- WO-A-03/055878

## Beschreibung

Die Erfindung betrifft neue 2-Heteroarylcarbonsäureamide, Verfahren zu ihrer Herstellung sowie ihre Verwendung zur Herstellung von Arzneimitteln zur Behandlung und/oder Prophylaxe von Krankheiten und zur Verbesserung der Wahrnehmung, Konzentrationsleistung, Lernleistung und/oder Gedächtnisleistung.

Nikotinische Acetylcholin-Rezeptoren (nAChR) bilden eine große Familie von lonenkanälen, die durch den körpereigenen Botenstoff Acetylcholin aktiviert werden (Galzi et al., *Neuropharmacol.* 1995, *34*, 563-582). Ein funktioneller nAChR besteht aus fünf Untereinheiten, die Unterschiedlich (bestimmte Kombinationen von α1-9- und β1-4,γ,δ,ε-Untereinheiten) oder identisch (α7-9) sein können. Dies führt zur Bildung einer Vielfalt von Subtypen, die eine unterschiedliche Verteilung in der Muskulatur, dem Nervensystem und anderen Organen zeigen (McGehee et al., *Annu. Rev. Physiol.* 1995, *57,* 521-546). Aktivierung von nAChR führt zum Einstrom von Kationen in die Zelle und zur Stimulation von Nerven- oder Muskelzellen. Selektive Aktivierung einzelner nAChR-Subtypen beschränkt diese Stimulation auf die Zelltypen, die den entsprechenden Subtyp besitzen und kann so unerwünschte Nebeneffekte, wie z.B. die Stimulierung von nAChR in der Muskulatur, vermeiden. Klinische Experimente mit Nikotin und Experimente in verschiedenen Tiermodellen weisen auf eine Rolle von zentralen nikotinischen Acetylcholin-Rezeptoren bei Lern- und Gedächtnisvorgängen hin (z.B. Rezvani et al., *Biol. Psychiatry* 2001, *49,* 258-267). Nikotinische Acetylcholinrezeptoren des alpha7-Subtyps (α7-nAChR) haben eine besonders hohe Konzentration in für Lernen und Gedächtnis wichtigen Hirnregionen, wie dem Hippocampus und dem cerebralen Cortex (Séguéla et al., *J. Neurosci.* 1993, *13*, 596-604). Der α7-nAChR besitzt eine besonders hohe Durchlässigkeit für Calcium-Ionen, erhöht glutamaterge Neurotransmission, beeinflusst das Wachstum von Neuriten und moduliert auf diese Weise die neuronale Plastizität (Broide et al., *Mol. Neurobiol.* 1999, *20,* 1-16).

Bestimmte *N*-(1-Aza-bicyclo[2.2.2]oct-3-yl)-heteroarylcarbonsäureamide zur Behandlung von u.a. Psychosen sind in der DE-A 37 24 059 beschrieben.

*N*-(Aza-bicycloalkyl)-heteroarylcarbonsäureamide, insbesondere *N*-(1-Aza-bicyclo-[2.2.2]oct-4-yl)-benzothiophen-3-carbonsäureamide, werden in der WO 93/15073 bzw. in der EP-A 485 962 als Zwischenstufen für die Synthese von pharmazeutisch wirksamen Verbindungen offenbart.

Aus der US 4,605,652 und der EP-A 372 335 sind beispielsweise *N*-(1-Aza-bicyclo[2.2.2]oct-3-yl)-thiophen-2-carbonsäureamid und seine gedächtnisverbessernde Wirkung bekannt.

In JP-A 14 030 084 werden 1-Azabicyloalkane zur Behandlung von u. a. Demenz, Attention Deficit Hyperactivity Disorder und Lern- und Gedächtnisstörungen beschrieben.

Aus WO 02/44176, WO. 02/085901, WO 01/60821, EP-A 1 231 212 und EP-A 1 219 622 sind weitere α7-nicotinische Acetylcholinrezeptor-Agonisten zur Behandlung von Krankheiten des Zentralen Nervensystems bekannt.

Die vorliegende Erfindung betrifft Verbindungen der Formel 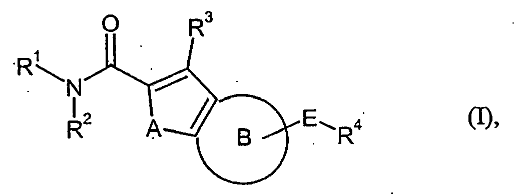 in welcher
- R¹: 1-Aza-bicyclo[2.2.2]oct-3-yl, welches gegebenenfalls über das Stickstoffatom mit einem Rest ausgewählt aus der Gruppe C₁-C₄-Alkyl, Benzyl und Oxy substituiert ist,
- R²: Wasserstoff oder C₁-C₆-Alkyl,
- R³: Wasserstoff, Halogen oder C₁-C₆-Alkyl,
- R⁴: Wasserstoff, Halogen, Cyano, Amino, Trifluormethyl, Trifluormethoxy, C₁₋C₆-Alkyl, C₁-C₆-Alkylcarbonyl, C₁-C₆-Alkylamino, Formyl, Hydroxycarbonyl, C₁-C₆-Alkoxy, C₁-C₆-Alkoxycarbonyl, C₁-C₆-Alkylthio, C₁-C₆₋Alkylcarbonylamino, C₁-C₆-Alkylaminocarbonyl, C₁-C₄-Alkylsulfonylamino, C₃-C₈-Cycloalkylcarbonylamino, C₃-C₆-Cycloalkylaminocarbonyl, Pyrrolyl, C₁-C₆-Alkylaminocarbonylamino, Heterocyclylcarbonyl, Heterocyclylcarbonylamino, Heteroarylcarbonylamino, Hydroxy, Phenyl oder Heterocyclyl,
wobei
C₁-C₆-Alkyl gegebenenfalls mit Hydroxy, Cyano, Amino, C₁-C₆-Alkylaminocarbonylamino, C₁-C₆-Alkylaminocarboxyl, Heterocyclyl oder Aryl,
C₁-C₆-Alkylaminocarbonyl gegebenenfalls mit C₁-C₆-Alkoxy oder C₁-C₆₋Alkylamino, C₁-C₆-Alkylcarbonylamino gegebenenfalls mit C₁-C₆-Alkoxy, und Heterocyclyl gegebenenfalls mit Oxo substituiert sein können,
- A: Sauerstoff oder Schwefel,
- der Ring B: Benzo oder Pyrido, die jeweils gegebenenfalls durch Reste aus der Reihe Halogen, Cyano, Formyl, Trifluormethyl, Trifluormethoxy, Nitro, Amino, C₁-C₆-Alkyl und C₁-C₆-Alkoxy substituiert sind,
und
- E: C≡C, Aryl und Heteroaryl, wobei Aryl und Heteroaryl durch Reste aus der Reihe Halogen, Cyano, Trifluormethyl, Trifluormethoxy, Nitro, Amino, C₁₋C₆-Alkoxy und C₁-C₆-Alkyl substituiert sein können,
bedeuten, sowie die Solvate, Salze oder Solvate der Salze dieser Verbindungen.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Als Salze sind im Rahmen der Erfindung physiologisch unbedenkliche Salze der erfindungsgemäßen Verbindungen bevorzugt.

Physiologisch unbedenkliche Salze der Verbindungen (I) können Säureadditionssalze der Verbindungen mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Oxalsäure, Milchsäure, Weinsäure, Zitronensäure, Fumarsäure, Maleinsäure oder Benzoesäure.

Als Salze können aber auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Monoethanolamin, Diethanolamin, Triethanolamin, Arginin, Lysin, Dimethylaminoethanol, Diethylamin, Triethylamin, Ethyldiisopropylamin, Prokain, Dibenzylamin, *N*-Methylmorpholin, Dihydroabiethylamin, 1-Ephenamin oder Methylpiperidin.

Die erfindungsgemäßen Verbindungen können in stereoisomeren Formen (Enantiomere, Diastereomere) existieren. Die Erfindung betrifft daher sowohl die Enantiomeren oder Diastereomeren als auch deren jeweilige Mischungen. Diese Enantiomer- und Diastereomer-Mischungen lassen sich in bekannter Weise in die stereoisomer einheitlichen Bestandteile trennen.

Im Rahmen der vorliegenden Erfindung haben die Substituenten im Allgemeinen die folgende Bedeutung:
C₁-C₆- und C₁-C₄-Alkoxy stehen für einen geradkettigen oder verzweigten Alkoxyrest mit 1 bis 6, bevorzugt 1 bis 4, besonders bevorzugt 1 bis 3 Kohlenstoffatomen. Nicht-limitierende Beispiele umfassen Methoxy, Ethoxy, n-Propoxy, Isopropoxy, tert.-Butoxy, n-Pentoxy und n-Hexoxy.
C₁-C₆- und C₁-C₄-Alkyl stehen für einen geradkettigen oder verzweigten Alkylrest mit 1 bis 6, bevorzugt 1 bis 4, besonders bevorzugt 1 bis 3 Kohlenstoffatomen. Nicht-limitierende Beispiele umfassen Methyl, Ethyl, n-Propyl, Isopropyl, tert.-Butyl, n-Pentyl und n-Hexyl.
C₁-C₆- und C₁-C₄-Alkylamino steht für einen geradkettigen oder verzweigten Mono- oder Dialkylaminorest mit 1 bis 6, bevorzugt mit 1 bis 4 Kohlenstoffatomen pro Alkylrest. Nicht-limitierende Beispiele umfassen Methylamine, Dimethylamino, Ethylamino, Diethylamino, n-Propylamino, Di-n-propylamino, Isopropylamino, Diisopropylamino, tert.-Butylamino, Di-tert.-butylamino, n-Pentylamino, Di-n-pentylamino, n-Hexylamino, Di-n-hexylamino, Ethylmethylamino, Isopropylmethylamino, n-Butylethylamino, n-Hexyl-i-pentylamino.
C₁-C₆- und C₁-C₄-Alkylcarbonylamino steht für einen geradkettigen oder verzweigten Alkylcarbonylaminorest mit 1 bis 6, bevorzugt mit 1 bis 4 und besonders bevorzugt mit 1 bis 3 Kohlenstoffatomen. Nicht-limitierende Beispiele umfassen Methylcarbonylamino, Ethylcarbonylamino, n-Propylcarbonylamino, Isopropylcarbonylamino, tert.-Butylcarbonylanuno, n-Pentylcarbonylamino und n-Hexylcarbonylamino.
C₁-C₆- und C₁-C₄-Alkylaminocarboxyl steht für einen geradkettigen oder verzweigten Mono- oder Dialkylaminocarboxylrest mit 1 bis 6, bevorzugt mit 1 bis 4, besonders bevorzugt mit 1-bis 3 Kohlenstoffatomen pro Alkylrest. Nicht-limitierende Beispiele umfassen Methylaminocarboxyl, Dimethylaminocarboxyl, Ethylaminocarboxyl, Diethylaminocarboxyl, n-Propylaminocarboxyl, Di-n-propylaminocarboxyl, Isopropylaminocarboxyl, Diisopropylaminocarboxyl, tert.-Butylaminocarboxyl, Di-tert.-butylaminocarboxyl, n-Pentylaminocarboxyl, Di-n-pentylaminocarboxyl, n-Hexylaminocarboxyl, Di-n-hexylaminocarboxyl, Ethylmethylaminocarboxyl, Isopropylmethylaminocarboxyl, n-Butylethylaminocarboxyl, n-Hexyl-i-pentylaminocarboxyl.
C₁-C₆- und C₁-C₄-Alkylaminocarbonyl steht für einen geradkettigen oder verzweigten Mono- oder Dialkylaminocarbonylrest mit 1 bis 6, bevorzugt mit 1 bis 4, besonders bevorzugt mit 1 bis 3 Kohlenstoffatomen pro Alkylrest. Nicht-limitierende Beispiele umfassen Methylaminocarbonyl, Dimethylaminocarbonyl, Ethylaminocarbonyl, Diethylaminocarbonyl, n-Propylaminocarbonyl, Di-n-propylaminocarbonyl, Isopropylaminocarbonyl, Diisopropylaminocarbonyl, tert.-Butylaminocarbonyl, Di-tert.-butylaminocarbonyl, n-Pentylaminocarbonyl, Di-n-pentylaminocarbonyl, n-Hexylaminocarbonyl, Di-n-hexylaminocarbonyl, Ethylmethylaminocarbonyl, Isopropylmethylaminocarbonyl, n-Butylethylaminocarbonyl, n-Hexyl-i-pentylaminocarbonyl.
C₁-C₆- und C₁-C₄-Alkylaminocarbonylamino steht für einen geradkettigen oder verzweigten Mono- oder Dialkylaminocarbonylaminorest mit 1 bis 6, bevorzugt mit 1 bis 4, besonders bevorzugt mit 1 bis 3 Kohlenstoffatomen pro Alkylrest. Nicht-limitierende Beispiele umfassen Methylaminocarbonylamino, Dimethylaminocarbonylamino, Ethylaminocarbonylamino, Diethylaminocarbonylamino, n-Propylaminocarbonylamino, Di-n-propylaminocarbonylamino, Isopropylaminocarbonylamino, Diisopropylaminocarbonylamino, tert.-Butylaminocarbonylamino, Di-tert.-butylaminocarbonylamino, n-Pentylaminocarbonylamino, Di-n-pentylaminocarbonylamino, n-Hexylaminocarbonylamino, Di-n-hexylaminocarbonylamino, Ethylmethylaminocarbonylamino, Isopropylmethylaminocarbonylamino, n-Butylethylaminocarbonylamino, n-Hexyl-i-pentylaminocarbonylamino.
C₁-C₆-Alkylcarbonyl steht für einen geradkettigen oder verzweigten Alkylcarbonylrest mit 1 bis 6, bevorzugt mit 1 bis 4 Kohlenstoffatomen.. Nicht-limitierende Beispiele umfassen: Acetyl, Ethylcarbonyl, Propylcarbonyl, Isopropylcarbonyl, Butylcarbonyl, Isobutylcarbonyl, tert.-Butylcarbonyl, Pentylcarbonyl und Hexylcarbonyl.
C₁-C₄-Alkylsulfonylamino stehen für einen geradkettigen oder verzweigten Alkylsulfonylaminorest mit 1 bis 4, bevorzugt mit 1 bis 3 Kohlenstoffatomen. Beispielsweise und vorzugsweise seien genannt: Methansulfonylamino, Ethansulfonylamino, n-Propansulfonylamino, Isopropansulfonylamino, tert.-Butansulfonylamino.
C₁-C₆- und C₁-C₄-Alkoxycarbonyl steht für einen geradkettigen oder verzweigten Alkoxycarbonylrest mit 1 bis 6, bevorzugt mit 1 bis 4 und besonders bevorzugt mit 1 bis 3 Kohlenstoffatomen. Nicht-limitierende Beispiele umfassen Methoxycarbonyl, Ethoxycarbonyl, n-Propoxycarbonyl, Isopropoxycarbonyl und tert.-Butoxycarbonyl.
C₁-C₆- und C₁-C₄-Alkoxycarbonylamino steht für einen geradkettigen oder verzweigten Alkoxycarbonylaminorest mit 1 bis 6, bevorzugt mit 1 bis 4 und besonders bevorzugt mit 1 bis 3 Kohlenstoffatomen. Nicht-limitierende Beispiele umfassen Methoxycarbonylamino, Ethoxycarbonylamino, n-Propoxycarbonylamino, Isopropoxycarbonylamino, tert.-Butoxycarbonylamino, n-Pentoxycarbonylamino und n-Hexoxycarbonylamino.
C₃-C₆-Cycloalkylaminocarbonyl steht für einen 3- bis 6-gliedrigen, vorzugsweise 5-bis 6-gliedrigen Cycloalkylaminocarbonylrest. Nicht-limitierende Beispiele umfassen Cyclopropylaminocarbonyl, Cyclobutylaminocarbonyl, Cyclopentylaminocarbonyl, Cyclohexylaminocarbonyl, Cycloheptylaminocarbonyl und Cyclooctylaminocarbonyl.
C₃-C₈- und C₅-C₆-Cycloalkylcarbonylamino steht für einen 3- bis 8-gliedrigen, vorzugsweise 5- bis 6-gliedrigen Cycloalkylcarbonylamino-Rest. Nicht-limitierende Beispiele umfassen Cyclopropylcarbonylamino, Cyclobutylcarbonylamino, Cyclopentylcarbonylamino, Cyclohexylcarbonylamino, Cycloheptylcarbonylamino und Cyclooctylcarbonylamino.
Heterocyclyl steht für einen mono- oder polycyclischen, vorzugsweise mono- oder bicyclischen, nicht-aromatischen Rest mit in der Regel 4 bis 10, vorzugsweise 5 bis 8 Ringatomen und bis zu 3, vorzugsweise bis zu 2 Hetero-Ringgliedern aus der Reihe N, O, S, SO, SO₂. Die Heterocyclyl-Reste können gesättigt oder teilweise ungesättigt sein. Nicht-limitierende Beispiele umfassen 5- bis 8-gliedrige monocyclische gesättigte Heterocyclylreste mit bis zu zwei Hetero-Ringatomen aus der Reihe O, N und S wie Tetrahydrofuran-2-yl, Piperazinyl, N-Methylpiperazinyl, Pyrrolidin-2-yl; Pyrrolidin-3-yl, Pyrrolinyl, Piperidinyl, Morpholinyl und Perhydroazepinyl.
Heteroaryl steht für einen aromatischen, mono- oder bicyclischen Rest mit 5 bis 10 Ringatomen und bis zu 5 Heteroatomen aus der Reihe S, O und/oder N. Bevorzugt sind 5- bis 6-gliedrige Heteroaryle mit bis zu 4 Heteroatomen. Der Heteroarylrest kann über ein Kohlenstoff- oder Heteroatom gebunden sein. Nicht-limitierende Beispiele umfassen: Thienyl, Furyl, Pyrrolyl, Thiazolyl, Oxadiazolyl, Oxazolyl, Isoxazolyl, Imidazolyl, Tetrazolyl, Pyridyl, Pyrimidinyl, Pyridazinyl, Indolyl, Indazolyl, Benzofuranyl, Benzothiophenyl, Chinolinyl, Isochinolinyl.
Heterocyclylcarbonylamino steht für eine Carbonylaminogruppe, die mit einem mono- oder polycyclischen, vorzugsweise mono- oder bicyclischen, nicht-aromatischen Rest mit in der Regel 4 bis 10, vorzugsweise 5 bis 8 Ringatomen und bis zu 3, vorzugsweise bis zu 2 Hetero-Ringgliedern aus der Reihe N, O, S, SO, SO₂ verknüpft ist. Die Heterocyclyl-Reste können gesättigt oder teilweise ungesättigt sein. Nicht-limitierende Beispiele umfassen Carbonylaminogruppen verknüpft mit 5- bis 8-gliedrigen monocyclischen gesättigten Heterocyclylresten mit bis zu zwei Hetero-Ringatomen aus der Reihe O, N und S wie Tetrahydrofuran-2-ylcarbonylamino, Piperazinylcarbonylamino, N-Methylpiperazinylcarbonylamino, Pyrrolidin-2-ylcarbonylamino, Pyrrolidin-3-ylcarbonylamino, Pyrrolinylcarbonylamino, Piperidinylcarbonylamino, Morpholinylcarbonylamino und Perhydroazepinylcarbonylamino.
Heteroarylcarbonylamino steht für eine Carbonylaminogruppe, die mit einem mono- oder bicyclischen aromatischen, mono- oder bicyclischen Rest mit 5 bis' 10 Ringatomen und bis zu 5 Heteroatomen aus der Reihe S, O und/oder N. Bevorzugt sind 5-bis 6-gliedrige Heteroaryle mit bis zu 4 Heteroatomen. Der Heteroarylrest kann über ein Kohlenstoff- oder Heteroatom an die Carbonylaminogruppe gebunden sein. Nicht-limitierende Beispiele umfassen: Thienylcarbonylamino, Furylcarbonylamino, Pyrrolylcarbonylamino, Thiazolylcarbonylamino, Isoxazolylcarbonylamino, Oxadiazolylcarbonylamino, Oxazolylcarbonylamino, Imidazolylcarbonylamino, Tetrazolylcarbonylamino, Pyridylcarbonylamino, Pyrimidinylcarbonylamino, Pyridazinylcarbonylamino, Indolylcarbonylamino, Indazolylcarbonylamino, Benzofuranylcarbonylamino, Benzothiophenylcarbonylamino, Chinolinylcarbonylamino, Isochinolinylcarbonylamino.
Heterocyclylcarbonyl steht für eine Carbonylgruppe, die mit einem mono- oder polycyclischen, vorzugsweise mono- oder bicyclischen, nicht-aromatischen Rest mit in der Regel 4 bis 10, vorzugsweise 5 bis 8 Ringatomen und bis zu 3, vorzugsweise bis zu 2 Hetero-Ringgliedern aus der Reihe N, O, S, SO, SO₂ verknüpft ist. Die Heterocyclyl-Reste können gesättigt oder teilweise ungesättigt sein. Nicht-limitierende Beispiele umfassen Carbonylgruppen verknüpft mit 5- bis 8-gliedrigen monocyclischen gesättigten Heterocyclylresten mit bis zu zwei Hetero-Ringatomen aus der Reihe O, N und S wie Tetrahydrofuran-2-ylcarbonyl, Piperazinylcarbonyl, N-Methylpiperazinylcarbonyl, Pyrrolidin-2-ylcarbonyl, Pyrrolidin-3-ylcarbonyl, Pyrrolinylcarbonyl, Piperidinylcarbonyl, Morpholinylcarbonyl und Perhydroazepinylcarbonyl.
Aryl steht für einen mono- bis tricyclischen aromatischen, carbocyclischen Rest mit in der Regel 6 bis 10 Kohlenstoff-Ringatomen. Nicht-limitierende Beispiele umfassen Phenyl und Naphthyl.
Halogen steht für Fluor, Chlor, Brom und Iod. Bevorzugt sind Fluor, Chlor und Brom, besonders bevorzugt Fluor und Chlor.
C₁-C₆- und C₁-C₄-Alkylthio stehen für einen geradkettigen oder verzweigten Alkylthio-Rest mit 1 bis 6, bevorzugt 1 bis 4, besonders bevorzugt 1 bis 3 Kohlenstoffatomen. Nicht-limitierende Beispiele umfassen Methylthio, Ethylthio, n-Propylthio, Isopropylthio, tert.-Butylthio, n-Pentylthio und n-Hexylthio.

Wenn Reste in den erfindungsgemäßen Verbindungen gegebenenfalls substituiert sind, können die Reste, soweit nichts anderes angegeben ist, ein- oder mehrfach gleich oder verschieden substituiert sein.

Bevorzugt sind Verbindungen der Formel (I), in welcher R¹ (3*R*)-1-Aza-bicyclo-[2.2.2]oct-3-yl bedeutet und R², R³, R⁴, A, E und der Ring B die oben angegebenen Bedeutungen besitzen, sowie die Solvate, Salze oder Solvate der Salze dieser Verbindungen.

Ebenfalls bevorzugt sind Verbindungen der Formel (I), in welcher R² Wasserstoff oder Methyl bedeutet und R¹, R³, R⁴, A, E und der Ring B die oben angegebenen Bedeutungen besitzen, sowie die Solvate, Salze oder Solvate der Salze dieser Verbindungen.

Besonders bevorzugt sind Verbindungen der Formel (I), in welcher R² Wasserstoff bedeutet und R¹, R³, R⁴, A, E und der Ring B die oben angegebenen Bedeutungen besitzen, sowie die Solvate, Salze oder Solvate der Salze dieser Verbindungen.

Ebenfalls bevorzugt sind Verbindungen der Formel (I), in welcher R³ Wasserstoff oder Methyl bedeutet und R¹, R², R⁴, A, E und der Ring B die oben angegebenen Bedeutungen besitzen, sowie die Solvate, Salze oder Solvate der Salze dieser Verbindungen.

Besonders bevorzugt sind Verbindungen der Formel (I), in welcher R³ Wasserstoff bedeutet und R¹, R², R⁴, A, E und der Ring B die oben angegebenen Bedeutungen besitzen, sowie die Solvate, Salze oder Solvate der Salze dieser Verbindungen.

Ebenfalls bevorzugt sind Verbindungen der Formel (I), in welcher R⁴ Wasserstoff, Fluor, Chlor, Brom, Trifluormethoxy, Hydroxymethyl, Methoxy oder 6-gliedriges Heterocyclyl bedeutet und R¹, R², R³, A, E und der Ring B die oben angegebenen Bedeutungen besitzen, sowie die Solvate, Salze oder Solvate der Salze dieser Verbindungen.

Ebenfalls bevorzugt sind Verbindungen der Formel (I), in welcher A ein Schwefelatom bedeutet und R¹, R², R³, R⁴, E und der Ring B die oben angegebenen Bedeutungen besitzen, sowie die Solvate, Salze oder Solvate der Salze dieser Verbindungen.

Ebenfalls bevorzugt sind Verbindungen der Formel (I), in welcher A ein Sauerstoffatom bedeutet und R¹, R², R³, R⁴, E und der Ring B die oben angegebenen Bedeutungen besitzen, sowie die Solvate, Salze oder Solvate der Salze dieser Verbindungen.

Ebenfalls bevorzugt sind Verbindungen der Formel (I), in welcher der Ring B Benzo bedeutet, das gegebenenfalls durch 1 bis 3 Reste aus der Reihe Halogen, Cyano, Trifluormethyl, Trifluormethoxy und C₁-C₄-Alkyl substituiert ist, und R¹, R², R³, R⁴, A und E die oben angegebenen Bedeutungen besitzen, sowie die Solvate, Salze oder Solvate der Salze dieser Verbindungen.

Ebenfalls bevorzugt sind Verbindungen der Formel (I), in welcher E Phenylen bedeutet, das gegebenenfalls durch Reste aus der Reihe Fluor, Chlor, Brom, Cyano, Trifluormethoxy, C₁-C₆-Alkyl und C₁-C₆-Alkoxy substituiert ist, und R¹, R², R³, R⁴, A und der Ring B die oben angegebenen Bedeutungen besitzen, sowie die Solvate, Salze oder Solvate der Salze dieser Verbindungen.

Ebenfalls bevorzugt sind Verbindungen der Formel (I), in welcher
in welcher
- R¹: 1-Aza-bicyclo[2.2.2]oct-3-yl,
- R²: Wasserstoff oder C₁-C₄-Alkyl,
- R³: Wasserstoff, Fluor, Chlor, Brom oder C₁-C₄-Alkyl,
- R⁴: Wasserstoff, Fluor; Chlor, Brom, Cyano, Amino, Trifluormethyl, Trifluormethoxy, C₁-C₄-Alkyl, C₁-C₄-Alkylcarbonyl, C₁-C₄-Alkylamino, Formyl, Hydroxycarbonyl, C₁-C₄-Alkoxy, C₁-C₄-Alkoxycarbonyl, C₁-C₄₋Alkylthio, C₁-C₄-Alkylcarbonylamino, C₁-C₄-Alkylaminocarbonyl, C₁-C₄₋Alkylsulfonylamino, C₃-C₆-Cycloalkylcarbonylamino, C₃-C₆-Cycloalkylaminocarbonyl, Pyrrolyl, C₁-C₄-Alkylaminocarbonylamino, Heterocyclylcarbonyl, Heterocyclylcarbonylamino, Heteroarylcarbonylamino, Hydroxy, Phenyl oder Heterocyclyl,
wobei C₁-C₄-Alkyl gegebenenfalls mit Hydroxy, Cyano, Amino, C₁-C₄₋Alkylaminocarbonylamino, C₁-C₄-Alkylaminocarboxyl, Heterocyclyl oder Aryl,
C₁-C₄-Alkylaminocarbonyl gegebenenfalls mit C₁-C₄-Alkoxy oder C₁₋C₄-Alkylamino,
C₁-C₄-Alkylcarbonylamino gegebenenfalls mit C₁-C₄-Alkoxy, und Heterocyclyl gegebenenfalls mit Oxo substituiert sein können,
- A: Sauerstoff oder Schwefel,
- der Ring B: Benzo oder Pyrido, die jeweils gegebenenfalls durch Reste aus der Reihe Halogen, Cyano, Trifluormethyl, Trifluormethoxy und C₁-C₄₋Alkyl substituiert sind,
und
- E: C≡C, Aryl und Heteroaryl, wobei Aryl und Heteroaryl durch Reste aus der Reihe Halogen, Cyano, Trifluormethyl, Trifluormethoxy, Nitro, Amino, C₁₋C₄-Alkoxy und C₁-C₄-Alkyl substituiert sein können,
bedeuten, sowie die Solvate, Salze oder Solvate der Salze dieser Verbindungen.

Ebenfalls bevorzugt sind Verbindungen der Formel (I), in welcher
in welcher
- R¹: 1-Aza-bicyclo[2.2.2]oct-3-yl,
- R² und R³: Wasserstoff,
- R⁴: Wasserstoff, Fluor, Chlor, Brom, Cyano, Amino, Trifluormethyl, Trifluormethoxy, C₁-C₄-Alkyl, C₁-C₄-Alkylcarbonyl, C₁-C₄-Alkylamino, Formyl, Hydroxycarbonyl, C₁-C₄-Alkoxy, C₁-C₄-Alkoxycarbonyl, C₁-C₆₋Alkylthio, C₁-C₄-Alkylcarbonylamino, C₁-C₄-Alkylaminocarbonyl, C₁-C₄₋Alkylsulfonylamino, C₃-C₆-Cycloalkylcarbonylamino, C₃-C₆-Cycloakylaminocarbonyl, Pyrrolyl, C₁-C₄-Alkylaminocarbonylamino, Heterocyclylcarbonyl, Heterocyclylcarbonylamino, Heteroarylcarbonylamino, Hydroxy, Phenyl oder Heterocyclyl,
wobei C₁-C₄-Alkyl gegebenenfalls mit Hydroxy, Cyano, Amino, C₁-C₄₋Alkylaminocarbonylamino, C₁-C₄-Alkylaminocarboxyl, Heterocyclyl oder Aryl,
C₁-C₄-Alkylaminocarbonyl gegebenenfalls mit C₁-C₄-Alkoxy oder C₁₋C₄-Alkylamino,
C₁-C₄-Alkylcarbonylamino gegebenenfalls mit C₁-C₄-Alkoxy, und Heterocyclyl gegebenenfalls mit Oxo substituiert sein können,
- A: Sauerstoff,
- der Ring B: Benzo oder Pyrido, die jeweils gegebenenfalls durch Reste aus der Reihe Halogen, Cyano, Trifluormethyl, Trifluormethoxy und C₁-C₄₋Alkyl substituiert sind,
und
- E: C≡C, Aryl und Heteroaryl, wobei Aryl und Heteroaryl durch Reste aus der Reihe Halogen, Cyano, Trifluormethyl, Trifluormethoxy, Nitro, Amino, C₁₋C₄-Alkoxy und C₁-C₄-Alkyl substituiert sein können,
bedeuten, sowie die Solvate, Salze oder Solvate der Salze dieser Verbindungen.

Ebenfalls bevorzugt sind Verbindungen der Formel (I), in welcher
- R¹: 1-Aza-bicyclo[2.2.2]oct-3-yl,
- R²: Wasserstoff oder C₁-C₆-Alkyl,
- R³: Wasserstoff, Halogen oder C₁-C₆-Alkyl,
- R⁴: Wasserstoff, Halogen, Cyano, Amino, Trifluormethyl, Trifluormethoxy, C₁₋C₆-Alkyl, C₁-C₆-Alkylcarbonyl, C₁-C₆-Alkylamino, Formyl, Hydroxycarbonyl, C₁-C₆-Alkoxy, C₁-C₆-Alkoxycarbonyl, C₁-C₆-Alkylthio, C₁-C₆₋Alkylcarbonylamino, C₁-C₄-Akylsulfonylamino, C₃-C₈-Cycloalkylcarbonylamino, Pyrrolyl, C₁-C₆-Alkylaminocarbonylamino, Heterocyclylcarbonyl, Phenyl oder Heterocyclyl,
wobei C₁-C₆-Alkyl gegebenenfalls mit Hydroxy, Amino, C₁-C₆- Alkylaminocarbonylamino, C₁-C₆-Alkylaminocarboxyl, Heterocyclyl oder Aryl,
C₁-C₆-Alkylcarbonylamino gegebenenfalls mit C₁-C₆-Alkoxy, und Heterocyclyl gegebenenfalls mit Oxo substituiert sein können,
- A: Sauerstoff oder Schwefel,
- der Ring B: Benzo oder Pyrido, die jeweils gegebenenfalls durch Reste aus der Reihe Halogen, Cyano, Formyl, Trifluormethyl, Trifluormethoxy, Nitro, Amino, C₁-C₆-Alkyl und C₁-C₆-Alkoxy substituiert sind,
und
- E: C≡C, Aryl und Heteroaryl, wobei Aryl und Heteroaryl durch Reste aus der Reihe Halogen, Cyano, Trifluormethyl, Trifluormethoxy, Nitro, Amino, C₁₋C₆-Alkoxy und C₁-C₆-Alkyl substituiert sein können,
bedeuten, sowie die Solvate, Salze oder Solvate der Salze dieser Verbindungen.

Ebenfalls bevorzugt sind Verbindungen der Formel (I), in welcher
- R¹: 1-Aza-bicyclo[2.2.2]oct-3-yl,
- R²: Wasserstoff oder C₁-C₆-Alkyl,
- R³: Wasserstoff, Halogen oder C₁-C₆-Alkyl,
- R⁴: Wasserstoff, Halogen, Cyano, Trifluormethyl, Trifluormethoxy, C₁-C₆-Alkyl, C₁-C₆-Alkoxy oder Heterocyclyl, wobei Alkyl gegebenenfalls durch einen Rest Hydroxy substituiert ist,
- A: Sauerstoff oder Schwefel,
- der Ring B: Benzo oder Pyrido, die jeweils gegebenenfalls durch Reste aus der Reihe Halogen, Cyano, Trifluormethyl, Trifluormethoxy, Nitro, Amino, C₁-C₆₋Alkyl und C₁-C₆-Alkoxy substituiert sind,
und
- E: C=C, Arylen oder Heteroarylen, wobei Arylen und Heteroarylen durch Reste aus der Reihe Halogen, Cyano, Trifluormethyl, Trifluormethoxy, Nitro, Amino, C₁-C₆-Alkyl und C₁-C₆-Alkoxy substituiert sein können,
bedeuten, sowie die Solvate, Salze oder Solvate der Salze dieser Verbindungen.

Ebenfalls bevorzugt sind Verbindungen der Formel 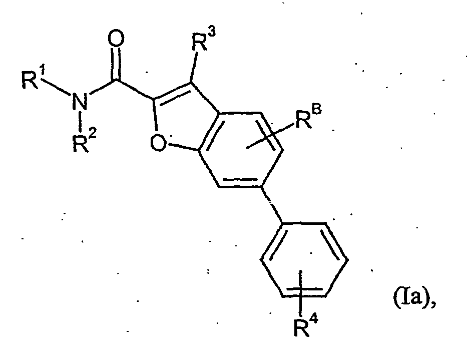 in welcher
- R¹: (3*R*)-1-Aza-bicyclo[2.2.2]oct-3-yl,
- R² und R³: unabhängig voneinander Wasserstoff oder Methyl,
- R⁴: Wasserstoff, Halogen, Cyano, Trifluormethyl, Trifluormethoxy, C₁-C₆-Alkyl, C₁-C₆-Akoxy oder Heterocyclyl, wobei Alkyl gegebenenfalls durch einen Hydroxyrest substituiert ist,
und
- R^{B}: Wasserstoff, Halogen, Cyano, Trifluormethyl, Trifluormethoxy, Nitro, Amino, C₁-C₆-Alkyl oder C₁-C₆-Alkoxy
bedeuten, sowie die Solvate, Salze oder Solvate der Salze dieser Verbindungen.

Ebenfalls ganz besonders bevorzugt sind Verbindungen der Formel (Ia), in welcher
- R¹: (3*R*)-1-Aza-bicyclo[2.2.2]oct-3-yl,
- R² und: R³ Wasserstoff,
- R⁴: Wasserstoff, Fluor, Chlor, Brom, Trifluormethoxy, Hydroxymethyl, Methoxy oder 6-gliedriges Heterocyclyl und
- R^{B}: Wasserstoff, Halogen, Cyano, Trifluormethyl, Trifluormethoxy oder C₁-C₄₋Alkyl
bedeuten, sowie die Solvate, Salze oder Solvate der Salze dieser Verbindungen.

Ebenfalls bevorzugt sind Verbindungen der Formel 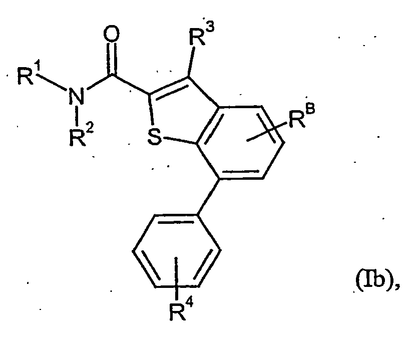 in welcher
- R¹: (3*R*)-1-Aza-bicyclo[2.2.2]oct-3-yl,
- R² und R³: unabhängig voneinander Wasserstoff oder Methyl,
- R⁴: Wasserstoff, Halogen, Cyano, Trifluormethyl, Trifluormethoxy, C₁-C₆-Alkyl, C₁-C₆-Alkoxy oder Heterocyclyl, wobei Alkyl gegebenenfalls durch einen Hydroxyrest substituiert ist, und
- R^{B}: Wasserstoff, Halogen, Cyano, Trifluormethyl, Trifluormethoxy, Nitro, Amino, C₁-C₆-Alkyl und C₁-C₆-Alkoxy
bedeuten, sowie die Solvate, Salze oder Solvate der Salze dieser Verbindungen.

Ebenfalls bevorzugt sind Verbindungen der Formel (Ib), in welcher
- R¹: (3*R*)-1-Aza-bicyclo[2.2.2]oct-3-yl,
- R² und R³: Wasserstoff,
- R⁴: Wasserstoff, Fluor, Chlor, Brom, Trifluormethoxy, Hydroxymethyl, Methoxy oder 6-gliedriges Heterocyclyl und
- R^{B}: Wasserstoff, Halogen, Cyano, Trifluormethyl, Trifluormethoxy oder C₁-C₄₋Alkyl
bedeuten, sowie die Solvate, Salze oder Solvate der Salze dieser Verbindungen.

Ebenfalls bevorzugt sind Verbindungen der Formel 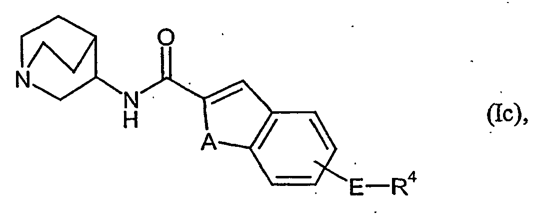 in welcher
- E: Phenylen,
- R⁴: C₁-C₆-Alkoxy, Aminomethylen, Hydroxycarbonyl, C₃-C₈-Cycloalkylcarbonylamino, eine Gruppe der Formel 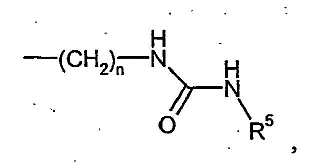 wobei
R⁵ C₁-C₆-Alkyl,
n null, 1, 2, 3 oder 4, oder
5- bis 6-gliedriges Heterocyclyl, das gegebenenfalls mit Oxo substituiert ist,
- A: Schwefel oder Sauerstoff,
bedeuten, sowie deren Solvate, Salze oder Solvate der Salze.

Bevorzugt betrifft die Erfindung Verbindungen der Formel (I), in welcher
- E: Phenylen,
- R⁴: C₁-C₄-Akoxy, Aminomethylen, Hydroxycarbonyl, C₃-C₆-Cycloalkylcarbonylamino, eine Gruppe der Formel 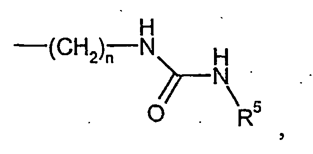 wobei
R⁵ C₁-C₄-Alkyl,
n null, 1 oder 2, oder
5- bis 6-gliedriges Heterocyclyl, das gegebenenfalls mit Oxo substituiert ist,
- A: Schwefel oder Sauerstoff,
bedeuten, sowie deren Solvate, Salze oder Solvate der Salze.

Besonders bevorzugt betrifft die Erfindung Verbindungen der folgenden Formeln 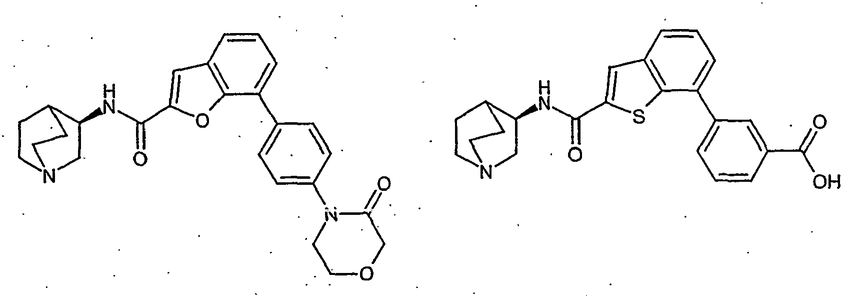 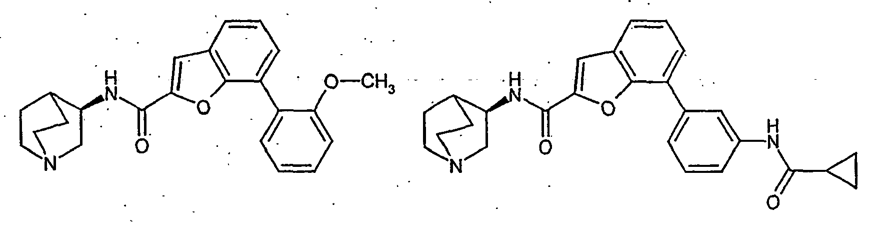 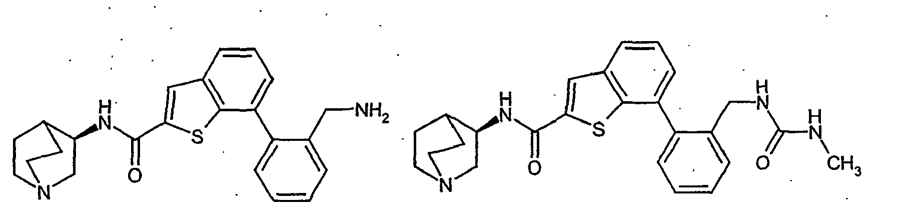 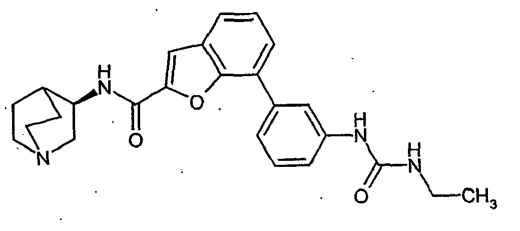 sowie die Solvate, Salze oder Solvate der Salze dieser Verbindungen.

Ganz besonders bevorzugt sind Kombinationen von zwei oder mehreren der oben genannten Vorzugsbereiche.

Die Erfindung betrifft weiterhin Verfahren zur Herstellung der erfindungsgemäßen Verbindungen, wonach man Verbindungen der Formel

X¹-E-R⁴ (II),

in welcher
- R⁴: die oben genannten Bedeutungen hat und
oder
- X¹: im Falle, dass E Arylen oder Heteroarylen bedeutet, für -B(OH)₂ oder 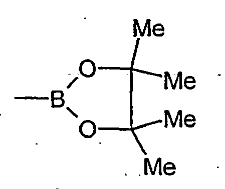 steht,
und im Falle, dass E -C≡C- bedeutet, für Wasserstoff steht,
mit einer Verbindung der Formel 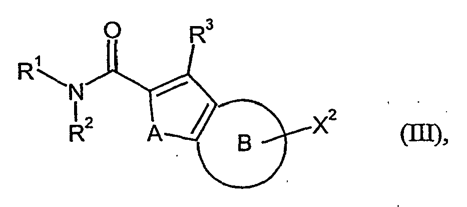 in welcher
- R¹, R², R³,: A und der Ring B die oben genannten Bedeutungen besitzen und
- X²: für Triflat oder Halogen, bevorzugt Chlor, Brom oder Iod, steht,
und gegebenenfalls
[A] die resultierenden Verbindungen (I) mit entsprechenden Alkylierungsreagentien am Chinuklidinstickstoffatom alkyliert, oder
[B] die resultierenden Verbindungen (I) mit geeigneten Oxidationsmitteln am Chinuklidinstickstoffatom oxidiert,
und die resultierenden Verbindungen (I) gegebenenfalls mit den entsprechenden (I) Lösungsmitteln und/oder (ii) Basen oder Säuren zu ihren Solvaten, Salzen oder Solvaten der Salze umsetzt.

Die Umsetzung der Verbindungen (II) und (III) findet im allgemeinen in einem inerten Lösungsmittel in Gegenwart eines Übergangsmetallkatalysators, in Gegenwart einer Base und gegebenenfalls in Gegenwart von Kupfer(I)iodid statt.

Vorzugsweise wird das erfindungsgemäße Verfahren in einem Temperaturbereich von 70°C bis 110°C bei Normaldruck durchgeführt.

Inerte Lösungsmittel sind beispielsweise Ether wie Dioxan, Tetrahydrofuran oder 1,2-Dimethoxyethan, Kohlenwasserstoffe wie Benzol, Xylol oder Toluol, Nitroaromaten wie Nitrobenzol, gegebenenfalls *N*-alkylierte Carbonsäureamide wie Dimethylformamid, Dimethylacetamid, Alkylsulfoxide wie Dimethylsulfoxid oder cyclische Lactame wie *N*-Methylpyrrolidon. Bevorzugt sind Lösungsmittel aus der Reihe Dimethylformamid, Dimethylacetamid, Dimethylsulfoxid und 1,2-Dimethoxyethan.

Als Übergangsmetallkatalysatoren werden bevorzugt Palladium(0)- oder Palladium-(II)-verbindungen, insbesondere Bis-(diphenylphosphanferrocenyl)-palladium(II)-chlorid, Dichlorbis(triphenylphosphin)-palladium oder Tetrakis(triphenylphosphin)-palladium(0), verwendet.

Als Basen werden Alkalihydroxide oder -salze wie Kaliumacetat, Natriumhydroxid, Natriumhydrogencarbonat oder Natriumcarbonat, gegebenenfalls in Form ihrer wässrigen Lösungen, bevorzugt.

Verfahrensschritte [A] und [B] können in inerten Lösungsmittel und bei Temperaturen von -30 bis 50 °C und bei Normaldruck durchgeführt werden.

Als Basen für Verfahrensschritt [A] können Alkalihydride wie Kalium- oder Natriumhydird, Alkalihydroxide wie Natrium- oder Kaliumhydroxid oder Alkalicarbonate, wie Natrium- oder Kaliumcarbonat eingesetzt werden.

Als Alkylierungsreagentien für Verfahrensschritt [A] können Alkylhalogenide wie Methyliodid oder Benzylhalogenide wie Benzylbromid eingesetzt werden.

Als Oxidationmittel für Verfahrensschritt [B] eignet sich besonders Wasserstoffperoxid oder Metachlorperbenzoesäure.

Die übergangsmetallkatalysierten Reaktionen können analog literaturbekannten Verfahren durchgeführt werden, z. B. Umsetzung mit Alkinen: vgl. N. Krause et al., *J. Org. Chem.* 1998, *63,* 8551; mit Ketonen, Aromaten und Alkenen: vgl. z.B. A. Suzuki, *Acc. Chem. Res.* 1982, *15,* 178ff; Miyaura et al. *J*. *Am. Chem. Soc.* 1989, *111,* 314; J. K. Stille, *Angew. Chem.* 1986, *98,* 504 und mit substituierten Aminen: vgl. S. L. Buchwald et al., *J*. *Organomet. Chem.* 1999, *576,* 125ff. (siehe auch J. Tsuji, Palladium Reagents and Catalysts, Wiley, New York, 1995).

Die Verbindungen (II) sind bekannt oder lassen sich analog bekannten Verfahren aus den entsprechenden Edukten synthetisieren.

Die Verbindungen (III) können durch Umsetzung von Verbindungen der Formel

R¹R²NH (IV),

in welcher R¹ und R² die oben genannten Bedeutungen besitzen,
mit einer Verbindung der Formel 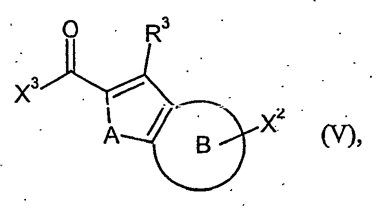 in welcher
- R³, X²,: A und der Ring B die oben genannten Bedeutungen besitzen und
- X³: für Hydroxy oder Halogen, bevorzugt Brom oder Chlor, steht,
hergestellt werden.

Die Umsetzung der Verbindungen (IV) und (V) erfolgt, falls X³ für Halogen steht, im Allgemeinen in inerten Lösungsmitteln, gegebenenfalls in Gegenwart einer Base, bevorzugt in einem Temperaturbereich von 0°C bis 50°C bei Normaldruck.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, Nitroaromaten wie Nitromethan, Carbonsäureester wie Ethylacetat, Ketone wie Aceton oder 2-Butanon, gegebenenfalls *N*-alkylierte Carbonsäureamide wie Dimethylformamid oder Dimethylacetamid, Alkylsulfoxide wie Dimethylsulfoxid, Carbonsäurenitrile wie Acetonitril oder Heteroaromaten wie Pyridin. Bevorzugt sind Dioxan, Dimethylformamid oder Methylenchlorid.

Basen sind beispielsweise Alkalihydroxide wie Natrium- oder Kaliumhydroxid, Alkalicarbonate und -hydrogencarbonate wie Cäsiumcarbonat, Natriumhydrogencarbonat, Natrium- oder Kaliumcarbonat, oder Amide wie Lithiumdiisopropylamid, Alkylamine wie Triethylamin oder Diisopropylethylamin, bevorzugt Diisopropylethylamin oder Triethylamin, und andere Basen wie DBU.

Die Umsetzung erfolgt, falls X³ für Hydroxy steht, im allgemeinen in inerten Lösungsmitteln, in Gegenwart von Kondensationsmitteln, gegebenenfalls in Gegenwart einer Base, bevorzugt in einem Temperaturbereich von 20 bis 50°C bei Normaldruck.

Der Begriff "inerte Lösungsmittel" umfasst beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, Nitroaromaten wie Nitromethan, Carbonsäureester wie Ethylacetat, Ketone wie Aceton, gegebenenfalls *N*-alkylierte Carbonsäureamide wie Dimethylformamid oder Dimethylacetamid, Alkylsulfoxide wie Dimethylsulfoxid, Carbonsäurenitrile wie Acetonitril und Heteroaromaten wie Pyridin. Bevorzugt sind Tetrahydrofuran, Dimethylformamid, 1,2-Dichlorethan oder Methylenchlorid.

Kondensationsmittel im Sinne der Erfindung sind beispielsweise Carbodiimide wie z.B. N,N'-Diethyl-, N,N,'-Dipropyl-, N,N'-Diisopropyl-, N,N'-Dicyclohexylcarbodiimid, *N*-(3-Dimethylaminoisopropyl)-N'-ethylcarbodiimid-Hydrochlorid (EDC), *N-*Cyclohexylcarbodiimid-*N'*-propyloxymethyl-Polystyrol (PS-Carbodiimid); Carbonylverbindungen wie Carbonyldiimidazol; 1,2-Oxazoliumverbindungen wie 2-Ethyl-5-phenyl-1,2-oxazolium-3-sulfat oder 2-tert.-Butyl-5-methyl-isoxazolium-perchlorat; Acylaminoverbindungen wie 2-Ethoxy-1-ethoxycarbonyl-1,2-dihydrochinolin; weiterhin Propanphosphonsäureanhydrid, Isobutylchloroformat, Bis-(2-oxo-3-oxazolidinyl)-phosphorylchlorid, Benzotriazolyloxy-tri(dimethylamino)phosphoniumhexafluorophosphat, O-(Benzotriazol-1-yl)-N,N,N',N'-tetra-methyluronium-hexafluorophosphat (HBTU), 2-(2-Oxo-1-(2H)-pyridyl)-1,1,3,3-tetramethyluroniumtetrafluoroborat (TPTU), O-(7-Azabenzotriazol-1-yl)-NNN',N'-tetramethyl-uroniumhexafluorophosphat (HATU), Benzotriazol-1-yloxytris(dimethylamino)-phosphoniumhexafluoro-phosphat (BOP), und deren Mischungen.

Gegebenenfalls kann es vorteilhaft sein, das Kondensationsmittel in Gegenwart eines Hilfsnukleophils wie beispielsweise 1-Hydroxybenztriazol (HOBt) zu verwenden.

Basen sind beispielsweise Alkalicarbonate und -hydrogencarbonate, wie z.B. Natrium- oder Kaliumcarbonat oder -hydrogencarbonat, organische Basen wie Alkylamine z.B. Triethylamin, oder *N*-Methylmorpholin, *N*-Methylpiperidin, 4- Dimethylaminopyridin oder Diisopropylethylamin.

Besonders bevorzugt ist die Kombination von *N*-(3-Dimethylaminoisopropyl)-*N*'-ethylcarbodiimid-Hydrochlorid (EDC), 1-Hydroxybenztriazol (HOBt) und Triethylamin in Dimethylformamid oder von O-(7-Azabenzotriazol-1-yl)-*N*,*N*,*N*',*N*'-tetramethyl-uroniumhexafluorophosphat (HATU) und Diisopropylethylamin in Dimethylformamid.

Die Verbindungen (IV) und (V) sind bekannt oder lassen sich analog bekannten Verfahren aus den entsprechenden Edukten synthetisieren (vgl. z.B. "Comprehensive Heterocyclic Chemistry", Katritzki et al., Hrsg.; Elsevier, 1996).

So können beispielsweise substituierte Benzothiophen-2-carbonsäuren aus entsprechend substituierten 2-Halogenbenzaldehyden durch Reaktion mit Mercaptoessigsäuremethylester (siehe z.B. A. J. Bridges et al., *Tetrahedron Lett.* 1992, *33*, 7499) und anschließender Verseifung des Esters erhalten werden: 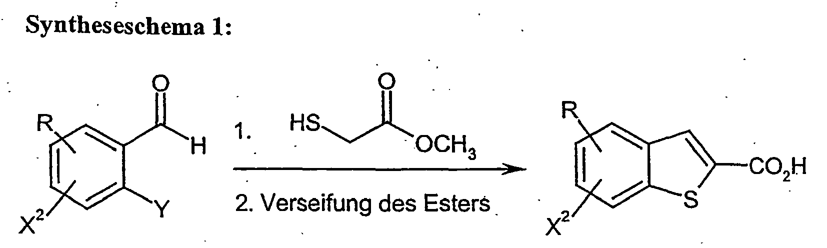 Y = F, Cl, Br
X² = Halogen oder Triflat

Zur Synthese der entsprechenden Pyrido-Derivate ist ausgehend von 2-Halogenbenzonitrilen eine Reaktion mit Mercaptoessigsäuremethylester zu den 3-Aminobenzothiophen-2-carbonsäureestern möglich:

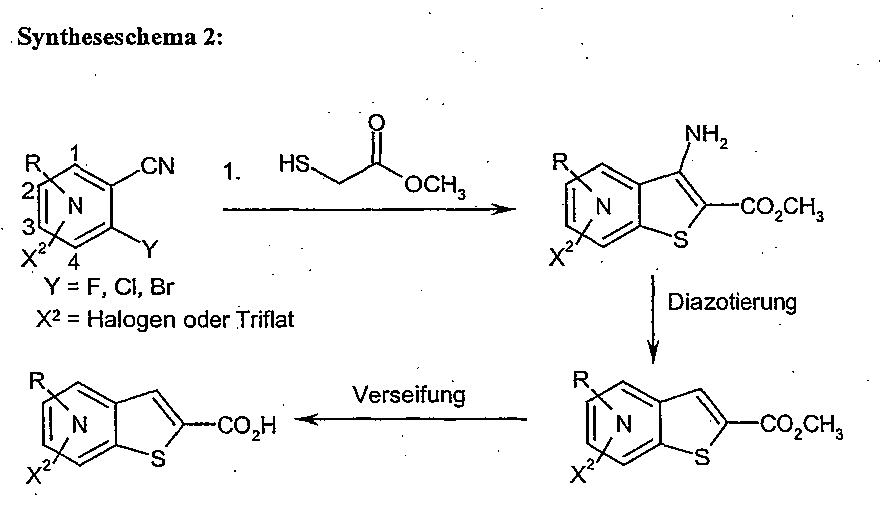

Das in den Ring gezeichnete Stickstoffatom kann an einer der Positionen 1 bis 4 im Aromaten eine CH-Gruppe ersetzen.

Die Aminofunktion kann durch Diazotierung entfernt werden. Schließlich kann der Ester zur Zielverbindung verseift werden.

Substituierte Benzofuran-2-carbonsäuren sind z. B. gemäß D. Bogdal et al., *Tetrahedron* 2000, *56,* 8769 zugänglich.

Die erfindungsgemäßen Verbindungen eignen sich zur Verwendung als Arzneimittel zur Behandlung und/oder Prophylaxe von Krankheiten bei Menschen und Tieren.

Sie wirken als Agonisten am α7-nAChR und zeigen ein nicht vorhersehbares, wertvolles pharmakologisches Wirkspektrum.

Die erfindungsgemäßen Verbindungen können aufgrund ihrer pharmakologischen Eigenschaften allein oder in Kombination mit anderen Wirkstoffen zur Behandlung und/oder Prävention von kognitiven Störungen, insbesondere der Alzheimerschen Krankheit eingesetzt' werden. Wegen ihrer selektiven Wirkung als α7-nAChR-Agonisten eignen sie sich besonders zur Verbesserung der Wahrnehmung, Konzentrationsleistung, Lernleistung oder Gedächtnisleistung; insbesondere nach kognitiven Störungen, wie sie beispielsweise bei "Mild cognitive impairment", Alters-assoziierte Lern- und Gedächtnisstörungen, Alters-assoziierte Gedächtnisverluste, vaskuläre Demenz, Schädel-Hirn-Trauma, Schlaganfall, Demenz, die nach Schlaganfällen auftritt ("post stroke dementia"), post-traumatisches Schädel-Hirn- Trauma, allgemeine Konzentrationsstörungen, Konzentrationsstörungen bei Kindern mit Lern- und Gedächtnisproblemen, Attention Deficit Hyperactivity Disorder, Alzheimersche Krankheit, Demenz mit Lewy-Körperchen, Demenz mit Degeneration der Frontallappen einschließlich des Pick's Syndroms, Parkinsonsche Krankheit, Progressive nuclear palsy, Demenz mit corticobasaler Degeneration, Amyotrophe Lateralsklerose (ALS), Huntingtonsche Krankheit, Multiple Sklerose, Thalamische Degeneration, Creutzfeld-Jacob-Demenz, HIV-Demenz, Schizophrenie, Schizophrenie mit Demenz oder Korsakoff-Psychose auftreten.

Die erfindungsgemäßen Verbindungen können allein oder in Kombination mit anderen Wirkstoffen zur Prophylaxe und Behandlung von akuten und/oder chronischen Schmerzen (für eine Klassifizierung siehe "Classification of Chronic Pain, Descriptions of Chronic Pain Syndromes and Definitions of Pain Terms", 2. Aufl., Meskey und Begduk, Hrsg.; IASP-Press, Seattle, 1994) eingesetzt werden, insbesondere zur Behandlung von Krebs-induzierten Schmerzen und chronischen neuropathischen Schmerzen, wie zum Beispiel bei diabetischer Neuropathie, postherpetischer Neuralgie, peripheren Nervenbeschädigungen, zentralem Schmerz (beispielsweise als Folge von cerebraler Ischämie) und trigeminaler Neuralgie, und anderen chronischen Schmerzen, wie zum Beispiel Lumbago, Rückenschmerz (low back pain) oder rheumatischen Schmerzen. Daneben eignen sich diese Wirkstoffe auch zur Therapie von primär akuten Schmerzen jeglicher Genese und von daraus resultierenden sekundären Schmerzzuständen, sowie zur Therapie chronifizierter, ehemals akuter Schmerzzustände. Die erfindungsgemäßen Verbindungen können allein oder in Kombination mit anderen Wirkstoffen zur Behandlung von Schizophrenie eingesetzt werden.

Die *in vitro-*Wirkung der erfindungsgemäßen Verbindungen kann in folgenden Assays gezeigt werden:

### 1. Bestimmung der Affinität von Testsubstanzen für α7-nAChR durch Inhibition von [³H]Methyllycaconitine-Bindung an Rattenhirnmembranen

Der [³H]-Methyllycaconitine-Bindungstest ist eine Modifikation der von Davies et al. in *Neuropharmacol.* 1999, *38,* 679-690 beschriebenen Methode.

Rattenhirngewebe (Hippocampus oder Gesamthirn) wird in Homogenisierungspuffer (10 % w/v, 0.32 M Sucrose, 1 mM EDTA, 0.1 mM Phenylmethylsulfonylfluorid (PMSF), 0,01 % (w/v) NaN₃, pH 7.4, 4°C) bei 600 rpm in einem Glashomogenisator homogenisiert. Das Homogenisat wird zentrifugiert (1000 x g, 4°C, 10 min) und der Überstand wird abgenommen. Das Pellet wird erneut suspendiert (20 % w/v) und die Suspension wird zentrifugiert (1000 x g, 4°C, 10 min). Die beiden Überstände werden vereinigt und zentrifugiert (15.000 x g, 4°C, 30 min). Das so erhaltene Pellet wird als P2-Fraktion bezeichnet.

Das P2-Pellet wird zweimal in Bindungspuffer (50 mM Tris-HCl, 1 mM MgCl₂, 120 mM NaCl, 5 mM KCl, 2 mM CaCl₂, pH 7.4) suspendiert und die Suspension wird zentrifugiert (15.000 x g, 4°C, 30 min).

Der Rückstand wird in Bindungspuffer resuspendiert und in einem Volumen von 250µl (Membranproteinmenge 0.1 - 0.5 mg) in Gegenwart von 1-5 nM [³H]-Methyllycaconitin 0.1 % (w/v) BSA (bovines Serumalbumin) und verschiedenen Konzentrationen der Testsubstanz für 2.5 h bei 21°C inkubiert. Anschließend wird in Gegenwart von 1 µM α-Bungarotoxin oder 100 µM Nicotin oder 10 µM MLA (Methyllycaconitin) inkubiert.

Die Inkubation wird durch Zugabe von 4 ml PBS (20 mM Na₂HPO₄, 5 mM KH₂PO₄, 150 mM NaCl, pH 7.4, 4°C) und Filtration durch Typ A/E glass fibre filters (Gelman Sciences), die vorher 3 h in 0.3 % (v/v) Polyethylenimin (PEI) eingelegt waren, beendet. Die Filter werden zweimal mit 4 ml PBS (4°C) gewaschen und die gebundene Radioaktivität wird durch Szintillationsmessung bestimmt. Alle Tests werden als Dreifachbestimmungen durchgeführt. Aus dem IC_{5O}-Wert der Verbindungen (Konzentration der Testsubstanz, bei der 50 % des am Rezeptor gebundenen Liganden verdrängt werden), der Dissoziationskonstante K_{D} und der Konzentration L von [³H]Methyllycaconitin wurde die Dissoziationskonstante K; der Testsubstanz nach der Gleichung Kᵢ = IC₅₀ /(1+L/K_{D}) bestimmt.

Anstelle von [³H]-Methyllycaconitin können auch andere α7-nAChR-selektive Radioliganden wie z.B. [¹²⁵I]-α-Bungarotoxin oder unselektive nAChR-Radioliganden gemeinsam mit Inhibitoren anderer nAChR eingesetzt werden.

Repräsentative in-vitro-Wirkdaten für die erfindungsgemäßen Verbindungen sind in Tabelle A wiedergegeben:

**Tabelle A**

| **Beispiel Nr.** | **K**_{**i**}**-Wert (nM)** |
|---|---|
| 3 | 60 |
| 4 | 24 |
| 17 | 17 |
| 19 | 20 |
| 20 | 1.6 |
| 73 | <1 |
| 75 | <0.1 |
| 76 | 3.3 |
| 90 | 14 |
| 102 | 62 |
| 108 | 17 |
| 116 | 17 |
| 130 | 26 |
| 149 | 97 |
| 150 | 35 |
| 151 | 88 |
| 154 | 3 |
| 163 | 14 |
| 175 | 8.3 |
| 186 | 120 |

Die Eignung der erfindungsgemäßen Verbindungen zur Behandlung von kognitiven Störungen kann in folgenden Tiermodellen gezeigt werden:

### 2. Objekt-Wiedererkennungstest

Der Objekt-Wiedererkennungstest ist ein Gedächtnistest. Er misst die Fähigkeit von Ratten (und Mäusen), zwischen bekannten und unbekannten Objekten zu unterscheiden.

Der Test ist bei Blokland et al., *Neuro Report* 1998, *9*, 4205-4208; A. Ennaceur et al,. *Behav. Brain Res.* 1988, *31*, 47-59; A. Ennaceur et al., *Psychopharmacology* 1992, *109,* 321-330; und Prickaerts et al., *Eur. J. Pharmacol.* 1997, *337*, 125-136 beschrieben.

In einem ersten Durchgang wird eine Ratte in einer ansonsten leeren größeren Beobachtungsarena mit zwei identischen Objekten konfrontiert. Die Ratte wird beide Objekte ausgiebig untersuchen, d.h. beschnüffeln und berühren. In einem zweiten Durchgang, nach einer Wartezeit von 24 Stunden, wird die Ratte erneut in die Beobachtungsarena gesetzt. Nun ist eines der bekannten Objekte durch ein neues, unbekanntes Objekt ersetzt. Wenn eine Ratte das bekannte Objekt wiedererkennt, wird sie vor allem das unbekannte Objekt untersuchen. Nach 24 Stunden hat eine Ratte jedoch normalerweise vergessen, welches Objekt sie bereits im ersten Durchgang untersucht hat, und wird daher beide Objekte gleichstark inspektieren. Die Gabe einer Substanz mit lern- und gedächtnisverbessemder Wirkung kann dazu führen, dass eine Ratte das bereits 24 Stunden vorher im ersten Durchgang gesehene Objekt wiedererkennt. Sie wird dann das neue unbekannte Objekt ausführlicher untersuchen als das bereits bekannte. Diese Gedächtnisleistung wird in einem Diskriminationsindex ausgedrückt. Ein Diskiminationsindex von Null bedeutet, dass die Ratte beide Objekte, das alte und das neue, gleichlang untersucht; d.h. sie hat das alte Objekt nicht wiedererkannt und reagiert auf beide Objekte als wären sie neu. Ein Diskriminationsindex größer Null bedeutet, dass die Ratte das neue Objekt länger inspektiert als das alte; d.h. die Ratte hat das alte Objekt wiedererkannt.

### 3. Sozialer Wiedererkennungstest:

Der Soziale Wiedererkennungstest ist ein Test zur Prüfung der lern- oder gedächtnisverbessernden Wirkung von Testsubstanzen.

Adulte Ratten, die in Gruppen gehalten werden, werden 30 Minuten vor Testbeginn einzeln in Testkäfige gesetzt. Vier Minuten vor Testbeginn wird das Testtier in eine Beobachtungsbox gebracht. Nach dieser Adaptationszeit wird ein juveniles Tier zu dem Testtier gesetzt und 2 Minuten lang die Zeit gemessen, die das adulte Tier das juvenile Tier investigiert (Trial 1). Gemessen werden alle deutlich auf das Jungtier gerichteten Verhaltensweisen, d.h. ano-genitale Inspektion, Verfolgen sowie Fellpflege, bei denen das Alttier einen Abstand von höchstens 1 cm zu dem Jungtier hat. Danach wird das juvenile Tier herausgenommen und das adulte in seinem Testkäfig belassen (bei 24 Stunden Retention wird das Tier in seinen Heimkäfig zurückgesetzt). Vor oder nach dem ersten Test wird das adulte Testtier mit Testsubstanz behandelt. Je nach Zeitpunkt der Behandlung kann das Erlernen oder das Speichern der Information über das Jungtier durch die Substanz beeinflusst werden. Nach einem festgelegten Zeitraum (Retention) wird der Test wiederholt (Trial 2). Je größer die Differenz zwischen den in Trials 1 und 2 ermittelten Investigationszeiten, desto besser hat sich das adulte Tier an das Jungtier erinnert.

Die erfindungsgemäßen Verbindungen eignen sich zur Verwendung als Arzneimittel für Menschen und Tiere.

Zur vorliegenden Erfindung gehören auch pharmazeutische Zubereitungen, die neben inerten, nicht-toxischen, pharmazeutisch geeigneten Hilfs- und Trägerstoffen eine oder mehrere erfindungsgemäße Verbindungen enthalten, oder die aus einem oder mehreren erfindungsgemäße Verbindungen bestehen, sowie Verfahren zur Herstellung dieser Zubereitungen.

Die erfindungsgemäßen Verbindungen sollen in diesen Zubereitungen in einer Konzentration von 0,1 bis 99,5 Gew.%, bevorzugt von 0,5 bis 95 Gew.-% der Gesamtmischung vorhanden sein.

Neben den erfindungsgemäßen Verbindungen können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Formulierung vorhanden sein, d.h. in Mengen, die ausreichend sind, um den angegebenen Dosierungsspielraum zu erreichen.

Die Formulierungen werden beispielsweise durch Verstrecken der Wirkstoffe mit Lösungsmitteln und/oder Trägerstoffen, gegebenenfalls unter Verwendung von Emulgiermitteln und/oder Dispergiermitteln, hergestellt, wobei z.B. im Fall der Benutzung von Wasser als Verdünnungsmittel gegebenenfalls organische Lösungsmittel als Hilfslösungsmittel verwendet werden können.

Die Applikation kann in üblicher Weise, vorzugsweise oral, transdermal oder parenteral, insbesondere perlingual oder intravenös, erfolgen. Sie kann aber auch durch Inhalation über Mund oder Nase, beispielsweise mit Hilfe eines Sprays, oder topisch über die Haut erfolgen.

Im Allgemeinen hat es sich als vorteilhaft erwiesen, Mengen von etwa 0,001 bis 10 mg/kg, bei oraler Anwendung vorzugsweise etwa 0,005 bis 3 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Soweit nicht anders angegeben, beziehen sich alle Mengenangaben auf GewichtsProzente. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10 % w/v": 100 ml Lösung oder Suspension enthalten 10 g Substanz.

### Abkürzungen:

- DAD: Dioden-Array-Detektor
- DBU: 1,5-Diazabicyclo[4.3.0]non-5-en
- DCI: direkte chemische Ionisation (bei MS)
- DMAP: 4-*N,N*-Dimethylaminopyridin
- DMF: *N,N*-Dimethylformamid
- DMSO: Dimethylsulfoxid
- d.Th.: der Theorie (bei Ausbeute)
- EDC: *N*'-(3-Dimethylaminopropyl)-*N*-ethylcarbodiimid x HCl
- eq.: Äquivalent(e)
- ESI: Elektrospray-Ionisation (bei MS)
- HATU: *O*-(7-Azabenzotriazol-1-yl)-*N,N,N',N*'-tetramethyluroniumhexafluorophosphat
- HOBt: 1-Hydroxy-1H-benzotriazol x H₂O
- HPLC: Hochdruck- / Hochleistungsflüssigchromatographie
- konz.: konzentriert
- LC-MS: Flüssigchromatographie mit gekoppelter Massenspektroskopie
- MS: Massenspektroskopie
- NMR: Kernresonanzspektroskopie
- PBS: phosphate buffered saline (Phosphat-gepufferte Kochsalz-Lösung)
- PdCl₂(dppf): Bis-(diphenylphosphanferrocenyl)-palladium(II)chlorid
- PdCl₂(PPh₃)₂: Dichlor-bis-(triphenylphosphin)-palladium
- Pd(PPh₃)₄: Tetrakis-(triphenylphosphin)-palladium(0)
- Ph: Phenyl
- RT: Raumtemperatur
- Rₜ: Retentionszeit (bei HPLC)
- TBTU: *O*-(Benzotriazol-1-yl)-*N,N,N',N*'-tetramethyluroniumtetrafluoroborat
- THF: Tetrahydrofuran
- TRIS: Tris-(hydroxymethyl)aminomethan

### HPLC- und LC-MS-Methoden:

### Methode 1 (HPLC):

Instrument: HP 1100 mit DAD-Detektion; Säule: Kromasil RP-18, 60 mm x 2 mm, 3.5 µm; Eluent: A = 5 mL HClO₄ / L H₂O, Eluent B = Acetonitril; Gradient: 0 min 2% B, 0.5 min 2% B, 4.5 min 90% B, 6.5 min 90% B; Fluss: 0.75 mL/min; Temperatur: 30°C; Detektion: UV 210 nm.

### Methode 2 (LC-MS):

Gerätetyp MS: Micromass ZQ; Gerätetyp HPLC: Waters Alliance 2790; Säule: Symmetry, C 18, 50 mm x 2.1 mm, 3.5 µm; Eluent B: Acetonitril + 0.05% Ameisensäure, Eluent A: Wasser + 0.05% Ameisensäure; Gradient: 0 min 5% B → 4.5 min 90% B → 5.5 min 90% B; Ofen: 50°C; Fluss: 1.0 mL/min; UV-Detektion: 210 nm.

### Methode 3 (LC-MS):

Instrument: Micromass Platform LCZ, HP1100; Säule: Symmetry C18, 50 mm x 2.1 mm, 3.5 µm; Eluent A: Wasser + 0.05% Ameisensäure, Eluent B: Acetonitril + 0.05% Ameisensäure; Gradient: 0 min 90% A → 4.0 min 10% A → 6.0 min 10% A; Ofen: 40°C; Fluss: 0.5 mL/min; UV-Detektion: 208-400 nm.

### Methode 4 (LC-MS):

Gerätetyp MS: Micromass ZQ; Gerätetyp HPLC: Waters Alliance 2790; Säule: Grom-Sil 120 ODS-4 HE 50 mm x 2 mm, 3.0 µm; Eluent B: Acetonitril + 0.05% Ameisensäure, Eluent A: Wasser + 0.05% Ameisensäure; Gradient: 0.0 min 5% B → 2.0 min 40% B → 4.5 min 90% B → 5.5 min 90% B; Ofen: 45°C; Fluss: 0.0 min 0.75 mL/min → 4.5 min 0.75 mL/min → 5.5 min 1.25 mL/min; UV-Detektion: 210 nm.

### Methode 5 (LC-MS):

Instrument MS: Micromass TOF (LCT); Instrument HPLC: 2-Säulen-Schaltung, Waters 2690; Säule: YMC-ODS-AQ, 50 mm x 4.6 mm, 3.0 µm; Eluent A: Wasser + 0.1% Ameisensäure, Eluent B: Acetonitril + 0.1% Ameisensäure; Gradient: 0.0 min 100% A → 0.2 min 95% A → 1.8 min 25% A → 1.9 min 10% A → 3.2 min 10% A; Ofen: 40°C; Fluss: 3.0 mL/min; UV-Detektion: 210 nm.

### Methode 6. (LC-MS):

Flow-injection, Instrument: Micromass Platform LCZ + Quattro LCZ; Eluent A: Wasser + 0.05% Ameisensäure, Eluent B: Acetonitril + 0.05% Ameisensäure; Gradient: 0.0 min 30% A → 1.0 min 30% A; Fluss: 0.2 - 0.3 mL/min; HPLC: Instrument HP 1100; UV-Detektion: DAD.

### Methode 7 (HPLC):

Instrument: HP 1100 mit DAD-Detektion; Säule: Kromasil RP-18, 60 mm x 2 mm, 3.5 µm; Eluent A: 5 mL HClO₄ / L H₂O, Eluent B: Acetonitril; Gradient: 0 min 2% B → 0.5 min 2% B → 4.5 min 90% B → 9 min 90% B; Fluss: 0.75 mL/min; Temperatur: 30°C; UV-Detektion: 210 nm.

### Ausgangsverbindungen:

### Allgemeine Arbeitsvorschrift A

### Synthese von 1-Benzothiophen-2-carbonsäuremethylestern:

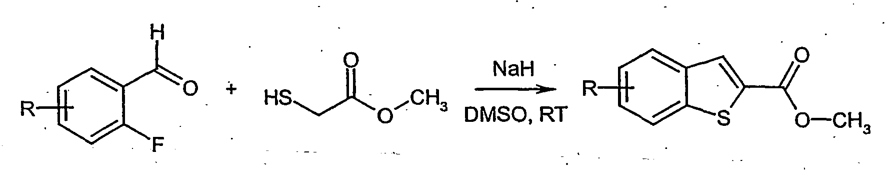

Unter einer Argonatmosphäre werden 1.5 Äquivalente Natriumhydrid (60%-ig in Paraffinöl) in absolutem DMSO (0.60-1.26 M Suspension) vorgelegt. Bei Raumtemperatur werden langsam 1.1 Äquivalente Mercaptoessigsäuremethylester zur Reaktionsmischung hinzugetropft, und man lässt bis zur Beendigung der Wasserstoffentwicklung (ca. 15 min) bei Raumtemperatur rühren. 1.0 Äquivalente des entsprechenden Benzaldehyds werden in absolutem DMSO gelöst (1.60-3.36 M Lösung) und bei Raumtemperatur zur Reaktionsmischung gegeben. Die Reaktionsmischung wird bis zur Beendigung der Reaktion (ca. 5-10 min) gerührt und anschließend in Eiswasser gegossen. Der entstandene Niederschlag wird abgesaugt, über Nacht im Vakuum bei 40°C getrocknet und roh weiter umgesetzt.

### Allgemeine Arbeitsvorschrift B

### Synthese von 1-Benzothiophen-2-carbonsäuren:

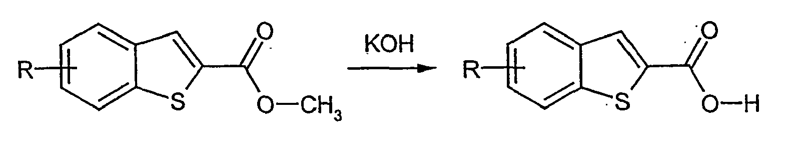

Der entsprechende 1-Benzothiophen-2-carbonsäuremethylester wird mit einer Mischung aus gleichen Teilen THF und 2 N wässriger Kaliumhydroxid-Lösung (0.28-0.47 M Lösung) versetzt. Man lässt die Reaktionsmischung bei Raumtemperatur über Nacht rühren. Im Vakuum wird das THF entfernt und die wässrige Reaktionsmischung mit konzentrierter Salzsäure sauer gestellt. Der entstandene Niederschlag wird abgesaugt und im Vakuum bei 40°C getrocknet.

### Allgemeine Arbeitsvorschrift C

### Amidknüpfung zwischen 3-Chinuklidinamin und 2-Benzothiophen- bzw. 2-Benzofurancarbonsäuren:

1.0 eq. des entsprechenden enantiomeren 3-Chinuklidinamin-Hydrochlorids werden zusammen mit 1 eq. der Carbonsäure und 1.2 eq. HATU bei 0°C in DMF vorgelegt. Nach Zugabe von 1.2 eq. *N,N*-Diisopropylethylamin wird das Gemisch bei RT gerührt. Nach 30 min. werden weitere 2.4 eq. *N,N*-Diisopropylethylamin zugegeben und über Nacht bei RT nachgerührt.

### Beispiel 1A

### 6-Brom-1-benzofuran-2-carbonsäure

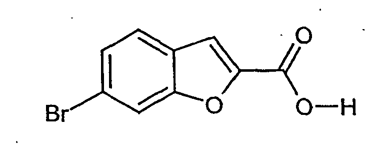

8.0 g (39.8 mmol) 4-Brom-2-hydroxybenzaldehyd und 1.47 g (3.98 mmol) Tetra-n-butylammoniumiodid werden zusammen mit 22 g (159.19 mmol) wasserfreiem Kaliumcarbonat vorgelegt. Es werden 9.07 g (83.57 mmol) Chloressigsäuremethylester zugegeben. Das Reaktionsgemisch wird 4 h auf 130°C erhitzt und anschließend mittels eines Eisbades auf 0°C abgekühlt. 100 mL THF und eine Lösung von 13.4 g (238.8 mmol) Kaliumhydroxid in 50 mL Wasser werden zugegeben und die Mischung wird anschließend über Nacht bei RT gerührt. Das THF wird unter reduziertem. Druck entfernt. Die zurückbleibende wässrige Phase wird mit Wasser verdünnt und mit konz. Salzsäure sauer gestellt. Das ausgefallene Produkt wird abfiltriert und im Hochvakuum getrocknet. Zur Feinreinigung wird über Kieselgel 60 (Merck, Darmstadt; Eluent: Toluol, Toluol-Essigsäure 50:1, Toluol-Essigsäure-Essigsäuremethylester 35:1:5) gereinigt. Das Solvens wird unter reduziertem Druck entfernt. Schließlich werden letzte Lösungsmittelreste im Hochvakuum entfernt. Es werden 3.8 g (40 % d.Th.) der Titelverbindung isoliert.
¹H-NMR (400 MHz, Methanol-d₄): δ = 7.91 (m, 1H), 7.61-7.51 (m, 3H).
HPLC (Methode 1): Rₜ = 4.1 min.
MS (ESIpos): m/z = 258 (M+NH₄)⁺.

### Beispiel 2A

### N-[(3R)-1-Azabicyclo[2.2.2]oct-3-yl]-6-brom-1-benzofuran-2-carboxamid

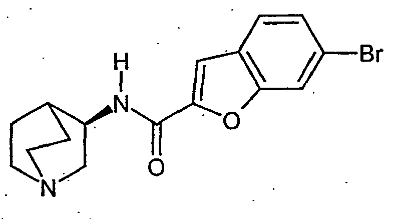

3.8 g (15.77 mmol) 6-Brombenzofuran-2-carbonsäure (Beispiel 1A), 3.14 g (15.77 mmol) (*R*)-3-Aminochinuklidin-Dihydrochlorid, 7.19 g (18.92 mmol) HATU, 7.34 g (56.76 mmol) *N,N-*Diisopropylethylamin und 50 mL DMF werden gemäß der allgemeinen Arbeitsvorschrift C umgesetzt. Das Rohprodukt wird in Methanol aufgenommen und zusammen mit saurem Ionenaustauscher (Dowex^{®} WX2-200) etwa 20 min. lang geschüttelt. Der beladene Ionenaustauscher wird sukzessive mit Methanol, Dichlormethan und erneut mit Methanol gewaschen. Das Produkt wird mit Methanol-Triethylamin 90:10 eluiert. Das Solvens wird unter reduziertem Druck am Rotationsverdampfer entfernt. Schließlich werden letzte Lösungsmittelreste im Hochvakuum entfernt. Es werden 5.14 g (85 % d.Th.) der Titelverbindung isoliert. Für die Analytik wird eine kleine Menge mittels 4 N Chlorwasserstoff in Dioxan in das Hydrochlorid überführt.
¹H-NMR (200 MHz, DMSO-d₆): δ = 10.55 (br. s, 1H), 9.22 (d, 1H), 8.05 (s, 1H), 7.75-7.55 (m, 3H), 4.43-4.29 (m, 1H), 3.70-3.55 (m, 1H), 3.45-3.10 (m, 5H), 2.25-2.00 (m, 2H), 1.98-1.82 (m, 2H), 1.80-1.60 (m, 1H).
HPLC (Methode 1): Rₜ = 3.9 min.
MS (ESIpos): m/z = 349 (M+H)⁺.
LC-MS (Methode 2): Rₜ = 1.49 min.
MS (ESIpos): m/z = 349 (M+H)⁺.

### Beispiel 3A

### N-[(3R)-1-Azabicyclo[2.2.2]oct-3-yl]-5-brom-1-benzofuran-2-carboxamid-Hydrochlorid

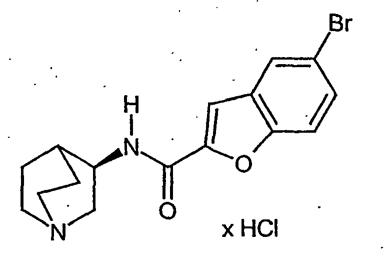

240 mg (0.98 mmol) 5-Brombenzofuran-2-carbonsäure, 200 mg (0.98 mmol) *(R)*-3-Aminochinuklidin-Dihydrochlorid, 450 mg (1.18 mmol) HATU, 460 mg (3.54 mmol) *N,N*-Diisopropylethylamin und 2.0 mL DMF werden gemäß der allgemeinen Arbeitsvorschrift C umgesetzt. Das Reaktionsgemisch wird durch präparative HPLC gereinigt. Abschließend wird das Produkt mit einem Überschuss an 1 N Salzsäure versetzt. Das Solvens wird unter reduziertem Druck entfernt. Es werden 202 mg (53% d.Th.) der Titelverbindung isoliert.
¹H-NMR (200 MHz, DMSO-d₆): δ = 9.38 (br. s, 1H), 8.88 (d, 1H), 7.60 (s, 1H), 7.38-7.20 (m, 2H), 7.09 (dd, 1H), 4.43-4.29 (m, 1H), 3.70-3.55 (m, 1H), 3.45-3.10 (m, 5H), 2.25-2.00 (m, 2H), 1.98-1.82 (m, 2H), 1.80-1.60 (m, 1H).
MS (ESIpos): m/z = 349 (M+H)⁺ (freie Base).
LC-MS (Methode 3): Rₜ = 2.71 min.
MS (ESIpos): m/z = 349 (M+H)⁺ (freie Base).

### Beispiel 4A

### 7-Brom-5-fluor-1-benzofuran-2-carbonsäure

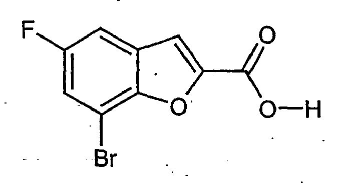

1.0 g (5.24 mmol) 2-Brom-4-fluorphenol werden in 4.0 mL Trifluoressigsäure vorgelegt. Es werden portionsweise innerhalb von 20 min. 1.47 g (10.47 mmol) Hexamethylentetramin zugegeben. Anschließend wird 28 h unter Rückfluss gekocht. Bei RT werden 6 mL Wasser und 3 mL 50%-ige Schwefelsäure zugegeben. Nach 2 h wird zweimal mit insgesamt 60 mL Essigsäureethylester extrahiert. Die vereinigten organischen Phasen werden viermal mit 1 N Salzsäure und einmal mit Wasser gewaschen. Es wird über Magnesiumsulfat getrocknet und das Solvens unter reduziertem Druck entfernt. Schließlich werden letzte Lösungsmittelreste im Hochvakuum entfernt. Das Rohprodukt (ohne weitere Reinigung) und 0.19 g (0.52 mmol) Tetra-n-butylammoniumiodid werden zusammen mit 2.9 g (20.96 mmol) wasserfreiem Kaliumcarbonat vorgelegt. Es werden 1.19 g (11.0 mmol) Chloressigsäuremethylester zugegeben. Das Reaktionsgemisch wird 4 h auf 130°C erhitzt und anschließend mittels eines Eisbades auf 0°C abgekühlt. Es werden 18 mL THF und eine Lösung von 1.76 g (31.44 mmol) Kaliumhydroxid in 18 mL Wasser zugegeben. Es wird über Nacht bei RT gerührt. Das Solvens wird unter reduziertem Druck entfernt. Es wird mit Wasser verdünnt und mit konzentrierter Salzsäure sauer gestellt. Es wird zweimal mit Essigsäureethylester extrahiert. Die vereinigten organischen Phasen werden über Magnesiumsulfat getrocknet und das Solvens wird unter reduziertem Druck am Rotationsverdampfer entfernt. Es wird über Kieselgel 60 (Merck, Darmstadt; Eluent: Toluol-Essigsäure 40:1) gereinigt. Das Solvens wird unter reduziertem Druck entfernt. Schließlich werden letzte Lösungsmittelreste im Hochvakuum entfernt. Es werden 257 mg (19 % d.Th. über beide Stufen) der Titelverbindung isoliert.
¹H-NMR (400 MHz, Methanol-d₄): δ =7.60 (m, 1H), 7.48-7.35 (m, H).
HPLC (Methode 1): Rₜ = 4.1 min.
MS (ESIpos): m/z = 276 (M+NH₄)⁺.

### Beispiel 5A

### N-[(3R)-1-Azabicyclo[2.2.2]oct-3-yl]-5-fluor-7-brom-1-benzofuran-2-carboxamid

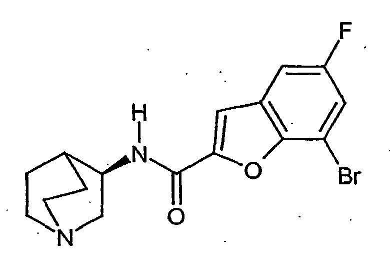

143 mg (0.55 mmol) 5-Fluor-7-brom-1-benzofuran-2-carbonsäure (Beispiel 4A), 100 mg (0.50 mmol) *(R)*-3-Aminochinuklidin-Dihydrochlorid, 229.14 mg (0.6 mmol) HATU, 234 mg (1.81 mmol) *N,N*-Diisopropylethylamin und 2.0 mL DMF werden gemäß der allgemeinen Arbeitsvorschrift C umgesetzt. DMF wird unter reduziertem Druck entfernt und das Rohprodukt in 1 N Natronlauge gelöst. Die wässrige Phase wird mit Essigsäureethylester extrahiert und mit gesättigter wässriger Natriumchlorid-Lösung gewaschen. Die vereinigten organischen Phasen werden über Magnesiumsulfat getrocknet und das Solvens wird unter reduziertem Druck am Rotationsverdampfer entfernt. Das Rohprodukt wird in Methanol aufgenommen und zusammen mit saurem Ionenaustauscher (Dowex^{®} WX2-200) etwa 20 min. lang geschüttelt. Der beladene Ionenaustauscher wird dreimal mit je 30 mL Methanol, dann mit Wasser, erneut mit Methanol, mit Dichlormethan und schließlich wieder mit Methanol gewaschen. Das Produkt wird mit Methanol-Triethylamin 95:5 eluiert. Das Solvens wird unter reduziertem Druck am Rotationsverdampfer entfernt. Es werden 181 mg (98 % d.Th.) der Titelverbindung isoliert.
¹H-NMR (400 MHz, Methanol-d₄): δ = 7.59 (d; 1H), 7.53-7.46 (m, 2H), 4.24-4.18 (m, 1H), 3.34-3.29 (m, 1H); 3.07-2.97 (m, 1H), 2.93-2.77 (m, 4H), 2.13-2.05 (m, 1H), 1.98-1.86 (m, 1H), 1.84-1.75 (m, 2H), 1.63-1.53 (m, 1H).
MS (ESIpos): m/z = 367 (M+H)⁺.
LC-MS (Methode 3): Rₜ = 2.92 min.
MS (ESIpos): m/z = 367 (M+H)⁺.

### Beispiel 6A

### 7-Brom-1-benzothiophen-2-carbonsäuremethylester

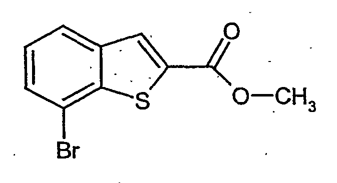

Ausgehend von 27.8 g (137.1 mmol) 3-Brom-2-fluorbenzaldehyd werden mit 8.2 g (205.7 mmol) Natriumhydrid (60%-ig in Paraffinöl) und 16.0 g (150.9 mmol) Mercaptoessigsäuremethylester 20.57 g eines Gemisches aus der Titelverbindung und der korrespondierenden Säure (ca. 1:1) nach der allgemeinen Arbeitsvorschrift A erhalten.

### Beispiel 7A

### 7-Brom-1-benzothiophen-2-carbonsäure

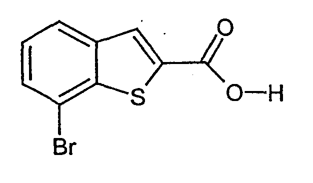

Ausgehend von 10.0 g (36.9 mmol) 7-Brom-1-benzothiophen-2-carbonsäuremethylester werden 8.99 g (91.0 % d.Th.) des gewünschten Produkts nach der allgemeinen Arbeitsvorschrift B erhalten.
¹H-NMR (200 MHz, DMSO-d₆): δ = 13.76 (br. s, 1H), 8.28 (s, 1H), 8.07 (d, 1H), 7.78 (d, 1H), 7.46 (dd, 1H).
HPLC (Methode 1): Rₜ= 4.4 min.

### Beispiel 8A

### N-(1-Azabicyclo[2.2.2]oct-3-yl)-7-brom-1-benzothiophen-2-carboxamid-Hydrochlorid

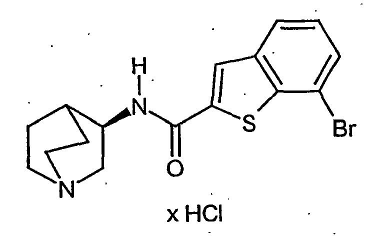

903.8 mg (3.52 mmol) 7-Brom-1-benzothiophen-2-carbonsäure (Beispiel 7A), 700 mg (3.52 mmol) (*R*)-3-Aminochinuklidin-Dihydrochlorid, 1604.0 mg (4.22 mmol) HATU, 1635.7 mg (12.66 mmol) *N,N*-Diisopropylethylamin und 7.0 mL DMF werden gemäß der allgemeinen Arbeitsvorschrift C umgesetzt. Das Reaktionsgemisch wird durch präparative HPLC gereinigt. Das Produkt wird in einem 1:1-Gemisch aus 4 M Chlorwasserstoff in Dioxan und 1 N Salzsäure gelöst, anschließend eingeengt und im Hochvakuum getrocknet. Es werden 1087 mg (77 % d.Th.) der Titelverbindung erhalten.
¹H-NMR (200 MHz, DMSO-d₆): δ = 10.01 (br. s, 1H), 9.15 (d, 1H), 8.47 (s, 1H), 8.02 (m, 1H), 7.74 (m, 1H), 7.43 (dd, 1H), 4.34 (m, 1H), 3.80-3.10 (m, 6H), 2.22 (m, 1H), 2.14 (m, 1H), 1.93 (m, 2H), 1.78 (m, 1H).
HPLC (Methode 1): Rₜ = 4.1 min.
MS (ESIpos): m/z = 365 (M+H)⁺ (freie Base).

### Beispiel 9A

### 6-Brom-1-benzothiophen-2-carbonsäuremethylester

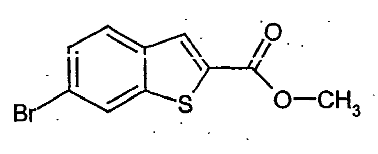

Ausgehend von 6.54 g (32.2 mmol) 4-Brom-2-fluorbenzaldehyd werden mit 1.93 g (48.3 mmol) Natriumhydrid (60%-ig in Paraffinöl) und 3.76 g (35.5 mmol) Mercaptoessigsäuremethylester nach der allgemeinen Arbeitsvorschrift A 4.06 g (46 % d.Th.) der Titelverbindung erhalten.
¹H-NMR (200 MHz, DMSO-d₆): δ = 8.42 (d, 1H), 8.22 (s, 1H), 7.98 (d, 1H), 7.65 (dd, 1H), 3.90 (s, 3H).
HPLC (Methode 1): Rₜ = 5.3 min.
MS (ESIpos): m/z = 270 (M⁺).

### Beispiel 10A

### 6-Brom-1-benzothiophen-2-carbonsäure

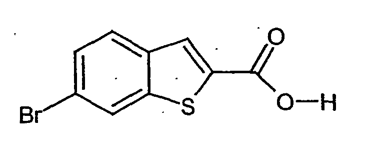

Ausgehend von 4.0 g (14.8 mmol) 6-Brom-1-benzothiophen-2-carbonsäuremethylester (aus Beispiel 9A) werden nach der allgemeinen Arbeitsvorschrift B 3.55 g (94 % d.Th.) des gewünschten Produkts erhalten.
¹H-NMR (400 MHz, DMSO-d₆): δ = 13.48 (br. s, 1H), 8.38 (s, 1H), 8.22 (s, 1H), 7.96 (d, 1H), 7.63 (m, 1H).
HPLC (Methode 1): Rₜ = 4.5 min.

### Beispiel 11A

### N-[(3R)-1-Azabicyclo[2.2.2]oct-3-yl]-6-brom-1-benzothiophen-2-carboxamid-Hydrochlorid

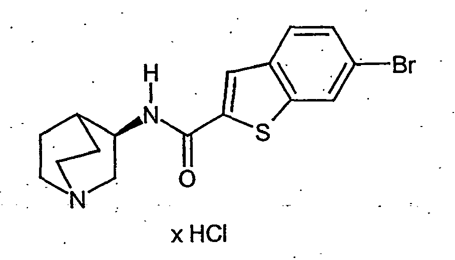

900.0 mg (3.50 mmol) 4-Brom-1-benzothiophen-2-carbonsäure (Beispiel 10A), 697.0 mg (3.50 mmol) *(R)*-3-Aminochinuklidin-Dihydrochlorid, 1597.1 mg (4.20 mmol) HATU, 1628.7 mg (12.60 mmol) *N,N*-Diisopropylethylamin und 8.0 mL DMF werden gemäß der allgemeinen Arbeitsvorschrift C umgesetzt. Das Reaktionsgemisch wird durch präparative HPLC gereinigt. Das Produkt wird in einem 1:1-Gemisch aus 4 M Chlorwasserstoff in Dioxan und 1 N Salzsäure gelöst und die Lösung wird anschließend eingeengt. Umkristallisation aus Methanol/Ethanol (1:10) ergibt 594 mg (42 % d.Th.) der Titelverbindung in Form gelb-brauner Kristalle.
¹H-NMR (300 MHz, DMSO-d₆): δ = 9.81 (br. s, 1H), 8.76 (m, 1H), 8.33 (s, 1H), 8.22 (s, 1H), 7.91 (d, 1H), 7.59 (dd, 1H), 4.15 (m, 1H), 3.51-2.93 (m, 6H), 2.12-1.92 (m, 2H), 1.79 (m, 2H), 1.58 (m, 1H).
HPLC (Methode 1): Rₜ = 4.1 min.
MS (ESIpos): m/z = 364 (M⁺) (freie Base).

### Beispiel 12A

### 5-Brom-1-benzothiophen-2-carbonsäuremethylester

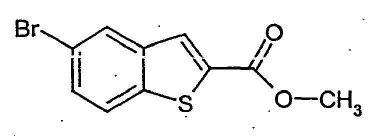

Ausgehend von 2.99 g (14.7 mmol) 5-Brom-2-fluorbenzaldehyd werden mit 0.88 g (22.1 mmol) Natriumhydrid (60%-ig) und 1.72 g (16.2 mmol) Mercaptoessigsäuremethylester nach der allgemeinen Arbeitsvorschrift A 2.76 g (69.1 % d.Th) der Titelverbindung erhalten.
¹H-NMR (200 MHz, DMSO-d₆): δ = 8.29 (d, 1H), 8.18 (s, 1H), 8.08 (d, 1H), 7.69 (dd, 1H), 3.90 (s, 3H).
HPLC (Methode 1): Rₜ = 5.2 min.
MS (ESIpos): m/z = 270 (M⁺).

### Beispiel 13A

### 5-Brom-1-benzothiophen-2-carbonsäure

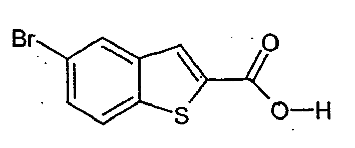

Ausgehend von 2.7 g (9.96 mmol) 5-Brom-1-benzothiophen-2-carbonsäuremethylester (aus Beispiel 12A) werden nach der allgemeinen Arbeitsvorschrift B 2.41 g (94 % d.Th.) des gewünschten Produkts erhalten.
¹H-NMR (400 MHz, DMSO-d₆): δ = 13.67 (br. s, 1H), 8.27 (m, 1H), 8.10 (s, 1H), 8.05 (d, 1H), 7.66 (dd, 1H).
HPLC (Methode 1): Rₜ = 4.5 min.

### Beispiel 14A

### N-[(3R)-1-Azabicyclo[2.2.2]oct-3-yl]-5-brom-1-benzothiophen-2-carboxamid-Hydrochlorid

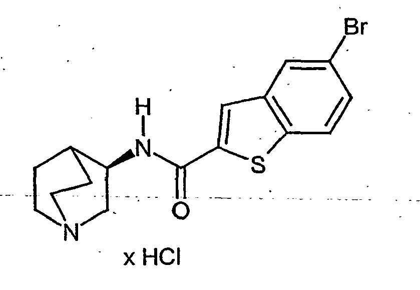

133.7 mg (0.52 mmol) 5-Brom-1-benzothiophen-2-carbonsäure (Beispiel 13A), 155.4 mg (0.78 mmol) *(R)*-3-Aminochinuklidin-Dihydrochlorid, 296.7 mg (0.78 mmol) HATU, 369.8 mg (2.86 mmol) *N,N*-Diisopropylethylamin und 1.5 mL DMF werden gemäß der allgemeinen Arbeitsvorschrift C umgesetzt. Das Reaktionsgemisch wird durch präparative HPLC gereinigt. Das Produkt wird in Acetonitril gelöst und mit einem Überschuss an 1 N Salzsäure versetzt. Schließlich wird das Solvens entfernt. Es werden 175 mg (84 % d.Th.) der Titelverbindung isoliert.
¹H-NMR (200 MHz, DMSO-d₆): δ = 9.44 (br. s, 1H), 8.95 (d, 1H), 8.30-8.10 (m, 2H), 8.03 (d, 1H), 7.60 (m, 1H), 4.38-4.20 (m, 1H), 3.80-3.55 (m, 1H), 3.42-3.05 (m, 5H), 2.25-2.00 (m, 2H), 1.98-1.62 (m, 3H).
HPLC (Methode 1): Rₜ = 4.1 min.
MS (ESIpos): m/z = 365 (M+H)⁺ (freie Base).

### Beispiel 15A

### 4-(4-Bromphenyl)morpholin

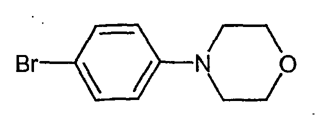

Zu einer Lösung von 20 g (122.5 mmol) N-Phenylmorpholin in 170 mL Essigsäure wird bei Raumtemperatur eine Lösung von 6.94 mL (134.8 mmol) Brom in 25 mL Essigsäure über einen Zeitraum von 40 min. langsam zugetropft. Nach 30 min. Rühren bei Raumtemperatur wird das Reaktionsgemisch in 750 mL Wasser eingerührt und mit 45%-iger Natronlauge auf pH 11 eingestellt. Der entstehende Niederschlag wird abgesaugt, mit Wasser gewaschen und im Hochvakuum getrocknet. Nach Umkristallisation aus Ethanol erhält man 18.6 g (62.9 % d.Th.) der Titelverbindung.
¹H-NMR (200 MHz, DMSO-d₆): δ= 7.37 (m, 2H), 6.89 (m, 2H), 3.73 (m, 4H), 3.08 (m, 4H).
HPLC (Methode 1): Rₜ = 3.9 min.
MS (ESIpos): m/z = 242 (M+H)⁺.

### Beispiel 16A

### 4-(4-Bromphenyl)-3-morpholinon

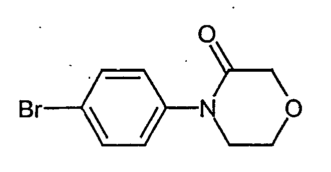

Zu einer Lösung von 500 mg (2.07 mmol) 4-(4-Bromphenyl)morpholin (Beispiel 15A) in 10 mL Dichlormethan werden 1.41 g (6.20 mmol) Benzyltriethylammoniumchlorid und 0.98 g (6.20 mmol) Kaliumpermanganat gegeben. Nach 5 h unter Rückfluss wird der Kolbeninhalt im Vakuum eingeengt und der Rückstand mittels präparativer HPLC aufgereinigt. Das eingeengte Produkt wird im Hochvakuum getrocknet. Man erhält 217 mg (35.7 % d.Th.) der Titelverbindung.
LC-MS (Methode 4): Rₜ = 2.9 min, m/z = 255 (M⁺).

### Beispiel 17A

### 3-(4-Morpholinyl)phenyl-trifluormethansulfonat

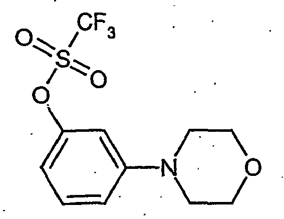

Zu einer auf -10°C gekühlten Lösung von 1.54 g (8.6 mmol) 3-(4-Morpholinyl)-phenol und 3.59 mL (25.8 mmol). Triethylamin in 10 mL Dichlormethan werden 2.18mL (12.9 mmol) Trifluormethansulfonsäureanhydrid langsam zugetropft. Es wird 30 min. bei -10°C und anschließend 30 min. bei 0°C nachgerührt. Es wird sukzessive mit 10%-iger Natriumhydrogencarbonat-Lösung, Wasser und gesättigter Kochsalz-Lösung gewaschen, über Natriumsulfat getrocknet, im Vakuum eingeengt und der Rückstand im Hochvakuum getrocknet. Man erhält 2.41 g (90.1 % d.Th.) der Titelverbindung.
¹H-NMR (200 MHz, CDCl₃): δ = 7.28 (m, 1H), 6.88 (m, 1H), 6.73 (m, 2H), 3.86 (m, 4H), 3.18 (m, 4H).
HPLC (Methode 1): Rₜ = 4.8 min.
MS (ESIpos): m/z = 312 (M+H)⁺.

### Beispiel 18A

### 4-(4-Morpholinylcarbonyl)phenyl-trifluormethansulfonat

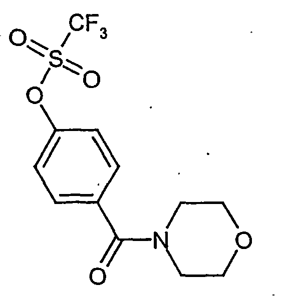

Zu einer auf -10°C gekühlten Lösung von 1.0 g (4.83 mmol) 4-(4-Morpholinylcarbonyl)phenol und 2.02 mL (14.48 mmol) Triethylamin in 20 mL Dichlormethan werden 1.23 mL (7.24 mmol) Trifluormethansulfonsäureanhydrid langsam zugetropft. Es wird 30 min. bei -10°C und anschließend 30 min. bei 0°C nachgerührt. Es wird sukzessive mit 10%-iger Natriumhydrogencarbonat-Lösung, Wasser und gesättigter Kochsalz-Lösung gewaschen, über Natriumsulfat getrocknet, im Vakuum eingeengt und der Rückstand im Hochvakuum getrocknet. Man erhält 1.71 g (94.6 % d.Th.) der Titelverbindung.
¹H-NMR (400 MHz, Methanol-d₄): δ= 7.62 (m, 2H), 7.49 (m, 2H), 3.86-3.34 (m, 8H).
HPLC (Methode 1): Rₜ= 4.2 min.
MS (ESIpos): m/z = 357 (M+NH₄)⁺.

### Beispiel 19A

### 7-[4-(4-Morpholinyl)phenyl]-1-benzothiophen-2-carbonsäuremethylester

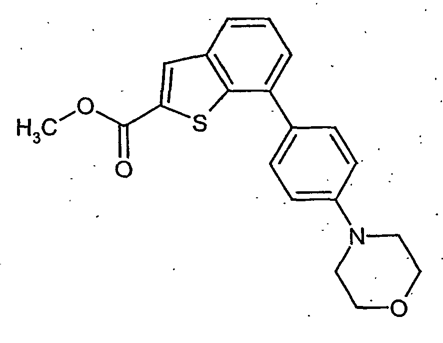

Zu einer Lösung von 619.1 mg (2.28 mmol) 7-Brom-1-benzothiophen-2-carbonsäuremethylester (Beispiel 6A) und 520 mg (2.51 mmol) 4-(4-Morpholinyl)phenylboronsäure in 10 mL DMF werden sukzessive 3.42 mL 2 M Natriumcarbonat-Lösung sowie 83.5 mg (0.11 mmol) PdCl₂(dppf) zugegeben. Es wird 16 h auf 80°C erhitzt. Nach dem Abkühlen wird über Kieselgur filtriert und mittels präparativer HPLC aufgereinigt. Das eingeengte Produkt wird im Hochvakuum getrocknet. Man erhält 146.7 mg (16.4 % d.Th.) der Titelverbindung.
HPLC (Methode 1): Rₜ = 4.6 min.
MS (ESIpos): m/z = 354 (M+H)⁺.

### Beispiel 20A

### 7-[4-(4-Morpholinyl)phenyl]-1-benzothiophen-2-carbonsäure

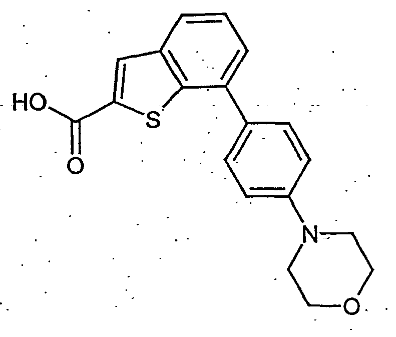

Eine Lösung von 330 mg (0.77 mmol) 7-[4-(4-Morpholinyl)phenyl]-1-benzothiophen-2-carbonsäuremethylester (Beispiel 19A) in 6 mL einer 1:1-Mischung aus Methanol und 2 N Kalilauge wird 2 h bei Raumtemperatur und 1 h bei 50°C gerührt. Das Reaktionsgemisch wird im Vakuum eingeengt, mit Wasser versetzt und anschließend mit konz. Salzsäure angesäuert. Der entstehende Niederschlag wird abgesaugt, zweimal mit Wasser nachgewaschen und im Hochvakuum getrocknet. Man erhält 292 mg Rohprodukt, das ohne weitere Reinigung umgesetzt wird.

### Beispiel 21A

### 7-(2-Methoxyphenyl)-1-benzofuran-2-carbonsäure

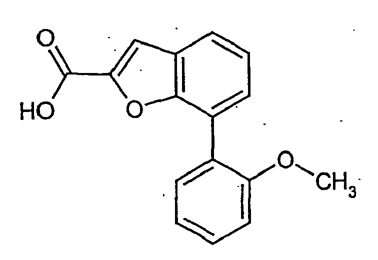

5.0 g (20.7 mmol) 7-Brom-1-benzofuran-2-carbonsäure (Beispiel 29A) und 3.78 g (24.9 mmol) 2-Methoxyphenylboronsäure werden in 50 mL DMF vorgelegt. Nach Zugabe von 31.1 mL 2 M Natriumcarbonat-Lösung und 1.2 g (1.04 mmol) Pd(PPh₃)₄ wird auf 90°C erhitzt. Nach 18 h wird das Lösungsmittel abdestilliert. Der Rückstand wird zwischen 1 N Salzsäure und Essigsäureethylester verteilt und dreimal mit je 200 mL Essigsäureethylester extrahiert. Die organische Phase wird über Natriumsulfat getrocknet und im Vakuum eingeengt. Der Rückstand wird mittels Flash-Chromatographie (Kieselgel, Laufmittel: Dichlormethan/Methanol/Essigsäure 100:10:1) aufgereinigt. Nach Einengen und Trocknen im Hochvakuum erhält man 2.97 g (53.2 % d.Th.) der Titelverbindung.
¹H-NMR (300 MHz, DMSO-d₆): δ = 13.46 (s, 1H), 7.73 (dd, 1H), 7.59 (s, 1H), 7.48-7.33 (m, 4H), 7.20 (d, 1H), 7.09 (m, 1H), 3.75 (s, 3H).
HPLC (Methode 1): Rₜ = 4.5 min.
MS (ESIpos): m/z = 286 (M+NH₄)⁺.

### Beispiel 22A

### N-[(3S)-1-Azabicyclo[2.2.2]oct-3-yl]-7-brom-1-benzothiophen-2-carboxamid-Hydrochlorid

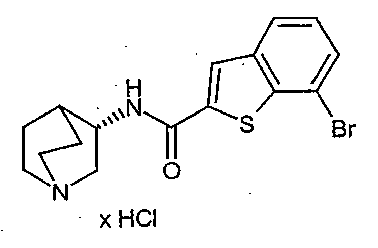

Zu einer auf 0°C gekühlten Lösung von 4.0 g (15.6 mmol) 7-Brom-1-benzothiophen-2-carbonsäure (Beispiel 7A) und 3.10 g (15.6 mmol) *(S)*-3-Aminochinuklidin-Dihydrochlorid in 50 mL DMF werden 3.58 g (18.7 mmol) EDC, 2.52 g (18.7 mmol) HOBt und 7.8 mL (56 mmol) Triethylamin zugegeben. Bei Raumtemperatur wird 18 h gerührt. Die Reaktion wird durch Zugabe von 10%-iger Natriumhydrogencarbonat-Lösung abgebrochen. Der nach der Zugabe von Essigsäureethylester entstehende Niederschlag wird abfiltriert. Die wässrige Phase wird mit Essigsäureethylester extrahiert, die vereinigten organischen Phasen werden über Natriumsulfat getrocknet, eingeengt und der Rückstand im Hochvakuum getrocknet, Man erhält 4.70 g (68 % d.Th.) der Titelverbindung.
Die spektroskopischen Daten stimmen mit denen der enantiomeren Verbindung ; (Beispiel 8A) überein.

### Beispiel 23A

### N-[(3R)-1-Azabicyclo[2.2.2]oct-3-yl]-6-(4-formylphenyl)-1-benzothiophen-2-carboxamid-Hydrochlorid

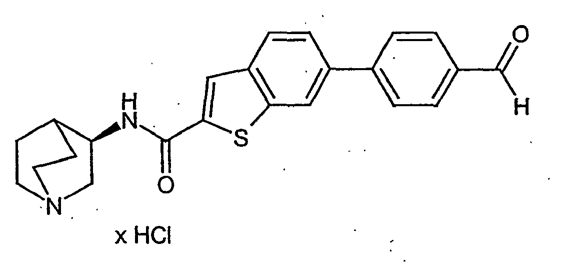

200 mg (0.50 mmol) *N*-[(3R)-1-Azabicyclo[2.2.2]oct-3-yl]-6-brom-1-benzothiophen-2-carboxamid-Hydrochlorid (Beispiel 11A) und 74.6 mg (0.50 mmol) 4-Formylphenylboronsäure werden in 2 mL DMF vorgelegt. Nach Addition von 0.75 mL 2 M Natriumcarbonat-Lösung und 20.3 mg (0.02 mmol) PdCl₂(dppf) wird auf 80°C erhitzt. Nach 18 h wird das Reaktionsgemisch über Kieselgur filtriert und mittels präparativer HPLC aufgereinigt. Die Produktfraktionen werden eingeengt, mit einer 5:1-Mischung aus Methanol und 4 N Chlorwasserstoff in Dioxan versetzt und erneut eingeengt. Nach Trocknen im Hochvakuum erhält man 163.8 mg (75.0 % d.Th.) der Titelverbindung.
¹H-NMR (200 MHz, DMSO-d₆): δ = 10.09 (s, 1H), 10.07 (br. s, 1H), 9.10 (d, 1H), 8.50 (m, 1H), 8.37 (s, 1H), 8.15-7.97 (m, 5H), 7.87 (dd, 1H), 4.33 (m, 1H), 3.68 (m, 1H), 3.45-3.12 (m, 5H), 2.23 (m, 1H), 2.16 (m, 1H), 1.91 (m, 2H), 1.76 (m, 1H). HPLC (Methode 1): Rₜ = 4.1 min.
MS (ESIpos): m/z = 391 (M+H)⁺ (freie Base).

### Beispiel 24A

### 6-Cyano-1-benzothiophen-2-carbonsäuremethylester

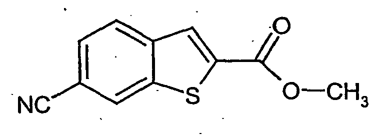

4.08 g (23.2 mmol) 4-Cyano-2-nitrobenzaldehyd, 2.46 g (23.2 mmol) Mercaptoessigsäuremethylester und 6.46 mL (46.4 mmol) Triethylamin werden in 12.3 mL DMSO für 2.5 h auf 80°C erhitzt. Die Reaktionslösung wird auf 400 mL Eiswasser gegeben. Nach der Addition von 4 mL Essigsäure wird der entstandene Niederschlag abgesaugt, zweimal mit Wasser gewaschen und über Nacht bei 50°C im Vakuum getrocknet. Man erhält 4.19 g (83.2 % d.Th.) der Titelverbindung.
¹H-NMR (200 MHz, DMSO-d₆): δ = 8.73 (d, 1H), 8.32 (s, 1H), 8.21 (d, 1H), 7.85 (dd, 1H), 3.92 (s, 3H).
HPLC (Methode 1): Rₜ = 4.4 min.
MS (ESIpos): m/z = 218 (M+H)⁺.

### Beispiel 25A

### 6-Cyano-1-benzothiophen-2-carbonsäure

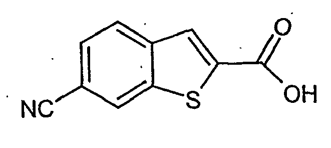

Entsprechend der allgemeinen Arbeitsvorschrift B werden ausgehend von 0.6 g (2.76 mmol) 6-Cyano-1-benzothiophen-2-carbonsäuremethylester (Beispiel 24A) 0.49 g (61.6 % d.Th.) des gewünschten Produkts erhalten.
HPLC (Methode 1): Rₜ = 3.9 min.
MS (ESIpos): m/z = 222 (M+H)⁺.

### Beispiel 26A

### N-[(3R)-1-Azabicyclo[2.2.2]oct-3-yl]-6-cyano-1-benzothiophen-2-carboxamid-Hydrochlorid

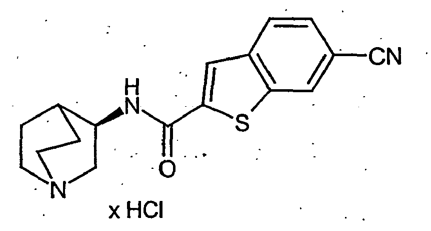

320.8 mg (1.1 mmol) 6-Cyano-1-benzothiophen-2-carbonsäure (Beispiel 25A), 200 mg (1.0 mmol) *(R)*-3-Aminochinuklidin-Dihydrochlorid, 458.3 mg (1.21 mmol) HATU, 467.3 mg (3.62 mmol) *N*,*N*-Diisopropylethylamin und 4.0 mL DMF werden gemäß der allgemeinen Arbeitsvorschrift C umgesetzt. Das Reaktionsgemisch wird mittels präparativer HPLC gereinigt. Das Produkt wird in einem Gemisch aus Methanol und 4 M Chlorwasserstoff in Dioxan gelöst, anschließend eingeengt und im Hochvakuum getrocknet. Man erhält 222.1 mg (64 % d.Th.) der Titelverbindung.
¹H-NMR (300 MHz, DMSO-d₆): δ = 9.80 (m, 1H), 9.12 (d, 1H), 8.68 (s, 1H), 8.37 (s, 1H), 8.16 (d, 1H), 7.83 (dd, 1H), 4.33 (m, 1H), 3.76-3.05 (m, 6H), 2.23 (m, 1H), 2.13 (m, 1H), 1.92 (m, 2H), 1.76 (m, 1H).
HPLC (Methode 1): Rₜ = 3.6 min.
MS (ESIpos): m/z = 312 (M+H)⁺ (freie Base).

### Beispiel 27A

### 6-[(Z)-Amino(hydroxyimino)methyl]-N-[(3R)-1-azabicyclo[2.2.2]oct-3-yl]-1-benzothiophen-2-carboxamid-Dihydrochlorid

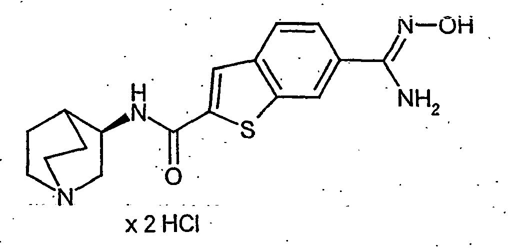

800 mg (2.0. mmol) *N*-[(3R)-1-Azabicyclo[2.2.2]oct-3-yl]-6-cyano-1-benzothiophen-2-carboxamid-Hydrochlorid (Beispiel 26A), 278.1 mg (4.0 mmol) Hydroxylamin-Hydrochlorid und 829.5 mg (6.0 mmol) Kaliumcarbonat werden in 8 mL eines 8:1-Gemisches aus Wasser und Ethanol für 3 h auf 80°C erhitzt. Das Gemisch wird säulenchromatographisch an Kieselgel aufgereinigt (Laufmittel: Dichlormethan/-Methanol/25%-ige Ammoniaklösung 100:20:4). Die Produktfraktionen werden vereinigt, eingeengt, mit Methanol und 4 M Chlorwasserstoff in Dioxan versetzt, anschließend erneut eingeengt und im Hochvakuum getrocknet. Man erhält 447.3 mg (53.6 % d.Th.) der Titelverbindung.
¹H-NMR (200 MHz, DMSO-d₆): δ = 11.15 (m, 1H), 10.22 (m, 1H), 9.36 (d, 1H), 8.52 (s, 1H), 8.46 (m, 1H), 8.14 (d, 1H), 7.73 (dd, 1H), 4.33 (m, 1H), 3.93-3.10 (m, 6H), 2.32-2.05 (m, 2H), 1.93 (m, 2H), 1.75 (m, 1H).
HPLC (Methode 1): Rₜ = 2.9 min.
MS (ESIpos): m/z = 345 (M+H)⁺ (freie Base).

### Beispiel 28A

### 3-Brom-2-hydroxybenzaldehyd

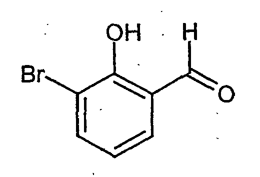

20.0 g (115.6 mmol) 2-Bromphenol werden in 500 mL trockenem Acetonitril vorgelegt. Es werden 16.84 g (176.87 mmol) trockenes Magnesiumchlorid, 23.4 g Paraformaldehyd-Granulat und 41.9 mL (300.6 mmol) Triethylamin zugegeben. Das Reaktionsgemisch wird 4 h unter Rückfluss erhitzt, dann auf 0°C gekühlt und mit 300 mL 2 N Salzsäure versetzt. Die wässrige Phase wird dreimal mit je 200 mL Diethylether extrahiert. Die organische Phase wird über Magnesiumsulfat getrocknet und das Solvens im Vakuum entfernt. Es werden 24 g (64 % d.Th., Gehalt 62 % nach HPLC) der Titelverbindung isoliert, die ohne weitere Aufreinigung weiter umgesetzt wird.
HPLC (Methode 1): Rₜ = 4.25 min.
MS (ESIpos): m/z = 202 (M+H)⁺.

### Beispiel 29A

### 7-Brom-1-benzofuran-2-carbonsäure

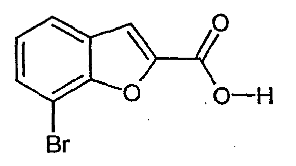

13.5 g (40.3 mmol) 3-Brom-2-hydroxybenzaldehyd (Beispiel 28A, Gehalt 62 %) werden zusammen mit 9.18 g (84.62 mmol) Chloressigsäuremethylester, 1.49 g (4.03 mmol) Tetra-n-butylammoniumiodid und 22.28 g (161.18 mmol) Kaliumcarbonat für 6 h auf 130°C erhitzt. Nach Abkühlung auf RT werden 100 mL Wasser und 100 mL THF sowie 13.57 g (241.77 mmol) Kaliumhydroxid zugegeben und über Nacht bei RT gerührt. Das Solvens wird unter reduziertem Druck entfernt, der Rückstand in 400 mL Wasser aufgenommen und viermal mit insgesamt 400 mL Diethylether gewaschen. Unter Eiskühlung wird mit konzentrierter Salzsäure auf pH 0 gestellt und fünfmal mit insgesamt 700 mL Essigsäureethylester extrahiert. Die organische Phase wird mit 100 mL gesättigter Natriumchlorid-Lösung gewaschen und anschließend über Magnesiumsulfat getrocknet. Das Rohprodukt wird im Hochvakuum von Lösungsmittelresten vollständig befreit und mit 80 mL Diethylether verrührt. Das Produkt wird abfiltriert und mit wenig eiskaltem Diethylether nachgewaschen. Es werden 4.8 g (47 % d.Th.) der Titelverbindung isoliert.
¹H-NMR (200 MHz, DMSO-d₆): δ = 13.5 (br. s, 1H), 7.86-7.72 (m, 2H), 7.79 (s, 1H), 7.31 (t, 1H).
MS(DCI/NH₃):m/z=258(M+NH₄)⁺.

### Beispiel 30A

### N-[(3R)-1-Azabicyclo[2.2.2]oct-3-yl]-7-brom-1-benzofuran-2-carboxamid

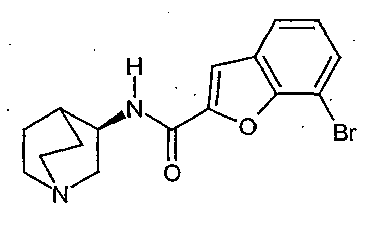

5.20 g (21.57 mmol) 7-Brombenzofuran-2-carbonsäure (Beispiel 29A), 4.3 g (21.57 mmol) *(R)*-3-Aminochinuklidin-Dihydrochlorid, 9.84 g (25.89 mmol) HATU, 13.53 mL (74.68 mmol) *N,N*-Diisopropylethylamin und 21 mL DMF werden gemäß der allgemeinen Arbeitsvorschrift C umgesetzt. Das Solvens wird unter reduziertem Druck entfernt, das Rohprodukt in 100 mL Essigsäureethylester aufgenommen und 15-mal mit insgesamt 1.5 L 1 N Natronlauge gewaschen. Die organische Phase wird über magnesiumsulfat getrocknet und vom Solvens befreit. Es werden 5.2 g (69 % d.Th.) der Titelverbindung isoliert.
¹H-NMR (300 MHz, DMSO-d₆): δ = 8.48 (d, 1H), 7.85-7.65 (m, 3H), 7.25 (t, 1H), 3.95 (m, 1H), 3.15 (m, 1H), 2.95 (m, 1H), 2.80-2.60 (m, 4H), 1.90 (m, 1H), 1.70 (m, 1H), 1.58 (m, 2H), 1.35 (m, 1H).
HPLC (Methode 1): Rₜ = 3.79 min.
MS (ESIpos): m/z = 349 (M+H)⁺
[α]²⁰_{D} = 26.9° (c = 0.50, Methanol).
In einigen Ausführungsbeispielen wird das entsprechende Hydrochlorid eingesetzt, welches durch Versetzen der Titelverbindung mit einer 5:1-Mischung aus Methanol und 1 N Salzsäure und anschließendes Einengen und Trocknen im Hochvakuum erhalten wird.

### Beispiel 31A

### N-[(3S)-1-Azabicylo[2.2.2]oct-3-yl]-7-brom-1-benzofuran-2-carboxamid

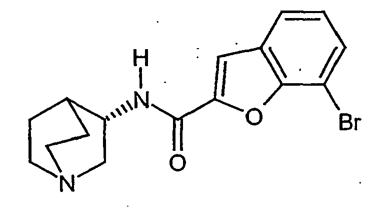

4.0 g (16.59 mmol) 7-Brombenzofuran-2-carbonsäure (Beispiel 29A), 3.3 g (16.59 mmol) *(S)*-3-Aminochinuklidin-Dihydrochlorid, 7.57 g (19.91 mmol) HATU, 10.41 mL (59.74 mmol) *N,N*-Diisopropylethylamin und 21 mL DMF werden gemäß der allgemeinen Arbeitsvorschrift C umgesetzt. Das Solvens wird unter reduziertem Druck entfernt, das Rohprodukt in 100 mL Essigsäureethylester aufgenommen und 15-mal mit insgesamt 1.5 L 1 N Natronlauge gewaschen. Die organische Phase wird über Magnesiumsulfat getrocknet und vom Solvens befreit. Es werden 5.0 g (85 % d.Th.) der Titelverbindung isoliert.
Die analytischen Daten stimmen mit denen aus Beispiel 30A überein.
[α]²⁰_{D} = -28.0° (c = 0.1, Methanol).

### Beispiel 32A

### 2-(4-Morpholinyl)phenyl-trifluormethansulfonat

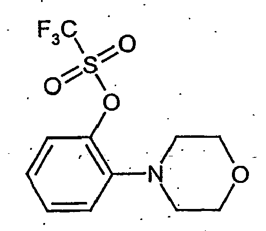

Zu einer auf -10°C gekühlten Lösung von 2 g (10.9 mmol) 2-(4-Morpholinyl)phenol und 4.57 mL (32.8 mmol) Triethylamin in 15 mL Dichlormethan werden 2.78 mL (16.4 mmol) Trifluormethansulfonsäureanhydrid langsam zugetropft. Es wird 30 min, bei -10°C und anschließend 30 min. bei 0°C nachgerührt. Es wird sukzessive mit 10%-iger Natriumhydrogencarbonat-Lösung, Wasser und gesättigter Kochsalz-Lösung gewaschen, über Natriumsulfat getrocknet, im Vakuum eingeengt und der Rückstand im Hochvakuum getrocknet. Man erhält 3.48 g (87.6 % d.Th.) der Titelverbindung.
HPLC (Methode 1): Rₜ = 4.9 min.
MS (ESIpos): m/z = 312 (M+H)⁺.

### Beispiel 33A

### N-[(3R)-1-Azabicyclo[2.2.2]oct-3-yl]-6-(3-formylphenyl)-1-benzothiophen-2-carboxamid-Hydrochlorid

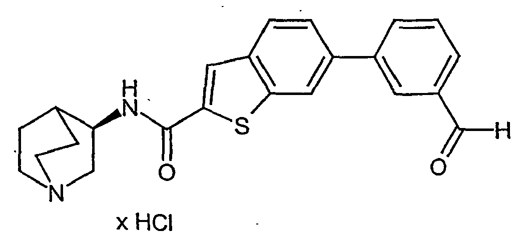

200 mg (0.50 mmol) *N*-[(3R)-1-Azabicyclo[2.2.2]oct-3-yl]-6-brom-1-benzothiophen-2-carboxamid-Hydrochlorid (Beispiel 11A) und 74.6 mg (0.50 mmol) 3-Formylphenylboronsäure werden in 1 mL DMF vorgelegt. Nach Zugabe von 0.75 mL 2 M Natriumcarbonat-Lösung und 20.3 mg (0.02 mmol) PdCl₂(dppf) wird auf 80°C erhitzt. Nach 18 h wird das Reaktionsgemisch über Kieselgur filtriert und mittels präparativer HPLC aufgereinigt. Die Produktfraktionen werden eingeengt, mit einer 5:1-Mischung aus Methanol und 4 N Chlorwasserstoff in Dioxan versetzt und erneut eingeengt. Nach Trocknen im Hochvakuum erhält man 92.4 mg (39.5 % d.Th.) der Titelverbindung.
HPLC (Methode 1): Rₜ = 4.11 min.
MS (ESIpos): m/z = 391 (M+H)⁺ (freie Base).

### Ausführungsbeispiele:

### Allgemeine Arbeitsvorschrift D:

1.5 eq. Bis(pinacolato)dibor, 3.25 eq. trockenes Kaliumacetat, 1.3 eq. des substituierten Halogenaromaten öder des substituierten Aryltrifluormethansulfonats werden in DMF (ca. 1 mL/mmol Halogenaromat bzw. Aryltrifluormethansulfonat) gelöst. Es wird für 15 Minuten Argon durch das Reaktionsgemisch geleitet, anschließend mit 0.05 eq. PdCl₂(dppf) versetzt und für 2 h auf 90°C erhitzt. Anschließend werden 1.0 eq. des entsprechenden Brom-substituierten *N*-[(3R)-1-Azabicyclo[2.2.2]oct-3-yl]-benzothiophen-2-carboxamids oder *N*-[(3R)-1-Azabicyclo[2.2.2]oct-3-yl]-benzokran-2-carboxamids, 5 eq. wässrige 2 M Natriumcarbonat-Lösung und weitere 0.05 eq. PdCl₂(dppf) zugegeben. Das Reaktionsgemisch wird 6-12 h auf 90°C erhitzt. Die Aufreinigung erfolgt durch präparative HPLC. Das erhaltene Produkt (freie Base) wird in Methanol gelöst und mit einem Überschuss an 1 N Salzsäure versetzt. Das Lösungsmittel wird unter reduziertem Druck entfernt.

### Allgemeine Arbeitsvorschrift E:

Zu einer 2 N Lösung eines Amins (0.23 mmol) in DMF wird eine Lösung aus 50 mg (0.11 mmol) 3-(2-{[(3R)-1-Azabicyclo[2.2.2]oct-3-yl-amino]carbonyl}-1-benzothien-7-yl)-benzoesäure-Hydrochlorid (Beispiel 75), 103 mg (0.27 mmol) HATU und 52.5 mg (0.41 mmol) *N,N*-Diisopropylethylamin in 0.5 mL DMF zugetropft. Nach 16 h bei Raumtemperatur wird das Reaktionsgemisch mit 0.1 mL Wasser versetzt, filtriert und mittels präparativer HPLC aufgereinigt. Die Produktfraktionen werden vereinigt, mit 2 mL 1 N Salzsäure versetzt, im Vakuum eingeengt und im Hochvakuum getrocknet.

### Allgemeine Arbeitsvorschrift F:

Zu einer Lösung aus 50 mg (0.12 mmol) *N*-((3R)-Chinuclidin-3-yl)-[7-(3-aminophenyl)benzo[b]thiophen-2-yl]carboxamid-Hydrochlorid (Beispiel 21) in 0.5 mL DMF werden 0.24 mmol Säurechlorid und 84 µL (0.60 mmol) Triethylamin hinzugefügt. Nach einer Stunde bei Raumtemperatur wird das Reaktionsgemisch mit 0.5 mL 1 N Natronlauge und 15. mL Essigsäureethylester versetzt und filtriert. Die eingeengte organische Phase wird mittels präparativer HPLC aufgereinigt. Die Produktfraktionen werden vereinigt, mit 1 N Salzsäure versetzt, im Vakuum erneut eingeengt und im Hochvakuum getrocknet.

### Beispiel 1

### N-[(3R)-1-Azabicyclo[2.2.2]odt-3-yl]-6-[2-(hydroxymethyl)phenyl]-1-benzofuran-2-carboxamid

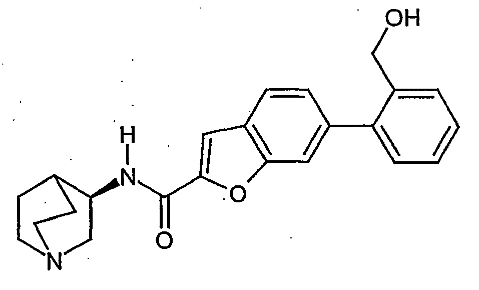

Eine Mischung aus 130 mg (0.86 mmol) 2-(Hydroxymethyl)phenylboronsäure, 200 mg (0.57 mmol) *N*-[(3R)-1-Azabicyclo[2.2.2]oct-3-yl]-6-brom-1-benzofuran-2-carboxamid (Beispiel 2A), 1.72 mL (1.72 mmol) 1 N Natronlauge, 40 mg (0.06 mmol) 1,1'-Bis(diphenylphosphino)ferrocenpalladium(II)chlorid und 2 mL DMF wird 18 h auf 80-85°C erhitzt. Das Solvens wird unter reduziertem Druck entfernt. Das Rohprodukt wird über Kieselgel 60 (Merck, Darmstadt; Eluent: Dichlormethan, Dichlormethan-Methanol 20:1, Dichlormethan-Methanol-Ammoniak 80:20:2) gereinigt. Das Solvens wird unter reduziertem Druck entfernt. Schließlich werden letzte Lösungsmittelreste im Hochvakuum entfernt. Es werden 149 mg (63 % d.Th.) der Titelverbindung isoliert.
¹H-NMR (200 MHz, CDCl₃): δ = 7.71-7.28 (m, 8H), 6.77 (d, 1H), 4.62 (s, 2H), 4.28-4.12 (m, 1H), 3.56-3.38 (m, 1H), 3.04-2.78 (m, 4H), 2.75-2.59 (m, 1H), 2.16-2.02 (m, 1H), 1.93-1.66 (m, 3H), 1.66-1.45 (m, 1H).
HPLC (Methode 1): Rₜ = 3.6 min.
LC-MS (Methode 2): Rₜ = 1.49 min.
MS (ESIpos): m/z = 377 (M+H)⁺.

### Beispiel 2

### N-[(3R)-1-Azabicyclo[2.2.2]oct-3-yl]-6-[3-(hydroxymethyl)phenyl]-1-benzofuran-2-carboxamid

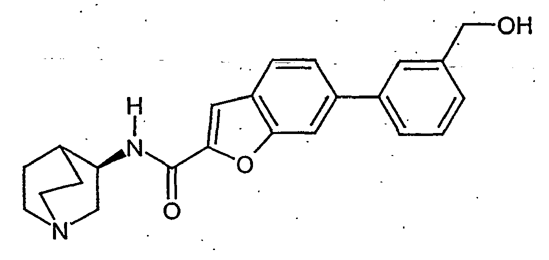

Eine Mischung aus 130 mg (0.86 mmol) 3-(Hydroxymethyl)phenylboronsäure, 200 mg (0.57 mmol) *N*-[(3R)-1-Azabicyclo[2.2.2]oct-3-yl]-6-brom-1-benzofuran-2-carboxamid (Beispiel 2A), 1.72 mL (1.72 mmol) 1 N Natronlauge, 40 mg (0.06 mmol) 1,1'-Bis(diphenylphosphino)ferrocenpalladium(II)chlorid und 2 mL DMF wird 18 h auf 80-85°C erhitzt. Das Solvens wird unter reduziertem Druck entfernt. Das Rohprodukt wird über Kieselgel 60 (Merck, Darmstadt; Eluent: Dichlormethan, Dichlormethan-Methanol 20:1, Dichlormethan-Methanol-Ammoniak 80:20:2) gereinigt. Das Solvens wird unter reduziertem Druck entfernt. Schließlich werden letzte Lösungsmittelreste im Hochvakuum entfernt. Es werden 127 mg (54 % d.Th.) der Titelverbindung isoliert.
¹H-NMR (200 MHz, CDCl₃): δ = 7.86 (d, 1H), 7.72-7.28 (m, 7H), 6.77 (d, 1H), 4.62 (s, 2H), 4.28-4.12 (m, 1H), 3.56-3.38 (m, 1H), 3.04-2.78 (m, 4H), 2.75-2.59 (m, 1H), 2.16-2.02 (m, 1H), 1.93-1.66 (m, 3H), 1.66-1.45 (m, 1H).
HPLC (Methode 1): Rₜ = 3.5 min.
LC-MS (Methode 2): Rₜ = 1.50 min.
MS (ESIpos): m/z = 377 (M+H)⁺.

### Beispiel 3

### N-[(3R)-1-Azabicyclo[2.2.2]oct-3-yl]-6-[4-(hydroxymethyl)phenyl]-1-benzofuran-2-carboxamid

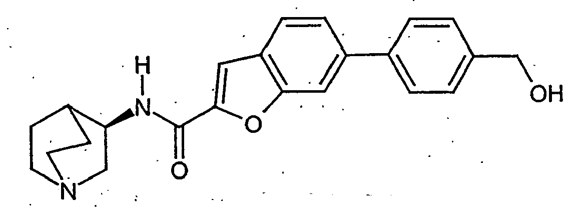

Eine Mischung aus 130 mg (0.86 mmol) 4-(Hydroxymethyl)phenylboronsäure, 200 mg (0.57 mmol) *N*-[(3R)-1-Azabicyclo[2.2.2]oct-3-yl]-6-brom-1-benzofuran-2-carboxamid (Beispiel 2A), 1.72 mL (1.72 mmol) 1 N Natronlauge, 40 mg (0.06 mmol) 1,1'-Bis(diphenylphosphino)ferrocenpalladium(II)chlorid und 2 mL DMF wird 18 h auf 80-85°C erhitzt. Das Solvens wird unter reduziertem Druck entfernt. Das Rohprodukt wird über Kieselgel 60 (Merck, Darmstadt; Eluent: Dichlormethan, Dichlormethan-Methanol 20:1, Dichlormethan-Methanol-Ammoniak 80:20:2) gereinigt. Das Solvens wird unter reduziertem Druck entfernt. Schließlich werden letzte Lösungsmittelreste im Hochvakuum entfernt. Es werden 55 mg (23 % d.Th.) der Titelverbindung isoliert.
¹H-NMR (200 MHz, CDCl₃): δ = 7.83 (d, 1H), 7.69-7.28 (m, 7H), 6.77 (d, 1H), 4.62 (s, 2H), 4.28-4.12 (m, 1H), 3.56-3.38 (m, 1H), 3.04-2.78 (m, 4H), 2.75-2.59 (m, 1H), 2.16-2.02 (m, 1H), 1.93-1.66 (m, 3H), 1.66-1.45 (m, 1H).
HPLC (Methode 1): Rₜ = 3.5 min.
LC-MS (Methode 2): Rₜ =1.46 min.
MS (ESIpos): m/z = 377 (M+H)⁺.

### Beispiel 4

### N-[(3R)-1-Azabicyclo[2.2.2]oct-3-yl]-6-[4-(4-morpholinyl)-phenyl]-1-benzofuran-2-carboxamid

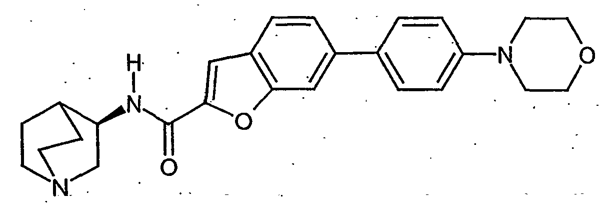

Eine Mischung aus 180 mg (0.86 mmol) 4-(4-Morpholinyl)-phenylboronsäure, 200 mg (0.57 mmol) *N*-[(3R)-1-Azabicyclo[2.2.2]oct-3-yl]-6-brom-1-benzofuran-2-carboxamid (Beispiel 2A), 1.72 mL (1.72 mmol) 1 N Natronlauge, 40 mg (0.06 mmol) 1,1'-Bis(diphenylphosphino)ferrocenpalladium(II)chlorid und 2 mL DMF wird 18 h auf 80-85°C erhitzt. Das Solvens wird unter reduziertem Druck entfernt. Das Rohprodukt wird über Kieselgel 60 (Merck, Darmstadt; Eluent: Dichlormethan, Dichlormethan-Methanol 20:1, Dichlormethan-Methanol-Ammoniak 80:20:2) gereinigt. Das Solvens wird unter reduziertem Druck entfernt. Schließlich werden letzte Lösungsmittelreste im Hochvakuum entfernt. Es werden 79 mg (32 % d.Th.) der Titelverbindung isoliert.
¹H-NMR (200 MHz, CDCl₃): δ = 7.84-7.29 (m, 7H), 6.99 (d, 1H), 6.84-6.70 (m, 1H), 4.28-4.13 (m, 1H), 3.97-3.83 (m, 2H), 3.59-3.36 (m, 1H), 3.29-3.13 (m, 2H), 3.04-2.78 (m, 4H), 2.75-2.59 (m, 1H), 2.16-2.02 (m, 1H), 1.93-1.66 (m, 3H), 1.66-1.45 (m, 1H).
HPLC (Methode 1): Rₜ = 3.5 min.
LC-MS (Methode 2): Rₜ =1.74 min.
MS (ESIpos): m/z = 432 (M+H)⁺.

### Beispiel 5

### N-[(3R)-1-Azabicyclo[2.2.2]oct-3-yl]-6-[4-(methoxy)-phenyl]-1-benzofuran-2-carboxamid

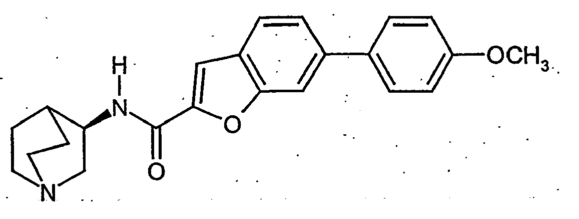

Eine Mischung aus 130 mg (0.86 mmol) 4-Methoxyphenylboronsäure, 200 mg (0.57 mmol) *N*-[(3R)-1-Azabicyclo[2.2.2]oct-3-yl]-6-brom-1-benzofuran-2-carboxamid (Beispiel 2A), 1.72 mL (1.72 mmol) 1 N Natronlauge, 40 mg (0.06 mmol) 1,1'-Bis(diphenylphosphino)ferrocenpalladium(II)chlorid und 2 mL DMF wird 18 h auf 80-85°C erhitzt. Das Solvens wird unter reduziertem Druck entfernt. Das Rohprodukt wird über Kieselgel 60 (Merck, Darmstadt; Eluent: Dichlormethan, Dichlormethan-Methanol 20:1, Dichlormethan-Methanol-Ammoniak 80:20:2) gereinigt. Das Solvens wird unter reduziertem Druck entfernt. Schließlich werden letzte Lösungsmittelreste im Hochvakuum entfernt. Es werden 160 mg (68 % d.Th.) der Titelverbindung isoliert.
¹H-NMR (200 MHz, CDCl₃): δ = 7.84-7.75 (m, 1H), 7.62-7.45 (m, 5H), 6.99 (m, 2H), 6.84-6.70 (m, 1H), 4.28-4.13 (m, 1H), 3.87 (s, 3H), 3.59-3.36 (m, 1H), 3.04-2.78 (m, 4H), 2.75-2.59 (m, 1H), 2.16-2.02 (m, 1H), 1.93-1.66 (m, 3H), 1.66-1.45 (m, 1H).
HPLC (Methode 1): Rₜ = 4.0 min.
LC-MS (Methode 3): Rₜ = 3.2 min.
MS (ESIpos): m/z = 377 (M+R)⁺.

### Beispiel 6

### N-[(3R)-1-Azabicyclo[2.2.2]oct-3-yl]-6-[3-(methoxy)-phenyl]-1-benzofuran-2-carboxamid

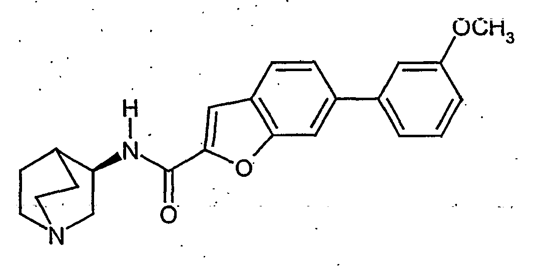

Ein Gemisch aus 130 mg (0.86 mmol) 3-Methoxyphenylboronsäure, 200 mg (0.57 mmol) *N*-[(3R)-1-Azabicyclo[2.2.2]oct-3-yl]-6-brom-1-benzofuran-2-carboxamid (Beispiel 2A), 1.72 mL (1.72 mmol) 1 N Natronlauge, 40 mg (0.06 mmol) 1,1'-Bis(diphenylphosphino)ferrocenpalladium(II)chlorid und 2 mL DMF wird 18 h auf 80-85°C erhitzt. Das Solvens wird unter reduziertem Druck entfernt. Das Rohprodukt wird über Kieselgel 60 (Merck, Darmstadt; Eluent: Dichlormethan, Dichlormethan-Methanol 20:1, Dichlormethan-Methanol-Ammoniak 80:20:2) gereinigt. Das Solvens wird unter reduziertem Druck entfernt. Schließlich werden letzte Lösungsmittelreste im Hochvakuum entfernt. Es werden 151 mg (64 % d.Th.) der Titelverbindung isoliert.
¹H-NMR (200 MHz, CDCl₃): δ = 7.90-7.80 (m, 1H), 7.72-7.08 (m, 5H), 6.95-6.85 (m, 1H), 6.84-6.70 (m, 1H), 4.28-4.13 (m, 1H), 3.87 (s, 3H), 3.59-3.36 (m, 1H), 3.04-2.78 (m, 4H), 2.75-2.59 (m, 1H), 2.16-2.02 (m, 1H), 1.93-1.66 (m, 3H), 1.66-1.45 (m, 1H).
HPLC (Methode 1): Rₜ = 4.0 min.
LC-MS (Methode 2): Rₜ = 1.87 min.
MS (ESIpos): m/z = 377 (M+H)⁺.

### Beispiel 7

### N-[(3R)-1-Azabicyclo[2.2.2]oct-3-yl]-6-(4-fluorphenyl)-1-benzofuran-2-carboxamid

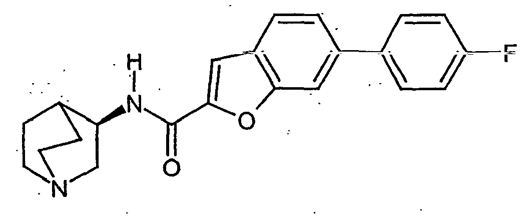

Eine Mischung aus 120 mg (0.86 mmol) 4-Fluorphenylboronsäure, 200 mg (0.57 mmol) *N*-[(3R)-1-Azabicyclo[2,2,2]oct-3-yl]-6-brom-1-benzofuran-2-carboxamid (Beispiel 2A), 1.72 mL (1.72 mmol) 1 N Natronlauge, 40 mg (0.06 mmol) 1,1'-Bis(diphenylphosphino)ferrocenpalladium(II)chlorid und 2 mL DMF wird 18 h auf 80-85°C erhitzt. Das Solvens wird unter reduziertem Druck entfernt. Das Rohprodukt wird über Kieselgel 60 (Merck, Darmstadt; Eluent: Dichlormethan, Dichlormethan-Methanol 20:1, Dichlormethan-Methanol-Ammoniak 80:20:2) gereinigt. Das Solvens wird unter reduziertem Druck entfernt. Schließlich werden letzte Lösungsmittelreste im Hochvakuum entfernt. Es werden 155 mg (68 % d.Th.) der Titelverbindung isoliert.
¹H-NMR (200 MHz, CDCl₃): δ = 7.99 (s, 1H), 7.64-7.46 (m, 5H), 7.22-7.07 (m, 2H), 6.84-6.70 (m, 1H), 4.28-4.13 (m, 1H), 3.59-3.36 (m, 1H), 3.04-2.78 (m, 4H), 2.75-2.59 (m, 1H), 2.16-2.02 (m, 1H), 1.93-1.66 (m, 3H), 1.66-1.45 (m, 1H).
HPLC (Methode 1): Rₜ = 4.1 min.
LC-MS (Methode 2): Rₜ =1.92 min.
MS (ESIpos): m/z = 365 (M+H)⁺.

### Beispiel 8

### N-[(3R)-1-Azabicyclo[2.2.2]oct-3-yl]-6-(4-trifluonnethoxyphenyl)-1-benzofuran-2-carboxamid

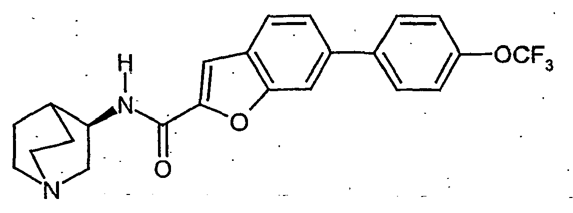

Eine Mischung aus 180 mg (0.86 mmol) 4-(Trifluormethoxy)phenylboronsäure, 200 mg (0.57 mmol) *N*-[(3R)-1-Azabicyclo[2.2.2]oct-3-yl]-6-brom-1-benzofuran-2-carboxamid (Beispiel 2A), 1.72 mL (1.72 mmol) 1 N Natronlauge, 40 mg (0.06 mmol) 1,1'-Bis(diphenylphosphino)ferrocenpalladium(II)chlorid und 2 mL DMF wird 18 h auf 80-85°C erhitzt. Das Solvens wird unter reduziertem Druck entfernt. Das Rohprodukt wird über Kieselgel 60 (Merck, Darmstadt; Eluent: Dichlormethan, Dichlormethan-Methanol 20:1, Dichlormethan-Methanol-Ammoniak 80:20:2) gereinigt. Das Solvens wird unter reduziertem Druck entfernt. Schließlich werden letzte Lösungsmittelreste im Hochvakuum entfernt. Es werden 155 mg (68 % d.Th.) der Titelverbindung isoliert.
¹H-NMR (200 MHz, CDCl₃): δ = 7.82 (s, 1H), 7.72-7.45 (m, 5H), 7.36-7.27 (m, 2H), 6.84-6.70 (m, 1H), 4.28-4.13 (m, 1H), 3.59-3.36 (m, 1H), 3.04-2.78 (m, 4H), 2.75-2.59 (m, 1H), 2.16-2.02 (m, 1H), 1.93-1.66 (m, 3H), 1.66-1.45 (m, 1H).
HPLC (Methode 1): Rₜ = 4.4 min.
LC-MS (Methode 2): Rₜ = 2.22 min.
MS (ESTpos): m/z = 431 (M+H)⁺.

### Beispiel 9

### N-[(3R)-1-Azabicyclo[2.2.2]oct-3-yl]-6-(3-hydroxy-1-propinyl)-1-benzofuran-2-carboxamid

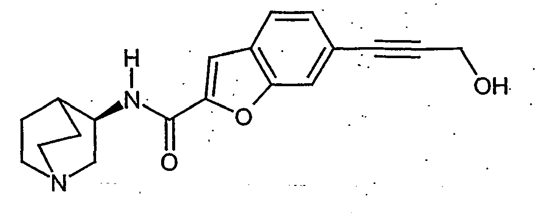

Eine Mischung aus 289 mg (5.15 mmol) Propargylalkohol, 150 mg (0.43 mmol) *N-*[(3R)-1-Azabicyclo[2.2.2]oct-3-yl]-6-brom-1-benzofuran-2-carboxamid (Beispiel 2A), 1.6 mg (0.01 mmol) Kupfer(I)iodid, 15 mg (0.02 mmol) Bis(triphenylphosphin)palladium(II)chlorid, 61 mg (0.86 mmol) Pyrrolidin und 1 mL THF wird über Nacht unter Rückfluss erhitzt. Das Rohprodukt wird mit 10 mL 1 N Natronlauge versetzt und dreimal mit insgesamt 100 mL Essigsäureethylester extrahiert. Die vereinigten organischen Phasen werden über Magnesiumsulfat getrocknet und das Solvens unter reduziertem Druck entfernt. Das Rohprodukt wird über Kieselgel 60 (Merck, Darmstadt; Eluent: Dichlormethan-Triethylamin 100:1, dann Dichlormethan-Methanol-Triethylamin 100:1:1 bis Dichlormethan-Methanol-Triethylamin-100:10:1) gereinigt. Das Solvens wird unter reduziertem Druck entfernt. Schließlich werden letzte Lösungsmittelreste im Hochvakuum entfernt. Es werden 40 mg (27 % d.Th.) der Titelverbindung isoliert.
HPLC (Methode 1): Rₜ = 3.3 min.
LC-MS (Methode 3): Rₜ = 2.6 min.
MS (ESIpos): m/z= 325 (M+H)⁺.

### Beispiel 10

### N-[(3R)-1-Azabicyclo[2.2.2]oct-3-yl]-5-fluor-7-(4-fluorphenyl)-1-benzofuran-2-carboxamid

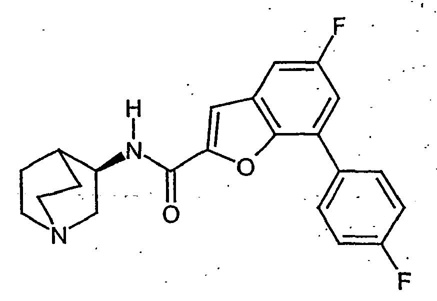

Ein Gemisch aus 40 mg (0.29 mmol) 4-Fluorphenylboronsäure, 70 mg (0.19 mmol) *N*-[(3R)-1-Azabicyclo[2.2.2]oct-3-yl]-5-fluor-7-brom-1-benzofuran-2-carboxamid (Beispiel 5A), 0.57 mL (0.57 mmol) 1 N Natronlauge, 14 mg (0.02 mmol) 1,1'-Bis(diphenylphosphino)ferrocenpalladium(II)chlorid und 2 mL DMF wird über Nacht auf 85°C erhitzt. Das Solvens wird unter reduziertem Druck entfernt. Das Rohprodukt wird mit 1 N Natronlauge versetzt und dreimal mit insgesamt 100 mL Essigsäureethylester extrahiert. Die vereinigten organischen Phasen werden über Magnesiumsulfat getrocknet und das Solvens unter reduziertem Druck entfernt. Das Rohprodukt wird in Methanol aufgenommen und zusammen mit saurem Ionenaustauscher (Dowex^{®} WX2-200) etwa 20 min. lang geschüttelt. Der beladene Ionenaustauscher wird dreimal mit je 30 mL Methanol, dann mit Wasser, wieder mit Methanol, mit Dichlormethan, wieder mit Methanol, mit THF und schließlich nochmals mit Methanol gewaschen. Das Produkt wird mit Methanol-Triethylamin 95:5 eluiert. Das Solvens wird unter reduziertem Druck am Rotationsverdampfer entfernt. Zur Feinreinigung wird über Kieselgel 60 (Merck, Darmstadt; Eluent: Dichlormethan-Triethylamin 100:1, dann Dichlormethan-Methanol-Triethylamin 100:1:1 bis Dichlormethan-Methanol-Triethylamin 100:10:1) gereinigt. Das Solvens wird unter reduziertem Druck entfernt. Schließlich werden letzte Lösungsmittelreste im Hochvakuum entfernt. Es werden 51 mg (70 % d.Th.) der Titelverbindung isoliert.
¹H-NMR (400 MHz, Methanol-d₄): δ = 7.99-7.90 (m, 2H), 7.59 (s, 1H), 7.45-7.35 (m, 2H), 7.30-7.22 (m, 2H), 4.24-4.18 (m, 1H), 3.34-3.29 (m, 1H), 3.07-2.97 (m, 1H), 2.93-2.77 (m, 4H), 2.13-2.05 (m, 1H), 1.98-1.86 (m, 1H), 1.84-1.75 (m, 2H), 1.63-1.53 (m, 1H).
HPLC (Methode 1): Rₜ = 4.3 min.
LC-MS (Methode 3): Rₜ = 3.08 min.
MS (ESIpos): m/z = 383 (M+H)⁺.

### Beispiel 11

### N-[(3R)-1-Azabicyclo[2.2.2]oct-3-yl]-5-fluor-7-(4-trifluormethoxyphenyl)-1-benzofuran-2-carboxamid

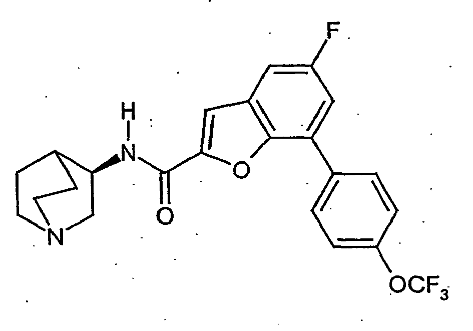

Ein Gemisch aus 40 mg (0.29 mmol) 4-(Trifluormethoxy)phenylboronsäure, 70 mg (0.19 mmol) *N*-[(3R)-1-Azabicyclo[2.2.2]oct-3-yl]-5-fluor-7-brom-1-benzofuran-2-carboxamid (Beispiel 5A), 0.57 mL (0.57 mmol) 1 N Natronlauge, 14 mg (0.02 mmol) 1,1'-Bis(diphenylphosphino)ferrocenpalladium(II)chlorid und' 2 niL DMF wird über Nacht auf 85°C erhitzt. Das Solvens wird unter reduziertem Druck entfernt. Das Rohprodukt wird mit 1 N Natronlauge versetzt und dreimal mit insgesamt 100 mL Essigsäureethylester extrahiert. Die vereinigten organischen Phasen werden über Magnesiumsulfat getrocknet und das Solvens unter reduziertem Druck entfernt. Das Rohprodukt wird in Methanol aufgenommen und zusammen mit saurem Ionenaustauscher (Dowex^{®} WX2-200) etwa 20 min. lang geschüttelt. Der beladene Ionenaustauscher wird dreimal mit je 30 mL Methanol, dann mit Wasser, wieder mit Methanol, mit Dichlormethan, wieder mit Methanol, mit THF und schließlich nochmals mit Methanol gewaschen. Das Produkt wird mit Methanol-Triethylamin 95:5 eluiert. Das Solvens wird unter reduziertem Druck am Rotationsverdampfer entfernt. Zur Feinreinigung wird über Kieselgel 60 (Merck, Darmstadt; Eluent: Dichlormethan-Triethylamin 100:1, dann Dichlormethan-Methanol-Triethylamin 100:1:1 bis Dichlormethan-Methanol-Triethylamin 100:10:1) gereinigt. Das Solvens wird unter reduziertem Druck entfernt. Schließlich werden letzte Lösungsmittelreste im Hochvakuum entfernt. Es werden 52 mg (61 % d.Th.) der Titelverbindung isoliert.
¹H-NMR (400 MHz, Methanol-d₄): δ = 8.08-8.00 (m, 2H), 7.59 (s, 1H), 7.49-741 (m, 4H), 4.24-4.18 (m, 1H), 3.34-3.29 (m, 1H), 3.07-2.97 (m, 1H), 2.93-2.77 (m, 4H), 2.13-2.05 (m, 1H), 1.98-1.86 (m, 1H), 1.84-1.75 (m, 2H), 1.63-1.53 (m, 1H).
HPLC (Methode 1): Rₜ = 4.6 min.
LC-MS (Methode 3): Rₜ = 3.37 min.
MS (ESIpos): m/z = 449 (M+H)⁺.

### Beispiel 12

### N-[(3R)-1-Azabicylo[2.2.2]oct-3-yl]-5-phenyl-1-benzothiophen-2-carboxamid-Hydrochlorid

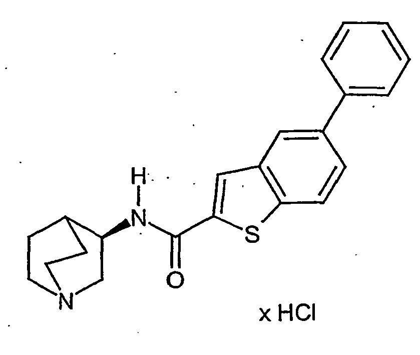

Zu einer Mischung aus 40 mg (0.10 mmol) *N*-[(3R)-1-Azabicyclo[2.2.2]oct-3-yl]-5-brom-1-benzothiophen-2-carboxamid-Hydrochlorid (Beispiel 14A) und 12.1 mg (0.10 mmol) Phenylboronsäure in 1 mL DMF werden 0.15 mL 2 M wässrige Natriumcarbonat-Lösung und 4.1 mg (0.005 mmol) PdCl₂(dppf) gegeben. Das Reaktionsgemisch wird 14 h auf 80°C erhitzt, 'über Kieselgur filtriert und zur Trockene eingeengt. Nach Reinigung des Rohprodukts mittels präparativer HPLC, anschließendem Versetzen mit 1 N Salzsäure sowie Trocknung im Hochvakuum erhält man 7.3 mg (18 % d.Th.) der Titelverbindung.
HPLC (Methode 1): Rₜ = 4.2 min.
MS (ESIpos): m/z = 363 (M+H)⁺ (freie Base).

### Beispiel 13

### N-[(3R)-1-Azabicyclo[2.2.2]oct-3-yl]-6-phenyl-1-benzothiophen-2-carboxamid-Hydrochlorid

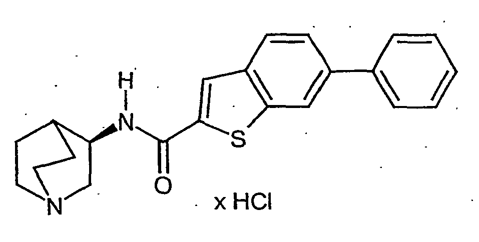

Zu einer Mischung aus 40 mg (0.10 mmol) *N*-[(3R)-1-Azabicyclo[2.2.2]oct-3-yl]-6-brom-1-benzothiophen-2-carboxamid-Hydrochlorid (Beispiel 11A) und 12.1 mg (0.10 mmol) Phenylboronsäure in 1 mL DMF werden 0.15 mL 2 M wässrige Natriumcarbonat-Lösung und 4.1 mg (0.005 mmol) PdCl₂(dppf) gegeben. Das Reaktionsgemisch wird 14 h auf 80°C erhitzt, über Kieselgur filtriert und zur Trockene eingeengt. Nach Reinigung des Rohprodukts mittels präparativer HPLC, anschließendem Versetzen mit 1 N Salzsäure sowie Trocknung im Hochvakuum erhält man 14.5 mg (37 % d.Th.) der Titelverbindung.
¹H-NMR (200 MHz, DMSO-d₆): δ = 9.91 (m, 1H), 9.02 (d, 1H), 8.38 (m, 1H), 8.32 (m, 1H), 8.06 (d, 1H), 7.78 (m, 3H), 7.58-7.37 (m, 3H), 4.32 (m, 1H), 3.78-3.03 (m, 6H), 2.28-2.05 (m, 2H), 1.93 (m, 2H), 1.78 (m, 1H).
HPLC (Methode 1): Rₜ = 4.2 min.
MS (ESIpos): m/z = 363 (M+H)⁺ (freie Base).

### Beispiel 14

### N-[(3R)-1-Azabicylo[2.2.2]oct-3-yl]-6-(3-methoxyphenyl)-1-benzothiophen-2-carboxamid-Hydrochlorid

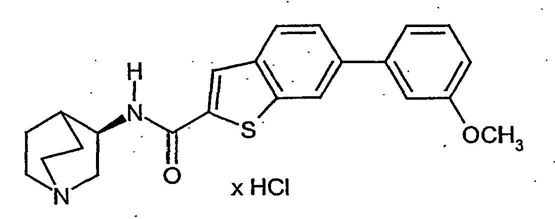

Zu einer Mischung aus 40 mg (0.10 mmol) *N*-[(3R)-1-Azabicyclo[2.2.2]oct-3-yl]-6-brom-1-benzothiophen-2-carboxamid-Hydrochlorid (Beispiel 11A) und 15.1 mg (0.10 mmol) 3-Methoxyphenylboronsäure in 1 mL DMF werden 0.3 mL 2 M wässrige Natriumcarbonat-Lösung und 4.1 mg (0.005 mmol) PdCl₂(dppf) gegeben. Das Reaktionsgemisch wird 14 h auf 80°C erhitzt, über Kieselgur filtriert und zur Trockene eingeengt. Nach Reinigung des Rohprodukts mittels präparativer HPLC, anschließendem Versetzen mit 1 N Salzsäure sowie Trocknung im Hochvakuum erhält man 25.5 mg (57 % d.Th.) der Titelverbindung.
¹H-NMR (200 MHz, DMSO-d₆): δ = 9.70 (s, 1H), 8.97 (d, 1H), 8.38 (m, 1H), 8.28 (m, 1H), 7.99 (m, 1H), 7.78 (m, 1H), 7.37 (m, 3H), 6.98 (m, 1H), 4.33 (m, 1H), 3.86 (s, 3H), 3.79-3.12 (m, 6H), 2.28-2.00 (m, 2H), 1.99-1.68 (m, 3H).
HPLC (Methode 1): Rₜ = 4.2 min.
MS (ESIpos): m/z = 393 (M+H)⁺ (freie Base).

### Beispiel 15

### N-[(3R)-1-Azabicyclo[2.2.2]oct-3-yl]-6-(3-hydroxy-1-propinyl)-1-benzothiophen-2-carboxamid

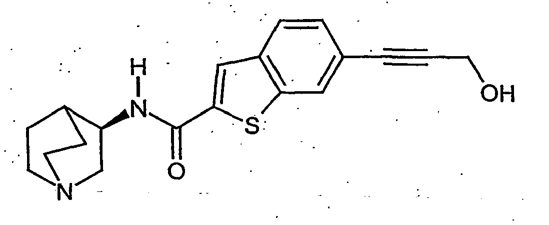

120 mg (0.30 mmol) *N*-[(3R)-1-Azabicyclo[2.2.2]oct-3-yl]-6-brom-1-benzothiophen-2-carboxamid-Hydrochlorid (Beispiel 11A), 10.5 mg (0.01 mmol) PdCl₂(PPh₃)₂ und 4.6 mg (0.02 mmol) Kupfer(I)iodid werden in 1.5 mL Triethylamin/DMF (2:1) unter Argon gelöst und für 1 h bei 60°C gerührt. Nach Zugabe von 25.1 mg (0.45 mmol) Propargylalkohol wird für weitere 16 h auf 70°C erhitzt. Nach dem Abkühlen wird über Kieselgur filtriert und mittels präparativer HPLC gereinigt, eingeengt und das Produkt im Hochvakuum getrocknet. Man erhält 12 mg (11 % d.Th.) der Titelverbindung.
¹H-NMR (200 MHz, DMSO-d₆): δ = 8.73 (d, 1H), 8.20 (m, 2H), 7.96 (d, 1H), 7.46 (dd, 1H), 4.34 (s, 2H), 4.09 (m, 1H), 3.32 (m, 1H), 3.16-2.77 (m, 5H), 1.99 (m, 1H), 1.91 (m, 1H), 1.70 (m, 2H), 1.49 (m, 1H).
HPLC (Methode 1): Rₜ = 3.4 min.
MS (ESIpos): m/z = 341 (M+H)⁺.

### Beispiel 16

### N-[(3R)-1-Azabicyclo[2.2.2]oct-3-yl]-7-phenyl-1-benzothiophen-2-carboxamid-Hydrochlorid

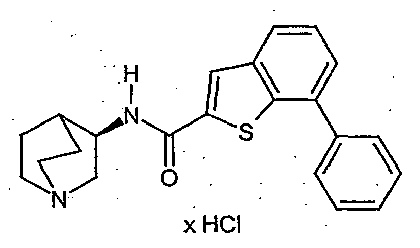

Zu einer Mischung aus 56 mg (0.14 mmol) *N*-[(3R)-1-Azabicyclo[2.2.2]oct-3-yl]-7-brom-1-benzothiophen-2-carboxamid-Hydrochlorid (Beispiel 8A) und 18.7 mg (0.15 mmol) Phenylboronsäure in 1 mL DMF werden 0.14 mL 2 M wässrige Natriumcarbonat-Lösung und 5.7 mg (0.007 mmol) PdCl₂(dppf) gegeben. Das Reaktionsgemisch wird auf 80°C erhitzt. Nach 3 h bei dieser Temperatur werden weitere 5.7 mg (0.007 mmol) PdCl₂(dppf) hinzugefügt und es wird für weitere 12 h bei 80°C gerührt. Das Reaktionsgemisch wird über Kieselgur filtriert und zur Trockene eingeengt. Nach Reinigung des Rohprodukts mittels präparativer HPLC, anschließendem Versetzen mit 1 N Salzsäure sowie Trocknung im Hochvakuum erhält man 10.6 mg (18 % d.Th.) der Titelverbindung.
HPLC (Methode 1): Rₜ = 4.2 min.
MS (ESIpos): m/z = 363 (M+H)⁺ (freie Base).

### Beispiel 17

### N-[(3R)-1-Azabicyclo[2.2.2]oct-3-yl]-7-(3-methoxyphenyl)-1-benzothiophen-2-carboxamid-Hydrochlorid

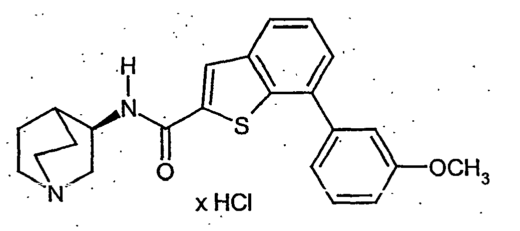

Zu einer Mischung aus 49 mg (0.1.0 mmol) *N*-[(3R)-1-Azabicyclo[2.2.2]oct-3-yl]-7-brom-1-benzothiophen-2-carboxamid-Hydrochlorid (Beispiel 8A) und 15.8 mg (0.10 mmol) 3-Methoxyphenylboronsäure in 1 mL DMF werden 0.16 mL 2 M wässrige Natriumcarbonat-Lösung und 4.2 mg (0.005 mmol) PdCl₂(dppf) gegeben. Das Reaktionsgemisch wird 14 h auf 80°C erhitzt, dann über Kieselgur filtriert und zur Trockene eingeengt. Nach Reinigung des Rohprodukts mittels präparativer HPLC, anschließendem Versetzen mit 1 N Salzsäure sowie Trocknung im Hochvakuum erhält man 8.0 mg (18 % d.Th.) der Titelverbindung.
HPLC (Methode 1): Rₜ = 4.2 min.
MS (ESIpos): m/z = 393 (M+H)⁺ (freie Base).

### Beispiel 18

### N-[(3R)-1-Azabicyclo[2.2.2]oct-3-yl]-7-(2-methoxyphenyl)-1-benzothiophen-2-carboxamid-Hydrochlorid

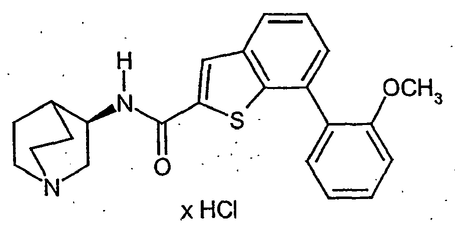

Zu einer Mischung aus 60 mg (0.15 mmol) *N*-[(3R)-1-Azabicyclo[2.2.2]oct-3-yl]-7-brom-1-benzothiophen-2-carboxamid-Hydrochlorid (Beispiel 8A) und 22.7 mg (0.15 mmol) 2-Methoxyphenylboronsäure in 1 mL DMF werden 0.22 mL 2 M wässrige Natriumcarbonat-Lösung und 6.1 mg (0.007 mmol) PdCl₂(dppf) gegeben. Das Reaktionsgemisch wird 14 h auf 80°C erhitzt, dann über Kieselgur filtriert und zur Trockene eingeengt. Nach Reinigung des Rohprodukts mittels präparativer HPLC, anschließendem Versetzen mit 1 N Salzsäure sowie Trocknung im Hochvakuum erhält man 12.8 mg (18 % d.Th.) der Titelverbindung.
HPLC (Methode 1): Rₜ = 4.2 min.
MS (ESIpos): m/z = 393 (M+H)⁺ (freie Base).

### Beispiel 19

### N-[(3R)-1-Azabicyclo[2.2.2]oct-3-yl]-7-[4-(4-morpholinyl)phenyl]-1-benzothiophen-2-carboxamid-Hydrochlorid

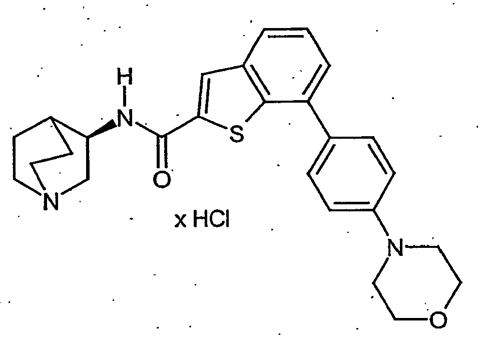

Zu einer Mischung aus 60 mg (0.15 mmol) *N*-[(3R)-1-Azabicylo[2.2.2]oct-3-yl]-7-brom-1-benzothiophen-2-carboxamid-Hydrochlorid (Beispiel 8A) und 30.9 mg (0.15 mmol) 4-(4-Morpholinyl)-phenylboronsäure in 1 mL DMF werden 0.22 mL 2 M wässrige Natriumcarbonat-Lösung und 6.1 mg (0.007 mmol) PdCl₂(dppf) gegeben. Das Reaktionsgemisch wird auf 80°C erhitzt. Nach 4.5 h werden nochmals 6.1 mg (0.007 mmol) PdCl₂(dppf) hinzugefügt. Nach weiteren 12 h wird das Reaktionsgemisch über Kieselgur filtriert und zur Trockene eingeengt. Die Reinigung des Rohprodukts erfolgt mittels präparativer HPLC. Das Produkt wird in Methanol gelöst und mit einem Überschuss an 4 N Chlorwasserstoff in Dioxan versetzt. Nach dem Trocknen im Hochvakuum erhält man 20.6 mg (25 % d.Th.) der Titelverbindung.
¹H-NMR (300 MHz, Methanol-d₄): δ = 8.21 (s, 1H), 7.97 (m, 2H), 7.93 (s, 1H), 7.83 (m, 2H), 7.57 (dd, 1H), 7.52 (m, 1H), 4.46 (m, 1H), 4.13 (m, 4H), 3.83 (m, 1H), 3.78 (m, 4H), 3.49 (m, 1H), 3.43-3.17 (m, 4H), 2.38 (m, 1H), 2.28 (m, 1H), 2.10 (m, 2H), 1.96 (m, 1H).
HPLC (Methode 1): Rₜ = 4.3 min.
MS (ESIpos): m/z = 448 (M+H)⁺ (freie Base).

### Beispiel 20

### N-[(3R)-1-Azabicyclo[2.2.2]oct-3-yl]-7-[4-hydroxymethyl)phenyl]-1-benzothiophen-2-carboxamid-Hydrochlorid

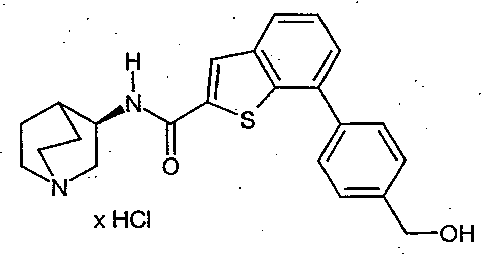

60 mg (0.15 mmol) *N-*[(3R)-1-Azabicyclo[2.2.2]oct-3-yl]-7-brom-1-benzothiophen-2-carboxamid-Hydrochlorid (Beispiel 8A) und 22.7 mg (0.15 mmol) 4-(Hydroxymethyl)phenylboronsäure werden in 1 mL DMF vorgelegt. Nach Addition von 0.22 mL 2 M wässriger Natriumcarbonat-Lösung und 6.1 mg (0.01 mmol) PdCl₂(dppf) wird auf 80°C erhitzt. Nach 14 h wird das Reaktionsgemisch über Kieselgur filtriert und zur Trockene eingeengt. Die Reinigung des Rohprodukts erfolgt mittels präparativer HPLC. Das Produkt wird in Methanol gelöst und mit einem Überschuss an 4 N Chlorwasserstoff in Dioxan versetzt. Nach dem Trocknen im Hochvakuum erhält man 9 mg (9 % d.Th.) der Titelverbindung.
HPLC (Methode 1): Rₜ = 3.8 min.
MS (ESIpos): m/z = 393 (M+H)⁺ (freie Base).
Auf analoge Weise werden die in der folgenden Tabelle aufgeführten Verbindungen erhalten:

**Tabelle 2:**

| | | | |
|---|---|---|---|
| 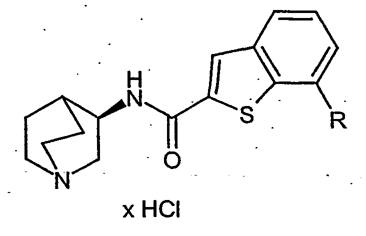 | | | |

| **Beispiel-Nr.** | **R** | **R**_{**t**} **[min.]** **(Methode 5)** | **MS (ESIpos): m/z [M+H]**^{**+**} **(freie Base)** |
|---|---|---|---|
| **21** | 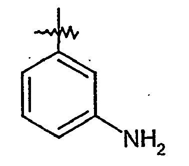 | 1.27 | 378 |
| **22** | 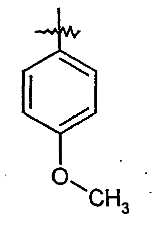 | 1.51 | 393 |
| **23** | 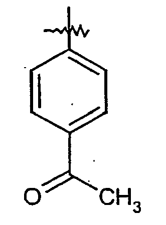 | 1.46 | 405 |
| **24** | 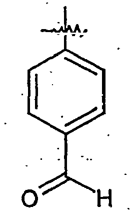 | 1.44 | 391 |
| **25** | 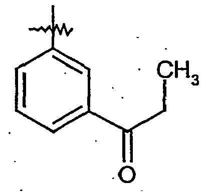 | 1.51 | 420 |
| **26** | 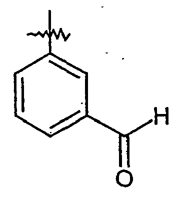 | 1.45 | 391 |
| **27** | 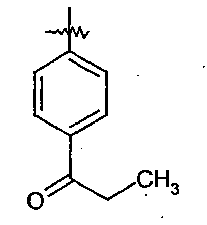 | 1.53 | 420 |
| **28** | 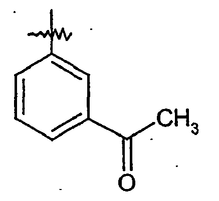 | 1.46 | 405 |
| **29** | 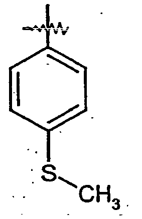 | 1.59 | 410 |
| **30** | 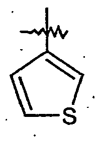 | 1.48 | 369 |
| **31** | 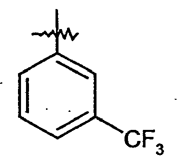 | 1.61 | 431 |
| **32** | 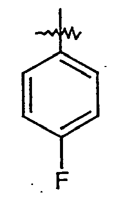 | 1.52 | 381 |
| **33** | 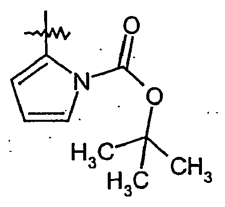 | 1.6 | 453 |
| **34** | 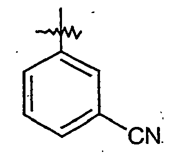 | 1.48 | 388 |
| **35** | 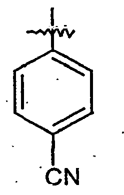 | 1.45 | 388 |
| **36** | 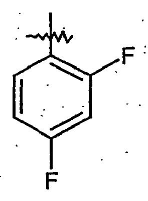 | 1.53 | 399 |
| **37** | 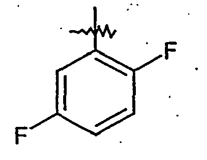 | 1.52 | 399 |
| **38** | 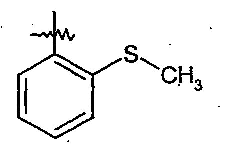 | 1.54 | 410 |
| **39** | 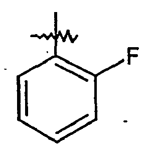 | 1.51 | 381 |
| **40** | 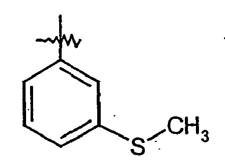 | 1.6 | 410 |
| **41** | 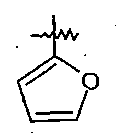 | 1.43 | 353 |
| **42** | 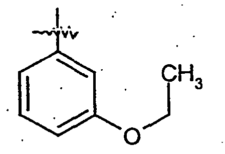 | 1.58 | 408 |
| **43** | 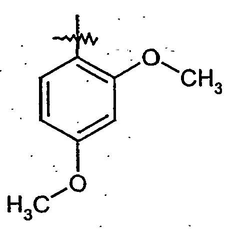 | 1.52 | 424 |
| **44** | 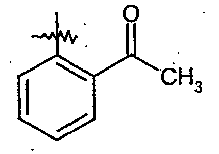 | 1.42 | 405 |
| **45** | 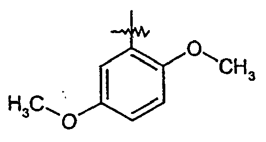 | 1.48 | 424 |
| **46** | 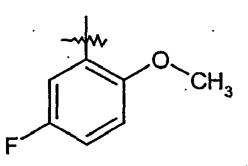 | 1.53 | 411 |
| **47** | 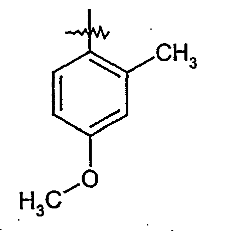 | 1.56 | 408 |
| **48** | 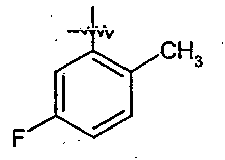 | 1.58 | 395 |
| **49** | 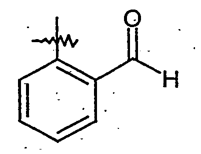 | 1.43 | 391 |
| **50** | 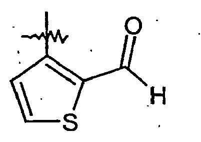 | 1.38 | 397 |

### Beispiel 51

### 7-[3-(Acetylamino)phenyl]-N-[(3R)-1-azabicyclo[2.2.2]oct-3-yl]-1-benzothiophen-2-carboxamid-Hydrochlorid

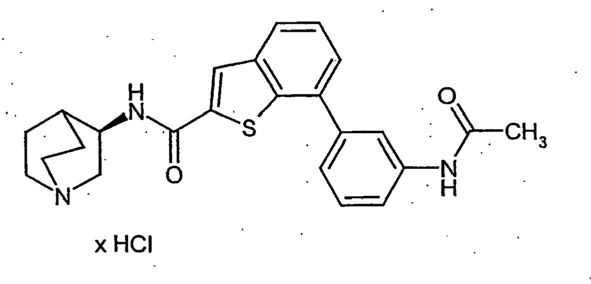

Zu einer Mischung aus 200 mg (0.50 mmol) *N*-[(3R)-1-Azabicyclo[2.2.2]oct-3-yl]-7-brom-1-benzothiophen-2-carboxamid-Hydrochlorid (Beispiel 8A) und 89.1 mg (0.50 mmol) 3-(Acetamido)phenylboronsäure in 2 mL DMF werden 0.75 mL 2 M wässrige Natriumcarbonat-Lösung und 20.3 mg (0.02 mmol) PdCl₂(dppf) gegeben. Das Reaktionsgemisch wird 17 h auf 80°C erhitzt. Es werden weitere 89.1 mg (0.50 mmol) 3-(Acetamido)phenylboronsäure, 1.5 mL 1 N Natronlauge sowie 81.3 mg (0.1 mmol) PdCl₂(dppf) zugegeben und weitere 18 h auf 80°C erhitzt. Nach Abkühlen wird über Kieselgur filtriert und mittels präparativer HPLC aufgereinigt. Das erhaltene Rohprodukt wird via Flash-Chromatographie über Kieselgel (Laufmittel: Dichlormethan/Methanol/Ammoniak 100:10:2) weiter aufgereinigt. Die Produktfraktionen werden eingeengt, in einem 5: 1-Gemisch aus Methanol und 1 N Salzsäure aufgenommen und erneut eingeengt. Nach Trocknen im Hochvakuum erhält man 243.2 mg (86.6 % d:Th.) der Titelverbindung.
¹H-NMR (300 MHz, DMSO-d₆): δ = 10.16 (s, 1H), 10.10 (br. s, 1H), 9.03 (d, 1H), 8.38 (s, 1H), 8.06 (m, 1H), 7.98 (dd, 1H), 7.65 (m, 1H), 7.59 (dd, 1H), 7.51 (m, 1H), 7.48 (m, 1H), 7.37 (m, 1H), 4.33 (m, 1H), 3.66 (m, 1H), 3.45-3.13 (m, 5H), 2.22 (m, 1H), 2.14 (m, 1H), 2.08 (s, 3H), 1.91 (m, 2H), 1.75 (m, 1H).
HPLC (Methode 1): Rₜ = 3.9 min.
MS (ESIpos): m/z = 420 (M+H)⁺ (freie Base).

### Beispiel 52

### N-[(3R)-1-Azabicyclo[2.2.2]oct-3-yl]-6-[4-(4-morpholinyl)phenyl]-1-benzothiophen-2-carboxamid-Hydrochlorid

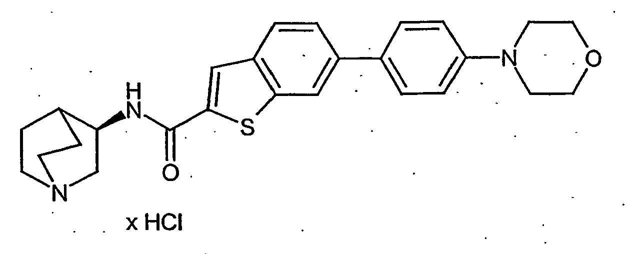

100 mg (0.25 mmol) *N*-[(3R)-1-Azabicyclo[2.2.2]oct-3-yl]-6-brom-1-benzothiophen-2-carboxamid-Hydrochlorid (Beispiel 11A) und 51.5 mg (0.25 mmol) 4-Morpholinophenylboronsäure werden in 1 inL DMF vorgelegt. Nach Addition von 0.37 mL 2 M Natriumcarbonat-Lösung und 10.2 mg (0.01 mmol) PdCl₂(dppf) wird auf 80°C erhitzt. Nach 16 h werden weitere 51.5 mg (0.25 mmol) 4-Morpholinophenylboronsäure, 0.37 mL 2 M Natriumcarbonat-Lösung sowie 10.2 mg (0.01 mmol) PdCl₂(dppf) zugegeben. Es wird für weitere 4 h auf 80°C erhitzt. Nach Abkühlen wird das Reaktionsgemisch über Kieselgur filtriert und mittels präparativer HPLC gereinigt. Die Pröduktfraktionen werden eingeengt, mit einer 5:1-Mischung aus Methanol und 4 M Chlorwasserstoff in Dioxan versetzt und erneut eingeengt. Nach Trocknen im Hochvakuum erhält man 83 mg (69 % d.Th.) der Titelverbindung.
¹H-NMR (200 MHz, DMSO-d₆): δ = 10.20 (br. s, 1H), 9.07 (d, 1H), 8.33 (s, 1H), 8.27 (s, 1H), 7.97 (d, 1H), 7.71 (m, 3H), 7.10 (m, 2H), 4.33 (m, 1H), 3.78 (m, 4H), 3.73-3.07 (m, 10H), 2.22 (m, 1H), 2.17 (m, 1H), 1.93 (m, 2H), 1.75 (m, 1H).
HPLC (Methode 1): Rₜ = 3.8 min.
MS (ESIpos): m/z= 448 (M+H)⁺ (freie Base).

### Beispiel 53

### N-[(3R)-1-Azabicylo[2.2.2]oct-3-yl]-6-(5-methyl-1,2,4-oxadiazol-3-yl)-1-benzothiophen-2-carboxamid-Hydrochlorid

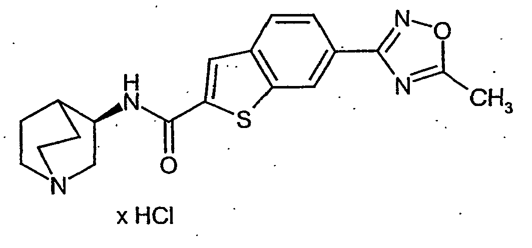

154 mg (0.37 mmol) 6-[Amino(hydroxyimino)methyl]-*N*-[(3R)-1-azabicyclo[2.2.2]-oct-3-yl]-1-benzothiophen-2-carboxamid-Dihydrochlorid (Beispiel 27A) werden in 2 mL DMF und 0.75 mL THF gelöst. Man versetzt mit 250 mg 4 Å Molekularsieb und rührt 30 min. bei Raumtemperatur. Nach der Zugabe von 44.4 mg (1.11 mmol) Natriumhydrid (60%-ige Suspension in Mineralöl) wird 20 min. auf 60°C erhitzt und anschließend auf Raumtemperatur abgekühlt. Anschließend wird eine Lösung von 90 µL (1.11 mmol) Essigsäuremethylester in 1 mL THF zum Reaktionsgemisch gegeben, dann wird für 14 h auf 80°C erhitzt. Nach Zugabe von weiteren 29.6 mg (0.74 mmol) Natriumhydrid (60%-ige Suspension in Mineralöl) und 0.88 mL (11.1 mmol) Essigsäuremethylester in 1 mL THF wird für weitere 24 h auf 70°C erhitzt, Die Reaktion wird durch Zugabe von Wasser abgebrochen. Die Reinigung erfolgt mittels präparativer HPLC. Die Produktfraktionen werden eingeengt, mit einer 5:1-Mischung aus Methanol und 4 N Chlorwasserstoff in Dioxan versetzt und erneut eingeengt. Nach Trocknen im Hochvakuum erhält man 41.9 mg (23.6 % d.Th.) der Titelverbindung.
¹H-NMR (300 MHz, DMSO-d₆): δ = 10.15 (br. s, 1H), 9.14 (d, 1H), 8.70 (m, 1H), 8.41 (s, 1H), 8.13 (d, 1H), 8.05 (m, 1H), 4.34 (m, 1H), 3.75-3.13 (m, 6H), 2.70 (s, 3H), 2.23 (m, 1H), 2.15 (m, 1H), 1.92 (m, 2H), 1.77 (m, 1H).
HPLC (Methode 1): Rₜ= 3.8 min.
MS (ESIpos): m/z = 369 (M+H)⁺ (freie Base).

### Beispiel 54

### N-[(3R)-1-Azabicyclo[2.2.2]oct-3-yl]-6-[2-(hydroxymethyl)phenyl]-1-benzothiophen-2-carboxamid-Hydrochlorid

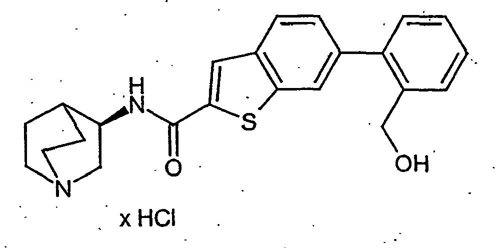

100 mg (0.25 mmol) *N*-[(3R)-1-Azabicyclo[2.2.2]oct-3-yl]-6-brom-1-benzothiophen-2-carboxamid-Hydrochlorid (Beispiel 11A) und 37.8 mg (0.25 mmol) 2-(Hydroxymethyl)phenylboronsäure werden in 1 mL DMF vorgelegt. Nach Addition von 0.37 mL 2 M Natriumcarbonat-Lösung und 10.2 mg (0.01 mmol) PdCl₂(dppf) wird auf 80°C erhitzt. Nach 14 h wird das Reaktionsgemisch über Kieselgur filtriert und durch zweimalige Trennung via präparativer HPLC aufgereinigt. Die Produktfraktionen, werden eingeengt, mit einer 5:1-Mischung aus Methanol und 4 N Chlorwasserstoff in Dioxan versetzt und erneut eingeengt. Nach Trocknen im Hochvakuum erhält man 27 mg (24.4 % d.Th.) der Titelverbindung.
¹H-NMR (200 MHz, DMSO-d₆): δ = 10.10 (br. s, 1H), 9.06 (d, 1H), 8.36 (s, 1H), 8.02 (m, 2H), 7.61 (m, 1H), 7.54-7.36 (m, 4H), 4.43 (s, 2H), 4.33 (m, 1H), 3.67 (m, 1H), 3.55-3.12 (m, 5H), 2.23 (m, 1H), 2.16 (m, 1H), 1.92 (m, 2H), 1.77 (m, 1H).
HPLC (Methode 1): Rₜ = 3.9 min.
MS (ESIpos):m/z = 393 (M+H)⁺ (freie Base).

### Beispiel 55

### N-[(3R)-1-Azabicyclo[2.2.2]oct-3-yl]-6-(5-phenyl-1,2,4-oxadiazol-3-yl)-1-benzothiophen-2-carboxamid-Hydrochlorid

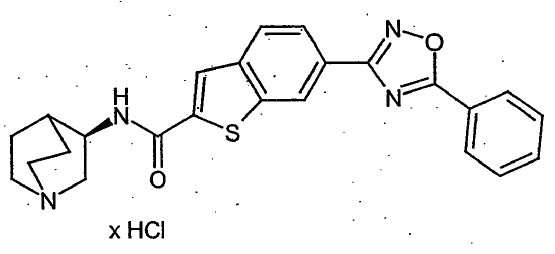

110 mg (0.26 mmol) 6-[Amino(hydroxyimino)methyl]-*N*-[(3R)-1-azabicyclo[2.2.2]-oct-3-yl]-1-benzothiophen-2-carboxamid-Dihydrochlorid (Beispiel 27A) werden in 2 mL DMF und 0.75 mL THF gelöst. Man versetzt mit 250 mg 4 Å Molekularsieb und rührt 30 min. bei Raumtemperatur. Nach der Addition von 31.2 mg (0.79 mmol) Natriumhydrid (60%-ige Suspension in Mineralöl) wird 20 min. auf 60°C erhitzt und anschließend auf Raumtemperatur abgekühlt. Eine Lösung von 100 µL (0.79 mmol) Benzoesäuremethylester in 1 mL THF wird zum Reaktionsgemisch gegeben, und es wird für 14 h auf 80°C erhitzt. Es werden weitere 20.8 mg (0.52 mmol) Natriumhydrid (60%-ige Suspension in Mineralöl) und 0.99 mL (7.91 mmol) Benzoesäuremethylester in 1 mL THF zugegeben und für weitere 24 h auf 70°C erhitzt. Die Reaktion wird durch Zugabe von Wasser abgebrochen. Die Reinigung erfolgt mittels präparativer HPLC. Die Produktfraktionen werden eingeengt, mit einer 5:1-Mischung aus Methanol und 4 N Chlorwasserstoff in Dioxan versetzt und erneut eingeengt. Nach Trocknen im Hochvakuum erhält man 45.7 mg (32 % d.Th.) der Titelverbindung.
HPLC (Methode 1): Rₜ = 4.5 min.
MS (ESIpos): m/z = 431 (M+H)⁺ (freie Base).

### Beispiel 56

### N-[(3R)-1-Azabicylo[2.2.2]oct-3-yl]-6-(5-benzyl-1,2,4-oxadiazol-3-yl)-1-benzothiophen-2-carboxamid-Hydrochlorid

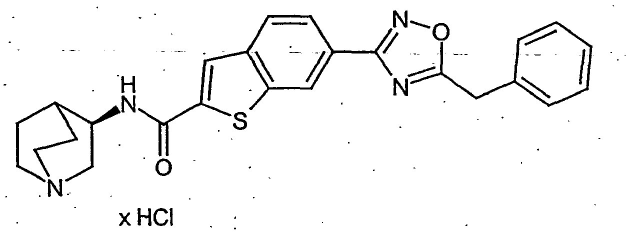

110 mg (0.26 mmol) 6-[Amino(hydroxyimino)methyl]-*N*-[(3R)-1-azabicyclo[2.2.2]-oct-3-yl]-1-benzothiophen-2-carboxamid-Dihydrochlorid (Beispiel 27A) werden in 2 mL DMF und 0.75 mL THF gelöst. Man versetzt mit 250 mg 4 Å Molekularsieb und rührt 30 min. bei Raumtemperatur. Nach Zugabe von 31.2 mg (0.79 mmol) Natriumhydrid (60%-ige Suspension in Mineralöl) wird 20 min. auf 60°C erhitzt und anschließend auf Raumtemperatur abgekühlt. Es wird eine Lösung von 110 µL (0.79 mmol) Phenylessigsäuremethylester in 1 mL THF zugegeben und für 14 h auf 80°C erhitzt. Nach Zugabe von weiteren 20.8 mg (0.52 mmol) Natriumhydrid (60%-ige Suspension in Mineralöl) und 1.14 mL (7.91 mmol) Phenylessigsäuremethylester in 1 mL THF wird für weitere 24 h auf 70°C erhitzt. Die Reaktion wird durch Addition von Wasser abgebrochen. Die Reinigung erfolgt mittels präparativer HPLC. Die Produktfraktionen werden eingeengt, mit einer 5:1-Mischung aus Methanol und 4 N Chlorwasserstoff in Dioxan versetzt und erneut eingeengt. Nach Trocknen im Hochvakuum erhält man 4.1 mg (3 % d.Th.) der Titelverbindung.
¹H-NMR (300 MHz, Methanol-d₄): δ = 8.63 (s, 1H), 8.11 (m, 2H), 8.04 (d, 1H), 7.43-7.27 (m, 5H), 4.47 (m, 1H), 4.38 (s, 2H), 3.87 (m, 1H), 3.52-3.20 (m, 5H), 2.40 (m, 1H), 2.28 (m, 1H), 2.11 (m, 2H), 1.97 (m, 1H).
HPLC (Methode 1): Rₜ = 4.4 min.
MS (ESIpos): m/z = 445 (M+H)⁺ (freie Base).

### Beispiel 157

### N-[(3R)-1-Azabicyclo[2.2.2]oct-3-yl]-6-[4-(4-morpholinylmethyl)phenyl]-1-benzothiophen-2-carboxamid-Dihydrochlorid

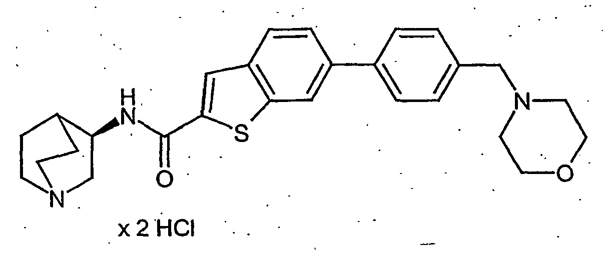

Zu einer Lösung von 80 mg (0.19 mmol) *N*-[(3R)-1-Azabicyclo[2.2.2]oct-3-yl]-6-(4-formylphenyl)-1-benzothiophen-2-carboxamid-Hydrochlorid (Beispiel 23 A) in 1.5 mL einer 6:1-Mischung aus Methanol und Essigsäure werden sukzessive 330 mg (3.75 mmol) Morpholin und 40 mg (0.56 mmol) Natriumcyanoborhydrid zugegeben. Nach 2 h bei Raumtemperatur und 6 h bei 80°C wird mittels präparativer HPLC aufgereinigt. Die Produktfraktionen werden eingeengt, mit einer 5:1-Mischung aus Methanol und 1 N Salzsäure versetzt und erneut eingeengt. Nach Trocknen im Hochvakuum erhält man 87.7 mg (88 % d.Th.) der Titelverbindung.
¹H-NMR (400 MHz, DMSO-d₆): δ = 11.26 (br. s, 1H), 10.29 (br. s, 1H), 9.18 (d, 1H), 8.41 (m, 2H), 8.06 (d, 1H), 7.89 (m, 2H), 7.81 (d, 1H), 7.74 (m, 2H), 4.39 (m, 2H), 4.34 (m, 1H), 4.05-3.03 (m, 6H), 2.23 (m, 1H), 2.16 (m, 1H), 1.92 (m, 2H), 1.76 (m, 1H).
HPLC (Methode 1): Rₜ = 3.5 min.
MS (ESIpos): m/z = 462 (M+H)⁺ (freie Base).

### Beispiel 58

### N-[(3R)-1-Azabicyclo[2.2.2]oct-3-yl]-7-[4-(dimethylamino)phenyl]-1-benzothiophen-2-carboxamid-Hydrochlorid

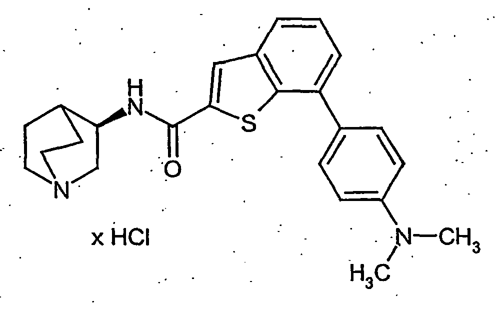

Zu einer Mischung aus 100 mg (0.22 mmol) *N*-[(3R)-1-Azabicyclo[2.2.2]oct-3-yl]-7-brom-1-benzothiophen-2-carboxamid-Hydrochlorid (Beispiel 8A) und 36.8 mg (0.22 mmol) 4-(Dimethylamino)phenylboronsäure in 1 mL DMF werden 0.33 mL 2 M Natriumcarbonat-Lösung und 9.1 mg (0.01 mmol) PdCl₂(dppf) gegeben. Das Reaktionsgemisch wird 16 h auf 80°C erhitzt. Es werden weitere 36.8 mg (0.22 mmol) 4-(Dimethylamino)phenylboronsäure, 36.5 mg (0.04 mmol) PdCl₂(dppf) sowie 0.67 mL 1 N Natronlauge zugegeben und weitere 3 h auf 80°C erhitzt. Nach Abkühlen wird über Kieselgur filtriert und mittels präparativer HPLC aufgereinigt. Die Produktfraktionen werden eingeengt, in einem 5:1-Gemisch aus Methanol und 1 N Salzsäure aufgenommen und erneut eingeengt. Nach Trocknen im Hochvakuum erhält man 50.6 mg (47 % d.Th.) der Titelverbindung.
¹H-NMR (300 MHz, Methanol-d₄): δ = 8.26 (s, 1H), 7.95 (m, 3H), 7.80 (m, 2H), 7.58 (dd, 1H), 7.53 (m, 1H), 4.46 (m, 1H), 3.82 (m, 1H), 3.51 (m, 1H), 3.45-3.16 (m, 10H), 2.37 (m, 1H), 2.29 (m, 1H), 2.10 (m, 2H), 1.95 (m, 1H).
HPLC (Methode 1): Rₜ = 3.6 min.
MS (ESIpos): m/z = 406 (M+H)⁺ (freie Base).

### Beispiel 59

### N-[(3R)-1-Azabicyclo[2.2.2]oct-3-yl]-7-(2-thienyl)-1-benzothiophen-2-carboxamid-Formiat

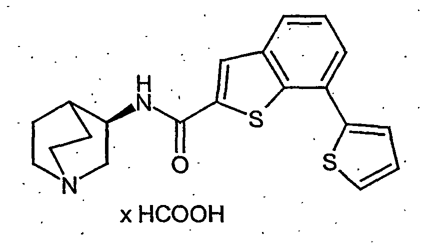

100 mg (0.25 mmol) *N*-[(3R)-1-Azabicyclo[2.2.2]oct-3-yl]-7-brom-1-benzothiophen-2-carboxamid-Hydrochlorid (Beispiel 8A) und 31.9 mg (0.25 mmol) 2-Thiophenboronsäure werden in 1.5 mL DMF vorgelegt. Nach Addition von 0.37 mL 2 M Natriumcarbonat-Lösung und 9.11 mg (0.01 mmol) PdCl₂(dppf) wird auf 85°C erhitzt. Nach 14 h wird das Reaktionsgemisch über Kieselgur filtriert und mittels präparativer HPLC aufgereinigt (Eluent A: Acetonitril, Eluent B: Wasser + 0.1%. Ameisensäure; Gradient: 10% A → 95% A). Die Produktfraktionen werden eingeengt und im Hochvakuum getrocknet. Man erhält 30 mg (28 % d.Th.) der Titelverbindung.
¹H-NMR (200 MHz, DMSO-d₆): δ = 8.80 (d, 1H), 8.33 (s, 1H), 8.23 (s, 1H), 7.97 (m, 1H); 7.72 (m, 3H), 7.55 (dd, 1H), 7.28 (dd, 1H), 4.12 (m, 1H), 3.36 (m, 1H), 3.18-2.80 (m, 5H), 2.03 (m, 1H), 1.95 (m, 1H), 1.74 (m, 2H), 1.52 (m, 1H).
HPLC (Methode 1): Rₜ = 4.2 min.
MS (ESIpos): m/z = 369 (M+H)⁺ (freie Base).

### Beispiel 60

### 7-(5-Acetyl-2-thienyl)-N-[(3R)-1-azabicyclo[2.2.2]oct-3-yl]-1-benzothiophen-2-carboxamid-Hydrochlorid

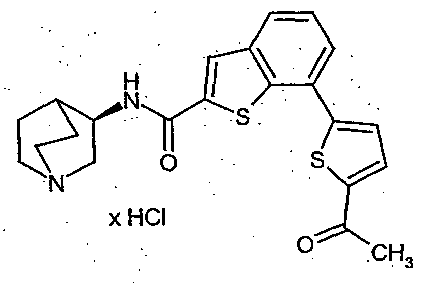

100 mg (0.25 mmol) *N*-[(3R)-1-Azabicydo[2.2.2]oct-3-yl]-7-brom-1-benzothiophen-2-carboxamid-Hydrochlorid (Beispiel 8A) und 42.3 mg (0.25 mmol) 5-Acetyl-2-thienylboronsäure werden in 1.5 mL DMF vorgelegt. Nach Addition von 0.37 mL 2 M Natriumcarbonat-Lösung und 9.11 mg (0.01 mmol) PdCl₂(dppf) wird auf 85°C erhitzt. Nach 14 h wird das Reaktionsgemisch über Kieselgur filtriert und mittels präparativer HPLC aufgereinigt. Die Produktfraktionen werden eingeengt, in einem 5:1-Gemisch aus Methanol und 1 N Salzsäure aufgenommen, erneut eingeengt und im Hochvakuum getrocknet. Man erhält 52 mg (46 % d.Th.) der Titelverbindung.
¹H-NMR (200 MHz, DMSO-d₆): δ = 10.25 (br. s, 1H), 9.23 (d, 1H), 8.49 (s, 1H), 8.08 (d, 1H), 8.06 (m, 1H), 7.89 (m, 1H), 7.82 (d, 1H), 7.61 (dd, 1H), 4.36 (m, 1H), 3.73-3.13 (m, 5H), 2.60 (s, 3H), 2.23 (m, 1H), 2.15 (m, 1H), 1.93 (m, 2H), 1.76 (m, 1H).
HPLC (Methode 1): Rₜ= 4.1 min.
MS (ESIpos): m/z = 411 (M+H)⁺ (freie Base).

### Beispiel 61

### N-[(3R)-1-Azabicyclo[2.2.2]oct-3-yl]-7-[4-(6-oxo-2-piperidinyl)phenyl]-1-benzothiophen-2-carboxamid-Hydrochlorid

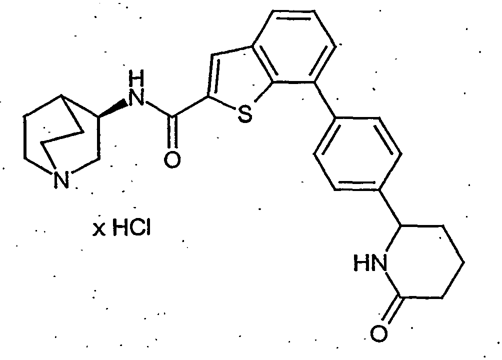

Gemäß der allgemeinen Arbeitsvorschrift D werden 123.4 mg (0.49 mmol) 6-(4-Bromphenyl)-2-piperidinon, 142.2 mg (0.56 mmol) Bis(pinacolato)dibor, 146.6 mg (1.49 mmol) Kaliumacetat, 13.7 mg (0.02 mmol) PdCl₂(dppf), 150 mg (0.37 mmol) *N*-[(3R)-1-Azabicyclo[2.2.2]oct-3-yl]-7-brom-1-benzothiophen-2-carboxamid-Hydrochlorid (Beispiel 8A), 0.93 mL 2 M Natriumcarbonat-Lösung und weitere 13.7 mg (0.02 mmol) PdCl₂(dppf) in 2 mL DMF umgesetzt. Nach Trocknen im Hochvakuum werden 7.3 mg (4 % d.Th.) der Titelverbindung erhalten.
HPLC (Methode 1): Rₜ = 3.9 min.
MS (ESIpos): m/z = 460 (M+H)⁺ (freie Base).

### Beispiel 62

### N-[(3R)-1-Azabicyclo[2.2.2]oct-3-yl]-7-[3-(hydroxymethyl)phenyl]-1-benzothiophen-2-carboxamid-Hydrochlorid

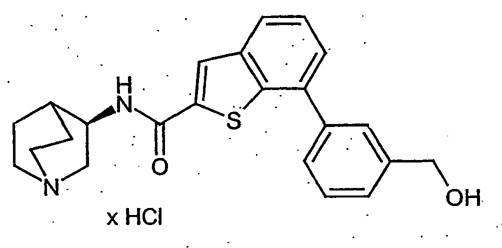

200 mg (0.45 mmol) *N*-[(3R)-1-Azabicyclo[2.2.2]oct-3-yl]-7-brom-1-benzothiophen-2-carboxamid-Hydrochlorid (Beispiel 8A) und 74.6 mg (0.49 mmol) 3-(Hydroxymethyl)phenylboronsäure werden in 3 mL DMF vorgelegt. Nach Addition von 0.67 mL 2 M Natriumcarbonat-Lösung und 18.2 mg (0.02 mmol) PdCl₂(dppf) wird auf 80°C erhitzt. Nach 14 h wird das Reaktionsgemisch über Kieselgur filtriert und durch Trennung mittels präparativer HPLC aufgereinigt. Die Produktfraktionen werden eingeengt, mit einer 5:1-Mischung aus Methanol und 4 N Chlorwasserstoff in Dioxan versetzt und erneut eingeengt. Nach Trocknen im Hochvakuum erhält man 40 mg (19% d.Th.) der Titelverbindung.
¹H-NMR (300 MHz, DMSO-d₆): δ = 10.47 (br. s, 1H), 9.17 (d, 1H), 8.47 (s, 1H), 7.96 (d, 1H), 7.67 (s, 1H), 7.63-7.47 (m, 4H), 7.43 (d, 1H), 4.61 (s, 2H), 4.33 (m, 1H), 3.62 (m, 1H), 3.39 (m, 2H), 3.20 (m, 3H), 2.11 (m, 1H), 2.15 (m, 1H), 1.91 (m, 2H), 1.75 (m, 1H).
HPLC (Methode 1): Rₜ = 3.9 min.
MS (ESIpos): m/z = 393 (M+H)⁺ (freie Base).

### Beispiel 63

### N-[(3R)-1-Azabicyclo[2.2.2]oct-3-yl]-7-[2-(4-morpholinyl)phenyl]-1-benzothiophen-2-carboxamid-Hydrochlorid

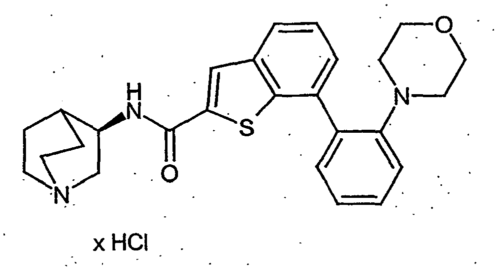

Gemäß der allgemeinen Arbeitsvorschrift D werden 100 mg (0.41 mmol) 4-(2-Bromphenyl)morpholin, 121.0 mg (0.48 mmol) Bis(pinacolato)dibor, 101.3 mg (1.03 mmol) Kaliumacetat, 11.6 mg (0.02 mmol) PdCl₂(dppf), 127.6 mg (0.32 mmol) *N*-[(3R)-1-Azabicyclo[2.2.2]oct-3-yl]-7-brom-1-benzothiophen-2-carboxamid-Hydrochlorid (Beispiel 8A), 0.79 mL 2 M Natriumcarbonat-Lösung und weitere 11.6 mg (0.02 mmol) PdCl₂(dppf) in 2 mL DMF umgesetzt. Nach Trocknen im Hochvakuum werden 38.9 mg (48 % d.Th.) der Titelverbindung erhalten.
¹H-NMR (200 MHz, DMSO-d₆): δ = 10.20 (br. s, 1H), 9.04 (d, 1H), 8.36 (s, 1H), 7.93 (dd, 1H), 7.55 (m, 2H), 7.45 (d, 1H), 7.37 (dd, 1H), 7.17 (m, 2H), 4.32 (m, 1H), 3.62 (m, 1H), 3.48-3.10 (m, 9H), 2.69 (m, 4H), 2.19 (m, 1H), 2.10 (m, 1H), 1.90 (m, 2H), 1.73 (m, 1H).
HPLC (Methode 1): Rₜ = 4.3 min.
MS (ESIpos): m/z = 448 (M+H)⁺ (freie Base).

### Beispiel 64

### 2-(2-{[(3R)-1-Azabicyclo[2.2.2]oct-3-ylamino]carbonyl}-1-benzothien-7-yl)-benzyl ethylcarbamat-Hydrochlorid

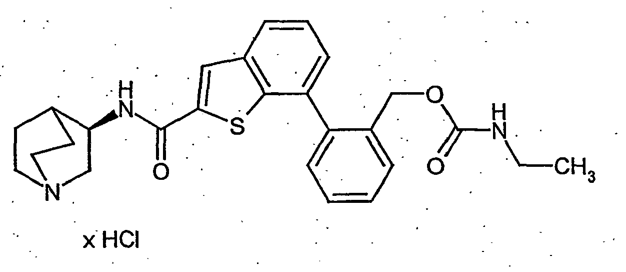

Zu einer Suspension von 50 mg (0.12 mmol) *N*-[(3R)-1-Azabicylo[2.2.2]oct-3-yl]-7-[2-(hydroxymethyl)phenyl]-1-benzothiophen-2-carboxamid-Hydrochlorid (Beispiel 131) in 1 mL einer 5:1-Mischung aus THF und DMF werden 32.5 µL (0.23 mmol) Triethylamin und 16.6 mg (0.23 mmol) Ethylisocyanat gegeben. Nach 18 h bei Raumtemperatur werden weitere 16.6 mg (0.23 mmol) Ethylisocyanat sowie eine katalytische Menge 4-*N,N*-Dimethylaminopyridin hinzugefügt. Es wird weitere 18 h bei Raumtemperatur gerührt. Die Reaktionsmischung wird im Vakuum eingeengt und mittels präparativer HPLC aufgereinigt. Die Produktfraktionen werden eingeengt, mit einer 5:1-Mischung aus Methanol und 1 N Salzsäure versetzt und erneut eingeengt. Nach Trocknen im Hochvakuum erhält man 28 mg (47 % d.Th.) der Titelverbindung.
HPLC (Methode 1): Rₜ = 4.2 min.
MS (ESIpos): m/z = 464 (M+H)⁺ (freie Base).

### Beispiel 65

### 2-(2-{[(3R)-1-Azabicyclo[2.2.2]oct-3-ylamino]carbonyl}-1-benzothien-7-yl)-benzyl methylcarbamat-Hydrochlorid

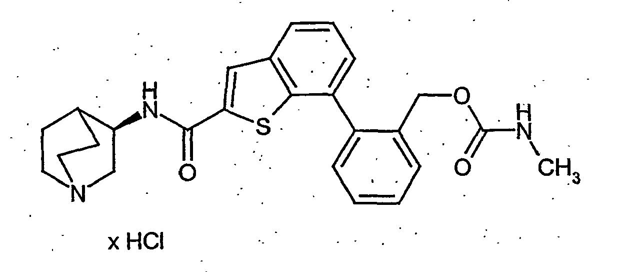

Zu einer Suspension von 50 mg (0.12 mmol) *N*-[(3R)-1-Azabicyclo[2.2.2]oct-3-yl]-7-[2-(hydroxymethyl)phenyl]-1-benzothiophen-2-carboxamid-Hydrochlorid (Beispiel 131) in 1 mL einer 5:1-Mischung aus THF und DMF werden 32.5 µL (0.23 mmol) Triethylamin und 13.3 mg (0.23 mmol) Methylisocyanat gegeben. Nach 18 h bei Raumtemperatur werden weitere 16.6 mg (0.23 mmol) Methylisocyanat sowie eine katalytische Menge 4-*N,N*-Dimethylaminopyridin hinzugefügt. Es wird weitere 18 h bei Raumtemperatur gerührt. Die Reaktionsmischung wird im Vakuum eingeengt und mittels präparativer HPLC aufgereinigt. Die Produktfraktionen werden eingeengt, mit einer 5:1-Mischung aus Methanol und 1 N Salzsäure versetzt und erneut eingeengt. Nach Trocknen im Hochvakuum erhält man 18 mg (30 % d.Th.) der Titelverbindung.
¹H-NMR (400 MHz, Methanol-d₄): δ =8.18 (s, 1H), 7.95 (m, 1H), 7.63-7.42 (m, 4H), 7.38 (m, 2H), 4.42 (m, 1H), 3.81 (m, 1H), 3.47 (m, 1H), 3.40-3.25 (m, 4H), 2.57 (m, 3H), 2.37 (m, 1H), 2.26 (m, 1H), 2.09 (m, 2H), 1.94 (m, 1H).
LC-MS (Methode 4): Rₜ = 2.7 min., m/z = 464 (M+H)⁺ (freie Base).

### Beispiel 66

### N-[(3R)-1-Azabicyclo[2.2.2]oct-3-yl]-7-[4-(2-oxo-1-pyrrolidinyl)phenyl]-1-benzothiophen-2-carboxamid-Hydrochlorid

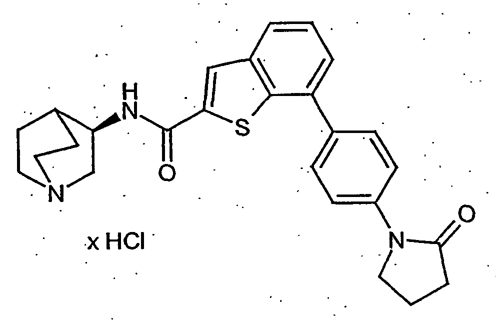

Gemäß der allgemeinen Arbeitsvorschrift D werden 116.5 mg (0.49 mmol) 1-(4-Bromphenyl)-2-pyrrolidinon, 142.2 mg (0.56 mmol) Bis(pinacolato)dibor, 119.1 mg (1.21 mmol) Kaliumacetat, 13.7 mg (0.02 mmol) PdCl₂(dppf), 150 mg (0.37 mmol) *N*-[(3R)-1-Azabicyclo[2.2..2]oct-3-yl]-7-brom-1-benzothiophen-2-carboxamid-Hydrochlorid (Beispiel 8A), 0.93 mL 2 M Natriumcarbonat-Lösung und weitere 13.7 mg (0.02 mmol) PdCl₂(dppf) in 2 mL DMF umgesetzt. Nach Trocknen im Hochvakuum werden 71 mg (39 % d.Th.) der Titelverbindung erhalten.
¹H-NMR (400 MHz, Methanol-d₄): δ = 8.16 (s; 1H), 7.91 (d, 1H), 7.80 (m, 2H), 7.75 (m, 2H), 7.54 (dd, 1H), 7.51 (dd, 1H), 4.46 (m, 1H), 4.01 (m, 2H), 3.85 (m, 1H), 3.47 (m, 1H), 3.42-3.26 (m, 4H), 2.65 (m, 2H), 2.39 (m, 1H), 2.24 (m, 3H), 2.10 (m, 2H), 1.96 (m, 1H).
HPLC (Methode 1): Rₜ = 4.0 min.
MS (ESIpos): m/z = 446 (M+H)⁺ (freie Base).

### Beispiel 67

### N-[(3R)-1-Azabicyclo[2.2.2]oct-3-yl]-7-[4-(1-piperazinyl)phenyl]-1-benzothiophen-2-carboxamid-Dihydrochlorid

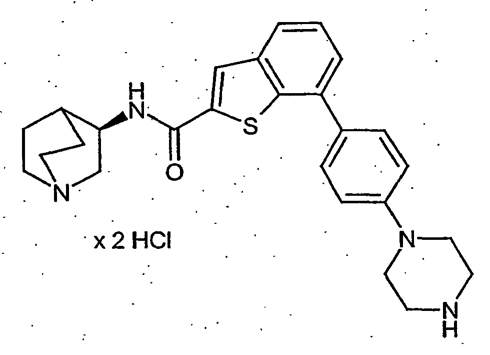

Gemäß der allgemeinen Arbeitsvorschrift D werden 165.6 mg (0.49 mmol) 4-(4-Bromphenyl)-1-piperazincarbonsäure-tert.-butylester, 142.2 mg (0.56 mmol) Bis-(pinacolato)dibor, 119.1 mg (1.21 mmol) Kaliumacetat, 13.7 mg (0.02 mmol) PdCl₂(dppf), 150 mg (0.37 mmol) *N*-[(3R)-1-Azabicyclo[2.2.2]oct-3-yl]-7-brom-1-benzothiophen-2-carboxamid-Hydrochlorid (Beispiel 8A), 0.93 mL 2 M Natriumcarbonat-Lösung und weitere 13.7 mg (0.02 mmol) PdCl₂(dppf) in 2 mL DMF umgesetzt. Die durch präparative HPLC gereinigte Verbindung wird in 3 mL Methanol gelöst, mit 3 mL 4 M Chlorwasserstoff in Dioxan versetzt und 30 min. bei Raumtemperatur gerührt. Der Kolbeninhalt wird im Vakuum eingeengt und der Rückstand zweimal mit Toluol azeotrop abdestilliert. Nach Trocknen im Hochvakuum werden 54 mg (28 % d.Th.) der Titelverbindung erhalten.
¹H-NMR (300 MHz, Methanol-d₄): δ = 8.17 (s, 1H), 7.87 (dd, 1H), 7.66 (m, 2H), 7.52 (dd, 1H), 7.45 (dd, 1H), 7.18 (m, 2H), 4.45 (m, 1H), 3.83 (m, 1H), 3.75-3.13 (m, 13H), 2.38 (m, 1H), 2.28 (m, 1H), 2.10 (m, 2H), 1.95 (m, 1H).
HPLC (Methode 1): Rₜ = 3.7 min.
MS (ESIpos): m/z = 447 (M+H)⁺ (freie Base).

### Beispiel 68

### N-[(3R)-1-Azabicyclo[2.2.2]oct-3-yl]-7-[4-(3-oxo-4-morpholinyl)phenyl]-1-benzothiophen-2-carboxamid-Hydrochlorid

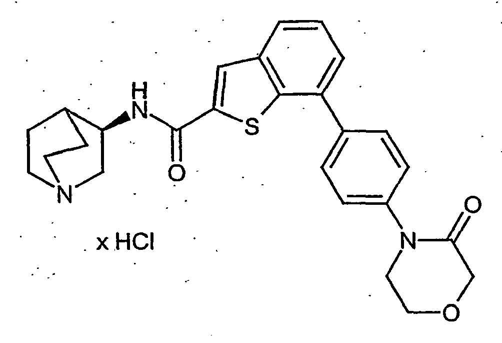

Gemäß der allgemeinen Arbeitsvorschrift D werden 120 mg (0.39 mmol) 4-(4-Bromphenyl)-3-morpholinon, 115.3 mg (0.45 mmol) Bis(pinacolato)dibor, 96.6 mg (0.98 mmol) Kaliumacetat, 11.1 mg (0.02 mmol) PdCl₂(dppf), 121.6 mg (0.30 mmol) *N*-[(3R)-1-Azabicyclo[2.2.2]oct-3-yl]-7-brom-1-benzothiophen-2-carboxamid-Hydrochlorid (Beispiel 8A), 0.76 mL 2 M Natriumcarbonat-Lösung und weitere 11.1 mg (0.02 mmol) PdCl₂(dppf) in 2 mL DMF umgesetzt. Nach Trocknen im Hochvakuum werden 24 mg (16 % d.Th.) der Titelverbindung erhalten.
¹H-NMR (400 MHz, Methanol-d₄): δ = 8.17 (s, 1H), 7.93 (d, 1H), 7.80 (m, 2H), 7.55 (m, 4H), 4.46 (m, 1H), 4.33 (s, 2H), 4.09 (m, 2H), 3.88 (m, 2H), 3.84 (m, 1H), 3.47 (m, 1H), 3.41-3.26 (m, 4H), 2.39 (m, 1H), 2.28 (m, 1H), 2.10 (m, 2H), 1.95 (m, 1H).
HPLC (Methode 1): Rₜ = 3.8 min.
MS (ESIpos): m/z = 462 (M+H)⁺ (freie Base).

### Beispiel 69

### N-[(3R)-1-Azabicyclo[2.2.2]oct-3-yl]-7-[3-(1-pyrrolidinyl)phenyl]-1-benzothiophen-2-carboxamid-Hydrochlorid

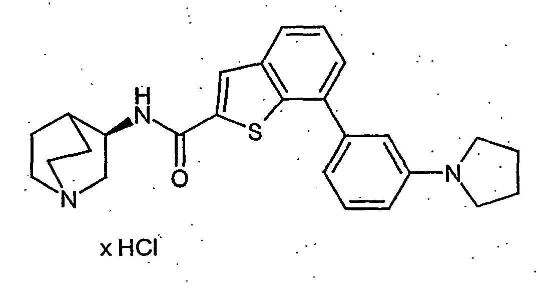

Gemäß der allgemeinen Arbeitsvorschrift D werden 109.8 mg (0.49 mmol) 1-(3-Bromphenyl)pyrrolidin, 142.2 mg (0.56 mmol) Bis(pinacolato)dibor, 119.1 mg (1.21 mmol) Kaliumacetat, 13.7 mg (0.02 mmol) PdCl₂(dppf), 150.0 mg (0.37 mmol) *N-*[(3R)-1-Azabicyclo[2.2.2]oct-3-yl]-7-brom-1-benzothiophen-2-carboxamid-Hydrochlorid (Beispiel 8A), 0.93 mL 2 M Natriumcarbonat-Lösung und weitere 13.7 mg (0.02 mmol) PdCl₂(dppf) in 2 mL DMF umgesetzt. Nach Trocknen im Hochvakuum werden 88.4 mg (51 % d.Th.) der Titelverbindung erhalten.
¹H-NMR (200 MHz, DMSO-d₆): δ = 10.28 (br. s, 1H), 9.11 (d, 1H), 8.42 (s, 1H), 7.95 (dd, 1H), 7.55 (m, 2H), 7.36 (dd, 1H), 6.96 (d, 1H), 6.87 (s, 1H), 6.68 (m, 1H), 4.33 (m, 1H), 3.80-3.10 (m, 10H), 2.21 (m, 1H), 2.11 (m, 1H), 2.95 (m, 6H), 1.75 (m, 1H).
HPLC (Methode 1): Rₜ = 4.2 min.
MS (ESIpos): m/z = 432 (M+H)⁺ (freie Base).

### Beispiel 70

### N-[(3R)-1-Azabicyclo[2.2.2]oct-3-yl]-7-[3-(4-morpholinyl)phenyl]-1-benzothiophen-2-carboxamid-Hydrochlorid

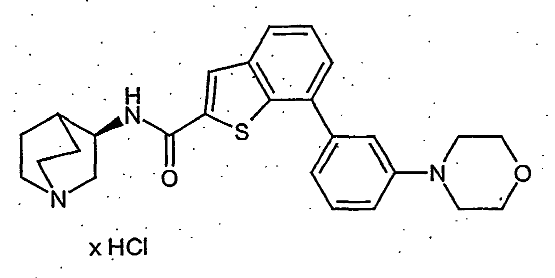

Gemäß der allgemeinen Arbeitsvorschrift D werden 151.1 mg (0.49 mmol) 3-(4-Morpholinyl)phenyl-trifluormethansulfonat (Beispiel 17A), 142.2 mg (0.56 mmol) Bis(pinacolato)dibor, 119.1 mg (1.21 mmol) Kaliumacetat, 13.7 mg (0.02 mmol) PdCl₂(dppf), 150.0 mg (0.37 mmol) *N*-[(3R)-1-Azabicyclo[2.2.2]oct-3-yl]-7-brom-1-benzotbiophen-2-carboxamid-Hydrochlorid (Beispiel 8A), 0.93 mL 2 M Natriumcarbonat-Lösung und weitere 13.7 mg (0.02 mmol) PdCl₂(dppf) in 2 mL DMF umgesetzt. Nach Trocknen im Hochvakuum werden 125.3 mg (68 % d.Th.) der Titelverbindung erhalten.
¹H-NMR (400 MHz, Methanol-d₄): δ = 8.26 (s, 1H), 7.99 (m, 2H), 7.86 (d, 1H), 7.75 (m, 2H), 7.59 (m, 2H), 4.47 (m, 1H), 4.10 (m, 4H), 3.83 (m, 1H), 3,76 (m, 4H), 3.73 (m, 1H), 3.52 (m, 1H), 3.37 (m, 3H), 2.38 (m, 1H), 2.29 (m, 1H), 2.10 (m, 2H), 1.96 (m, 1H).
HPLC (Methode 1): Rₜ = 3.9 min.
MS (ESIpos): m/z = 448 (M+H)⁺ (freie Base).

### Beispiel 71

### N-[(3R)-1-Azabicyclo[2.2.2]oct-3-yl]-7-[4-(1-pyrrolidinyl)phenyl]-1-benzothiophen-2-carboxamid-Dihydrochlorid

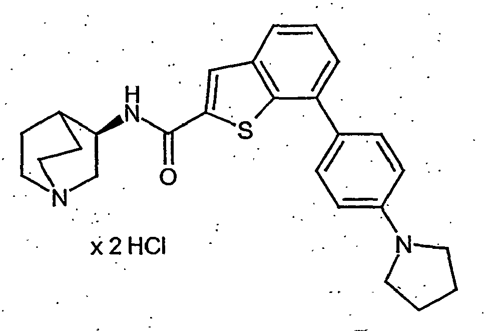

Gemäß der allgemeinen Arbeitsvorschrift D werden 109.8 mg (0.49 mmol) 1-(4-Bromphenyl)pyrrolidin, 142.2 mg (0.56 mmol) Bis(pinacolato)dibor, 119.1 mg (1.21 mmol) Kaliumacetat, 13.7 mg (0.02 mmol) PdCl₂(dppf), 150.0 mg (0.37, mmol) *N-*[(3R)-1-Azabicyclo[2.2.2]oct-3-yl]-7-brom-1-benzothiophen-2-carboxamid-Hydrochlorid (Beispiel 8A), 0.93 mL 2 M Natriumcarbonat-Lösung und weitere 13.7 mg (0.02 mmol) PdCl₂(dppf) in 2.5 mL DMF umgesetzt. Nach Trocknen im Hochvakuum werden 24.8 mg (13 % d.Th.) der Titelverbindung erhalten.
¹H-NMR (400 MHz, Methanol-d₄): δ = 8.26 (s, 1H), 7.98 (d, 1H), 7.92 (m, 2H), 7.75 (m, 2H), 7.58 (dd, 1H), 7.53 (d, 1H), 4.47 (m, 1H), 3.92-3.76 (m, 5H), 3.51 (m, 1H), 3.45-3.18 (m, 4H), 2.42-2.23 (m, 6H), 2.10 (m, 2H), 1.96 (m, 1H).
HPLC (Methode 1): Rₜ = 4.1 min.
MS (ESIpos): m/z = 432 (M+H)⁺ (freie Base).

### Beispiel 72

### N-[(3R)-1-Azabicyclo[2.2.2]oct-3-yl]-7-[4-(4-morpholinylcarbonyl)phenyl]-1-benzothiophen-2-carboxamid-Hydrochlorid

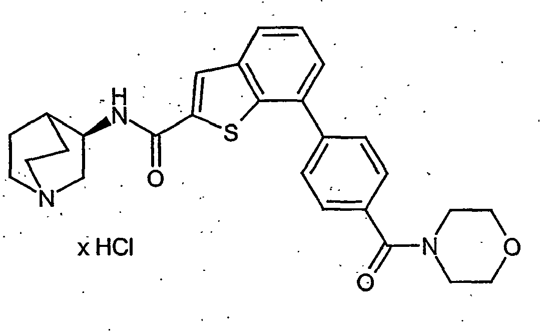

Gemäß der allgemeinen Arbeitsvorschrift D werden 164.7 mg (0.49 mmol) 4-(4-Morpholinylcarbonyl)phenyl-trifluormethansulfonat (Beispiel 18A), 142.2 mg (0.56 mmol) Bis(pinacolato)dibor, 119.1 mg (1.21 mmol) Kaliumacetat, 13.7 mg (0.02 mmol) PdCl₂(dppf), 150.0 mg (0.37 mmol) *N*-[(3R)-1-Azabicyclo[2.2.2]oct-3-yl]-7-brom-1-benzothiophen-2-carboxamid-Hydrochlorid (Beispiel 8A), 0.93 mL 2 M Natriumcarbonat-Lösung und weitere 13.7 mg (0.02 mmol) PdCl₂(dppf) in 2.5 mL DMF umgesetzt. Im Anschluss an eine erste Reinigung mittels präparativer HPLC wird eine Säulenchromatographie an Kieselgel (Laufmittel: Dichlormethan-Methanol-Ammoniak 90:9:1) durchgeführt. Nach Trocknen im Hochvakuum werden 24.8 mg (13 % d.Th.) der Titelverbindung erhalten.
¹H-NMR (400 MHz, Methanol-d₄): δ = 8.22 (s, 1H), 7.97 (d, 1H), 7.83 (m, 2H), 7.62 (m, 2H), 7.55 (m, 2H), 4.47 (m, 1H), 3.90-3.26 (m, 14H), 2.38 (m, 1H), 2.28 (m, 1H), 2.10 (m, 2H), 1.96 (m, 1H).
HPLC (Methode 1): Rₜ = 3.8 min.
MS (ESIpos): m/z = 476 (M+H)⁺ (freie Base).

### Beispiel 73

### 7-[2-(Aminomethyl)phenyl]-N-[(3R)-1-azabicyclo[2.2.2]oct-3-yl]-1-benzothiophen-2-carboxamid-Dihydrochlorid

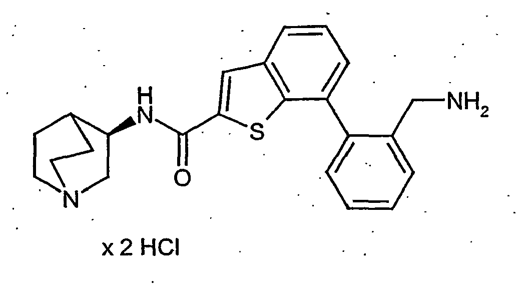

Gemäß der allgemeinen Arbeitsvorschrift D werden 534.2 mg (1.87 mmol) tert.-Butyl-2-brombenzylcarbamat, 474.1 mg (1.87 mmol) Bis(pinacolato)dibor, 397.0 mg (4.04 mmol) Kaliumacetat, 45.5 mg (0.06 mmol) PdCl₂(dppf), 500 mg (1.24 mmol) *N*-[(3R)-1-Azabicyclo[2.2.2]oct-3-yl]-7-brom-1-benzothiophen-2-carboxamid-Hydrochlorid (Beispiel 8A), 3.11 mL 2 M Natriumcarbonat-Lösung und weitere 45.5 mg (0.06 mmol) PdCl₂(dppf) in 6.0 mL DMF umgesetzt. Im Anschluss an die Reinigung mittels präparativer HPLC werden die vereinigten Produktfraktionen eingeengt, in Methanol aufgenommen, mit 1 N Salzsäure versetzt und 30 min. bei Raumtemperatur gerührt. Nach Einengen und Trocknen im Hochvakuum werden 121 mg (20 % d.Th.) der Titelverbindung erhalten.
HPLC (Methode 1): Rₜ = 3.6 min.
MS (ESIpos): m/z = 392 (M+H)⁺ (freie Base).

### Beispiel 74

### N-[(3R)-1-Azabicyclo[2.2.2]oct-3-yl]-7-{3-[(2,2-dimethylpropanoyl)amino]phenyl}-1-benzothiophen-2-carboxamid-Hydrochlorid

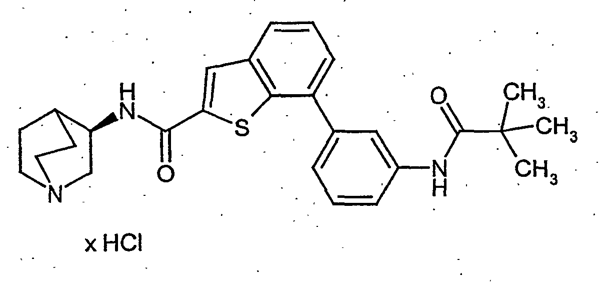

Gemäß der allgemeinen Arbeitsvorschrift D werden 143.5 mg (0.56 mmol) *N*-(3-Bromphenyl)-2,2-dimethylpropanamid, 142.2 mg (0.56 mmol) Bis(pinacolato)dibor, 119.1 mg (1.21 mmol) Kaliumacetat, 13.7 mg (0.02 mmol) PdCl₂(dppf), 150.0 mg (0.37 mmol) *N*-[(3R)-1-Azabicyclo[2.2.2]oct-3-yl]-7-brom-1-benzothiophen-2-carboxamid-Hydrochlorid (Beispiel 8A), 0.93 mL 2 M Natriumcarbonat-Lösung und weitere 13.7 mg (0.02 mmol) PdCl₂(dppf) in 2.0 mL DMF umgesetzt. Im Anschluss an eine erste Reinigung mittels präparativer HPLC wird eine Säulenchromatographie an Kieselgel (Laufmittel: Dichlormethan-Methanol-Ammoniak 90:9: 1) durchgeführt. Nach Trocknen im Hochvakuum werden 32.4 mg (17 % d.Th.) der Titelverbindung erhalten.
¹H-NMR (400 MHz, Methanol-d₄): δ = 8.16 (s, 1H), 8.01 (m, 1H), 7.92 (m, 1H), 7.63-7.48 (m, 3H), 7.47 (m, 2H), 4.44 (m, 1H), 3.84 (m, 1H), 3.47 (m, 1H), 3.41-3.27 (m, 4H), 2.38 (m, 1H), 2.28 (m, 1H), 2.10 (m, 2H), 1.96 (m, 1H), 1.32 (s, 9H).
HPLC (Methode 1): Rₜ = 4.3 min.
MS (ESIpos): m/z = 462 (M+H)⁺ (freie Base).

### Beispiel 75

### 3-(2-{[(3R)-1-Azabicyclo[2.2.2]oct-3-yl-amino]carbonyl}-1-benzothien-7-yl)-benzoesäure-Hydrochlorid

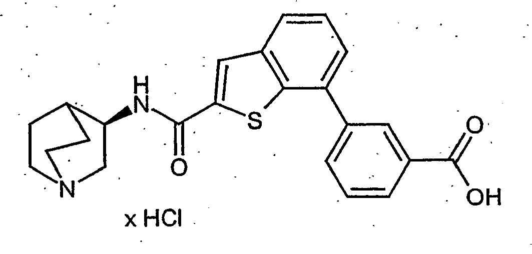

200 mg (0.50 mmol) *N*-[(3R)-1-Azabicyclo[2.2.2]oct-3-yl]-7-brom-1-benzothiophen-2-carboxamid-Hydrochlorid (Beispiel 8A) und 82.6 mg (0.50 mmol) 3-Carboxyphenylboronsäure werden in 1.5 mL DMF vorgelegt. Nach Zugabe von 0.78 mL 2 M Natriumcarbonat-Lösung und 20.3 mg (0.02 mmol) PdCl₂(dppf) wird auf 60°C erhitzt. Nach 18 h werden weitere 20.3 mg (0.02 mmol) PdCl₂(dppf) zugegeben und für weitere 18 h auf 90°C erhitzt. Nach dem Abkühlen wird das Reaktionsgemisch über Kieselgur filtriert und mittels präparativer HPLC aufgereinigt. Die Produktfraktionen werden eingeengt, mit einer 3:1-Mischung aus Acetonitril und 1 N Salzsäure versetzt und erneut eingeengt. Nach Trocknen im Hochvakuum erhält man 103 mg (45 % d.Th.) der Titelverbindung.
¹H-NMR (300 MHz, DMSO-d₆): δ = 10.28 (br. s, 1H), 9.13 (d, 1H), 8.46 (s, 1H), 8.29 (m, 1H), 8.08-7.95 (m, 3H), 7.71 (dd, 1H), 7.60 (m, 2H), 4.33 (m, 1H), 3.85-3.12 (m, 6H), 2.22 (m, 1H), 2.15 (m, 1H), 1.91 (m, 2H), 1.75 (m, 1H).
HPLC (Methode 1): Rₜ = 3.9 min.
MS (ESIpos): m/z = 407 (M+H)⁺ (freie Base).

### Beispiel 76

### N-[(3R)-1-Azabicyclo[2.2.2]oct-3-yl]-7-[2-({[(methylamino)carbonyl]amino}-methyl)phenyl]-1-benzothiophen-2-carboxamid-Hydrochlorid

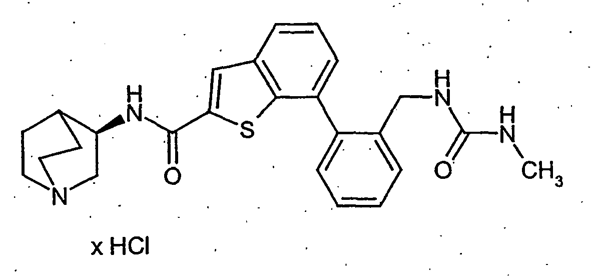

Zu einer Suspension von 85 mg (0.18 mmol) 7-[2-(Aminomethyl)phenyl]-*N*-[(3R)-1-azabicyclo[2.2.2]oct-3-yl]-1-benzothiophen-2-carboxamid-Dihydrochlorid (Beispiel 73) in 1 mL einer 5:1-Mischung aus THF und DMF werden 51.0 µL (0.37 mmol) Triethylamin und 43.5 µL (0.73 mmol) Methylisocyanat gegeben. Nach 18 h bei Raumtemperatur wird die Reaktionsmischung im Vakuum eingeengt und mittels präparativer HPLC aufgereinigt. Die Produktfraktionen werden eingeengt, mit einer 5:1-Mischung aus Methanol und 1 N Salzsäure versetzt und erneut eingeengt. Nach Trocknen im Hochvakuum erhält man 65.5 mg (74 % d.Th.) der Titelverbindung.
¹H-NMR (400 MHz, Methanol-d₄): δ = 8.18 (s, 1H), 7.94 (d, 1H), 7.58-7.44 (m, 3H), 7.43-7.29 (m, 3H), 4.43 (m, 1H), 4.15 (m, 2H), 3.82 (m, 1H); 3.47 (m, 1H), 3.41-3.27 (m, 4H), 2.62 (s, 3H), 2.37 (m, 1H), 2.26 (m, 1H), 2.08 (m, 2H), 1.94 (m, 1H).
HPLC (Methode 1): Rₜ = 3.8 min.
LC-MS (Methode 4): Rₜ = 2.5 min.
MS (ESIpos): m/z = 448 (M+H)⁺ (freie Base).

### Beispiel 77

### N-[(3R)-1-Azabicyclo[2.2.2]oct-3-yl]-7-[3-(1H-pyrrol-1-yl)phenyl]-1-benzothiophen-2-carboxamid-Hydrochlorid

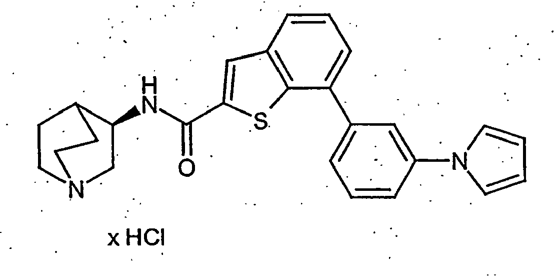

Gemäß der allgemeinen Arbeitsvorschrift D werden 124.4 mg (0.56 mmol) 1-(3-Bromphenyl)-1H-pyrrol, 142.2 mg (0.56 mmol) Bis(pinacolato)dibor, 119.1 mg (1.21 mmol) Kaliumacetat, 13.7 mg (0.02 mmol) PdCl₂(dppf), 150.0 mg (0.37 mmol) *N*-[(3R)-1-Azabicyclo[2.2.2]oct-3-yl]-7-brom-1-benzothiophen-2-carboxamid-Hydrochlorid (Beispiel 8A), 0.93 mL 2 M Natriumcarbonat-Lösung und weitere 13.7 mg (0.02 mmol) PdCl₂(dppf) in 2.0 mL DMF umgesetzt. Nach Trocknen im Hochvakuum werden 86.9 mg (48 % d.Th.) der Titelverbindung erhalten.
¹H-NMR (400 MHz, DMSO-d₆): δ = 9.92 (br. s, 1H), 9.03 (d, 1H), 8.39 (m, 1H), 8.02 (m, 1H), 7.88 (s, 1H), 7.77-7.57 (m, 5H), 7.49 (m, 2H), 6:31 (m, 2H), 4.32 (m, 1H), 3.67 (m, 1H), 3.5.7-3.13 (m, 5H), 2.21 (m, 1H), 2.13 (m, 1H), 1.90 (m, 2H), 1.75 (m, 1H).
HPLC (Methode 1): Rₜ = 4.5 min.
MS (ESIpos): m/z = 428 (M+H)⁺ (freie Base).

### Beispiel 78

### N-[(3R)-1-Azabicyclo[2.2.2]oct-3-yl]-7-[3-(4-morpholinylcarbonyl)phenyl]-1-benzothiophen-2-carboxamid-Hydrochlorid

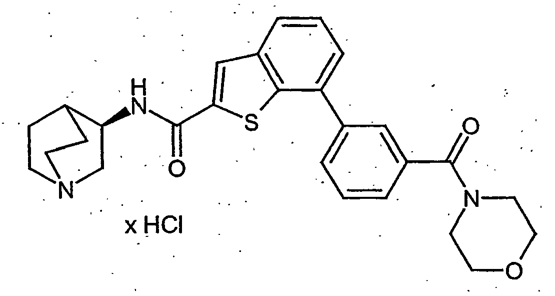

Zu einer Lösung von 50 mg (0.11 mmol) 3-(2-{[(3R)-1-Azabicyclo[2.2.2]oct-3-yl-amino]carbonyl}-1-benzothien-7-yl)-benzoesäure-Hydrochlorid (Beispiel 75) und 19.7 µL (0.23 mmol) Morpholin in 0.5 mL DMF werden bei 0°C 103 mg (0.27 mmol) HATU und 70.8 µL (0.41 mmol) *N,N*-Diisopropylethylamin gegeben. Bei Raumtemperatur wird 18 h gerührt. Nach Aufreinigung mittels präparativer HPLC werden die Produktfraktionen eingeengt, mit einer 3:1-Mischung aus Acetonitril und 1 N Salzsäure versetzt und erneut eingeengt. Nach Trocknen im Hochvakuum erhält man 43 mg (74 % d.Th.) der Titelverbindung.
¹H-NMR (200 MHz, DMSO-d₆): δ = 10.66 (br. s, 1H), 9.33 (d, 1H), 8.56 (s, 1H), 7.98 (dd, 1H), 7.87-7.45 (m, 6H), 4.34 (m; 1H), 3.87-3.06 (m, 14H), 2.18 (m, 2H), 1.90 (m, 2H), 1.74 (m, 1H).
HPLC (Methode 1): Rₜ = 3.8 min.
MS (ESIpos): m/z = 476 (M+H)⁺ (freie Base).

### Beispiel 79

### N-[(3S)-1-Azabicyclo[2.2.2]oct-3-yl]-7-[4-(4-morpholinyl)phenyl]-1-benzothiophen-2-carboxamid-Hydrochlorid

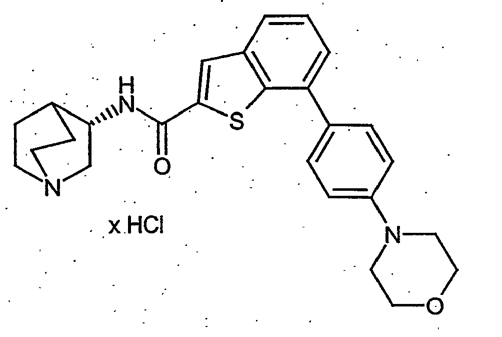

Zu einer Mischung aus 144.3 mg (0.72 mmol) *S*-3-Aminochinuclidin-Dihydrochlorid und 300 mg (0.72 mmol) 7-[4-(4-Morpholinyl)phenyl]-1-benzothiophen-2-carbonsäure (Beispiel 20A) in 3 mL DMF werden bei 0°C 330.7 mg (0.87 mmol) HATU und 112.4 mg (0.87 mmol) *N,N*-Diisopropylethylamin gegeben. Nach 30 min. bei 0°C werden weitere 224.8 mg (1.74 mol) *N,N*-Diisopropylethylamin hinzugefügt und 19 h bei Raumtemperatur nachgerührt. Die Reaktionslösung wird mit etwas Wasser und Acetonitril versetzt und mittels präparativer HPLC aufgereinigt. Die Produktfraktionen werden eingeengt, mit einer 3:1-Mischung aus Methanol und 1 N Salzsäure versetzt und erneut eingeengt. Nach Trocknen im Hochvakuum erhält man 158 mg (45 % d.Th.) der Titelverbindung.
Die spektroskopischen Daten stimmen mit denen der enantiomeren Verbindung (Beispiel 19) überein.

### Beispiel 80

### N-[(3R)-1-Azabicyclo[2.2.2]oct-3-yl]-7-[3-(4-morpholinyl)phenyl]-1-benzofuran-2-carboxamid-Hydrochlorid

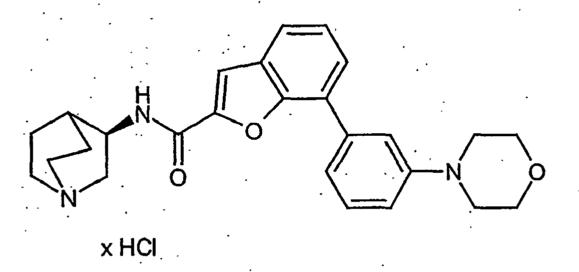

Gemäß der allgemeinen Arbeitsvorschrift D werden 151.1 mg (0.49 mmol) 3-(4-Morpholinyl)phenyl-trifluormethansulfonat (Beispiel 17A), 142.2 mg (0.56 mmol) Bis(pinacolato)dibor, 119.1 mg (1.21 mmol) Kaliumacetat, 13.7 mg (0.02 mmol) PdCl₂(dppf), 144 mg (0.37 mmol) *N*-[(3R)-1-Azabicyclo[2.2.2]oct-3-yl]-7-brom-1-benzofuran-2-carboxamid-Hydrochlorid (Beispiel 30A), 0.93 mL 2 M Natriumcarbonat-Lösung und weitere 13.7 mg (0.02 mmol) PdCl₂(dppf) in 2 mL DMF umgesetzt. Nach Trocknen im Hochvakuum werden 32 mg (18 % d.Th.) der Titelverbindung erhalten.
¹H-NMR (300 MHz, DMSO-d₆): δ = 10.27 (br. s, 1H), 8.96 (d, 1H), 7.85 (s, 1H), 7.78 (m, 1H), 7.68 (m, 1H); 7.62 (m, 1H), 7.43 (m, 3H), 7.17 (m, 1H), 4.36 (m, 1H), 3.82 (m, 4H), 3.63 (m, 1H), 3.44-3.10 (m, 9H), 2.22 (m, 1H), 2.12 (m, 1H), 1.91 (m, 2H), 1.75 (m, 1H).
HPLC (Methode 1): Rₜ = 3.8 min.
MS (ESIpos): m/z = 432 (M+H)⁺ (freie Base).

### Beispiel 81

### 7-(3-Aminophenyl)-N-[(3R)-1-azabicyclo[2.2.2]oct-3-yl]-1-benzothiophen-2-carboxamid-Hydrochlorid

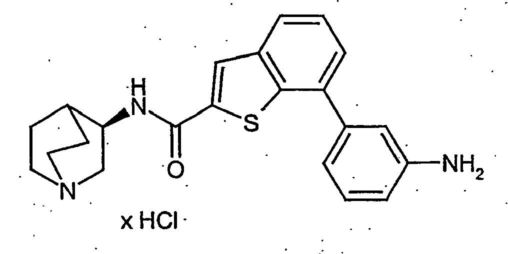

200 mg (0.50 mmol) *N*-[(3R)-1-Azabicyclo[2.2.2]oct-3-yl]-7-brom-1-benzothiophen-2-carboxamid-Hydrochlorid (Beispiel 8A) und 68.2 mg (0.50 mmol) 3-Aminophenylboronsäure werden in 1.5 mL DMF vorgelegt. Nach Addition von 0.78 mL 2 M Natriumcarbonat-Lösung und 20.3 mg (0.02 mmol) PdCl₂(dppf) wird 18 h auf 60°C erhitzt. Nach dem Abkühlen wird das Reaktionsgemisch über Kieselgur filtriert und durch Trennung mittels präparativer HPLC aufgereinigt. Die Produktfraktionen werden eingeengt, mit einer 3: 1-Mischung aus Acetonitril und 1 N Salzsäure versetzt und erneut eingeengt. Nach Trocknen im Hochvakuum erhält man 201 mg (98 % d.Th.) der Titelverbindung.
¹H-NMR (200 MHz, DMSO-d₆): δ = 10.63 (br. s, 1H), 9.33 (d, 1H), 8.57 (s, 1H), 8.00 (dd, 1H), 7.76-7.58 (m, 4H), 7.55 (m, 1H), 7.43 (m, 1H), 4.34 (m, 1H), 3.62 (m, 1H), 3.42 (m, 2H), 3.19 (m, 3H), 2.19 (m, 2H), 1.90 (m, 2H), 1.73 (m, 1H).
HPLC (Methode 1): Rₜ = 3.5 min.
MS (ESIpos): m/z = 378 (M+R)⁺ (freie Base).

### Beispiel 82

### N-[(3R)-1-Azabicyclo[2.2.2]oct-3-yl]-7-{3-[(methoxyacetyl)amino]phenyl}-1-benzothiophen-2-carboxamid-Hydrochlorid

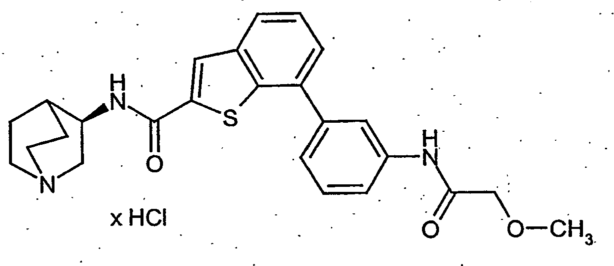

Zu einer Lösung von 50 mg (0.12 mmol) 7-(3-Aminophenyl)-*N*-[(3R)-1-azabicydo-[2.2.2]oct-3-yl]-1-benzothiophen-2-carboxamid-Hydröchlorid (Beispiel 81) und 18.5 µL (0.24 mmol) Methoxyessigsäure in 0.5 mL DMF werden bei 0°C 96.4 mg (0.25 mmol) HATU und 71.5 µL (0.41 mmol) *N,N*-Düsopropylethylamin gegeben. Bei Raumtemperatur wird 3 h nachgerührt. Nach Aufreinigung mittels präparativer HPLC werden die Produktfraktionen eingeengt, mit einer 3:1-Mischung aus Acetonitril und 1 N Salzsäure versetzt und erneut eingeengt. Nach Trocknen im Hochvakuum erhält man 7 mg (11 % d.Th.) der Titelverbindung.
¹H-NMR (300 MHz, DMSO-d₆): δ = 10.13 (br. s, 1H), 9.97 (s, 1H), 9.04 (d, 1H), 8.39 (s, 1H), 8.13 (m, 1H), 7.98 (d, 1H), 7.76 (d, 1H), 7.58 (dd, 1H), 7.50 (m, 2H), 7.42 (m, 1H), 4.33 (m, 1H), 4.03 (s, 2H), 3.66 (m, 1H), 3.56-3.12 (m, 8H), 2.22 (m, 1H), 2.14 (m, 1H), 1.91 (m, 2H), 1.75 (m, 1H).
HPLC (Methode 1): Rₜ = 4.0 min.
MS (ESIpos): m/z = 450 (M+H)⁺ (freie Base).

### Beispiel 83

### N-[(3R)-1-Azabicyclo[2.2.2]oct-3-yl]-7-[4-(2-oxo-1-pyrrolidinyl)phenyl]-1-benzofuran-2-carboxamid-Hydrochlorid

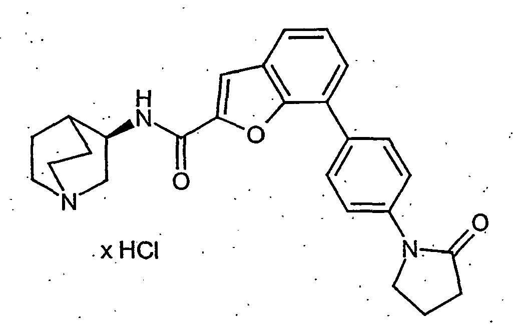

Gemäß der allgemeinen Arbeitsvorschrift D werden 161.9 mg (0.67 mmol) 1-(4-Bromphenyl)-2-pyrrolidinon, 197.5 mg (0.78 mmol) Bis(pinacolato)dibor, 165.4 mg (1.69 mmol) Kaliumacetat, 19.0 mg (0.03 mmol) PdCl₂(dppf), 200 mg (0.52 mmol) *N*-[(3R)-1-Azabicyclo[2.2.2]oct-3-yl]-7-brom-1-benzofuran-2-carboxamid-Hydrochlorid (Beispiel 30A), 1.30 mL 2 M Natriumcarbonat-Lösung und weitere 19.0 mg (0.03 mmol) PdCl₂(dppf) in 2.5 mL DMF umgesetzt. Nach Trocknen im Hochvakuum werden 166.6 mg (65 % d.Th.) der Titelverbindung erhalten.
¹H-NMR (200 MHz, DMSO-d₆): δ = 10.06 (br. s, 1H), 8.96 (d, 1H), 7.97 (m, 1H), 7.94 (s, 1H), 7.83 (m, 3H), 7.78 (dd, 1H), 7.69 (dd, 1H), 7.43 (dd, 1H), 4.33 (m, 1H), 4.00-3.75 (m, 2H), 3.66 (m, 1H), 3.49-3.10 (m, 5H), 2.55 (m, 2H), 2.23 (m, 1H), 2.10 (m, 3H), 1.91 (m, 2H), 1.75 (m, 1H).
HPLC (Methode 1): Rₜ = 4.0 min.
MS (ESIpos): m/z = 430.5 (M+H)⁺ (freie Base).

### Beispiel 84

### 7-[3-(Acetylamino)phenyl]-N-[(3S)-1-azabicyclo[2.2.2]oct-3-yl]-1-benzothiophen-2-carboxamid-Hydrochlorid

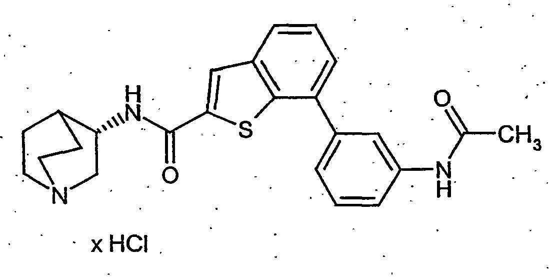

Zu einer Mischung aus 200 mg (0.45 mmol) *N*-[(3S)-1-Azabicyclo[2.2.2]oct-3-yl]-7-brom-1-benzothiophen-2-carboxamid-Hydrochlorid (Beispiel 22A) und 80.2 mg (0.45 mmol) 3-(Acetamido)phenylboronsäure in 2 mL DMF werden 0.67 mL 2 M Natriumcarbonat-Lösung und 18.3 mg (0.02 mmol) PdCl₂(dppf) gegeben. Das Reaktionsgemisch wird 17 h auf 80°C erhitzt. Es werden weitere 40.1 mg (0.22 mmol) 3-(Acetamido)phenylboronsäure, 1.34 mL 1 N Natronlauge sowie 73.2 mg (0.09 mmol) FdCl₂(dppf) zugegeben und weitere 18 h auf 70°C erhitzt. Nach dem Abkühlen wird über Kieselgur filtriert und mittels präparativer HPLC aufgereinigt. Die Produktfraktionen werden eingeengt, in einem 5:1-Gemisch aus Methanol und 1 N Salzsäure aufgenommen und erneut eingeengt. Nach Trocknen im Hochvakuum erhält man 124.5 mg (59 % d.Th.) der Titelverbindung.
Die spektroskopischen Daten stimmen mit denen der enantiomeren Verbindung (Beispiel 51) überein.

### Beispiel 85

### N-[(3S)-1-Azabicyclo[2.2.2]oct-3-yl]-7-(3-methoxyphenyl)-1-benzothiophen-2-carboxamid-Hydrochlorid

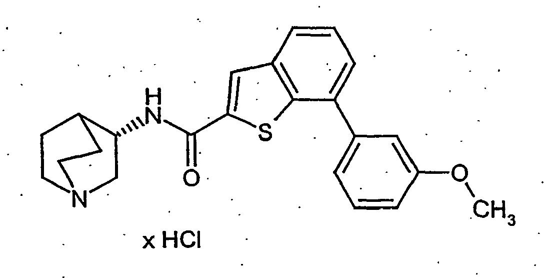

Zu einer Mischung aus 200 mg (0.45 mmol) *N*-[(3S)-1-Azabicyclo[2.2.2]oct-3-yl]-7-brom-1-benzothiophen-2-carboxamid-Hydrochlorid (Beispiel 22A) und 68.1 mg (0.45 mmol) 3-Methoxyphenylboronsäure in 2 mL DMF werden 0.67 mL 2 M wässrige Natriumcarbonat-Lösung und 18.3 mg (0.02 mmol) PdCl₂(dppf) gegeben. Das Reaktionsgemisch wird 17 h auf 80°C erhitzt, über Kieselgur filtriert und zur Trockne eingeengt. Im Anschluss an eine Aufreinigung mittels präparativer HPLC wird eine Säulenchromatographie an Kieselgel (Laufmittel: Dichlörmethan-Methanol-Ammoniak 90:9:1) durchgeführt. Die Produktfraktionen werden eingeengt, in einem 5:1-Gemisch aus Methanol und 1 N Salzsäure aufgenommen und erneut eingeengt. Nach Trocknen im Hochvakuum erhält man 97.7 mg (48 % d.Th.) der Titelverbindung.
¹H-NMR (200 MHz, DMSO-d₆): δ = 10.25 (br. s, 1H), 9.11 (d, 1H), 8.42 (s, 1H), 7.97 (dd, 1H), 7.56 (m, 2H), 7.47 (d, 1H), 7.30 (d, 1H), 7.24 (m, 1H), 7.06 (m, 1H), 4.32 (m, 1H), 3.83 (s, 3H), 3.63 (m, 1H), 3.49-3.10 (m, 5H), 2.20 (m, 1H), 2.13 (m, 1H), 1.90 (m, 2H), 1.74 (m, 1H).
Die analytischen Daten stimmen mit denen der enantiomeren Verbindung (Beispiel 17) überein.

### Beispiel 86

### 4-(2-{[(3R)-1-Azabicyclo[2.2.2]oct-3-yl-amino]carbonyl}-1-benzofuran-7-yl)-benzoesäure-Hydrochlorid

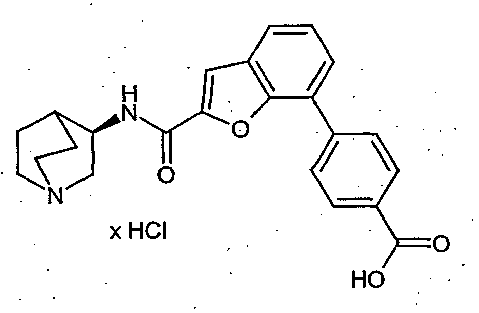

150 mg (0.43 mmol) *N*-[(3R)-1-Azabicyclo[2.2.2]oct-3-yl]-7-brom-1-benzofuran-2-carboxamid-Hydrochlorid (Beispiel 30A) und 71.3 mg (0.43 mmol) 4-Carboxyphenylboronsäure werden in 1.5 mL DMF vorgelegt. Nach Zugabe von 0.64 mL 2 M Natriumcarbonat-Lösung und 17.5 mg (0.02 mmol) PdCl₂(dppf) wird 18 h auf 80°C erhitzt. Nach Abkühlen wird das Reaktionsgemisch über Kieselgur filtriert, das Filtrat eingeengt und zwischen Wasser und Essigsäureethylester verteilt. Die wässrige Phase wird mit Essigsäureethylester gewaschen und anschließend eingeengt. Das Rohprodukt wird mittels präparativer HPLC aufgereinigt. Die Produktfraktionen werden vereinigt, eingeengt, mit einer 3:1-Mischung aus Acetonitril und 1 N Salzsäure versetzt und erneut eingeengt. Das Rohprodukt wird mit Acetonitril verrührt. Nach Absaugen des Niederschlags sowie Trocknen im Hochvakuum erhält man 37 mg (20 % d.Th.) der Titelverbindung.
¹H-NMR (300 MHz, DMSO-d₆): δ = 10.37 (br. s, 1H), 9.15 (d, 1H), 8.08 (m, 4H), 7.93 (s, 1H), 7.85 (dd, 1H), 7.76 (dd, 1H), 7.47 (dd, 1H), 4.36 (m, 1H), 3.77-3.32 (m, 3H), 3.32 (m, 3H), 2.23 (m, 1H), 2.12 (m, 1H), 1.92 (m, 2H), 1.76 (m, 1H). HPLC (Methode 1): Rₜ = 3.9 min.
MS (ESIpos): m/z = 391 (M+H)⁺ (freie Base).

### Beispiel 87

### N-[(3R)-1-Azabicyclo[2.2.2]oct-3-yl]-7-[3-(trifluormethoxy)phenyl]-1-benzofuran-2-carboxamid-Hydrochlorid

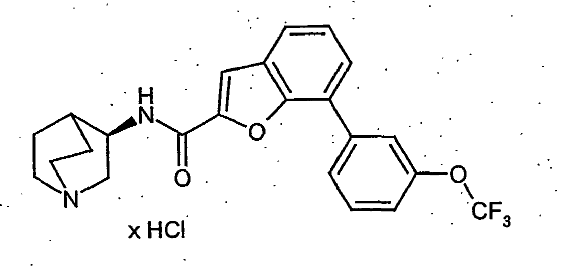

200 mg (0.52 mmol) *N*-[(3R)-1-Azabicyclo[2.2.2]oct-3-yl]-7-brom-1-benzofuran-2-carboxamid-Hydrochlorid (Beispiel 30A) und 106.8 mg (0.52 mmol) 3-(Trifluormethoxy)-phenylboronsäure werden in 2.0 mL DMF vorgelegt. Nach Zugabe von 0.78 mL 2 M Natriumcarbonat-Lösung und 21.2 mg (0.03 mmol) PdCl₂(dppf) wird 17 h auf 70°C erhitzt. Nach dem Abkühlen wird das Reaktionsgemisch über Kieselgur filtriert und mittels präparativer HPLC aufgereinigt. Die Produktfraktionen werden vereinigt, eingeengt, mit einer 5:1-Mischung aus Methanol und 1 N Salzsäure versetzt und erneut eingeengt. Nach Trocknen im Hochvakuum erhält man 48.8 mg (20 % d.Th.) der Titelverbindung.
¹H-NMR (200 MHz, DMSO-d₆): δ = 10.18 (br. s, 1H), 9.00 (d, 1H), 7.95 (m, 2H), 7.89 (s, 1H), 7.86 (m, 1H), 7.77 (m, 1H), 7.68 (m, 1H), 7.47 (m, 2H), 4.34 (m, 1H), 3.65 (m, 1H), 3.35 (m, 2H), 3.23 (m, 3H), 2.22 (m, 1H), 2.12 (m, 1H), 1.92 (m, 2H), 1.76 (in, 1H).
HPLC (Methode 1): Rₜ= 4.5 min.
MS (ESIpos): m/z = 431 (M+H)⁺ (freie Base).

### Beispiel 88

### N-[(3S)-1-Azabicyclo[2.2.2]oct-3-yl]-7-(2-methoxyphenyl)-1-benzothiophen-2-carboxamid-Hydrochlorid

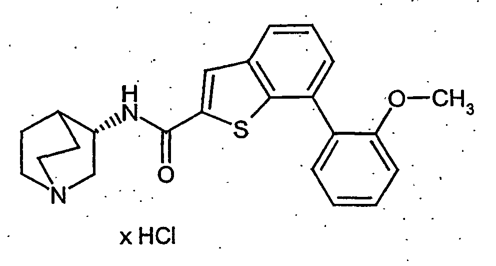

Zu einer Mischung aus 200 mg (0.45 mmol) *N*-[(3S)-1-Azabicyclo[2.2.2]oct-3-yl]-7-brom-1-benzothiophen-2-carboxamid-Hydrochlorid (Beispiel 22A) und 68.1 mg (0.45 mmol) 2-Methoxyphenylboronsäure in 2 mL DMF werden 0.67 mL 2 M Natriumcarbonät-Lösung und 18.3 mg (0.02, mmol) PdCl₂(dppf) gegeben. Das Reaktionsgemisch wird 17 h auf 70°C erhitzt, nach dem Abkühlen über Kieselgur filtriert und mittels präparativer HPLC aufgereinigt. Die Produktfraktionen werden eingeengt, in einem 5:1-Gemisch aus Methanol und 1 N Salzsäure aufgenommen und erneut eingeengt. Nach Trocknen im Hochvakuum erhält man 112 mg (58 % d.Th.) der Titelverbindung.
¹H-NMR (400 MHz, DMSO-d₆): δ = 10.38 (br. s, 1H), 9.07 (d, 1H), 8.38 (s, 1H), 7.95 (m, 1H), 7.50 (dd, 1H), 7.47 (m, 1H), 7.37 (m, 2H), 7.20 (d, 1H), 7.09 (dd, 1H), 4.31 (m, 1H), 3.73 (s, 3H), 3.62 (m, 1H), 3.35 (m, 2H), 3.19 (m, 3H), 2.19 (m, 1H), 2.13 (m, 1H), 1.90 (m, 2H), 1.73 (m, 1H).
Die übrigen analytischen Daten stimmen mit denen der enantiomeren Verbindung (Beispiel 18) überein.

### Beispiel 89

### N-[(3R)-1-Azabicyclo[2.2.2]oct-3-yl]-7-[3-(hydroxymethyl)phenyl]-1-benzofuran-2-carboxamid-Hydrochlorid

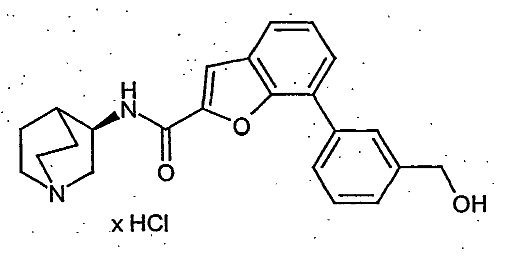

150 mg (0.43 mmol) *N*-[(3R)-1-Azabicyclo[2.2.2]oct-3-yl]-7-brom-1-benzofuran-2-carboxamid-Hydrochlorid (Beispiel 30A) und 65.3 mg (0.43 mmol) 3-(Hydroxymethyl)-phenylboronsäure werden in 1.5 mL DMF vorgelegt. Nach Addition von 0.64 mL 2 M Natriumcarbonat-Lösung und 17.5 mg (0.02 mmol) PdCl₂(dppf) wird 18 h auf 60°C erhitzt. Es werden weitere 17.5 mg (0,02 mmol) PdCl₂(dppf) hinzugefügt und weitere 18 h bei 90°C gerührt. Nach dem Abkühlen wird das Reaktionsgemisch über Kieselgur filtriert. Im Anschluss an eine erste Aufreinigung mittels präparativer HPLC wird eine Säulenchromatographie an Kieselgel (Laufmittel: Dichlormethan-Methanol-Ammoniak 90:9:1) durchgeführt. Die Produktfraktionen werden vereinigt, eingeengt, mit einer 5:1 -Mischung aus Methanol und 4 N Chlorwasserstoff in Dioxan versetzt und erneut eingeengt. Nach Trocknen im Hochvakuum erhält man 45 mg (24 % d.Th.) der Titelverbindung.
¹H-NMR (400 MHz, Methanol-d₄): δ = 7.98 (m, 1H), 7.74 (m, 2H), 7.65 (m, 2H), 7.52 (dd, 1H), 7.42 (m, 2H), 4.72 (s, 2H), 4.51 (m, 1H), 3.87-3.26 (m, 6H), 2.39 (m, 1H), 2.23 (m, 1H), 2.10 (m, 2H), 1.95 (m, 1H).
HPLC (Methode 1): Rₜ = 3.9 min.
MS (ESIpos): m/z = 377 (M+H)⁺ (freie Base).

### Beispiel 90

### N-[(3R)-1-Azabicyclo[2.2.2]oct-3-yl]-7-[4-(3-oxo-4-morpholinyl)phenyl]-1-benzofuran-2-carboxamid-Hydrochlorid

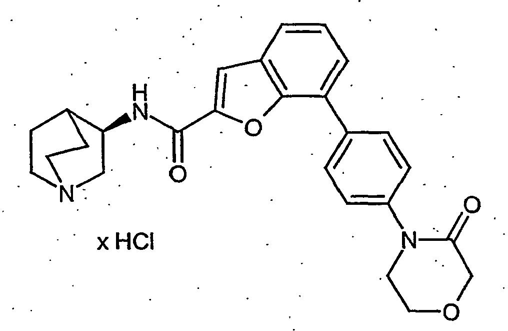

Gemäß der allgemeinen Arbeitsvorschrift D werden 205.4 mg (0.70 mmol) 4-(4-Bromphenyl)-3-morpholinon (Beispiel 16A), 204.4 mg (0.81 mmol) Bis(pinacolato)-dibor, 171.2 mg (1.74 mmol) Kaliumacetat, 19.6 mg (0.03 mmol) PdCl₂(dppf), 207 mg (0.54 mmol) *N*-[(3R)-1-Azabicyclo[2.2.2]oct-3-yl]-7-brom-1-benzofuran-2-carboxamid-Hydrochlorid (Beispiel 30A), 1.34 mL 2 M Natriumcarbonat-Lösung und weitere 19.6 mg (0.03 mmol) PdCl₂(dppf) in 2 mL DMF umgesetzt. Nach Trocknen im Hochvakuum werden 233 mg (85 % d.Th.) der Titelverbindung erhalten.
¹H-NMR (200 MHz, DMSO-d₆): δ = 10.32 (br. s, 1H), 9.07 (d, 1H), 7.95 (m, 3H), 7.80 (dd, 1H), 7.70 (dd, 1H), 7.59 (m, 2H), 7.44 (dd, 1H), 4.33 (m, 1H), 4.26 (s, 2H), 4.02 (m, 2H), 3.83 (m, 2H), 3.64 (m, 1H), 3.37 (m, 2H), 3.21 (m; 3H), 2.23 (m, 1H), 2:11 (m, 1H), 1.91 (m, 2H), 1.74 (m, 1H).
HPLC (Methode 1): Rₜ = 4.5 min.
MS (ESIpos): m/z = 446 (M+H)⁺ (freie Base).

### Beispiel 91

### N-[(3R)-1-Azabicyclo[2.2.2]oct-3-yl]-7-[2-(4-morpholinyl)phenyl]-1-benzofuran-2-carboxamid-Hydrochlorid

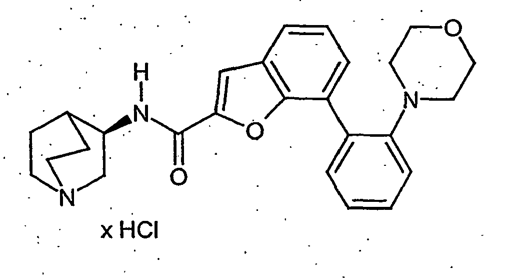

Gemäß der allgemeinen Arbeitsvorschrift D werden 225 mg (0.93 mmol) 2-(4-Morpholinyl)phenyl-trifluormethansulfonat (Beispiel 32A), 272 mg (1.07 mmol) Bis(pinacolato)dibor, 228 mg (2.33 mmol) Kaliumacetat, 26 mg (0.04 mmol) PdCl₂(dppf), 250 mg (0.72 mmol) *N*-[(3R)-1-Azabicyclo[2.2.2]oct-3-yl]-7-brom-1-benzofuran-2-carboxamid (Beispiel 30A), 1.8 mL 2 M Natriumcarbonat-Lösung und weitere 26 mg (0.04 mmol) PdCl₂(dppf) in 2 mL DMF umgesetzt. Nach Trocknen im Hochvakuum werden 82 mg (24 % d.Th.) der Titelverbindung erhalten.
¹H-NMR (400 MHz, DMSO-d₆): δ = 9.76 (s, 1H), 8.82 (d, 1H), 7.79 (s, 1H), 7.77 (d, 1H), 7.55 (d, 1H), 7.46-7.37 (m, 3H), 7.23-7.14 (m, 2H), 4.33 (m, 1H), 3.83-3.04 (m, 6H), 3.55 (s, 4H), 2.68 (s, 4H), 2.16 (m, 1H), 2.04 (m, 1H), 1.95-1.84 (m, 2H), 1.79-1.67 (m, 1H).
HPLC (Methode 1): Rₜ = 4.1 min.
MS (ESIpos): m/z = 432 (M+H)⁺ (freie Base).

### Beispiel 92

### N-[(3R)-1-Azabicyclo[2.2.2]oct-3-yl]-7-[4-(4-morpholinyl)phenyl]-1-benzofuran-2-carboxamid-Hydrochlorid

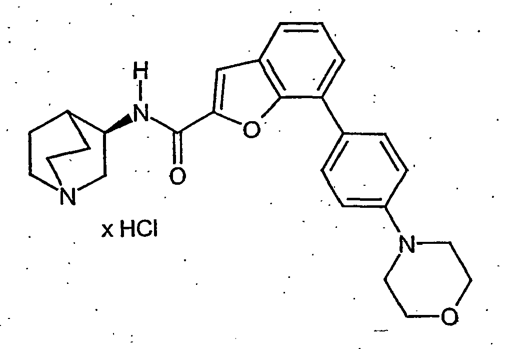

Zu einer Mischung aus 500 mg (0.143 mmol) *N*-[(3R)-1-Azabicyclo[2.2.2]oct-3-yl]-7-brom-1-benzofuran-2-carboxamid-Hydrochlorid (Beispiel 30A) und 105 mg (0,14 mmol) PdCl₂(dppf) in 5 mL DMF werden 444 mg (2.15 mmol) 4-(4-Morpholinyl)-phenylboronsäure und 4.3 mL 1 N Natronlauge gegeben. Das Reaktionsgemisch wird über Nacht auf 100°C erhitzt. Das Solvens wird unter reduziertem Druck entfernt und das Rohprodukt in Methanol aufgenommen und über Kieselgur filtriert. Die Reinigung erfolgt durch präparative HPLC. Die Produktfraktionen werden eingeengt, in Methanol gelöst und mit einem Überschuss an 1 N Salzsäure versetzt. Das Lösungsmittel wird unter reduziertem Druck entfernt. Nach Trocknen im Hochvakuum werden 393 mg (59 % d.Th.) der Titelverbindung erhalten.
¹H-NMR (200 MHz, DMSO-d₆): δ = 10.45 (s, 1H), 9.10 (d, 1H), 7.91 (s, 1H), 7.87 (d, 1H), 7.75-7.55 (m, 2H), 7.40 (t, 1H), 7.18 (d, 2H), 4.40 (m, 1H), 3.80 (m, 4H), 3.75-3.00 (m, 6H), 3.20 (m, 4H), 2.30-2.02 (m, 2H), 2.00-1.62 (m, 3H).
HPLC (Methode 1): Rₜ = 3.79 min.
MS (ESIpos): m/z = 432 (M+H)⁺ (freie Base).

### Beispiel 93

### N-[(3R)-1-Azabicyclo[2.2.2]oct-3-yl]-5-[4-(4-morpholinyl)phenyl]-1-benzofuran-2-carboxamid-Hydrochlorid

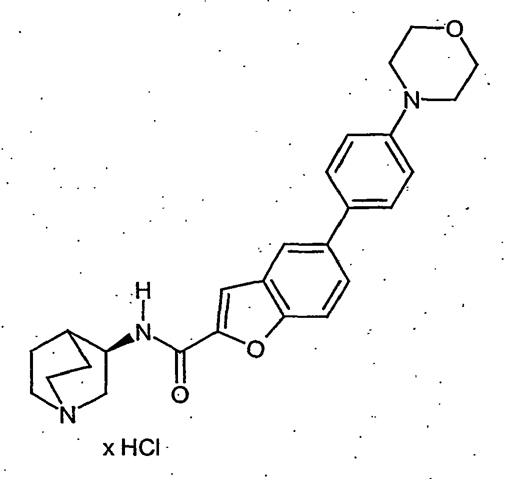

Zu einer Mischung aus 250 mg (0.72 mmol) *N*-[(3R)-1-Azabicyclo[2.2.2]oct-3-yl]-5-brom-1-benzofuran-2-carboxamid (Beispiel 3A) und 52 mg (0.07 mmol) PdCl₂(dppf) in 3 mL DMF werden 177 mg (0.86 mmol) 4-(4-Morpholinyl)phenylboronsäure und 2.15 mL 1 N Natronlauge gegeben. Das Reaktionsgemisch wird über Nacht auf 90°C erhitzt. Das Solvens wird unter reduziertem Druck entfernt und das Rohprodukt in Methanol aufgenommen und über Kieselgur filtriert. Die Reinigung erfolgt durch präparative HPLC. Die Produktfraktionen werden eingeengt, in Acetonitril/Wasser gelöst und mit einem Überschuss an 1 N Salzsäure versetzt. Das Lösungsmittel wird unter reduziertem Druck entfernt. Nach Trocknen im Hochvakuum werden 23 mg (7% d.Th.) der Titelverbindung erhalten.
¹H-NMR (200 MHz, DMSO-d₆): δ = 10.40 (s, 1H), 9.15 (d, 1H), 7.98 (s, 1H), 7.76-7.68 (m, 2H), 7.67-7.58 (m, 2H), 7.20-7.10 (d, 2H), 4.45 (m, 1H), 3.80 (m, 4H), 3.75-3.30 (m, 6H), 3.20 (m, 4H), 2.30-2.02 (m, 2H), 2.00-1.62 (m, 3H).
HPLC (Methode 1): Rₜ = 3.52 min.
MS (ESIpos): m/z = 432 (M+H)⁺ (freie Base).

### Beispiel 94

### N-[(3R)-1-Azabicyclo[2.2.2]oct-3-yl]-5-[4-(hydroxymethyl)phenyl]-1-benzofuran-2-carboxamid

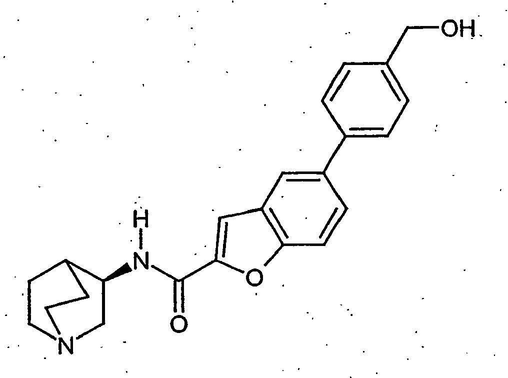

Zu einer Mischung aus 170 mg (0.49 mmol) *N*-[(3R)-1-Azabicyclo[2:2.2]oct-3-yl]-5-brom-1-benzofuran-2-carboxamid (Beispiel 3A) und 35 mg (0.05 mmol) PdCl₂(dppf) in 2 mL DMF werden 110 mg (0.73 mmol) 4-(Hydroxymethyl)phenylboronsäure und 1.46 mL 1 N Natronlauge gegeben. Das Reaktionsgemisch wird über Nacht auf 85°C erhitzt. Das Solvens wird unter reduziertem Druck entfernt. Nach Zugabe einer Mischung aus 1 N Natronlauge und Essigsäureethylester zum Rückstand wird die wässrige Phase nochmals mit Essigsäureethylester extrahiert. Die vereinigten organischen Phasen werden je zweimal mit 1 N Natronlauge und gesättigter Natriumchlorid-Lösung gewaschen, über Magnesiumsulfat getrocknet und unter reduziertem Druck am Rotationsverdampfer eingeengt. Das Rohprodukt wird in Methanol aufgenommen und zusammen mit saurem Ionenaustauscher (Dowex^{®} WX2-200) etwa 20 min. lang geschüttelt. Der beladene Ionenaustauscher wird dreimal mit je 30 mL Methanol, dann mit DMF, erneut mit Methanol, mit Dichlormethan, erneut mit Methanol, mit Wasser und schließlich wieder mit Methanol gewaschen. Das Produkt wird mit Methanol-Triethylamin 95:5 eluiert. Das Solvens wird unter reduziertem Druck am Rotationsverdampfer entfernt. Es werden 148 mg (80 % d.Th.) der Titelverbindung isoliert.
¹H-NMR (200 MHz, CDCl₃): δ = 7.85 (s, 1H), 7.70-7.35 (m, 7H), 6.77 (d, 1H), 4.62 (s, 2H), 4.28-4.12 (m, 1H), 3.56-3.38 (m, 1H), 3.04-2.78 (m, 4H), 2.75-2.59 (m, 1H), 2.16-2.02 (m, 1H), 1.93-1.66 (m, 3H), 1.66-1.50 (m, 1H).
HPLC (Methode 1): Rₜ = 3.65 min.
MS (ESIpos): m/z = 377 (M+H)⁺.

### Beispiel 95

### N-[(3R)-1-Azabicyclo[2.2.2]oct-3-yl]-5-[2-(hydroxymethyl)phenyl]-1-benzofuran-2-carboxamid

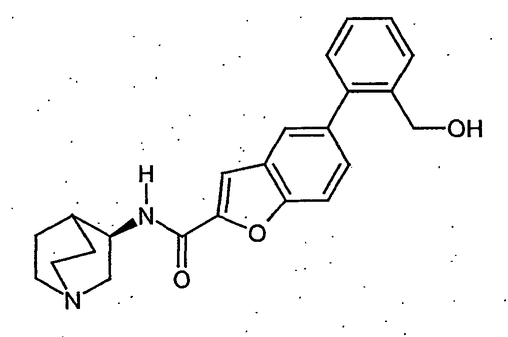

Zu einer Mischung aus 170 mg (0.49 mmol) *N*-[(3R)-1-Azabicyclo[2.2.2]oct-3-yl]-5-brom-1-benzofuran-2-carboxamid (Beispiel 3A) und 35 mg (0.05 mmol) PdCl₂(dppf) in 2 mL DMF werden 110 mg (0.73 mmol) 2-(Hydroxymethyl)phenylboronsäure und 1.46. mL 1 N Natronlauge gegeben. Das Reaktionsgemisch wird über Nacht auf 85°C erhitzt. Das Solvens wird unter reduziertem Druck entfernt. Nach Zugabe einer Mischung aus 1 N Natronlauge und Essigsäureethylester zum Rückstand wird die wässrige Phase nochmals mit Essigsäureethylester extrahiert. Die vereinigten organischen Phasen werden je zweimal mit 1 N Natronlauge und gesättigter Natriumchlorid-Lösung gewaschen, über Magnesiumsulfat getrocknet und unter reduziertem Druck am Rotationsverdampfer eingeengt. Das Rohprodukt wird in Methanol aufgenommen und zusammen mit saurem Ionenaustauscher (Dowex^{®} WX2-200) etwa 20 min. lang geschüttelt. Der beladene Ionenaustauscher wird dreimal mit je 30 mL Methanol, dann mit DMF, erneut mit Methanol, mit Dichlormethan, erneut mit Methanol, mit Wasser und schließlich wieder mit Methanol gewaschen. Das Produkt wird mit Methanol-Triethylamin 95:5 eluiert. Das Solvens wird unter reduziertem Druck am Rotationsverdampfer entfernt. Es werden 140 mg (76 % d.Th.) der Titelverbindung isoliert.
¹H-NMR (300 MHz, CDCl₃): δ = 7.70-7.25 (m, 8H), 6.75 (d, 1H), 4.62 (s, 2H), 4.28-4.12 (m, 1H), 3.56-3.38 (m, 1H), 3.04-2.78 (m, 4H), 2.75-2.59 (m, 1H), 2.16-2.02 (m, 1H), 1.93-1.66 (m, 3H), 1.66-1.50 (m, 1H).
HPLC (Methode 1): Rₜ = 3.76 min.
MS (ESIpos): m/z = 377 (M+H)⁺.

### Beispiel 96

### N-[(3R)-1-Azabicyclo[2.2.2]oct-3-yl]-5-[4-(dimethylamino)phenyl]-1-benzofuran-2-carboxamid

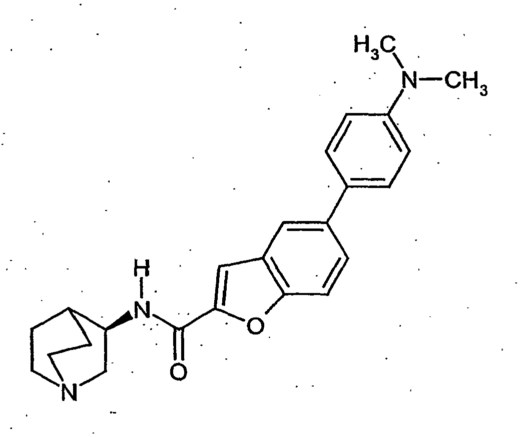

Zu einer Mischung aus 170 mg (0.49 mmol) *N*-[(3R)-1-Azabicyclo[2.2.2]oct-3-yl]-5-brom-1-benzofuran-2-carboxamid (Beispiel 3A) und 35 mg (0.05 mmol) PdCl₂(dppf) in 2 mL DMF werden 120 mg (0.73 mmol) 4-(Dimethylamino)phenylboronsäure und 1.46 mL 1 N Natronlauge gegeben. Das Reaktionsgemisch wird über Nacht auf 85°C erhitzt. Das Solvens wird unter reduziertem Druck entfernt. Nach Zugabe einer Mischung aus 1 N Natronlauge und Essigsäureethylester zum Rückstand wird .die wässrige Phase nochmals mit Essigsäureethylester extrahiert. Die vereinigten organischen Phasen werden je zweimal mit 1 N Natronlauge und gesättigter Natriumchlorid-Lösung gewaschen, über Magnesiumsulfat getrocknet und unter reduziertem Druck am Rotationsverdampfer eingeengt. Das Rohprodukt wird in Methanol aufgenommen und zusammen mit saurem Ionenaustauscher (Dowex^{®} WX2-200) etwa 20 min. lang geschüttelt. Der beladene Ionenaustauscher wird dreimal mit je 30 mL Methanol, dann mit DMF, erneut mit Methanol, mit Dichlormethan, erneut mit Methanol, mit Wasser und schließlich wieder mit Methanol gewaschen. Das Produkt wird mit Methanol-Triethylamin 95:5 eluiert. Das Solvens wird unter reduziertem Druck am Rotationsverdampfer entfernt. Es werden 138 mg (73 % d.Th.) der Titelverbindung isoliert.
¹H-NMR (200 MHz, CDCl₃): δ = 7.78 (d, 1H) 7.70-7.39 (m, 4H), 6.88-6.75 (m, 3H), 4.28-4.12 (m, 1H), 3.56-3.38 (m, 1H), 3.04-2.78 (m, 4H), 3.00 (s, 6H), 2.75-2.59 (m, 1H), 2.16-2.02 (m, 1H), 1.93-1.66 (m, 3H), 1.66-1.50 (m, 1H).
HPLC (Methode 1): Rₜ = 3.36 min.
MS (ESIpos): m/z = 390 (M+H)⁺.

### Beispiel 97

### N-[(3R)-1-Azabicyclo[2.2.2]oct-3-yl]-5-[4-(methoxy)phenyl]-1-benzofuran-2-carboxamid-Hydrochlorid

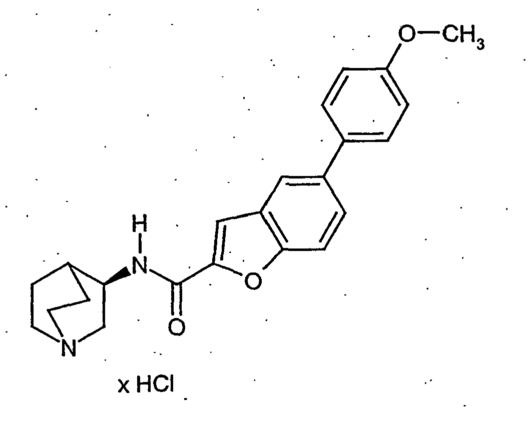

Zu einer Mischung aus 250 mg (0.72 mmol) *N*-[(3R)-1-Azabicyclo[2.2.2]oct-3-yl]-5-brom-1-benzofuran-2-carboxamid (Beispiel 3A) und 52 mg (0.07 mmol) PdCl₂(dppf) in 3 mL DMF werden 130 mg (0.86 mmol) 4-(Methoxy)phenylboronsäure und 2.15 mL 1 N Natronlauge gegeben. Das Reaktionsgemisch wird über Nacht auf 90°C erhitzt. Das Solvens wird unter reduziertem Druck entfernt und das Rohprodukt in Methanol aufgenommen und über Kieselgur filtriert. Die Reinigung erfolgt durch präparative HPLC. Die Produktfraktionen werden mit einem Überschuss an 1 N Salzsäure versetzt. Das Lösungsmittel wird unter reduziertem Druck entfernt. Nach Trocknen im Hochvakuum werden 127 mg (39 % d.Th.) der Titelverbindung erhalten.
¹H-NMR (200 MHz, DMSO-d₆): δ = 9.90 (s, 1H), 9.10 (d, 1H), 7.95 (m, 2H), 7.75-7.60 (m, 4H), 7.10-7.02 (m, 2H), 4.40 (m, 1H), 3.85 (m, 3H), 3.75-3.00 (m, 6H), 3.20 (m, 4H), 2.30-2.02 (m, 2H), 2.00-1.62 (m, 3H).
HPLC (Methode 1): Rₜ = 4.15 min.
MS (ESIpos): m/z = 377 (M+H)⁺ (freie Base).

### Beispiel 98

### N-[(3R)-1-Azabicyclo[2.2.2]oct-3-yl]-5-[3-(methoxy)phenyl]-1-benzofuran-2-carboxamid-Hydrochlorid

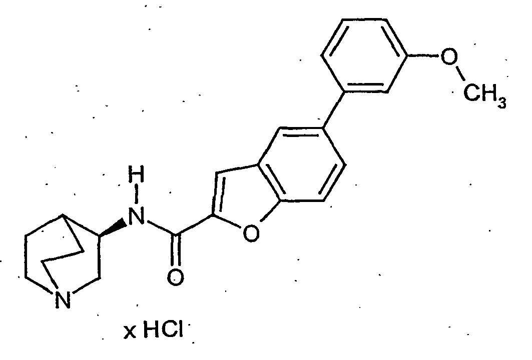

Zu einer Mischung aus 250 mg (0.72 mmol) *N*-[(3R)-1-Azabicyclo[2.2.2]oct-3-yl]-5-brom-1-benzofuran-2-carboxamid (Beispiel 3A) und 52 mg (0.07 mmol) PdCl₂(dppf) in 3 mL DMF werden 130 mg (0.86 mmol) 3-(Methoxy)phenylboronsäure und 2.15 mL 1 N Natronlauge gegeben. Das Reaktionsgemisch wird über Nacht auf 90°C erhitzt. Das Solvens wird unter reduziertem Druck entfernt und das Rohprodukt in Methanol aufgenommen und über Kieselgur filtriert. Die Reinigung erfolgt durch präparative HPLC. Die Produktfraktionen werden mit einem Überschuss an 1 N Salzsäure versetzt. Das Lösungsmittel wird unter reduziertem Druck entfernt. Nach Trocknen im Hochvakuum werden 208 mg (63 % d.Th.) der Titelverbindung erhalten. ¹H-NMR (300 MHz, DMSO-d₆): δ = 10.0 (s, 1H), 9.10 (d, 1H), 8.05 (s, 1H), 7.80-7.65 (m, 3H), 7.42-7.18 (m, 3H), 6.93 (m, 1H), 4.35 (m, 1H), 3.85 (s, 3H), 3.75-3.00 (m, 6H), 3.20 (m, 4H), 2.30-2.02 (m, 2H), 2.00-1.62 (m, 3H).
HPLC (Methode 1): Rₜ = 4.19 min.
MS (ESIpos): m/z = 377 (M+H)⁺ (freie Base).

### Beispiel 99

### N-[(3R)-1-Azabicyclo[2.2.2]oct-3-yl]-7-[4-(methoxy)phenyl]-1-benzofuran-2-carboxamid-Hydrochlorid

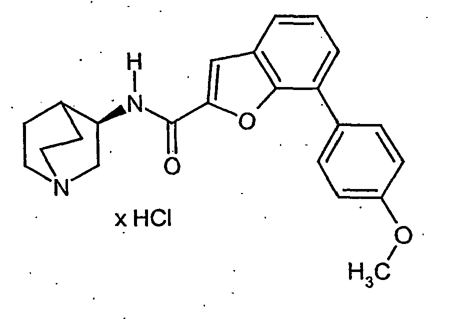

Zu einer Mischung aus 300 mg (0.86 mmol) *N*-[(3R)-1-Azabicyclo[2.2.2]oct-3-yl]-7-brom-1-benzofuran-2-carboxamid (Beispiel 30A) und 63 mg (0.09 mmol) PdCl₂(dppf) in 4 mL DMF werden 130 mg (0.86 mmol) 4-(Methoxy)phenylboronsäure und 2.58 mL 1 N Natronlauge gegeben. Das Reaktionsgemisch wird über Nacht auf 95°C erhitzt. Das Solvens wird unter reduziertem Druck entfernt und das Rohprodukt in Methanol aufgenommen und über Kieselgur filtriert. Die Reinigung erfolgt durch präparative HPLC. Die Produktfraktionen werden mit einem Überschuss an 1 N Salzsäure versetzt. Das Lösungsmittel wird unter reduziertem Druck entfernt. Nach Trocknen im Hochvakuum werden 165 mg (47 % d.Th.) der Titelverbindung erhalten.
¹H-NMR (300 MHz, DMSO-d₆): δ = 10.4 (s, 1H), 9.00 (d, 1H), 8.05 (s, 1H), 7.90-7.82. (m, 3H), 7.75 (d, 1H), 7.62 (d, 1H), 7.40 (t, 1H), 7.15-7.05 (m, 2H), 4.35 (m, 1H), 3.85 (s, 3H), 3.65 (m, 1H), 3.48-3.30 (m, 2H), 3.25-3.10 (m, 3H), 2.25 (m, 1H), 2.18-2.05 (m, 1H), 1.98-1.88 (m, 2H), 1.80-1.63 (m, 1H).
HPLC (Methode 1): Rₜ = 4.18 min.
MS (ESIpos): m/z = 377 (M+H)⁺ (freie Base).

### Beispiel 100

### N-[(3S)-1-Azabicyclo[2.2.2]oct-3-yl]-7-(2-methoxyphenyl)-1-benzofuran-2-carboxamid-Hydrochlorid

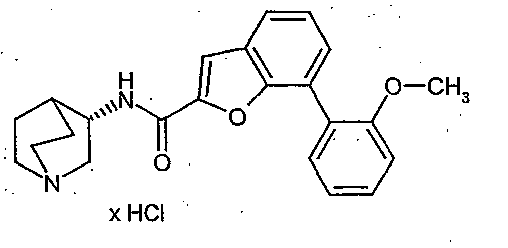

Zu einer Mischung aus 890 mg (4.47 mmol) *S*-3-Aminochinuclidin-Dihydrochlorid und 1000 mg (3.73 mmol) 7-(2-Methoxyphenyl)-1-benzofuran-2-carbonsäure (Beispiel 21A) in 10 mL DMF werden bei 0°C 3.4 g (8.95 mmol) HATU und 2.34 mL (13.42 mmol) *N,N*-Diisopropylethylamin gegeben. Nach 18 h Rühren bei Raumtemperatur wird die Reaktionslösung mittels präparativer HPLC aufgereinigt. Die Produktfraktionen werden eingeengt, mit 5 mL 1 N Salzsäure versetzt und erneut eingeengt. Nach Trocknen im Hochvakuum erhält man 209 mg (13.6 % d.Th.) der Titelverbindung.
Die analytischen Daten stimmen mit denen der enantiomeren Verbindung (Beispiel 102) überein.

### Beispiel 101

### N-[(3R)-1-Azabicyclo[2.2.2]oct-3-yl]-7-[3-(methoxy)phenyl]-1-benzofuran-2-carboxamid-Hydrochlorid

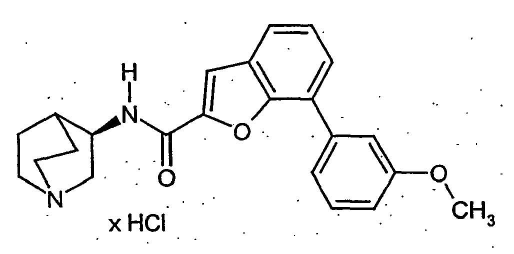

Zu einer Mischung aus 150 mg (0.43 mmol) *N*-[(3R)-1-Azabicyclo[2.2.2]oct-3-yl]-7-brom-1-benzofuran-2-carboxamid (Beispiel 30A) und 31 mg (0.04 mmol) PdCl₂(dppf) in 2 mL DMF werden 98 mg (0.64 mmol) 3-(Methoxy)phenylboronsäure und 1.29 mL 1 N Natronlauge gegeben. Das Reaktionsgemisch wird über Nacht auf 90°C erhitzt. Das Solvens wird unter reduziertem Druck entfernt und das Rohprodukt in Methanol aufgenommen und über Kieselgur filtriert. Die Reinigung erfolgt durch präparative HPLC. Das Produkt wird in Methanol gelöst und mit einem Überschuss an 1 N Salzsäure versetzt. Das Lösungsmittel wird unter reduziertem Druck entfernt und der Rückstand aus wenig Isopropanol umkristallisiert. Nach Trocknen im Hochvakuum werden 159 mg (85 % d.Th.) der Titelverbindung erhalten.
¹H-NMR (400 MHz, DMSO-d₆): δ = 10.05 (s, 1H), 8.95 (d, 1H), 7.85 (s, 1H), 7.80 (d, 1H), 7.70 (d, 1H), 7.50-7.40 (m, 4H), 7.00 (m, 1H), 4.35 (m, 1H), 3.85 (s, 3H), 3.65 (m, 1H), 3.48-3.30 (m, 2H), 3.25-3.15 (m, 3H), 2.25 (m, 1H), 2.18-2.05 (m, 1H), 1.98-1.88 (m, 2H), 1.80-1.63 (m, 1H).
HPLC (Methode 1): Rₜ = 4.21 min.
MS (ESIpos): m/z = 377 (M+H)⁺ (freie Base).

### Beispiel 102

### N-[(3R)-1-Azabicyclo[2.2.2]oct-3-yl]-7-[2-(methoxy)phenyl]-1-benzofüran-2-carboxamid-Hydrochlorid

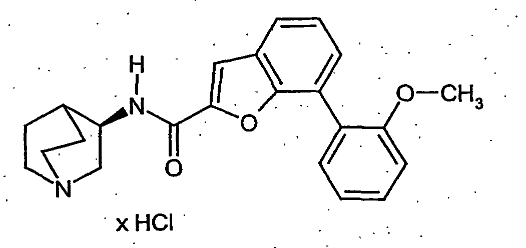

### Methode a):

Zu einer Mischung aus 150 mg (0.43 mmol) *N*-[(3R)-1-Azabicyclo[2.2.2]oct-3-yl]-7-brom-1-benzofuran-2-carboxamid (Beispiel 30A) und 31 mg (0.04 mmol) PdCl₂(dppf) in 2 mL DMF werden 98 mg (0.64 mmol) 2-(Methoxy)phenylboronsäure und 1.29 mL 1 N Natronlauge gegeben. Das Reaktionsgemisch wird über Nacht auf 85°C erhitzt. Das Solvens wird unter reduziertem Druck entfernt und das Rohprodukt in Methanol aufgenommen und über Kieselgur filtriert. Die weitere Reinigung erfolgt durch präparative HPLC. Das Produkt wird in Methanol gelöst und mit einem Überschuss an 1 N Salzsäure versetzt. Das Lösungsmittel wird unter reduziertem Druck entfernt und der Rückstand aus wenig Isopropanol umkristallisiert. Nach Trocknen im Hochvakuum werden 100 mg (62 % d.Th.) der Titelverbindung erhalten.
¹H-NMR (400 MHz, DMSO-d₆): δ = 10.08 (s, 1H), 8.91 (d, 1H), 7.87 (s, 1H), 7.84-7.74 (m, 2H), 7.53-7.33 (m, 3H), 7.25-7.00 (m, 2H), 4.35 (m, 1H), 3.75 (s, 3H), 3.65 (m, 1H), 3.48-3.30 (m, 2H), 3.25-3.15 (m, 3H), 2.25 (m; 1H), 2.18-2.05 (m, 1H), 1.98-1.88 (m, 2H), 1.80-1.63 (m, 1H).
HPLC (Methode 1): Rₜ = 4.16 min.
MS (ESIpos): m/z = 377 (M+R)⁺ (freie Base).

### Methode b):

Zu einer Lösung von 2.92 g (10.9 mmol) 7-(2-Methoxyphenyl)-1-benzofuran-2-carbonsäure (Beispiel 21A) und 2.17 g (10.9 mmol) *(R)*-3-Aminochinuclidin-Dihydrochlorid in 35 mL DMF werden bei 0°C 2.51 g (13.1 mmol) EDC, 1.77 g (13.1 mmol) HOBt und 5.47 mL (39.2 mmol) Triethylamin hinzugefügt. Nach 18-stündigem Rühren bei Raumtemperatur werden weitere 434 mg (2.2 mmol) *(R)*-3-Aminochinuclidin-Dihydrochlorid sowie 418 mg (2.2 mmol) EDC zugegeben. Nach 2 h bei 55°C wird die Reaktionslösung eingeengt und der Rückstand zwischen je 200 mL Essigsäureethylester und 2 N Natronlauge verteilt. Die organische Phase wird 15-mal mit je 100 mL 2 N Natronlauge gewaschen. Die vereinigten wässrigen Phasen werden mit 250 mL Essigsäureethylester rückextrahiert. Die vereinigten organischen Phasen werden über Natriumsulfat getrocknet, eingeengt, der Rückstand mit einer 5:1-Mischung aus Methanol und 1 N Salzsäure versetzt, erneut eingeengt und im Hochvakuum getrocknet. Nach Umkristallisation des Rückstands aus 10 mL eines 10:1-Gemisches aus Isopropanol und Ethanol werden 2.73 g (60.5 % d.Th.) der Titelverbindung erhalten.

### Beispiel 103

### N-[(3R)-1-Azabicyclo[2.2.2]oct-3-yl]-7-[4-(methoxy)-3-pyridinyl]-1-benzofuran-2-carboxamid-Hydrochlorid

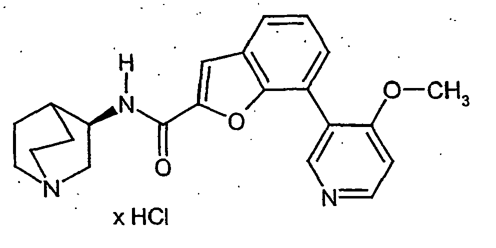

Zu einer Mischung aus 100 mg (0.29 mmol) *N*-[(3R)-1-Azabicyclo[2.2.2]oct-3-yl]-7-brom-1-benzofuran-2-carboxamid (Beispiel 30A) und 21 mg (0.03 mmol) PdCl₂(dppf) in 2 mL DMF werden 98 mg (0.64 mmol) 4-Methoxy-3-pyridinylboronsäure und 0.86 mL 1 N Natronlauge gegeben. Das Reaktionsgemisch wird über Nacht auf 85°C erhitzt. Das Solvens wird unter reduziertem Druck entfernt und das Rohprodukt in Methanol aufgenommen und über Kieselgur filtriert. Die Reinigung erfolgt durch präparative HPLC. Die Produktfraktionen werden mit einem Überschuss an 1 N Salzsäure versetzt. Das Lösungsmittel wird unter reduziertem Druck entfernt. Nach Trocknen im Hochvakuum werden 58 mg (49 % d.Th.) der Titelverbindung erhalten.
¹H-NMR (400 MHz, DMSO-d₆): δ = 10.55 (s, 1H), 9.20 (d, 1H), 9.05 (s, 1H), 8.95 (d, 1H), 7.96-7.89 (m, 2H), 7.82 (d, 1H), 7.65 (d, 1H), 7.50 (t, 1H), 4.35 (m, 1H), 4.10 (s, 3H), 3.65-3.15 (m, 6H), 2.20 (m, 1H), 2.18-2.05 (m, 1H), 1.98-1.88 (m, 2H), 1.80-1.63 (m, 1H).
HPLC (Methode 1): Rₜ = 3.35 min.
MS (ESIpos): m/z = 378 (M+H)⁺ (freie Base).

### Beispiel 104

### N-[(3R)-1-Azabicyclo[2.2.2]oct-3-yl]-7-[4-(4-morpholinylcarbonyl)phenyl]-1-benzofuran-2-carboxamid-Hydrochlorid

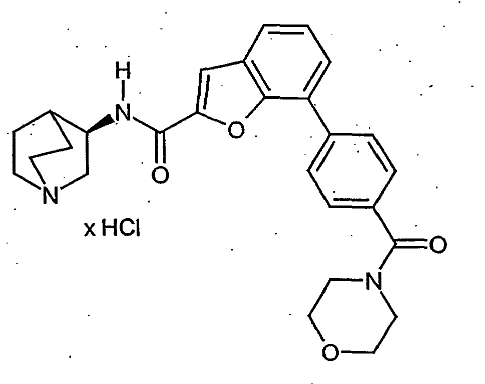

Gemäß der allgemeinen Arbeitsvorschrift D werden 631 mg (1.43 mmol) 4-(4-Morpholinylcarbonyl)phenyl-trifluormethansulfonat (Beispiel 18A), 545 mg (2.15 mmol) Bis(pinacolato)dibor, 456 mg (4.65 mmol) Kaliumacetat, 52 mg (0.07 mmol) PdCl₂(dppf), 500 mg (1.43 mmol) *N*-[(3R)-1-Azabieyclo[2.2.2]oct-3-yl]-7-brom-1-benzofuran-2-carboxamid (Beispiel 30A), 3.6 mL 2 M Natriumcarbonat-Lösung und weitere 52 mg (0.07 mmol) PdCl₂(dppf) in 8 mL DMF umgesetzt. Nach Trocknen im Hochvakuum werden 455 mg (61 % d.Th.) der Titelverbindung erhalten.
¹H-NMR (400 MHz, DMSO-d₆): δ = 10.45 (s, 1H), 9.10 (d, 1H), 8.02-7.92 (m, 3H), 7.78 (d, 1H), 7.71 (d, 1H), 7.60 (d, 2H), 7.45 (t, 1H), 4.35 (m, 1H), 3.75-3.35 (m, 11H), 3.25-3.15 (m, 3H), 2.25 (m, 1H), 2.18-2.05 (m, 1H), 1.98-1.88 (m, 2H), 1.80-1.63 (m, 1H).
BPLC (Methode 1): Rₜ = 3.79 min.
MS (ESIpos): m/z = 460 (M+H)⁺ (freie Base).

### Beispiel 105

### N-[(3R)-1-Azabicyclo[2.2.2]oct-3-yl]-7-[3-(1-piperidinyl)phenyl]-1-benzofuran-2-carboxamid-Dihydrochlorid

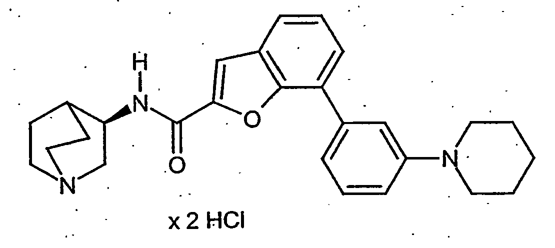

Zu einer Mischung aus 300 mg (0.86 mmol) *N*-[(3R)-1-Azabicyclo[2.2.2]oct-3-yl]-7-brom-1-benzofuran-2-carboxamid (Beispiel 30A) und' 63 mg (0.09 mmol) PdCl₂(dppf) in 4 mL DMF werden 311 mg (1.29 mmol) 3-(1-Piperidinyl)phenylboronsäure und 3.44 mL 1 N Natronlauge gegeben. Das Reaktionsgemisch wird über Nacht auf 95°C erhitzt. Das Solvens wird unter reduziertem Druck entfernt und das Rohprodukt in Methanol aufgenommen und über Kieselgur filtriert. Die Reinigung erfolgt durch präparative HPLC. Das Produkt wird in Methanol gelöst und mit einem Überschuss an 1 N Salzsäure versetzt. Das Lösungsmittel wird unter reduziertem Druck entfernt. Nach Trocknen im Hochvakuum werden 164 mg (41 % d.Th.) der Titelverbindung erhalten.
¹H-NMR (200 MHz, DMSO-d₆): δ = 10.55 (s, 1H), 9.10 (d, 1H), 8.75 (s, 1H), 7.95-7.63 (m, 7H), 7.45 (t, 1H), 4.35 (m, 1H), 4.13-3.40 (m, 7H), 3.35-3.10 (m, 3H), 2.15-1.50 (m, 11H).
HPLC (Methode 1): Rₜ = 3.72 min.
MS (ESIpos): m/z 430 (M+H)⁺ (freie Base).

### Beispiel 106

### N-[(3R)-1-Azabicyclo[2.2.2]oct-3-yl]-7-(3-pyridinyl)-1-benzofuran-2-carboxamid-Dihydrochlorid

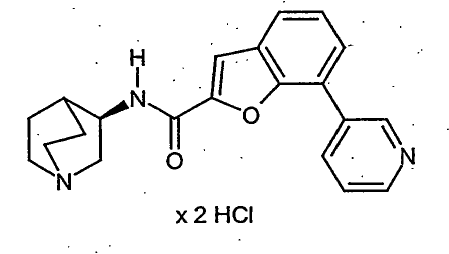

Zu einer Mischung aus 100 mg (0.86 mmol) *N*-[(3R)-1-Azabicyclo[2.2.2]oct-3-yl]-7-brom-1-benzofuran-2-carboxamid (Beispiel 30A) und 63 mg (0.09 mmol) PdCl₂(dppf) in 4 mL DMF werden 70 mg (0.57 mmol) 3-Pyridinboronsäure und 0.86 mL 1 N Natronlauge gegeben. Das Reaktionsgemisch wird über Nacht auf 95°C erhitzt. Das Solvens wird unter reduziertem Druck entfernt und das Rohprodukt in Methanol aufgenommen und über Kieselgur filtriert. Die Reinigung erfolgt durch präparative HPLC. Das Produkt wird in Methanol gelöst und mit einem Überschuss an 1 N Salzsäure versetzt. Das Lösungsmittel wird unter reduziertem Druck entfernt. Nach Trocknen im Hochvakuum werden 49 mg (45 % d.Th.) der Titelverbindung erhalten.
¹H-NMR (300 MHz, DMSO-d₆): δ = 10.55 (s, 1H), 9.51 (s, 1H), 9.30 (d, 1H), 9.20 (s, 1H), 8.94-8.80 (m, 2H), 8.07-7.80 (m, 4H), 7.55 (t, 1H), 4.35 (m, 1H), 3.65-3.15 (m, 6H), 2.20 (m, 1H), 2.18-2.05 (m, 1H), 1.98-1.88 (m, 2H), 1.80-1.63 (m, 1H). HPLC (Methode 1): Rₜ = 3.27 min.
MS (ESIpos): m/z = 348 (M+H)⁺ (freie Base).

### Beispiel 107

### N-[(3R)-1-Azabicyclo[2.2.2]oct-3-yl]-7-(3-{[(methylamino)carbonyl]amino}phenyl)-1-benzofuran-2-carboxamid-Hydrochlorid

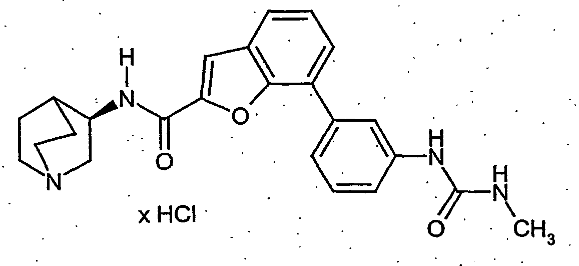

63 mg (0.18 mmol) 7-(3-Aminophenyl)-*N*-[(3R)-1-azabicyclo[2.2.2]oct-3-yl]-1-benzofuran-2-carboxamid (Beispiel 114), 40 mg (0.70 mmol) Methylisocyanat und 0.12 mL (0.88 mmol) Triethylamin werden in 3 mL THF/DMF (1:1) über Nacht auf 40°C erhitzt/Es werden weitere 40 mg (0.70 mmol) Methylisocyanat und eine katalytische Menge DMAP zugegeben und über Nacht auf 50°C erhitzt. Nach Abkühlen wird mit Wasser versetzt, filtriert und das Lösungsmittel unter reduziertem Druck entfernt. Die Reinigung erfolgt durch präparative HPLC. Das Produkt wird in Methanol gelöst und mit einem Überschuss an 1 N Salzsäure versetzt. Das Lösungsmittel wird unter reduziertem Druck entfernt. Nach Trocknen im Hochvakuum werden 18 mg (23 % d.Th.) der Titelverbindung erhalten.
¹H-NMR (400 MHz, DMSO-d₆): δ = 9.98 (s, 1H), 8.85 (s, 1H), 8.63 (d, 1H), 8.15 (s, 1H), 7.80 (m, 2H), 7.65 (d, 1H), 7.45 (t, 1H), 7.38 (s, 2H), 6.20 (m, 1H), 4.35 (m, 1H), 3.75-3.63 (m, 1H), 3.60-3.15 (m, 5H), 2.65 (s, 3H), 2.30 (m, 1H), 2.18-2.05 (m, 1H), 1.98-1..88 (m, 2H), 1.80-1.63 (m, 1H).
HPLC (Methode 1): Rₜ = 3.89 min.
MS (ESIpos): m/z = 419 (M+H)⁺ (freie Base).

### Beispiel 108

### N-[(3R)-1-Azabicyclo[2.2.2]oct-3-yl]-7-(3-{[(ethylamino)carbonyl]amino}phenyl)-1-benzofuran-2-carboxamid-Hydrochlorid

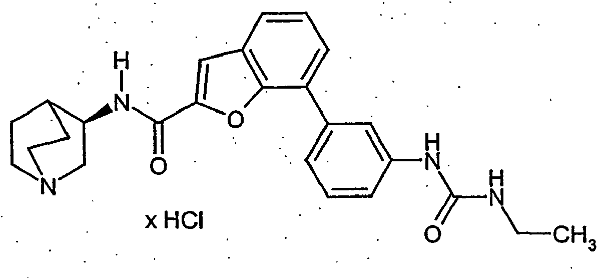

63 mg (0.18 mmol) 7-(3-Aminophenyl)-*N*-[(3R)-1-azabicyclo[2.2.2]oct-3-yl]-1-benzofuran-2-carboxamid (Beispiel 114), 50 mg (0.70 mmol) Ethylisocyanat und 0.12 mL (0.88 mmol) Triethylamin werden in 3 mL THF/DMF (1:1) über Nacht auf 40°C erhitzt. Es werden weitere 50 mg (0.70 mmol) Ethylisocyanat und eine katalytische Menge DMAP zugegeben und über Nacht auf 50°C erhitzt. Nach Abkühlen wird mit Wasser versetzt, filtriert und das Lösungsmittel unter reduziertem Druck entfernt. Die Reinigung erfolgt durch präparative HPLC. Das Produkt wird in Methanol gelöst und mit einem Überschuss an 1 N Salzsäure versetzt. Das Lösungsmittel wird unter reduziertem Druck entfernt. Nach Trocknen im Hochvakuum werden 15 mg (18 % d.Th.) der Titelverbindung erhalten.
¹H-NMR (400 MHz, DMSO-d₆): δ = 9.80 (s, 1H), 8.74 (s, 1H), 8.63 (d, 1H), 8.10 (s, 1H), 7.80 (m, 2H), 7.60 (d, 1H), 7.45 (t, 1H), 7.38 (s, 2H), 6.20 (m, 1H), 4.35 (m, 1H), 3.75-3.63 (m, 1H), 3.60-3.15 (m, 5H), 3.10 (m, 2H), 2.65 (s, 3H), 2.30 (m, 1H), 2.18-2.05 (m,1H), 1.98-1.88 (m, 2H), 1.80-1.63 (m, 1H), 1.05 (t, 3H).
HPLC (Methode 1): Rₜ = 4.01 min.
MS (ESIpos): m/z = 433 (M+H)⁺ (freie Base).

### Beispiel 109

### N-[(3R)-1-Azabicyclo[2.2.2]oct-3-yl]-7-[3-({[(1-Methylethyl)amino]carbonyl}-amino)phenyl]-1-benzofuran-2-carboxamid-Hydrochlorid

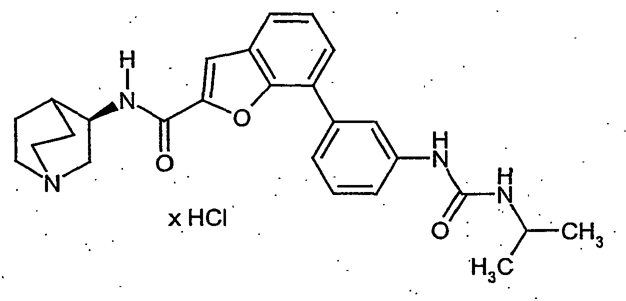

50 mg (0.14 mmol) 7-(3-Aminophenyl)-*N*-[(3R)-1-azabicyclo[2.2.2]oct-3-yl]-1-benzofuran-2-carboxamid (Beispiel 114), 47 mg (0.55 mmol) Isopropylisocyanat und 0.12 mL (0.88 mmol) Triethylamin werden in 3 mL THF/DMF (1:1) über Nacht auf 40°C erhitzt. Es werden weitere 47 mg (0.55 mmol) Isopropylisocyanat und eine katalytische Menge DMAP zugegeben und über Nacht auf 50°C erhitzt. Nach Abkühlen wird mit Wasser versetzt, filtriert und das Lösungsmittel unter reduziertem Druck entfernt. Die Reinigung erfolgt durch präparative HPLC. Das Produkt wird in Methanol gelöst und mit einem Überschuss an 1 N Salzsäure versetzt. Das Lösungsmittel wird unter reduziertem Druck entfernt. Nach Trocknen im Hochvakuum werden 13 mg (18% d.Th.) der Titelverbindung erhalten.
¹H-NMR (400 MHz, DMSO-d₆): δ = 9.80 (s, 1H), 8.70 (d, 1H), 8.65 (s, 1H), 8.05 (s, 1H), 7.80 (m, 2H), 7.60 (d, 1H), 7.45 (t, 1H), 7.38 (s, 2H), 6.20 (m, 1H), 4.35 (m, 1H), 3.80-3.72 (m, 1H), 3.70-3.63 (m, 1H), 3.50-3.05 (m, 5H), 2.30 (m, 1H), 2.18-2.05 (m, 1H), 1.98-1.88 (m, 2H), 1.80-1.63 (m, 1H), 1.10 (d, 6H).
HPLC (Methode 1): Rₜ = 4.12 min.
MS (ESIpos): m/z = 447 (M+H)⁺ (freie Base).

### Beispiel 110

### N-[(3R)-1-Azabicyclo[2.2.2]oct-3-yl]-7-[3-({[(1,1-dimethylethyl)amino]carbonyl}-amino)phenyl]-1-benzöfuran-2-carboxamid-Hydrochlorid

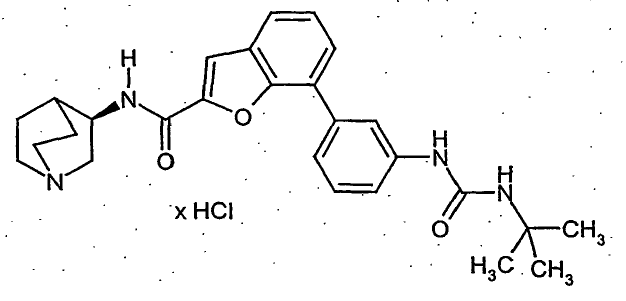

63 mg (0.14 mmol) 7-(3-Aminophenyl)-*N*-[(3R)-1-azabicyclo[2.2.2]oct-3-yl]-1-benzofuran-2-carboxamid (Beispiel 114), 69 mg (0.70 mmol) tert.-Butylisocyanat und 0.12 mL (0.88 mmol) Triethylamin werden in 3 mL THF/DMF (1:1) über Nacht auf 40°C erhitzt. Es werden weitere 69 mg (0.70 mmol) tert.-Butylisocyanat und eine katalytische Menge DMAP zugegeben und über Nacht auf 50°C erhitzt. Nach Abkühlen wird mit Wasser versetzt, filtriert und das Lösungsmittel unter reduziertem Druck entfernt. Die Reinigung erfolgt durch präparative HPLC. Das Produkt wird in Methanol gelöst und mit einem Überschuss an 1 N Salzsäure versetzt. Das Lösungsmittel wird unter reduziertem Druck entfernt. Nach Trocknen im Hochvakuum werden 8 mg (9 % d.Th.) der Titelverbindung erhalten.
¹H-NMR (400 MHz, DMSO-d₆); δ = 9.70 (s, 1H), 8.80 (d, 1H), 8.55 (s; 1H), 7.85 (s, 1H), 7.80 (m, 2H), 7.60 (d, 1H), 7.45 (t, 1H), 7.38 (s, 2H), 6.10 (m, 1H), 4.35 (m, 1H), 3.70-3.63 (m, 1H), 3.50-3.05 (m, 5H), 2.30 (m, 1H), 2.18-2.05 (m, 1H), 1.98-1.88 (m, 2H), 1.80-1.63 (m, 1H), 1.30 (s, 9H).
HPLC (Methode 1): Rₜ = 4.27 min.
MS (ESIpos): m/z = 461 (M+H)⁺ (freie Base).

### Beispiel 111

### N-[(3R)-1-Azabicyclo[2.2.2]oct-3-yl]-7-(3-[(methylsulfonyl)amino]phenyl)-1-benzofuran-2-carboxamid-Hydrochlorid

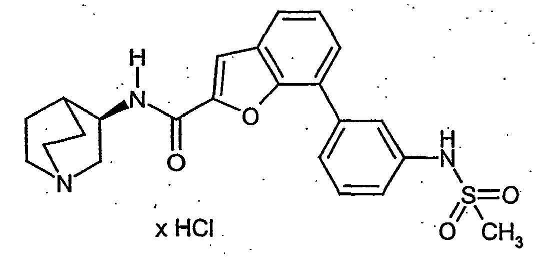

73 mg (0.20 mmol) 7-(3-Aminophenyl)-*N*-[(3R)-1-azabicyclo[2.2.2]oct-3-yl]-1-benzofuran-2-carboxamid (Beispiel 114), 69 mg (0.61 mmol) Metharisulfonsäurechlorid und 0.14 mL (1.01 mmol) Triethylamin werden in 3 mL THF/DMF (1:1) über Nacht auf 50°C erhitzt. Nach Abkühlen wird mit Wasser versetzt, filtriert und das Lösungsmittel unter reduziertem Druck entfernt. Die Reinigung erfolgt durch präparative HPLC. Das Produkt wird in Methanol gelöst und mit einem Überschuss an 1 N Salzsäure versetzt. Das Lösungsmittel wird unter reduziertem Druck entfernt. Nach Trocknen im Hochvakuum werden 16 mg (14 % d.Th.) der Titelverbindung erhalten.
¹H-NMR (300 MHz, DMSO-d₆): δ = 10.15 (s, 1H), 9.90 (s, 1H), 8.76 (d, 1H), 7.90 (s, 1H), 7.85-7.75 (m, 2H), 7.66-7.60 (m, 2H), 7.55-7.53 (m, 2H), 7.25 (m, 1H), 4.37 (m, 1H), 3.70-3.63 (m, 1H), 3.45-3.05 (m, 5H), 3.10 (s, 3H), 2.30 (m, 1H), 2.18-2.05 (m, 1H), 1.98-1.88 (m, 2H), 1.80-1.63 (m, 1H).
HPLC (Methode 1): Rₜ = 3.97 min.
MS (ESIpos): m/z = 440 (M+H)⁺ (freie Base).

### Beispiel 112

### N-[(3R)-1-Azabicyclo[2.2.2]oct-3-yl]-7-{2-[(cyclobutylcarbonyl)amino]phenyl}-1-benzofuran-2-carboxamid-Hydrochlorid

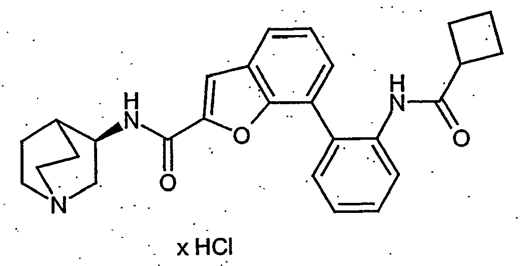

60 mg (0.15 mmol) 7-(2-Aminophenyl)-*N*-[(3R)-1-azabicyclo[2.2.2]oct-3-yl]-1-benzofuran-2-carboxamid (Beispiel 132), 26 mg (0.22 mmol) Cyclobutancarbonsäurechlorid und 0.06 mL (0.44 mmol) Triethylamin werden in 2 mL THF/DMF (1:1) über Nacht bei RT geschüttelt. Das Lösungsmittel wird unter reduziertem Druck entfernt. Die Reinigung erfolgt durch präparative HPLC. Das Produkt wird in Methanol gelöst und mit einem Überschuss an 1 N Salzsäure versetzt. Das Lösungsmittel wird unter reduziertem Druck entfernt. Nach Trocknen im Hochvakuum werden 39 mg (56 % d.Th.) der Titelverbindung erhalten.
¹H-NMR (300 MHz, DMSO-d₆): δ = 10.15 (s, 1H), 9.05 (s, 1H), 8.40 (d, 1H), 7.95 (s, 1H), 7.80-7.70 (m, 2H), 7.55-7.30 (m, 6H), 4.32 (m, 1H), 3.70-3.63 (m, 1H), 3.45-3.05 (m, 5H), 2.98-2.88 (m, 1H), 2.30 (m, 1H), 2.18-2.05 (m, 1H), 1.98-1.55 (m, 9H).
HPLC (Methode 1): Rₜ = 3.95 min.
MS (ESIpos): m/z.= 444 (M+H)⁺ (freie Base).

### Beispiel 113

### N-[(3R)-1-Azabicyclo[2.2.2]oct-3-yl]-5-(5-pyrimidinyl)-1-benzofuran-2-carboxamid-Hydrochlorid

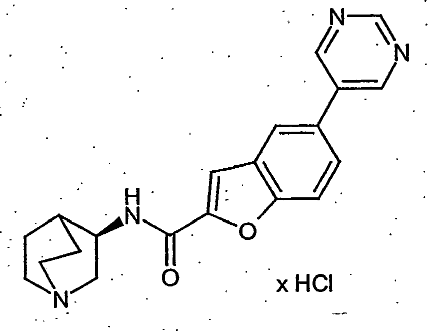

Zu einer Mischung aus 250 mg (0.72 mmol) *N*-[(3R)-1-Azabicyclo[2.2.2]oct-3-yl]-5-brom-1-benzofuran-2-carboxamid (Beispiel 3A) und 52 mg (0.07 mmol) PdCl₂(dppf) in 3 mL DMF werden 177 mg (0.86 mmol) 5-(4,4,5,5-Tetramethyl-1,3,2-dioxaborolan-2-yl)-pyrimidin und 2.15 mL 1 N Natronlauge gegeben. Das Reaktionsgemisch wird über Nacht auf 90°C erhitzt. Das Solvens wird unter reduziertem Druck entfernt und das Rohprodukt in Methanol aufgenommen und über Kieselgur filtriert. Die Reinigung erfolgt durch präparative HPLC. Das Produkt wird in Methanol gelöst und mit einem Überschuss an 1 N Salzsäure versetzt. Das Lösungsmittel wird unter reduziertem Druck entfernt. Nach Umkristallisieren des Rückstands aus Isopropanol und Trocknen im Hochvakuum werden 28 mg (10 % d.Th.) der Titelverbindung erhalten.
¹H-NMR (300 MHz, DMSO-d₆): δ = 10.30 (s, 1H), 9.22-9.13 (m, 4H), 8.24 (m, 1H), 7.93-7.81 (m, 3H), 4.39 (m, 1H), 3.68-3.48 (m, 1H), 3.45-3.13 (m, 5H), 2.28-2.20 (m, 1H), 2.18-2.07 (m, 1H), 1.97-1.88 (m, 2H), 1.80-1.57 (m, 1H).
HPLC (Methode 1): Rₜ = 3.26 min.
MS (ESIpos): m/z = 349 (M+H)⁺ (freie Base).

### Beispiel 114

### 7-(3-Aminophenyl)-N-[(3R)-1-azabicyclo[2.2.2]oct-3-yl]-1-benzofuran-2-carboxamid

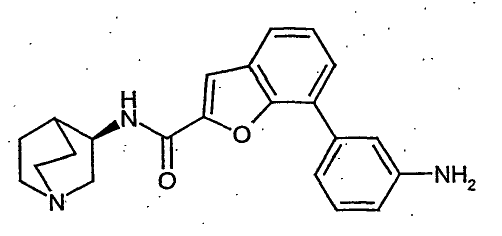

### Methode a):

Zu einer Mischung aus 978 mg (2.80 mmol) *N*-[(3R)-1-Azabicyclo[2.2.2]oct-3-yl]-7-brom-1-benzofuran-2-carboxamid (Beispiel 30A) und 205 mg (0.28 mmol) PdCl₂(dppf) in 15 mL DMF werden 622 mg (1.68 mmol) 3-Aminophenylboronsäure-Hemisulfat und 11.2 mL 1 N Natronlauge gegeben. Das Reaktionsgemisch wird über Nacht auf 95°C erhitzt. Das Rohprodukt wird über Kieselgur filtriert, mit DMF nachgewaschen und unter vermindertem Druck vom Lösungsmittel befreit. Der Rückstand wird mit 200 mL 1 N Natronlauge und 200 mL Essigsäureethylester versetzt. Nach Trennen der Phasen wird die organische Phase noch zweimal mit je 100 mL 1 N Natronlauge und anschließend noch einmal mit 100 mL einer gesättigten Kochsalz-Lösung gewaschen. Nach Trocknen über Magnesiumsulfat erfolgt die Reinigung des Rohproduktes durch präparative HPLC. Nach Entfernen des Lösungsmittels am Rotationsverdampfer können 875 mg (73 % d.Th.) der Titelverbindung durch zweimaliges Versetzen mit Dichlormethan und Wiedereinengen in Form eines weissen Schaumes erhalten werden.
¹H-NMR (300 MHz, DMSO-d₆): δ = 8.48 (d, 1H), 8.21 (s, 1H), 7.76-7.69 (m, 1H), 7.54 (d, 1H), 7.45-7:37 (m, 1H), 7.20-7.00 (m, 3H), 6.67-6.61 (m, 1H), 4.13-4.06 (m, 1H), 3.48-3.26 (m, 1H), 3.10-3.01 (m, 1H), 2.93-2.79 (m, 4H), 2.03 (m, 1H), 2.00-1.88 (m, 1H), 1.79-1.67 (m, 2H), 1.58-1.42 (m, 1H).
HPLC (Methode 1): Rₜ = 3.46 min.
MS (ESIpos): m/z = 362 (M+H)⁺.

### Methode b):

Zu einer Mischung aus 660 mg (1.89 mmol) *N*-[(3R)-1-Azabicyclo[2.2.2]oct-3-yl]-7-brom-1-benzofuran-2-carboxamid (Beispiel 30A) und 138 mg (0.19 mmol) PdCl₂(dppf) in 8 mL DMF werden 419 mg (1.13 mmol) Bis[3-(dihydroxyboranyl)-benzolaminium]sulfat und 7.56 mL 1 N Natronlauge gegeben. Das Reaktionsgemisch wird über Nacht auf 95°C erhitzt. Das Solvens wird unter reduziertem Druck entfernt, das Rohprodukt in Methanol aufgenommen und über Kieselgur filtriert. Die weitere Reinigung erfolgt durch präparative HPLC. Das Lösungsmittel wird aus den Produktfraktionen unter reduziertem Druck entfernt. Nach Trocknen im Hochvakuum werden 485 mg (71 % d.Th.) der Titelverbindung erhalten.
¹H-NMR (300 MHz, DMSO-d₆): δ = 8.48 (d, 1H), 8.21 (s, 1H), 7.70 (s, 1H), 7.55 (m, 1H), 7.40 (t, 1H), 7.18 (t, 1H), 7.10-7.00 (m, 2H), 6.66 (m, 1H), 4,20 (br. s, 2H), 4.05 (m, 1H), 3.25 (m, 1H), 3.05 (m, 1H), 2.90-2.70 (m, 4H), 2.05 (m, 1H), 1.70 (m, 1H), 1.65 (m, 2H), 1.45 (m, 1H).
HPLC (Methode 1): Rₜ = 3.50 min.
MS (ESIpos): m/z = 362 (M+H)⁺.

### Beispiel 115

### 7-[3-(Acetylamino)phenyl]-N-[(3R)-1-azabicyclo[2.2.2]oct-3-yl]-1-benzofuran-2-carboxamid-Hydrochlorid

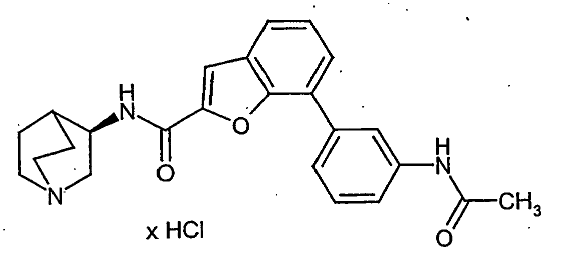

75 mg (0.16 mmol) 7-(3-Ammophenyl)-*N*-[(3R)-1-azabicyclo[2.2.2]oct-3-yl]-1-benzofuran-2-carboxamid (Beispiel 114), 18 µL (0.24 mmol) Acetylchlorid und 68 µL (0.49 mmol) Triethylamin werden in 2 mL THF über Nacht bei RT gerührt. Das Lösungsmittel wird unter reduziertem Druck entfernt. Die Reinigung erfolgt durch präparative HPLC. Das Produkt wird in Methanol gelöst und mit einem Überschuss an 1 N Salzsäure versetzt. Nach Entfernen des Lösungsmittels unter reduziertem Druck und Trocknen im Hochvakuum werden 51 mg (72 % d.Th.) der Titelverbindung erhalten.
¹H-NMR (300 MHz, DMSO-d₆): δ = 10.37 (s, 1H), 10.30 (br. s, 1H), 8.67-8.59 (m, 2H), 7.82-7.68 (m, 3H), 7.58-7.41 (m, 4M, 4.40 (m, 1H), 3.73-3.60 (m, 1H), 3.48-3.37 (m; 1H), 3.78-3.15 (m, 4H), 2.29 (m, 1H), 2.19-2.09 (m, 1H), 2.13 (s, 3H), 1.98-1.90 (m, 2H), 1.81-1.69 (m, 1H).
HPLC (Methode 1): Rₜ = 4.04 min.
MS (ESIpos): m/z = 404 (M+H)⁺ (freie Base).

### Beispiel 116

### N-[(3R)-1-Azabicyclo[2.2.2]oct-3-yl]-7-(3-[(cyclopropylcarbonyl)amino]phenyl)-1-benzofuran-2-carboxamid-Hydrochlorid

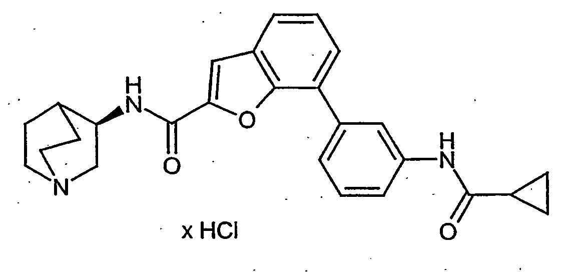

75 mg (0.21 mmol) 7-(3-Aminophenyl)-*N*-[(3R)-1-azabicyclo[2.2.2]oct-3-yl]-1-benzofuran-2-carboxamid (Beispiel 114), 28 µL (0.31 mmol) Cyclopropylcarbonsäurechlorid und 87 µL (0.62 mmol) Triethylamin werden in 2 mL THF über Nacht bei RT gerührt. Nach Versetzen mit Wasser wird das Lösungsmittel unter reduziertem Druck entfernt. Die Reinigung erfolgt durch präparative HPLC. Das Produkt wird in Methanol gelöst und mit einem Überschuss an 1 N Salzsäure versetzt. Nach Entfernen des Lösungsmittels unter reduziertem Druck und Trocknen im Hochvakuum werden 55 mg (57 % d.Th.) der Titelverbindung erhalten.
¹H-NMR (300 MHz, DMSO-d₆): δ = 10.53 (s, 1H), 10.08 (br. s, 1H), 8.83 (m, 1H), 8.28 (s, 1H), 7.82-7.75 (m, 2H), 7.71 (d, 1H), 7.62 (d, 1H), 7.58-7.50 (m, 1H), 7.48-7.41 (m, 2H), 4.38 (m, 1H), 3.71-3.60 (m, 1H), 3.50-3.15 (m, 5H), 2.27 (m, 1H), 2.21-2.11 (m, 1H), 1.99-1.82 (m, 3H), 1.80-1.71 (m, 1H), 0.88-0.77 (m, 4H).
HPLC (Methode 1): Rₜ= 4.07 min.
MS (ESIpos): m/z = 430 (M+H)⁺ (freie Base).

### Beispiel 117

### N-[(3R)-1-Azabicyclo[2.2.2]oct-3-yl]-7-(3-{[(methoxy)acetyl]amino}phenyl)-1-benzofuran-2-carboxamid-Hydrochlorid

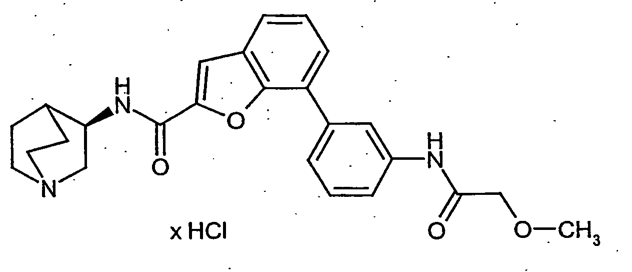

75 mg (0.21 mmol) 7-(3-Aminophenyl)-*N*-[(3R)-1-azabicyclo[2.2.2]oct-3-yl]-1-benzofuran-2-carboxamid (Beispiel 114), 21 µL (0.31 mmol) Methoxyessigsäurechlorid und 87 µL (0.62 mmol) Triethylamin werden in 2 mL THF über Nacht bei RT gerührt. Nach Versetzen mit Wasser wird das Lösungsmittel unter reduziertem Druck entfernt. Die Reinigung erfolgt durch präparative HPLC. Das Produkt wird in Methanol gelöst und mit einem Überschuss an 1 N Salzsäure versetzt. Nach Entfernen des Lösungsmittels unter reduziertem Druck und Trocknen im Hochvakuum werden 56 mg (55 % d.Th.) der Titelverbindung erhalten.
¹H-NMR (300 MHz, DMSO-d₆): δ = 10.17 (br. s, 1H), 10.10 (s, 1H), 8.63-8.55 (m, 2H), 7.82-7.68 (m, 3H), 7.64-7.57 (m, 2H), 7.52-7.41 (m, 2H), 4.39 (m, 1H), 4.10 (s, 2H), 3.71-3.61 (m, 1H), 3.49-3.14 (m, 5H), 3.41 (s, 3H), 2.29 (m, 1H), 2.19-2.05 (m, 1H), 1.99-1.89 (m, 2H), 1.80-1.69 (m, 1H).
HPLC (Methode 1): Rₜ = 4.07 min.
MS (ESIpos): m/z = 434 (M+H)⁺ (freie Base).

### Beispiel 118

### N-[(3R)-1-Azabicyclo[2.2.2]oct-3-yl]-7-{3-[(cyclobutylcarbonyl)amino]phenyl}-1-benzofuran-2-carboxamid-Hydrochlorid

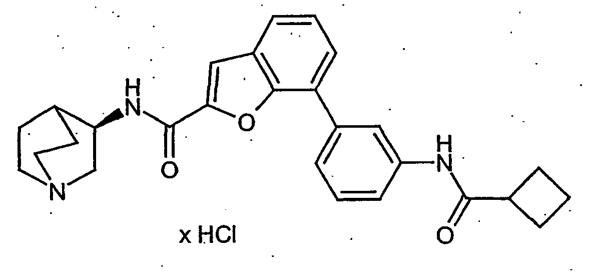

75 mg (0.21 mmol) 7-(3-Amkophenyl)-*N*-[(3R)-1-azabicyclo[2.2.2]oct-3-yl]-1-benzofuran-2-carboxamid (Beispiel 114), 37 mg (0.31 mmol) Cyclobutancarbonsäurechlorid und 87 µL (0.62 mmol) Triethylamin werden in 2 mL THF über Nacht bei RT gerührt. Nach Versetzen mit Wasser wird das Lösungsmittel unter reduziertem Druck entfernt. Die Reinigung erfolgt durch präparative HPLC. Das Produkt wird in Methanol gelöst und mit einem Überschuss an 1 N Salzsäure versetzt. Nach Entfernen des Lösungsmittels unter reduziertem Druck und Trocknen im Hochvakuum werden 57 mg (57 % d.Th.) der Titelverbindung erhalten.
¹H-NMR (300 MHz, DMSO-d₆): δ = 10.14 (br. s, 1H), 10.07 (s, 1H), 8.82 (d, 1H), 8.35 (s, 1H), 7.80-7.78 (m, 2H), 7.74-7.61 (m, 2H), 7.57-7.41 (m, 3H), 4.36 (m, 1H), 3.70-3.61 (m, 1H), 3.45-3.13 (m, 5H), 2.30-1.67 (m, 12H).
HPLC (Methode 1): Rₜ = 4.22 min.
MS (ESIpos): m/z = 444 (M+H)⁺ (freie Base).

### Beispiel 119

### N-[(3R)-1-Azabicyclo[2.2.2]oct-3-yl]-7-{2-[(cyclopropylcarbonyl)amino]phenyl}-1-benzofuran-2-carboxamid-Hydrochlorid

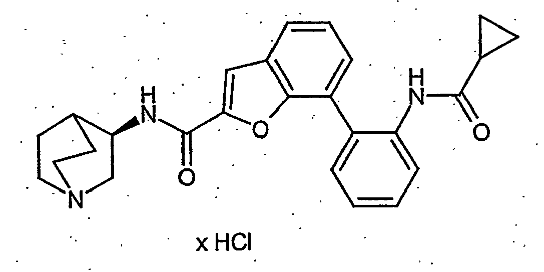

60 mg (0.15 mmol) 7-(2-Aminophenyl)-*N*-[(3R)-1-azabicyclo[2.2.2]oct-3-yl]-1-benzofuran-2-carboxamid (Beispiel 132), 20 µL (0.31 mmol) Cyclopropylcarbonsäurechlorid und 87 µL (0.62 mmol) Triethylamin werden in 2 mL THF/DMF (1:1) über Nacht bei RT gerührt. Nach Versetzen mit Wasser wird das Lösungsmittel unter reduziertem Druck entfernt. Die Reinigung erfolgt durch präparative HPLC. Das Produkt wird in Methanol gelöst und mit einem Überschuss an 1 N Salzsäure versetzt. Nach Entfernen des Lösungsmittels unter reduziertem Druck und Trocknen im Hochvakuum werden 27 mg (40 % d.Th.) der Titelverbindung erhalten.
¹H-NMR (300 MHz, DMSO-d₆): δ = 10.07 (br. s, 1H), 9.48 (s, 1H), 8.84 (d, 1H), 7.83-7.77 (m, 1H), 7.72 (m, 1H), 7:54-7.31 (m, 6H), 4.30 (m, 1H), 3.70-3.61 (m, 1H), 3.37-3.03 (m, 5H), 2.23 (m, 1H), 2.14-2.03 (m, 1H), 1.95-1.83 (m, 2H), 1.79-1.68 (m, 1H), 1.53 (m, 1H), 1.31-1.15 (m, 4H).
HPLC (Methode 1): Rₜ=3.85 min.
MS (ESIpos): m/z = 430 (M+H)⁺ (freie Base).

### Beispiel 120

### N-[(3R)-1-Azabicyclo[2.2.2]oct-3-yl]-7-(2-{[(methoxy)acetyl]amino}phenyl)-1-benzofuran-2-carboxamid-Hydrochlorid

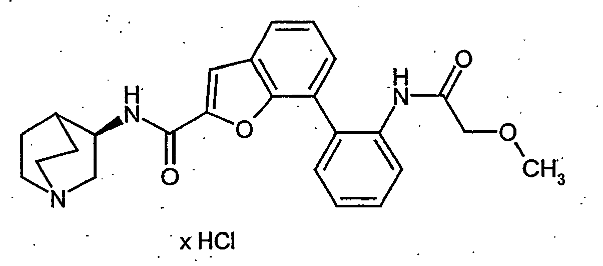

60 mg (0.15 mmol) 7-(2-Aminophenyl)-*N*-[(3R)-1-azabicyclo[2.2.2]oct-3-yl]-1-benzofuran-2-carboxamid (Beispiel 132), 20 µL (0.22 mmol) Methoxyessigsäurechlorid und 61 µL (0.44 mmol) Triethylamin werden in 2 mL THF/DMF (1:1) über Nacht bei RT gerührt. Nach Versetzen mit Wasser wird das Lösungsmittel unter reduziertem Druck entfernt. Die Reinigung erfolgt durch präparative HPLC. Das Produkt wird in Methanol gelöst und mit einem Überschuss an 1 N Salzsäure versetzt. Nach Entfernen des Lösungsmittels unter reduziertem Druck und Trocknen im Hochvakuum werden 29 mg (40 % d.Th.) der Titelverbindung erhalten.
¹H-NMR (300 MHz, DMSO-d₆): δ = 10.15 (br. s, 1H), 9.05 (s, 1H), 8.53 (d, 1H), 7.88-7.73 (m, 3H), 7.52-7.34 (m, 5H), 4.33 (m, 1H), 3.77 (s, 2H), 3.69-3.59 (m, 1H), 3.44-3.14 (m, 5H), 3.04 (s, 3H), 2.22 (m, 1H), 2.18-2.05 (m, 1H), 1.96-1.84 (m, 2H), 1.80-1.67 (m, 1H).
HPLC (Methode 1): Rₜ = 3.84 min.
MS (ESIpos): m/z = 434 (M+H)⁺ (freie Base).

### Beispiel 121

### N-[(3S)-1-Azabicyclo[2.2.2]oct-3-yl]-7-[4-(4-morpholinyl)phenyl]-1-benzofuran-2-carboxamid-Hydrochlorid

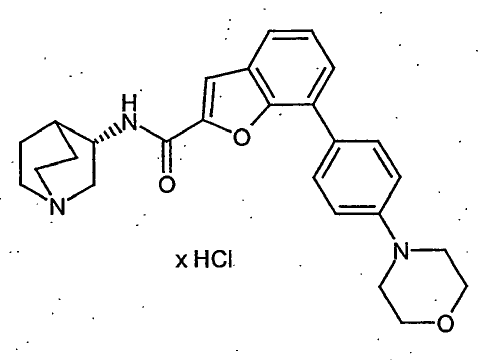

Zu einer Mischung aus 150 mg (0.43 mmol) *N*-[(3S)-1-Azabicyclo[2.2.2]oct-3-yl]-7-brom-1-benzofuran-2-carboxamid (Beispiel 31A) und 35 mg (0.04 mmol) PdCl₂(dppf) in 4 mL DMF werden 107 mg (0.52 mmol) 4-Morpholinophenylboronsäure und 1.72 mL 1 N Natronlauge gegeben. Das Reaktionsgemisch wird über Nacht auf 95°C erhitzt. Das Rohprodukt wird über Kieselgur filtriert, mit DMF nachgewaschen und unter vermindertem Druck vom Lösungsmittel befreit. Zur Abtrennung letzter Katalysatorreste wird nochmals über Kieselgel filtriert und mit Dichlormethan und Methanol nachgewaschen. Die Reinigung des Rohproduktes erfolgt durch präparative HPLC. Das Produkt wird in Methanol gelöst und mit einem Überschuss an 1 N Salzsäure versetzt. Nach Entfernen des Lösungsmittels unter reduziertem Druck und Trocknen im Hochvakuum werden 84 mg (42 % d.Th.) der Titelverbindung erhalten.
¹H-NMR (300 MHz, DMSO-d₆): δ = 10.48 (br. s, 1H), 9.05 (d, 1H), 7.90-7.82 (m, 3H), 7.70 (d, 1H), 7.63 (d, 1H), 7.42-7.35 (m, 1H), 7.20-7.12 (m, 2H), 4.40-4.31 (m, 1H), 3.84-3.77 (m, 4H), 3.69-3.57 (m, 1H), 3.48-3.12 (m, 9H), 2.22 (m, 1H), 2.19-2.08 (m, 1H), 1.96-1.85 (m, 2H), 1.80-1.71 (m, 1H).
HPLC (Methode 1): Rₜ = 3.82 min.
MS (ESIpos): m/z = 432 (M+H)⁺.

### Beispiel 122

### N-[(3S)-1-Azabicyclo[2.2.2]oct-3-yl]-7-[2-(hydroxymethyl)phenyl]-1-benzofuran-2-carboxamid

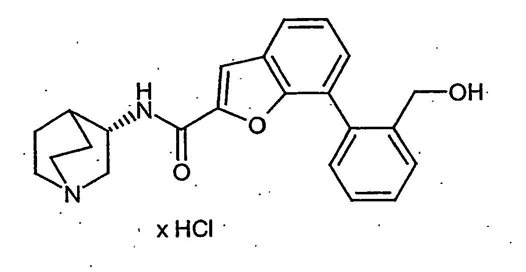

Zu einer Mischung aus 150 mg (0.43 mmol) *N*-[(3S)-1-Azabicyclo[2.2..2]oct-3-yl]-7-brom-1-benzofuran-2-carboxamid (Beispiel 31A) und 35 mg (0.04 mmol) PdCl₂(dppf) in 3 mL DMF werden 78 mg (0.52 mmol) 2-(Hydroxymethyl)phenylboronsäure und 1.72 mL 1 N Natronlauge gegeben. Das Reaktionsgemisch wird über Nacht auf 95°C erhitzt. Das Rohprodukt wird über Kieselgur filtriert, mit DMF nachgewaschen und unter vermindertem Druck vom Lösungsmittel befreit. Zur Abtrennung letzter Katalysatorreste wird nochmals über Kieselgel filtriert und mit Dichlormethan und Methanol nachgewaschen. Die Reinigung des Rohproduktes erfolgt durch präparative HPLC. Das Produkt wird in Methanol gelöst und mit einem Überschuss an 1 N Salzsäure versetzt. Nach Entfernen des Lösungsmittels unter reduziertem Druck und Trocknen im Hochvakuum werden 81 mg (46 % d.Th.) der Titelverbindung erhalten.
¹H-NMR (300 MHz, DMSO-d₆): δ = 10.12 (br. s, 1H), 8.94 (d, 1H), 7.91-7.79 (m, 3H), 7.69 (d, 1H), 7.53-7.32 (m, 5H), 4.33-4.22 (m, 3H), 3.68-3.57 (m, 1H), 3.48-3.12 (m, 5H), 2.19 (m, 1H), 2.15-2.04 (m, 1H), 1.94-1.83 (m, 2H), 1.79-1.67 (m, 1H).
HPLC (Methode 1): Rₜ = 3.87 min.
MS (ESIpos): m/z = 377 (M+H)⁺.

### Beispiel 123

### N-[(3S)-1-Azabicyclo[2.2.2]oct-3-yl]-7-(3-pyridinyl)-1-benzofuran-2-carboxamid-Hydrochlorid

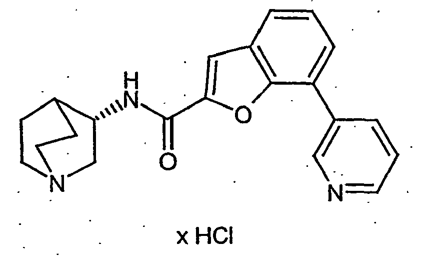

Zu einer Mischung aus 150 mg (0.43 mmol) *N*-[(3S)-1-Azabicyclo[2.2.2]oct-3-yl]-7-brom-1-benzofüran-2-carboxamid (Beispiel 31A) und 35 mg (0.04 mmol) PdCl₂(dppf) in 4 mL DMF werden 63 mg (0.57 mmol) 3-Pyridinboronsäure und 1.72 mL 1 N Natronlauge gegeben. Das Reaktionsgemisch wird über Nacht auf 95°C erhitzt. Das Solvens wird unter reduziertem Druck entfernt und das Rohprodukt in Methanol aufgenommen und über Kieselgur filtriert. Zur Abtrennung letzter Katalysatorreste wird nochmals über Kieselgel filtriert und mit Dichlormethan und Methanol nachgewaschen. Die Reinigung erfolgt durch präparative HPLC. Das Produkt wird in Methanol gelöst und mit einem Überschuss an 1 N Salzsäure versetzt. Das Lösungsmittel wird unter reduziertem Druck entfernt. Nach Trocknen .im Hochvakuum werden 76 mg (42 % d.Th.) der Titelverbindung erhalten.
¹H-NMR (300 MHz, DMSO-d₆): δ = 10.49 (s, 1H), 9.51 (s, 1H), 9.30 (d, 1H), 8.94-8.80 (m, 2H), 8.07-7.80 (m, 4H), 7.55 (t, 1H), 4.35 (m, 1H), 3.65-3.15 (m, 6H), 2.20 (m, 1H), 2.18-2.05 (m, 1H), 1.98-1.88 (m, 2H), 1.80-1.63 (m, 1H).
HPLC (Methode 1): Rₜ = 3.30 min.
MS (ESIpos): m/z = 348 (M+H)⁺ (freie Base).

### Beispiel 124

### N-[(3S)-1-Azabicyclo[2.2.2]oct-3-yl]-7-[4-(methoxy)phenyl]-1-benzofuran-2-carboxamid-Hydrochlorid

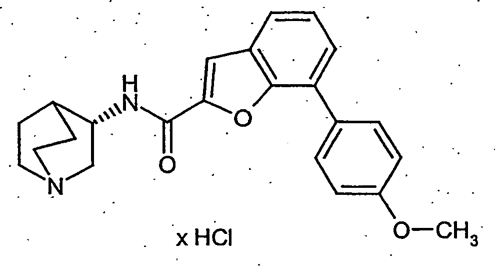

Zu einer Mischung aus 150 mg (0.43 mmol) *N*-[(3S)-1-Azabicyclo[2.2.2]oct-3-yl]-7-brom-1-benzofuran-2-carboxamid (Beispiel 31A) und 35 mg (0.04 mmol) PdCl₂(dppf) in 4 mL DMF werden 78 mg (0.52 mmol) 4-(Methoxy)phenylboronsäure und 1.72 mL 1 N Natronlauge gegeben. Das Reaktionsgemisch wird über Nacht auf 95°C erhitzt. Das Solvens wird unter reduziertem Druck entfernt, das Rohprodukt in Methanol aufgenommen und über Kieselgur filtriert. Zur Abtrennung letzter Katalysatorreste wird nochmals über Kieselgel filtriert und mit Dichlormethan und Methanol nachgewaschen. Die Reinigung erfolgt durch präparative HPLC. Die Produktfraktionen werden mit einem Überschuss an 1 N Salzsäure versetzt. Das Lösungsmittel wird unter reduziertem Druck entfernt. Nach Trocknen im Hochvakuum werden 27 mg (15 % d.Th.) der Titelverbindung erhalten.
Die analytischen Daten stimmen mit denen der enantiomeren Verbindung (Beispiel 99) überein.

### Beispiel 125

### N-[(3S)-1-Azabicyclo[2.2.2]oct-3-yl]-7-[3-(methoxy)phenyl]-1-benzofuran-2-carboxamid-Hydrochlorid

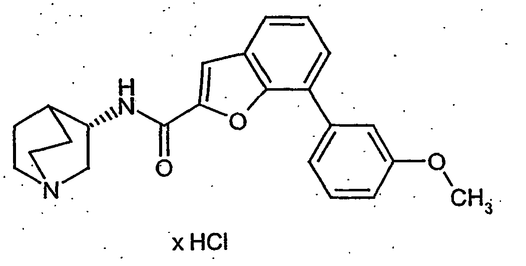

Zu einer Mischung aus 150 mg (0.43 mmol) *N*-[(3S)-1-Azabicyclo[2.2.2]oct-3-yl]-7-brom-1-benzofuran-2-carboxamid (Beispiel 31A) und 35 mg (0.04 mmol) PdCl₂(dppf) in 3 mL DMF werden 78 mg (0.52 mmol) 3-(Methoxy)phenylboronsäure und 1.72 mL 1 N Natronlauge gegeben. Das Reaktionsgemisch wird über Nacht auf 95°C erhitzt. Das Solvens wird unter reduziertem Druck entfernt, das Rohprodukt in Methanol aufgenommen und über Kieselgur filtriert. Zur Abtrennung letzter Katalysatorreste wird nochmals über Kieselgel filtriert und mit Dichlormethan und Methanol nachgewaschen. Die Reinigung erfolgt durch präparative HPLC. Das Produkt wird in Methanol gelöst und mit einem Überschuss an 1 N Salzsäure versetzt. Das Lösungsmittel wird unter reduziertem Druck entfernt. Nach Trocknen im Hochvakuum werden 23 mg (13 % d.Th.) der Titelverbindung erhalten.
Die analytischen Daten stimmen mit denen der enantiomeren Verbindung (Beispiel 101) überein.

### Beispiel 126

### N-[(3R)-1-Azabicyclo[2.2.2]oct-3-yl]-7-[2-({[(1-methylethyl)amino]carbonyl}-amino)phenyl]-1-benzofuran-2-carboxamid-Hydrochlorid

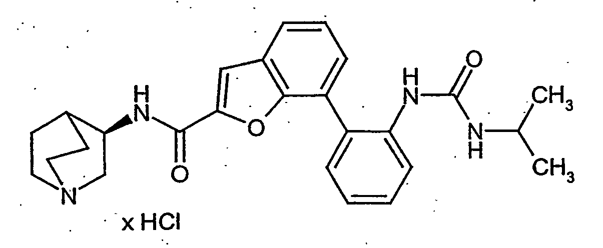

75 mg (0.16 mmol) 7-(2-Aminophenyl)-*N*-[(3R)-1-azabicyclo[2.2.2]oct-3-yl]-1-benzofuran-2-carboxamid (Beispiel 132), 65 µL (0.66 mmol) Isopropylisocyanat und 114 µL (0.82 mmol) Triethylamin werden in 3 mL THF/DMF (1:1) 48 h auf 50°C erhitzt. Nach Abkühlen wird mit Wasser versetzt, filtriert und das Lösungsmittel unter reduziertem Druck entfernt. Die Reinigung erfolgt durch präparative HPLC. Das Produkt wird in Methanol gelöst und mit einem Überschuss an 1 N Salzsäure versetzt. Das Lösungsmittel wird unter reduziertem Druck entfernt. Nach Trocknen im Hochvakuum werden 21 mg (26 % d.Th.) der Titelverbindung erhalten.
¹H-NMR (300 MHz, DMSO-d₆): δ = 10.09 (br. s, 1H), 8.60 (d, 1H), 7.88-7.77 (m, 3H), 7.46-7.33 (m, 3H), 7.29 (dd, 1H), 7.19-7.10 (m, 1H), 6.22 (br. s, 1H), 4.29 (m, 1H), 3.75-3.54 (m, 2H), 3.39-3.13 (m, 5H), 2.22 (m, 1H), 2.15-2.02 (m, 1H), 1.96-1.86 (m, 2H), 1.80-1.69 (m, 1H), 0.96 (d, 6H).
HPLC (Methode 1): Rₜ = 3.92 min.
MS (ESIpos): m/z = 447 (M+H)⁺ (freie Base).

### Beispiel 127

### N-[(3R)-1-Azabicyclo[2.2.2]oct-3-yl]-7-(2-{[(ethylamino)carbonyl]amino}phenyl)-1-benzofuran-2-carboxamid-Hydrochlorid

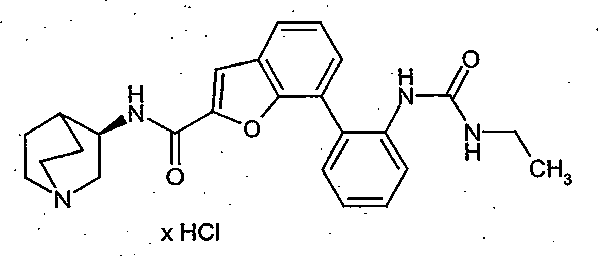

75 mg (0.16 mmol) 7-(2-Aminophenyl)-*N*-[(3R)-1-azabicyclo[2.2.2]oct-3-yl]-1-benzofuran-2-carboxamid (Beispiel 132), 50 µL (0.66 mmol) Ethylisocyanat und 110 µL (0.82 mmol) Triethylamin werden zusammen mit einer katalytischen Menge an DMAP in 3 mL THF/DMF (1:1) 48 h auf 50°C erhitzt. Nach Abkühlen wird mit Wasser versetzt, filtriert und das Lösungsmittel unter reduziertem Druck entfernt. Die Reinigung erfolgt durch präparative HPLC. Das Produkt wird in Methanol gelöst und mit einem Überschuss an 1 N Salzsäure versetzt. Das Lösungsmittel wird unter reduziertem Druck entfernt. Nach Trocknen im Hochvakuum werden 10 mg (12 % d.Th.) der Titelverbindung erhalten.
¹H-NMR (300 MHz, DMSO-d₆): δ = 10.41 (br. s, 1H), 9.30 (d, 1H), 8.69 (d, 1H), 7.96-7.78 (m, 3H), 7.59-7.11 (m, 4H), 4.30 (m, 1H), 3.69-3.54 (m, 1H), 3.40-3.13 (m, 5H), 2.99 (q, 2H), 2.24-2.19 (m, 1H), 2.17-2.04 (m, 1H), 1.96-1.84 (m, 2H), 1.80-1.64 (m, 1H), 0.92 (t, 3H).
HPLC (Methode 1): Rₜ = 3.86 min.
MS (ESIpos): m/z = 433 (M+H)⁺ (freie Base).

### Beispiel 128

### N-[(3R)-1-Azabicyclo[2.2.2]oct-3-yl]-7-(2-{[(methylanimo)carbonyl]ammo}phenyl)-1-benzofuran-2-carboxamid-Hydrochlorid

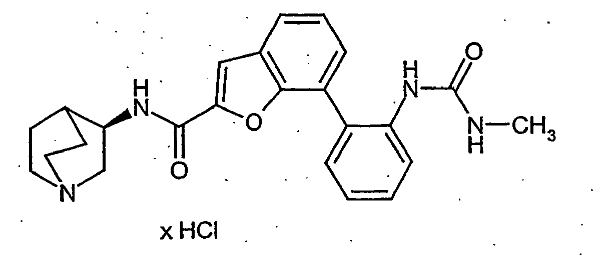

75 mg (0.16 mmol) 7-(2-Aminophenyl)-*N*-[(3R)-1-azabicyclo[2.2.2]oct-3-yl]-1-benzofuran-2-carboxamid (Beispiel 132), 37 mg (0.66 mmol) Methylisocyanat und 110 µL (0.82 mmol) Triethylamin werden zusammen mit einer katalytischen Menge an DMAP in 3 mL THF/DMF (1:1) über Nacht auf 50°C erhitzt. Nach Abkühlen wird mit Wasser versetzt, filtriert und das Lösungsmittel unter reduziertem Druck entfernt. Die Reinigung erfolgt durch präparative HPLC. Das Produkt wird in Methanol gelöst und mit einem Überschuss an 1 N Salzsäure versetzt. Das Lösungsmittel wird unter reduziertem Druck entfernt. Nach Trocknen im Hochvakuum werden 29 mg (35 % d.Th.) der Titelverbindung erhalten.
¹H-NMR (300 MHz, DMSO-d₆): δ = 10.22 (br. s, 1H), 8.60 (d, 1H), 8.69 (d, 1H), 7.86-7.74 (m, 3H), 7.51-7.28 (m, 5H), 7.18-7.11 (m, 1H), 6.21 (br. s, 1H), 4.29 (m, 1H), 3.69-3.58 (m, 1H), 3.38-3.13 (m, 5H), 2.51 (s, 3H), 2.28-2.22 (m, 1H), 2.18-2.04 (m, 1H), 1.99-1.87 (m, 2H), 1.82-1.68 (m, 1H).
HPLC (Methode 1): Rₜ = 3.72 min.
MS (ESIpos): m/z = 419 (M+H)⁺ (freie Base).

### Beispiel 129

### N-[(3R)-1-Azabicyclo[2.2.2]oct-3-yl]-7-[2-({[(1,1-dimethylethyl)amino]carbonyl}-amino)phenyl]-1-benzofuran-2-carboxamid-Hydrochlorid

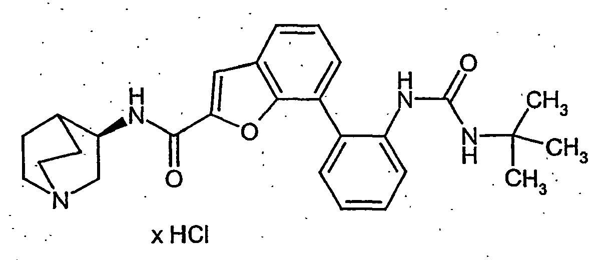

75 mg (0.16 mmol) 7-(2-Aminophenyl)-*N*-[(3R)-1-azabicyclo[2.2.2]oct-3-yl]-1-benzofuran-2-carboxamid (Beispiel 132), 65 mg (0.66 mmol) (1,1-Dimethylethyl)-isocyanat und 110 µL (0.82 mmol) Triethylamin werden zusammen mit einer katalytischen Menge an DMAP in 3 mL THF/DMF (1:1) über Nacht auf 50°C erhitzt. Nach Abkühlen wird mit Wasser versetzt, filtriert und das Lösungsmittel unter reduziertem Druck entfernt. Die Reinigung erfolgt durch präparative HPLC. Das Produkt wird in Methanol gelöst und mit einem Überschuss an 1 N Salzsäure versetzt. Das Lösungsmittel wird unter reduziertem Druck entfernt. Nach Trocknen im Hochvakuum werden 10 mg (12 % d.Th.) der Titelverbindung erhalten.
HPLC (Methode 1): Rₜ = 4.06 min.
MS (ESIpos): m/z = 461 (M+H)⁺ (freie Base).

### Beispiel 130

### N-[(3R)-1-Azabicyclo[2.2.2]oct-3-yl]-7-[2-(methoxy)phenyl]-1-benzofuran-2-carboxamid

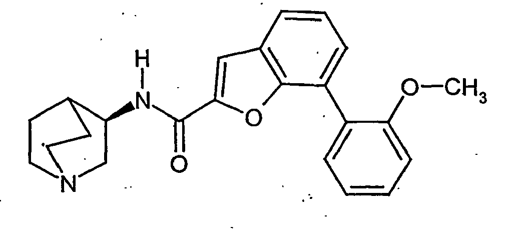

600 mg (1.45 mmol) *N*-[(3R)-1-Azabicyclo[2.2.2]oct-3-yl]-7-[2-(methoxy)phenyl]-1-benzofuran-2-carboxamid-Hydrochlorid (Beispiel 102) werden in 15 mL Essigsäureethylester gelöst und dreimal mit 1 N Natronlauge extrahiert. Die organische Phase wird über Natriumsulfat getrocknet und anschließend eingeengt. Nach Trocknen im Hochvakuum erhält man 534 mg (97.6% d.Th.) der Titelverbindung.
¹H-NMR (200 MHz, DMSO-d₆): δ = 8.34 (d, 1H), 7.72 (dd, 1H), 7.70 (s, 1H), 7.50-7.30 (m, 4H), 7.20 (m, 1H), 7.08 (m, 1H), 3.93 (m, 1H), 3.76 (s, 3H), 3.11 (m, 1H), 2.86 (m, 1H), 2.69 (m, 4H), 1.86 (m, 1H), 1.75 (m, 1H), 1.58 (m, 2H), 1.32 (m, 1H).
HPLC (Methode 1): Rₜ = 4.1 min.
MS (ESIpos): m/z = 377 (M+H)⁺.

### Beispiel 131

### N-[(3R)-1-Azabicyclo[2.2.2]oct-3-yl]-7-[2-(hydroxymethyl)phenyl]-1-benzothiophen-2-carboxamid-Hydrochlorid

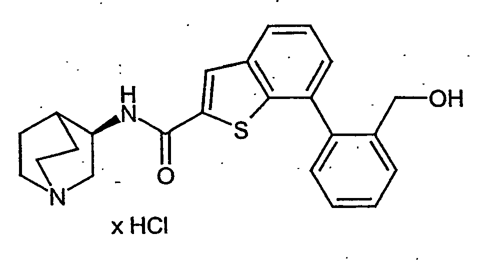

200 mg (0.45 mmol) *N*-[(3R)-1-Azabicyclo[2.2.2]oct-3-yl]-7-brom-1-benzothiophen-2-carboxamid-Hydrochlorid (Beispiel 8A) und 67.9 mg (0.45 mmol) 2-(Hydroxymethyl)phenylboronsäure werden in 2 mL DMF vorgelegt. Nach Addition von 0.67 mL 2 M Natriumcarbonat-Lösung und 18.2 mg (0.02 mmol) PdCl₂(dppf) wird auf 80°C erhitzt. Nach 18 h wird das Reaktionsgemisch über Kieselgur filtriert und durch Trennung mittels präparativer HPLC aufgereinigt. Die Produktfraktionen werden eingeengt, mit einer 5:1-Mischung aus Methanol und 4 N Chlorwasserstoff in Dioxan versetzt und erneut eingeengt. Nach Trocknen im Hochvakuum erhält man 148 mg (72 % d.Th.) der Titelverbindung.
¹H-NMR (300 MHz,DMSO-d₆): δ = 10.30 (br. s, 1H), 9.10 (d, 1H), 8.47 (s, 1H), 8.07 (d, 1H), 7.78 (d, 1H), 7.68-7.54 (m, 2H), 7.52-7.39 (m, 3H), 4.40 (m, 1H), 4.36 (s, 2H), 3.72 (m, 1H), 3.53-3.20 (m, 5H), 2.29 (m, 1H), 2.21 (m, 1H), 2.00 (m, 2H), 1.82 (m, 1H).
HPLC (Methode 1): Rₜ = 3.9 min.
MS (ESIpos): *m*/*z* = 393 (M+H)⁺ (freie Base).

### Beispiel 132

### 7-(2-Aminophenyl)-N-[(3R)-1-azabicyclo[2.2.2]oct-3-yl]-1-benzofuran-2-carboxamid

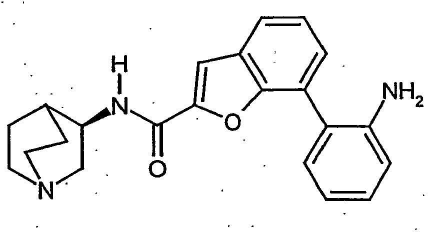

Zu einer Mischung aus 1.0 g (2.86 mmol) *N*-[(3R)-1-Azabicyclo[2.2.2]oct-3-yl]-7-brom-1-benzofuran-2-carboxamid (Beispiel 30A) und 234 mg (0.29 mmol) PdCl₂(dppf) in 15 mL DMF werden 752 mg (3.44 mmol) 2-(4,4,5,5-Tetramethyl-1,3,2-dioxaborolan-2-yl)phenylamin und 11.45 mL 1 N Natronlauge gegeben. Das Reaktionsgemisch wird über Nacht auf 95°C erhitzt und dann über Kieselgur filtriert. Anschließend wird das Solvens unter reduziertem Druck entfernt und der Rückstand in 100 mL Essigsäureethylester und 100 mL 1 N Natronlauge aufgenommen. Die organische Phase wird zweimal mit 1 N Natronlauge und einmal mit gesättigter Natriumchlorid-Lösung gewaschen. Die vereinigten organischen Phasen werden über Magnesiumsulfat getrocknet und das Solvens unter reduziertem Druck am Rotationsverdampfer abdestilliert. Das Rohprodukt wird in Methanol aufgenommen und zusammen mit saurem Ionenaustauscher (Dowex^{®} WX2-200) etwa 30 min. lang geschüttelt. Der beladene Ionenaustauscher wird dreimal mit je 30 mL Methanol, dann mit DMF gewaschen. Es wird nacheinander mit Methanol, Dichlormethan, Methanol, Wasser, Methanol, Dichlormethan, Methanol, THF und abschließend nochmals mit Methanol gewaschen. Das Produkt wird mit Methanol-Triethylamin 95:5 eluiert. Das Solvens wird unter reduziertem Druck am Rotationsverdampfer entfernt. Nach Trocknen im Hochvakuum werden 601 mg (48 % d.Th.) der Titelverbindung in ausreichender Reinheit für die weiteren Umsetzungen erhalten.
HPLC (Methode 1): Rₜ = 3.51 min.
MS (ESIpos): m/z = 362 (M+H)⁺.

### Beispiel 133

### N-[(3S)-1-Azabicyclo[2.2.2]oct-3-yl]-7-[4-(4-morpholinylcarbonyl)phenyl]-1-benzothiophen-2-carboxamid-Hydrochlorid

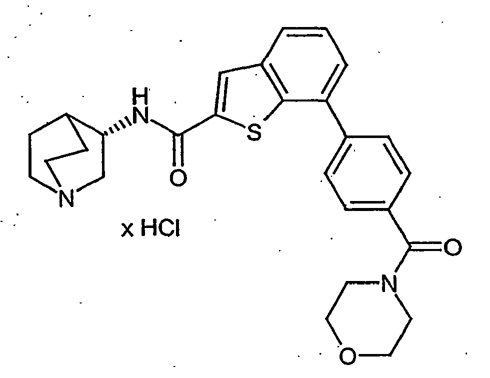

Gemäß der allgemeinen Arbeitsvorschrift D werden 244.0 mg (0.65 mmol) 4-(4-Morpholinylcarbonyl)phenyl-trifluormethansulfonat (Beispiel 18A), 189.6 mg (0.75 mmol) Bis(pinacolato)dibor, 158.8 mg (1.62 mmol). Kaliumacetat, 18.2 mg (0.02 mmol) PdCl₂(dppf), 200.0 mg (0.50 mmol) *N*-[(3S)-1-Azabicyclo[2.2.2]oct-3-yl]-7-brom-1-benzothiophen-2-carboxamid-Hydrochlorid (Beispiel 22A), 1.24 mL 2 M Natriumcarbonat-Lösung und weitere 18.2 mg (0.02 mmol) PdCl₂(dppf) in 2.5 mL DMF umgesetzt. Nach Trocknen im Hochvakuum werden 76.8 mg (30.1 % d.Th.) der Titelverbindung erhalten.
Die spektroskopischen Daten stimmen mit denen der enantiomeren Verbindung (Beispiel 72) überein.

### Beispiel 134

### N-[(3S)-1-Azabicyclo[2.2.2]oct-3-yl]-7-[3-(4-morpholinyl)phenyl]-1-benzothiophen-2-carboxamid-Hydrochlorid

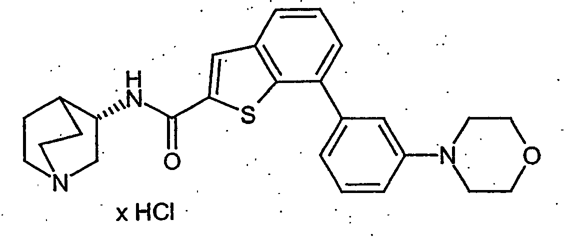

Gemäß der allgemeinen Arbeitsvorschrift D werden 143.3 mg (0.49 mmol) 3-(4-Morpholinyl)phenyl-trifluormethansulfonat (Beispiel 17A), 142.2 mg (0.56 mmol) Bis(pinacolato)dibor, 119.1 mg (1.21 mmol) Kaliumacetat, 13.7 mg (0.02 mmol) PdCl₂(dppf), 150 mg (0.37 mmol) *N*-[(3S)-1-Azabicyclo[2.2.2]oct-3-yl]-7-brom-1-benzothiophen-2-carboxamid-Hydrochlorid (Beispiel 22A), 0.93 mL 2 M Natriumcarbonat-Lösung und weitere 13.7 mg (0.02 mmol) PdCl₂(dppf) in 2.0 mL DMF umgesetzt. Nach Trocknen im Hochvakuum werden 67 mg (37.1 % d.Th.) der Titelverbindung erhalten.
Die spektroskopischen Daten stimmen mit denen der enantiomeren Verbindung (Beispiel 70) überein.

### Beispiel 135

### N-[(3R)-1-Azabicyclo[2.2.2]oct-3-yl]-7-{3-[(cyclopropylamino)carbonyl]phenyl}-1-benzothiophen-2-carboxamid-Hydrochlorid

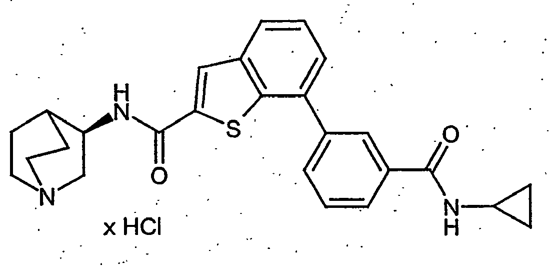

Gemäß der allgemeinen Arbeitsvorschrift E werden 50 mg (0.11 mmol) 3-(2-{[(3R)-1-Azabicyclo[2.2.2]oct-3-yl-amino]carbonyl}-1-benzothien-7-yl)-benzoesäure-Hydrochlorid (Beispiel 75) und 12.9 mg (0.23 mmol) Cyclopropylamin miteinander umgesetzt. Es werden 17.6 mg (31.1 % d.Th.) der Titelverbindung erhalten.
¹H-NMR (400 MHz, Methanol-d₄): δ = 8.18 (s, 1H), 8.15 (s, 1H), 7.93 (d, 1H), 7.88 (m, 2H), 7.62 (dd, 1H), 7.56 (m, 2H), 4.45 (m, 1H), 3.84 (m, 1H), 3.48 (m, 1H), 3.42-3.27 (m, 4H), 2.89 (m, 1H), 2.38 (m, 1H), 2.28 (m, 1H), 2.10 (m, 2H), 1.96 (m, 1H), 0.82 (m, 2H), 0.67 (m, 2H).
HPLC (Methode 1):Rₜ = 3.95 min.
LC-MS (Methode 6): Rₜ = 3.36 min.; m/z = 44.5 (M+H)⁺ (freie Base).

### Beispiel 136

### N-[(3R)-1-Azabicyclo[2.2.2]oct-3-yl]-7-(3-{[ethyl(methyl)amino]carbonyl}phenyl)-1-benzothiophen-2-carboxamid-Hydrochlorid

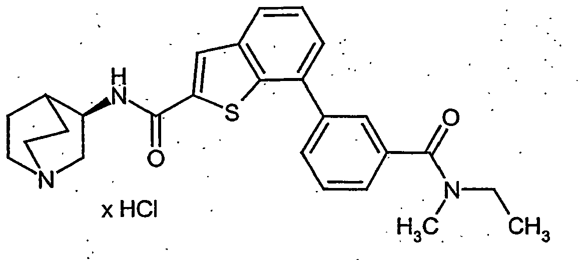

Gemäß der allgemeinen Arbeitsvorschrift E werden 50 mg (0.11 mmol) 3-(2-{[(3R)-1-Azabicyclo[2.2.2]oct-3-yl-amino]carbonyl}-1-benzothien-7-yl)-benzoesäure-Hydrochlorid (Beispiel 75) und 13.3 mg (0.23 mmol) Ethylmethylamin miteinander umgesetzt. Es werden 20.1 mg (36.8 % d.Th.) der Titelverbindung erhalten.
¹H-NMR (400 MHz, Methanol-d₄): δ = 8.19 (s, 1H), 7.95 (d, 1H), 7.82 (d, 1H), 7.73 (m, 1H), 7.64 (dd, 1H), 7.60-7.46 (m, 3H), 4.45 (m, 1H), 3.83 (m, 1H), 3.61 (m, 1H), 3.51-3.28 (m, 6H), 3.10 (m, 3H), 2.39 (m, 1H), 2.28 (m, 1H), 2.10 (m, 2H), 1.96 (m, 1H), 1.23 (m, 3H).
HPLC (Methode 1): Rₜ = 4.00 min.
LC-MS (Methode 6): Rₜ = 3.40 min.; m/z = 447 (M+H)⁺ (freie Base).

### Beispiel 137

### N-[(3R)-1-Azabicyclo[2.2.2]oct-3-yl]-7-[3-(2,5-dihydro-1H-pyrrol-1-ylcarbonyl)-phenyl]-1-benzothiophen-2-carboxamid-Hydrochlorid

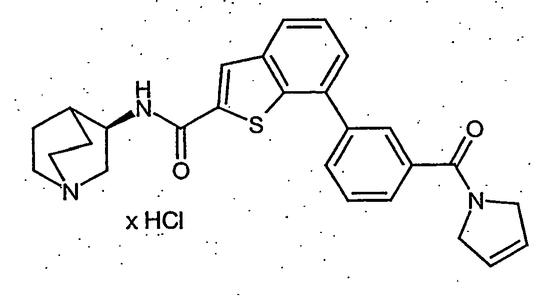

Gemäß der allgemeinen Arbeitsvorschrift E werden 50 mg (0.11 mmol) 3-(2-{[(3R)-1-Azabicyclo[2.2.2]oct-3-yl-amino]carbopyl}-1-benzothien-7-yl)-benzoesäure-Hydrochlorid (Beispiel 75) und 15.6 mg (0.23 mmol) 3-Pyrrolin miteinander umgesetzt. Es werden 20 mg (35.9 % d.Th.) der Titelverbindung erhalten.
HPLC (Methode 1): Rₜ = 4.00 min.
LC-MS (Methode 6): Rₜ =3.40 min.; m/z = 457 (M+H)⁺ (freie Base).

### Beispiel 138

### N-[(3R)-1-Azabicyclo[2.2.2]oct-3-yl]-7-(3-{[(3-methoxypropyl)amino]carbonyl}-phenyl)-1-benzothiophen-2-carboxamid-Hydrochlorid

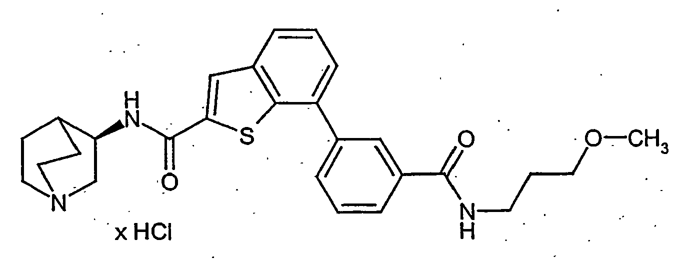

Gemäß der allgemeinen Arbeitsvorschrift E werden 50 mg (0.11 mmol) 3-(2-{[(3R)-1-Azabicyclo[2.2.2]oct-3-yl-amino]carbonyl}-1-benzothien-7-yl)-benzoesäure-Hydrochlorid (Beispiel 75) und 20.1 mg (0.23 mmol) 3-Methoxypropylamin miteinander umgesetzt. Es werden 29.2 mg (49.8 % d.Th.) der Titelverbindung erhalten.
HPLC (Methode 1): Rₜ = 3.94 min.
LC-MS (Methode 6): Rₜ = 3.37 min.; m/z = 477 (M+H)⁺ (freie Base).

### Beispiel 139

### N-[(3R)-1-Azabicyclo[2.2.2]oct-3-yl]-7-(3-{[(2-methoxyethyl)(methyl)amino]-carbonyl}phenyl)-1-benzothiophen-2-carboxamid-Hydrochlorid

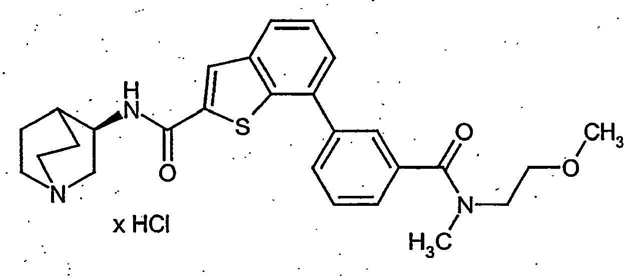

Gemäß der allgemeinen Arbeitsvorschrift E werden 50 mg (0.11 mmol) 3-(2-{[(3R)-1-Azabicyclo[2.2.2]oct-3-yl-amino]carbonyl}-1-benzothien-7-yl)-benzoesäure-Hydrochlorid (Beispiel 75) und 20.1 mg (0.23 mmol) (2-Methoxyethyl)methylamin miteinander umgesetzt. Es werden 20.5 mg (31.8 % d.Th.) der Titelverbindung erhalten.
HPLC (Methode 1): Rₜ = 3.93 min.
LC-MS (Methode 6): Rₜ = 3.35 min.; m/z = 477 (M+H)⁺ (freie Base).

### Beispiel 140

### N-[(3R)-1-Azabicyclo[2.2.2]oct-3-yl]-7-(3-{[(3-ethoxypropyl)amino]carbonyl}-phenyl)-1-benzothiophen-2-carboxamid-Hydrochlorid

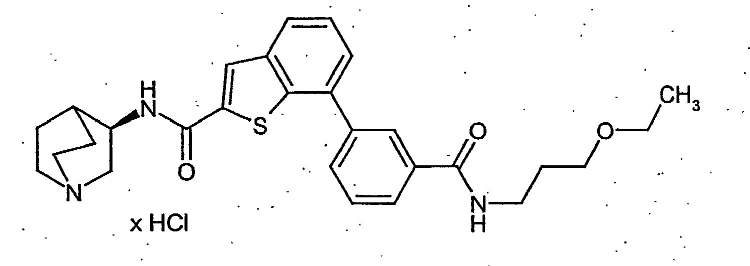

Gemäß der allgemeinen Arbeitsvorschrift E werden 50 mg (0.11 mmol) 3-(2-{[(3R)-1-Azabicyclo[2.2.2]oct-3-yl-amino]carbonyl}-1-benzothien-7-yl)-benzoesäure-Hydrochlorid (Beispiel 75) und 20.1 mg (0.23 mmol) 3-Ethoxypropylamin miteinander umgesetzt. Es werden 23.4 mg (37.1 % d.Th.) der Titelverbindung erhalten.
¹H-NMR (400 MHz, Methanol-d₄): δ = 8.18 (s, 1H), 8.15 (s, 1H), 7.94 (d, 1H), 7.89 (m, 2H), 7.64 (dd, 1H), 7.56 (m, 2H), 4.45 (m, 1H), 3.84 (m, 1H), 3.55 (m, 2H), 3.53-3.25 (m, 9H), 2.39 (m, 1H), 2.28 (m, 1H), 2.10 (m, 2H), 1.96 (m, 1H), 1.91 (m, 2H), 1.17 (m, 3H).
HPLC (Methode 1): Rₜ = 4.07 min.
LC-MS (Methode 6): Rₜ = 3.46 min.; m/z = 491 (M+H)⁺ (freie Base).

### Beispiel 141

### N-[(3R)-1-Azabicyclo[2.2.2]oct-3-yl]-7-{3-[(4-methyl-1-piperazinyl)carbonyl]-phenyl}-1-benzothiophen-2-carboxamid-Dihydrochlorid

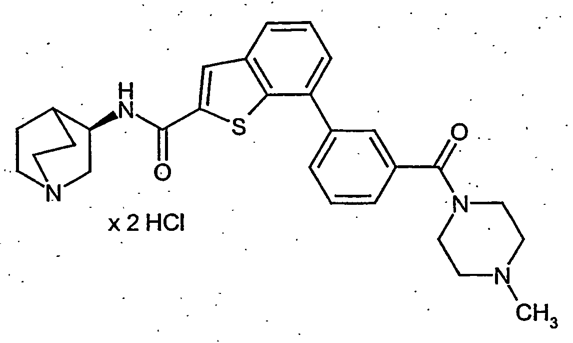

Gemäß der allgemeinen Arbeitsvorschrift E werden 50 mg (0.11 mmol) 3-(2-{[(3R)-1-Azabicyclo[2.2.2]oct-3-yl-amino]carbonyl}-1-benzothien-7-yl)-benzoesäure-Hydrochlorid (Beispiel 75) und 22.6 mg (0.23 mmol) *N*-Methylpiperazin miteinander umgesetzt. Es werden 4.2 mg (6.6 % d.Th.) der Titelverbindung erhalten.
¹H-NMR (400 MHz, Methanol-d₄): δ = 8.23 (s, 1H), 7.97 (dd, 1H), 7.88 (m, 2H), 7.69 (m, 1H), 7.57 (m, 3H), 4.46 (m, 1H), 3.84 (m, 1H), 3.50 (m, 1H), 3.46-3.25 (m, 12H), 2.97 (s, 3H), 2.39 (m, 1H), 2.29 (m, 1H), 2.10 (m, 2H), 1.96 (m, 1H).
HPLC (Methode 1): Rₜ = 3.62 min.
LC-MS (Methode 6): Rₜ = 2.94 min.; m/z = 488 (M+H)⁺ (freie Base).

### Beispiel 142

### N-[(3R)-1-Azabicyclo[2.2.2]oct-3-yl]-7-{3-[(cyclobutylcarbonyl)amino]phenyl}-1-benzothiophen-2-carboxamid-Hydrochlorid

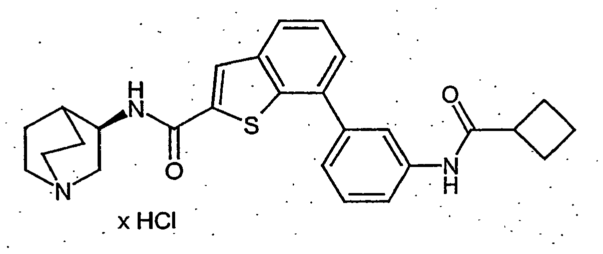

Gemäß der allgemeinen Arbeitsvorschrift F werden 50 mg (0.12 mmol) 7-(3-Aminophenyl)-*N*-[(3R)-1-azabicyclo[2.2.2]oct-3-yl]-1-benzothiophen-2-carboxamid-Hydrochlorid (Beispiel 21) und 28.6 mg (0.24 mmol) Cyclobutancarbonsäurechlorid miteinander umgesetzt. Es werden 38 mg (61.3 % d.Th.) der Titelverbindung erhalten.
HPLC (Methode 1): Rₜ = 4.22 min.
MS (ESIpos): m/z = 460 (M+H)⁺ (freie Base).

### Beispiel 143

### N-[3-(2-{[(3R)-1-Azabicyclo[2.2.2]oct-3-ylamino]carbonyl}-1-benzothien-7-yl)-phenyl]-5-isoxazolcarboxamid-Hydrochlorid

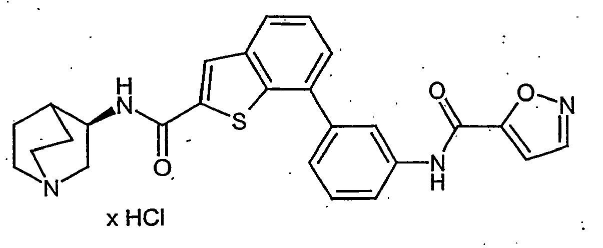

Gemäß der allgemeinen Arbeitsvorschrift F werden 50 mg (0.12 mmol) 7-(3-Aminophenyl)-*N*-[(3R)-1-azabicyclo[2.2.2]oct-3-yl]-1-benzothiophen-2-carboxamid-Hydrochlorid (Beispiel 21) und 31.8 mg (0.24 mmol) Isoxazol-5-carbonsäurechlorid miteinander umgesetzt. Es werden 44.4 mg (72.6 % d.Th.) der Titelverbindung erhalten.
¹H-NMR (400 MHz, Methanol-d₄): δ = 8.58 (d, 1H); 8.18 (s, 2H), 7.93 (dd, 1H), 7.79 (m, 1H), 7.55 (m, 4H), 7.13 (m, 1H), 4.45 (m, 1H), 3.83 (m, 1H), 3.48 (m, 1H), 3.42-3.27 (m, 4H), 2.39 (m, 1H), 2.28 (m, 1H), 2.10 (m, 2H), 1.96 (m, 1H).
HPLC (Methode 1): Rₜ = 4.12 min.
MS (ESIpos): m/z = 473 (M+H)⁺ (freie Base).

### Beispiel 144

### N-[(3R)-1-Azabicyclo[2.2.2]oct-3-yl]-7-{3-[(cyclopentylcarbonyl)amino]phenyl}-1-benzothiophen-2-carboxamid-Hydrochlorid

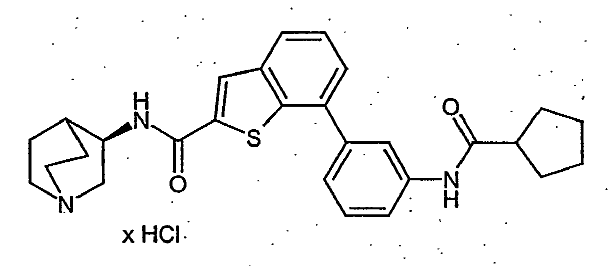

Gemäß der allgemeinen Arbeitsvorschrift F werden 50 mg (0.12 mmol) 7-(3-Aminophenyl)-*N*-[(3R)-1-azabicyclo[2.2.2]oct-3-yl]-1-benzothiophen-2-carboxamid-Hydrochlorid (Beispiel 21) und 32 mg (0.24 mmol) Cyclopentylcarbonsäurechlorid miteinander umgesetzt. Es werden 30.5 mg (52.8 % d.Th.) der Titelverbindung erhalten.
¹H-NMR (400 MHz, Methanol-d₄): δ = 8.17 (s, 1H), 8.03 (m, 1H), 7.91 (dd, 1H), 7.60 (m, 1H), 7.57-7.38 (m, 4H), 4.45 (m, 1H), 3.83 (m, 1H), 3.48 (m, 1H), 3.42-3.27 (m, 4H), 2.86 (m, 1H), 2.38 (m, 1H), 2.28 (m, 1H), 2.10 (m, 2H), 1.96 (m, 3H), 1.90-1.72 (m, 4H), 1.66.(m, 2H).
HPLC (Methode 1): Rₜ = 4.40 min.
MS (ESIpos): m/z = 474 (M+H)⁺ (freie Base).

### Beispiel 145

### N-[(3R)-1-Azabicyclo[2.2.2]oct-3-yl]-7-{3-[(cyclohexylcarbonyl)amino]phenyl}-1-benzothiophen-2-carboxamid-Hydrochlorid

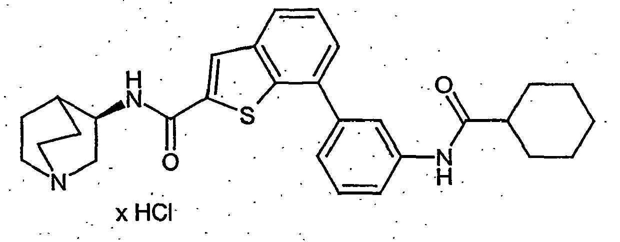

Gemäß der allgemeinen Arbeitsvorschrift F werden 50 mg (0.12 mmol) 7-(3-Aminophenyl)-*N*-[(3R)-1-azabicyclo[2.2.2]oct-3-yl]-1-benzothiophen-2-carboxamid-Hydrochlorid (Beispiel 21) und 35.4 mg (0.24 mmol) Cyclohexylcarbonsäurechlorid miteinander umgesetzt. Es werden 9.8 mg (16.2 % d.Th.) der Titelverbindung erhalten.
HPLC (Methode 1): Rₜ = 4.51 min.
MS (ESIpos): m/z = 488 (M+H)⁺ (freie Base).

### Beispiel 146

### N-[(3R)-1-Azabicyclo[2.2.2]oct-3-yl]-7-{3-[(tetrahydro-2-furanylcarbonyl)amino]-phenyl}-1-benzothiophen-2-carboxamid-Hydrochlorid

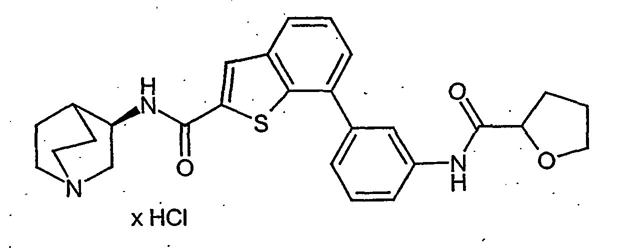

Gemäß der allgemeinen Arbeitsvorschrift F werden 50 mg (0.12 mmol) 7-(3-Aminophenyl)-*N*-[(3R)-1-azabicyclo[2.2.2]oct-3-yl]-1-benzothiophen-2-carboxamid-Hydrochlorid (Beispiel 21) und 32.5 mg (0.24 mmol) Tetrahydrofuran-2-carbonsäurechlorid miteinander umgesetzt. Es werden 40.9 mg (68.1 % d.Th.) der Titelverbindung erhalten.
¹H-NMR (400 MHz, Methanol-d₄): δ = 8.17 (s, 1H), 8.04 (s, 1H), 7.91 (d, 1H), 7.61 (m, 1H), 7.58-7.40 (m, 4H), 4.46 (m, 1H), 4.04 (dd, 1H), 3.92 (m, 2H), 3.83 (m, 2H), 3.48 (m, 1H), 3.42-3.27 (m, 4H), 3.23 (m, 1H), 2.38 (m, 1H), 2.28 (m, 1H), 2.22 (m, 2H), 2.10 (m, 2H), 1.96 (m, 1H).
HPLC (Methode 1): Rₜ = 3.99 min.
MS (ESIpos): m/z = 476 (M+H)⁺ (freie Base).

### Beispiel 147

### N-[(3R)-1-Azabicyclo[2.2.2]oct-3-yl]-7-[3-(isobutyrylamino)phenyl]-1-benzothiophen-2-carboxamid-Hydrochlorid

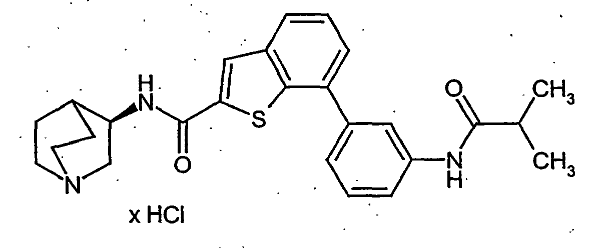

Gemäß der allgemeinen Arbeitsvorschrift F werden 50 mg (0.12 mmol) 7-(3-Aminophenyl)-*N*-[(3R)-1-azabicydo[2.2.2]oct-3-yl]-1-benzothiophen-2-carboxamid-Hydrochlorid (Beispiel 21) und 25.7 mg (0.24 mmol) Isobuttersäurechlorid miteinander umgesetzt. Es werden 35.1 mg (64.9 % d.Th.) der Titelverbindung erhalten.
¹H-NMR (400 MHz, Methanol-d₄): δ = 8.17 (s, 1H), 8.03 (s, 1H), 7.90 (d, 1H), 7.61 (m, 1H), 7.57-7.40 (m, 4H), 4.45 (m, 1H), 3.83 (m, 1H), 3.48 (m, 1H), 3.42-3.27 (m, 4H), 2.68 (m, 1H), 2.38 (m, 1H), 2.28 (m, 1H), 2.10 (m, 2H), 1.96 (m, 1H), 1.21 (d, 6H).
HPLC (Methode 1): Rₜ = 4.19 min.
MS (ESIpos): m/z = 448 (M+H)⁺ (freie Base).

### Beispiel 148

### N-[(3R)-1-Azabicyclo[2.2.2]oct-3-yl]-7-[3-(2-furoylamino)phenyl]-1-benzothiopheri-2-carboxamid-Hydrochlorid

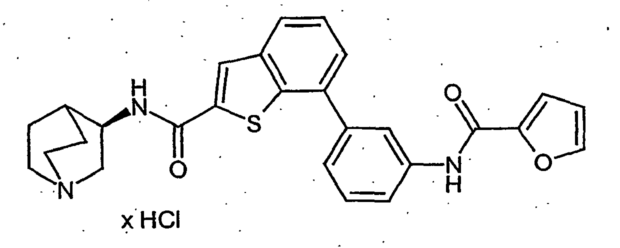

Gemäß der allgemeinen Arbeitsvorschrift F werden 50 mg (0.12 mmol) 7-(3-Aminophenyl)-*N*-[(3R)-1-azabicyclo[2.2.2]oct-3-yl]-1-benzothiophen-2-carboxamid-Hydrochlorid (Beispiel 21) und 31.5 mg (0.24 mmol) Furan-2-carbonsäurechlorid miteinander umgesetzt. Es werden 29.1 mg (51.1 % d.Th.) der Titelverbindung erhalten.
¹H-NMR (400 MHz, Methanol-d₄): δ = 8.17 (s, 2H), 7.91 (d, 1H), 7.74 (m, 2H), 7.58-7.47 (m, 4H), 7.29 (d, 1H), 6.65 (m, 1H), 4.44 (m, 1H), 3.83 (m, 1H), 3.47 (m, 1H), 3.42-3.27 (m, 4H), 2.38 (m, 1H), 2.28 (m, 1H), 2.10 (m, 2H), 1.96 (m, 1H).
HPLC (Methode 1): Rₜ = 4.19 min.
MS (ESIpos): m/z = 472 (M+H)⁺ (freie Base).

### Beispiel 149

### 3-(2-{[(3R)-1-Azabicyclo[2.2.2]oct-3-yl-amino]carbonyl}-1-benzofuran-7-yl)-benzoesäure-Hydrochlorid

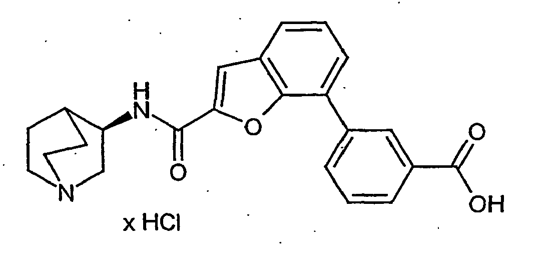

Zu einer Mischung aus 1104 mg (2.86 mmol) *N*-[(3R)-1-Azabicyclo [2.2.2] oct-3-yl]-7-brom-1-benzofuran-2-carboxamid-Hydrochlorid (Beispiel 30A) und 475 mg (2.86 mmol) 3-Carboxyphenylboronsäure in 10 mL DMF werden 4.3 mL 2 M wässrige Natriumcarbonat-Lösung und 116.9 mg (0.14 mmol) PdCl₂ (dppf) gegeben. Das Reaktionsgemisch wird 18h auf 90°C erhitzt, dann über Kieselgur filtriert und zur Trockene eingeengt. Nach Reinigung des Rohprodukts mittels präparativer HPLC, anschließendem Versetzen mit einem 3:1-Gemisch aus Acetonitril und 1 N Salzsäure, Einengen sowie Trocknen im Hochvakuum erhält man 724 mg (59.2 % d.Th.) der Titelverbindung.
¹H-NMR (400 MHz, Methanol-d₄): δ = 8.62 (s, 1H), 8.11 (d, 1H), 8.08 (d, 1H), 7.76 (d, 1H), 7.73-7.61 (m, 3H), 7.46 (dd, 1H), 4.49 (m, 1H), 3.83 (m, 1H), 3.48 (m, 1H), 3.43-3.27 (m, 4H), 2.40 (m, 1H), 2.28 (m, 1H), 2.10 (m, 2H), 1.96 (m, 1H).
HPLC (Methode 1): Rₜ = 3.89 min.
MS (ESIpos): m/z = 391 (M+H)⁺ (freie Base).

### Beispiel 150

### N-[(3R)-1-Azabicyclo[2.2.2]oct-3-yl]-7-[2-(hydroxymethyl)phenyl]-1-benzofuran-2-carboxamid-Hydrochlorid

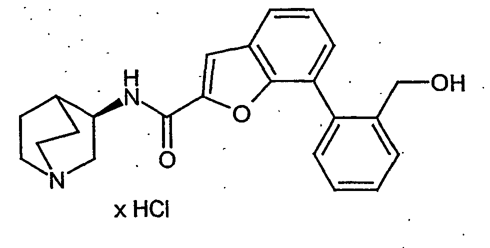

Zu einer Mischung aus 150 mg (0.43 mmol) *N*-[(3R)-1-Azabicyclo[2.2.2]oct-3-yl]-7-brom-1-benzofuran-2-carboxamid-Hydrochlorid (Beispiel 30A) und 65.3 mg (0.43 mmol) 2-(Hydroxymethyl)phenylboronsäure in 1.5 mL DMF werden 0.64 mL 2 M wässrige Natriumcarbonat-Lösung und 17.5 mg (0.02 mmol) PdCl₂(dppf) gegeben. Das Reaktionsgemisch wird 18 h auf 90°C erhitzt, dann über Kieselgur filtriert und zur Trockene eingeengt. Nach Reinigung des Rohprodukts mittels präparativer HPLC, anschließendem Versetzen mit einem 3:1-Gemisch aus Acetonitril und 1 N Salzsäure, Einengen sowie Trocknen im Hochvakuum erhält man 13 mg (7.1 % d.Th.) der Titelverbindung.
HPLC (Methode 1): Rₜ = 3.87 min.
MS (ESIpos): m/z = 377 (M+H)⁺ (freie Base).

### Beispiel 151

### N-[(3R)-1-Azabicyclo[2.2.2]oct-3-yl]-7-(2,5-dimethoxyphenyl)-1-benzofuran-2-carboxamid-Hydrochlorid

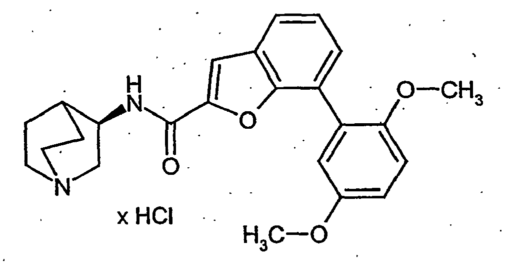

Zu einer Mischung aus 200 mg (0.52 mmol) *N*-[(3R)-1-Azabicyclo[2.2.2]oct-3-yl]-7-brom-1-benzofuran-2-carboxamid-Hydrochlorid (Beispiel 30A) und 94.4 mg (0.52 mmol) 2,5-Dimethoxyphenylboronsäure in 2 mL DMF werden 0.78 mL 2 M wässrige Natriumcarbonat-Lösung und 21.2 mg (0.03 mmol) PdCl₂(dppf) gegeben. Das Reaktionsgemisch wird 17 h auf 70°C erhitzt, dann über Kieselgur filtriert und zur Trockene eingeengt. Nach Reinigung des Rohprodukts mittels präparativer HPLC, anschließendem Versetzen mit einem 3:1-Gemisch aus Methanol und 1 N Salzsäure, Einengen sowie Trocknen im Hochvakuum erhält man 75 mg (31.7 % d.Th.) der Titelverbindung.
HPLC (Methode 1): Rₜ = 4.15 min.
MS (ESIpos): m/z = 407 (M+H)⁺ (freie Base).

### Beispiel 152

### 7-[2-(Aminomethyl)phenyl]-N-[(3R)-1-azabicyclo[2.2.2]oct-3-yl]-1-benzofuran-2-carboxamid-Dihydrochlorid

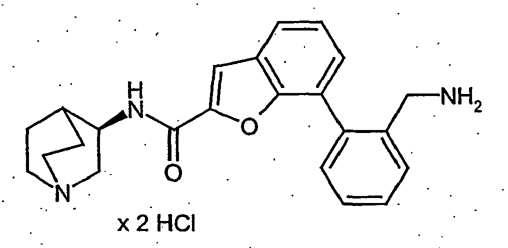

Gemäß der allgemeinen Arbeitsvorschrift D werden 500 mg (1.75 mmol) tert.-Butyl-2-brombenzylcarbamat, 512 mg (2.02 mmol) Bis(pinacolato)dibor, 428.7 mg (4.37 mmol) Kaliumacetat, 49.2 mg (0.07 mmol) PdCl₂(dppf), 518.4 mg (1.34 mmol) *N-*[(3R)-1-Azabicyclo[2.2.2]oct-3-yl]-7-brom-1-benzofuran-2-carboxamid-Hydrochlorid (Beispiel 30A), 3.36 mL 2 M Natriumcarbonat-Lösung und weitere 49.2 mg (0.07 mmol) PdCl₂(dppf) in 5 mL DMF umgesetzt. Das im Hochvakuum getrocknete Rohprodukt wird in 8 mL einer 1:1-Mischung aus Methanol und 4 M Chlorwasserstoff in Dioxan 2 h lang bei Raumtemperatur gerührt. Die Reaktionslösung wird eingeengt und das erhaltene Rohprodukt mittels präparativer HPLC aufgereinigt. Die Produktfraktionen werden eingeengt, mit einer 3:1-Mischung aus Methanol und 1 N Salzsäure versetzt, erneut eingeengt und im Hochvakuum getrocknet. Man erhält 245.5 mg (44.5 % d.Th.) der Titelverbindung.
¹H-NMR (300 MHz, DMSO-d₆): δ =10.48 (br. s, 1H), 9.26 (d, 1H), 8.47 (br. s, 3H), 8.05 (s, 1H), 7.88 (dd, 1H), 7.80 (d, 1H), 7.62-7.40 (m, 5H), 4.31 (m, 1H), 3.86 (m, 2H), 3.48 (m, 1H), 3.51-3.10 (m, 5H), 2.18 (m, 1H), 2.11 (m, 1H), 1.90 (m, 2H), 1.71 (m, 1H).
HPLC (Methode 7): Rₜ = 3.55 min.
MS (ESIpos): m/z = 376 (M+H)⁺ (freie Base).

### Beispiel 153

### 3-(2-{[(3S)-1-Azabicyclo[2.2.2]oct-3-yl-amino]carbonyl}-1-benzofuran-7-yl)-benzoesäure-Hydrochlorid

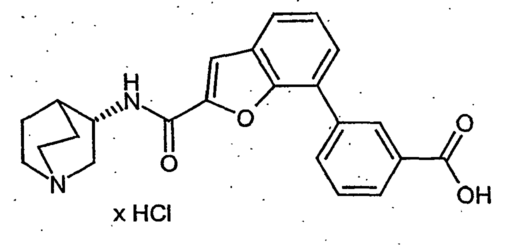

Zu einer Mischung aus 1000 mg (2.59 mmol) *N*-[(3S)-1-Azabicyclo[2.2.2]oct-3-yl]-7-brom-1-benzofuran-2-carboxamid-Hydrochlorid (freie Base: Beispiel 31A) und 430.2 mg (2.59 mmol) 3-Carboxyphenylboronsäure in 8 mL DMF werden 3.89 mL 2 M wässrige Natriumcarbonat-Lösung und 105.9 mg (0.13 mmol) PdCl₂(dppf) gegeben. Das Reaktionsgemisch wird 18 h auf 70°C erhitzt, dann über Kieselgur filtriert und zur Trockene eingeengt. Nach Reinigung des Rohprodukts mittels präparativer HPLC, anschließendem Versetzen mit einem 3:1-Gemisch aus Methanol und 1 N Salzsäure, Einengen sowie Trocknen im Hochvakuum erhält man 142.5 mg (12 % d.Th.) sowie weitere 627.9 mg (mit 80% Reinheit) der Titelverbindung.
Die spektroskopischen Daten stimmen mit denen der enantiomeren Verbindung (Beispiel 149) überein.

### Beispiel 154

### N-[(3R)-1-Azabicyclo[2.2.2]oct-3-yl]-7-{3-[(cyclopropylamino)carbonyl]phenyl}-1-benzofuran-2-carboxamid-Hydrochlorid

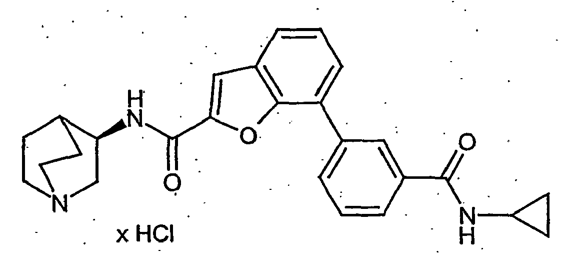

Gemäß der allgemeinen Arbeitsvorschrift E werden 50 mg (0.12 mmol) 3-(2-{[(3R)-1-Azabicyclo[2.2.2]oct-3-yl-amino]carbonyl}-1-benzofuran-7-yl)-benzoesäure-Hydrochlorid (Beispiel 149) und 13.4 mg (0.23 mmol) Cyclopropylamin miteinander umgesetzt. Es werden 20 mg (32.2 % d.Th.) der Titelverbindung erhalten.
HPLC (Methode 1): Rₜ = 3.93 min.
LC-MS (Methode 6): Rₜ = 3.33 min.; m/z = 429 (M+H)⁺ (freie Base).

### Beispiel 155

### N-[(3R)-1-Azabicyclo[2.2.2]oct-3-yl]-7-(3-{[ethyl(methyl)amino]carbonyl}phenyl)-1-benzofuran-2-carboxamid-Hydrochlorid

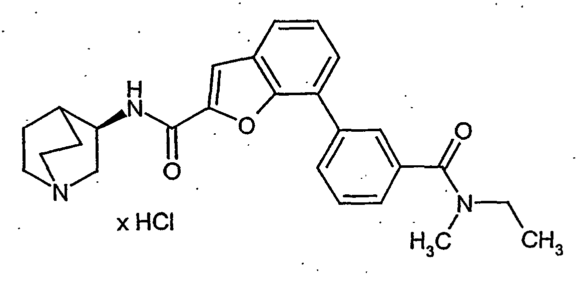

Gemäß der allgemeinen Arbeitsvorschrift E werden 50 mg (0.12 mmol) 3-(2-{[(3R)-1-Azabicyclo[2.2.2]oct-3-yl-amino]carbonyl}-1-benzofuran-7-yl)-benzoesäure-Hydrochlorid (Beispiel 149) und 13.9 mg (0.23 mmol) Methylethylamin miteinander umgesetzt. Es werden 19.8 mg (29.4 % d.Th.) der Titelverbindung erhalten.
HPLC (Methode 1): Rₜ = 4.03 min.
LC-MS (Methode 6): Rₜ = 3.38 min.; m/z = 431 (M+H)⁺ (freie Base).

### Beispiel 156

### N-[(3R)-1-Azabicyclo[2.2.2]oct-3-yl]-7-{3-[(butylamino)carbonyl]phenyl}-1-benzofuran-2-carboxamid-Hydrochlorid

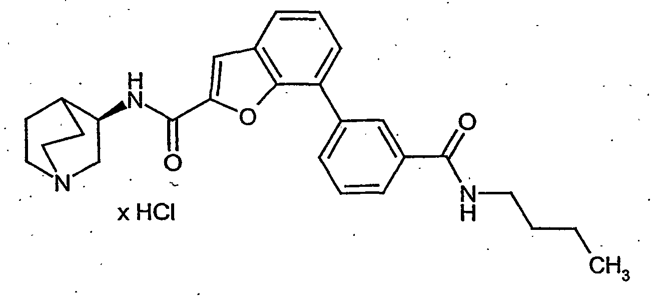

Gemäß der allgemeinen Arbeitsvorschrift E werden 50 mg (0.12 mmol). 3-(2-{[(3R)-1-Azabicyclo[2.2.2]oct-3-yl-amino]carbonyl}-1-benzofuran-7-yl)-benzoesäure-Hydrochlorid (Beispiel 149) und 17.1 mg (0.23 mmol) *n*-Butylamin miteinander umgesetzt. Es werden 15.2 mg (26.2 % d.Th.) der Titelverbindung erhalten.
HPLC (Methode 1): Rₜ = 4.21 min.
LC-MS (Methode 6): Rₜ = 3.49 min.; m/z = 445 (M+H)⁺ (freie Base).

### Beispiel 157

### N-[(3R)-1-Azabicyclo[2.2.2]oct-3-yl]-7-{3-[(isobutylamino)carbonyl]phenyl}-1-, benzofuran-2-carboxamid-Hydrochlorid

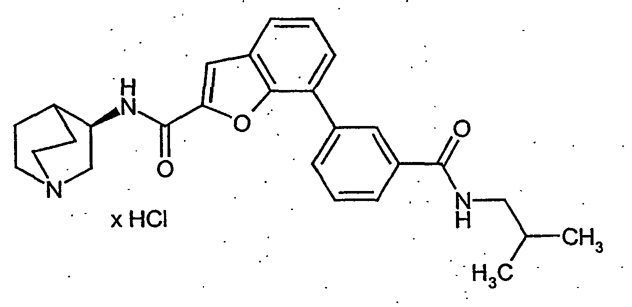

Gemäß der allgemeinen Arbeitsvorschrift E werden 50 mg (0.12 mmol) 3-(2-{[(3R)-1-Azabicyclo[2.2.2]oct-3-yl-amino]carbonyl}-1-benzofuran-7-yl)-benzoesäure-Hydrochlorid (Beispiel 149) und 17.1 mg (0.23 mmol) *iso*-Butylamin miteinander umgesetzt. Es werden 15.2 mg (26.9 % d.Th.) der Titelverbindung erhalten.
HPLC (Methode 1): Rₜ = 4.18 min.
LC-MS (Methode 6): Rₜ = 3.49 min.; m/z = 445 (M+H)⁺ (freie Base).

### Beispiel 158

### N-[(3R)-1-Azabicyclo[2.2.2]oct-3-yl]-7-[3-(1-piperidinylcarbonyl)phenyl]-1-benzofuran-2-carboxamid-Hydrochlorid

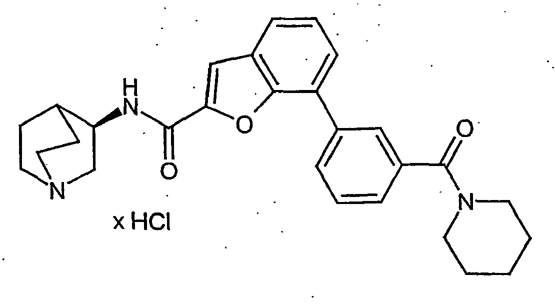

Gemäß der allgemeinen Arbeitsvorschrift E werden 50 mg (0.12 mmol) 3-(2-{[(3R)-1-Azabicyclo[2.2.2]oct-3-yl-amino]carbonyl}-1-benzofuran-7-yl)-benzoesäure-Hydrochlorid (Beispiel 149) und 20.0 mg (0.23 mmol) Piperidin miteinander umgesetzt. Es werden 16.4 mg (27.6 % d.Th.) der Titelverbindung erhalten.
HPLC (Methode 1): Rₜ = 4.22 min.
LC-MS (Methode 6): Rₜ = 3.51 min.; m/z = 457 (M+H)⁺ (freie Base).

### Beispiel 159

### N-[(3R)-1-Azabicyclo[2.2.2]oct-3-yl]-7-[3-({[2-(dimethylamino)ethyl]amino}-carbonyl)phenyl]-1-benzofuran-2-carboxamid-Hydrochlorid

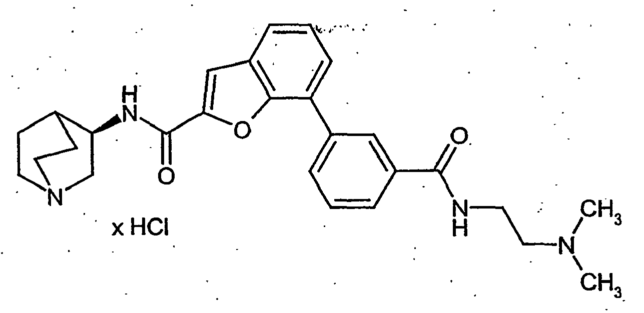

Gemäß der allgemeinen Arbeitsvorschrift E werden 50 mg (0.12 mmol) 3-(2-{[(3R)-1-Azabicyclo[2.2.2]oct-3-yl-amino]carbonyl}-1-benzofuran-7-yl)-benzoesäure-Hydrochlorid (Beispiel 149) und 20.7 mg (0.23 mmol) *N*-(2-Aminoethyl)-*N,N-*dimethylamin miteinander umgesetzt. Es werden 17.4 mg (24.8 % d.Th.) der Titelverbindung erhalten.
HPLC (Methode 1): Rₜ = 3.64 min.
LC-MS (Methode 6): Rₜ = 2.93 min.; m/z = 460 (M+H)⁺ (freie Base).

### Beispiel 160

### N-[(3R)-1-Azabicyclo[2.2.2]oct-3-yl]-7-(3-{[(3-methoxypropyl)amino]carbonyl}-phenyl)-1-benzofuran-2-carboxamid-Hydrochlorid

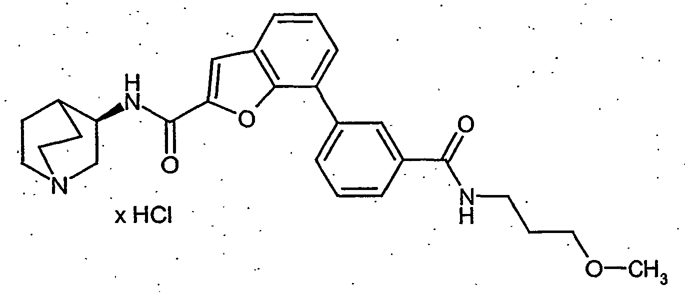

Gemäß der allgemeinen Arbeitsvorschrift E werden 50 mg (0.12 mmol) 3-(2-{[(3R)-1-Azabicylo[2.2.2]oct-3-yl-amino]carbonyl}-1-benzofuran-7-yl)-benzoesäure-Hydrochlorid (Beispiel 149) und 20.9 mg (0.23 mmol) 3-Methoxypropylamin miteinander umgesetzt. Es werden 22.7 mg (36.4 % d.Th.) der Titelverbindung erhalten.
HPLC (Methode 1): Rₜ = 3.93 min.
LC-MS (Methode 6): Rₜ = 3.36 min.; m/z = 461 (M+H)⁺ (freie Base).

### Beispiel 161

### N-[(3R)-1-Azabicyclo[2.2.2]oct-3-yl]-7-(3-{[(2-methoxyethyl)(methyl)amino]-carbonyl}phenyl)-1-benzofuran-2-carboxamid-Hydrochlorid

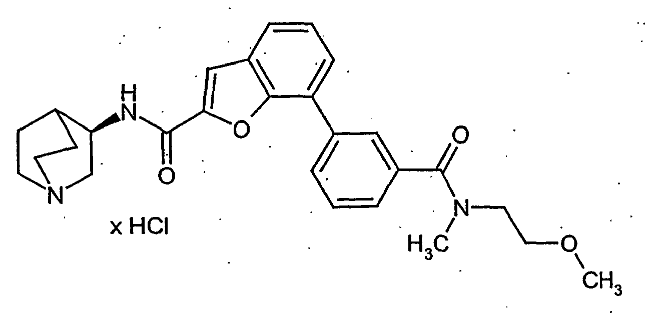

Gemäß der allgemeinen Arbeitsvorschrift E werden 50 mg (0.12 mmol) 3-(2-{[(3R)-1-Azabicyclo[2.2.2]oct-3-yl-amino]carbonyl}-1-benzofuran-7-yl)-benzoesäure-Hydrochlorid (Beispiel 149) und 20.9 mg (0.23 mmol) *N*-(2-Methoxyethyl)-*N-*methylamin miteinander umgesetzt. Es werden 20.4 mg (31.3 % d.Th.) der Titelverbindung erhalten.
HPLC (Methode 1): Rₜ = 3.96 min.
LC-MS (Methode 6): Rₜ = 3.34 min.; m/z = 461 (M+H)⁺ (freie Base).

### Beispiel 162

### N-[(3R)-1-Azabicyclo[2.2.2]oct-3-yl]-7-(3-{[(3-ethoxypropyl)amino]carbonyl}-phenyl)-1-benzofuran-2-carboxamid-Hydrochlorid

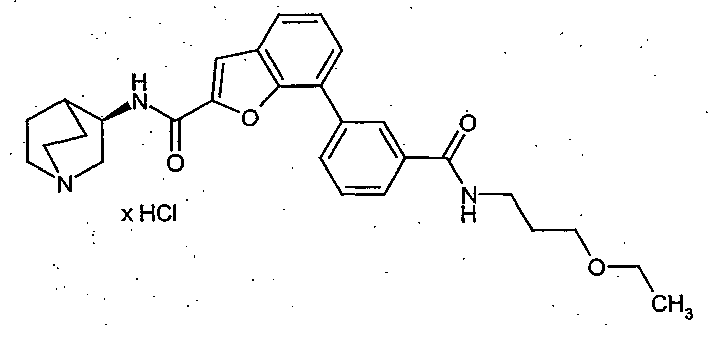

Gemäß der allgemeinen Arbeitsvorschrift E werden 50 mg (0.12 mmol) 3-(2-{[(3R)-1-Azabicyclo[2.2.2]oct-3-yl-amino]carbonyl}-1-benzofuran-7-yl)-benzoesäure-Hydrochlorid (Beispiel 149) und 24.2 mg (0.23 mmol) 3-Ethoxypropylamin miteinander umgesetzt. Es werden 17.8 mg (28.9 % d.Th.) der Titelverbindung erhalten.
HPLC (Methode 1): Rₜ = 4.06 min.
LC-MS (Methode 6): Rₜ = 3.43 min.; m/z = 475 (M+H)⁺ (freie Base).

### Beispiel 163

### N-[(3R)-1-Azabicyclo[2.2.2]oct-3-yl]-7-{3-[(4-methyl-1-piperazinyl)carbonyl]-phenyl}-1-benzofuran-2-carboxamid-Dihydrochlorid

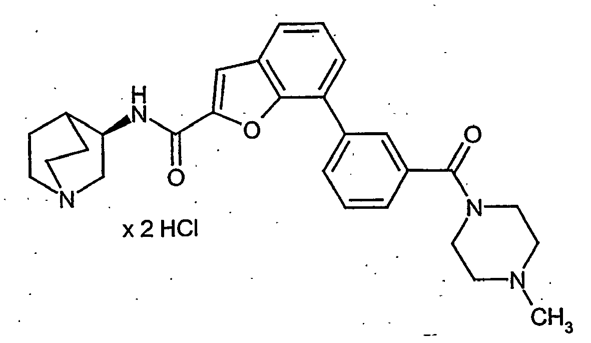

Gemäß der allgemeinen Arbeitsvorschrift E werden 50 mg (0.12 mmol) 3-(2-{[(3R)-1-Azabicyclo[2.2.2]oct-3-yl-amino]carbonyl}-1-benzofuran-7-yl)-benzoesäure-Hydrochlorid (Beispiel 149) und 23.5 mg (0.23 mmol) *N*-Methylpiperazin miteinander umgesetzt. Es werden 29.6 mg (41.9 % d.Th.) der Titelverbindung erhalten.
HPLC (Methode 1): Rₜ = 3.60 min.
LC-MS (Methode 6): Rₜ = 2.91 min.; m/z = 472 (M+H)⁺ (freie Base).

### Beispiel 164

### N-[(3S)-1-Azabicyclo[2.2.2]oct-3-yl]-7-(3-{[ethyl(methyl)amino]carbonyl}phenyl)-1-benzofuran-2-carboxamid-Hydrochlorid

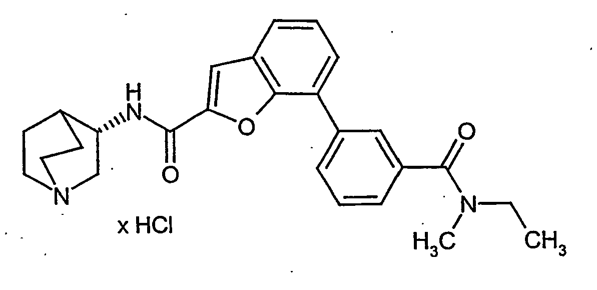

Gemäß der allgemeinen Arbeitsvorschrift E werden 50 mg (0.12 mmol) 3-(2-{[(3S)-1-Azabicyclo[2.2.2]oct-3-yl-amino]carbonyl}-1-benzofuran-7-yl)-benzoesäure-Hydrochlorid (Beispiel 153) und 13.9 mg (0.23 mmol) Methylethylamin miteinander umgesetzt. Es werden 50.1 mg (91.4 % d.Th.) der Titelverbindung erhalten.
Die spektroskopischen Daten stimmen mit denen der enantiomeren Verbindung (Beispiel 155) überein.

### Beispiel 165

### N-[(3S)-1-Azabicyclo[2.2.2]oct-3-yl]-7-{3-[(butylamino)carbonyl]phenyl}-1-benzofuran-2-carboxamid-Hydrochlorid

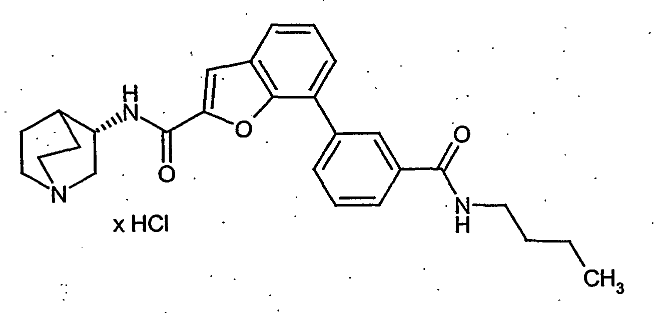

Gemäß der allgemeinen Arbeitsvorschrift E werden 50 mg (0.12 mmol) 3-(2-{[(3S)-1-Azabicyclo[2.2.2]oct-3-yl-amino]carbonyl}-1-benzofuran-7-yl)-benzoesäure-Hydrochlorid (Beispiel 153) und 17.1 mg (0.23 mmol) *n*-Butylamin miteinander umgesetzt. Es werden 49.4 mg (87.5 % d.Th.) der Titelverbindung erhalten.
Die spektroskopischen Daten stimmen mit denen der enantiomeren Verbindung (Beispiel 156) überein.

### Beispiel 166

### N-[(3S)-1-Azabicyclo[2.2.2]oct-3-yl]-7-{3-[(isobutylamino)carbonyl]phenyl}-1-benzofuran-2-carboxamid-Hydrochlorid

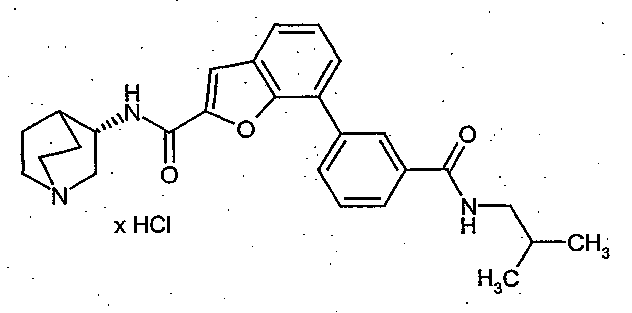

Gemäß der allgemeinen Arbeitsvorschrift E werden 50 mg (0.12 mmol) 3-(2-{[(3S)-1-Azabicyclo[2.2.2]oct-3-yl-amino]carbonyl}-1-benzofuran-7-yl)-benzoesäure-Hydrochlorid (Beispiel 153) und 17.1 mg (0.23 mmol) *iso*-Butylamin miteinander umgesetzt. Es werden 40.3 mg (71.4 % d.Th.) der Titelverbindung erhalten.
Die spektroskopischen Daten stimmen mit denen der enantiomeren Verbindung (Beispiel 157) überein.

### Beispiel 167

### N-[(3S)-1-Azabicyclo[2.2.2]oct-3-yl]-7-[3-(1-piperidinylcarbonyl)phenyl]-1-benzofuran-2-carboxamid-Hydrochlorid

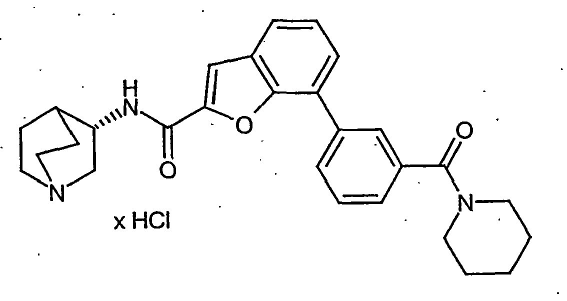

Gemäß der allgemeinen Arbeitsvorschrift E werden 50 mg (0.12 mmol) 3-(2-{[(3S)-1-Azabicyclo[2.2.2]oct-3-yl-amino]carbonyl}-1-benzofuran-7-yl)-benzoesäure-Hydrochlorid (Beispiel 153) und 20.0 mg (0.23 mmol) Piperidin miteinander umgesetzt. Es werden 29.7 mg (49.9 % d.Th.) der Titelverbindung erhalten.
Die spektroskopischen Daten stimmen mit denen der enantiomeren Verbindung (Beispiel 158) überein.

### Beispiel 168

### N-[(3S)-1-Azabicyclo[2.2.2]oct-3-yl]-7-[3-({[2-(dimethylamino)ethyl]amino}-carbonyl)phenyl]-1-benzofuran-2-carboxamid-Hydrochlorid

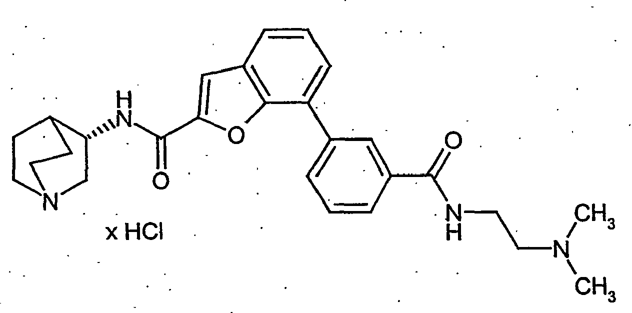

Gemäß der allgemeinen Arbeitsvorschrift E werden 50 mg (0.12 mmol) 3-(2-{[(3S)-1-Azabicyclo[2.2.2]oct-3-yl-amino]carbonyl}-1-benzofuran-7-yl)-benzoesäure-Hydrochlorid (Beispiel 153) und 20.7 mg (0.23 mmol) *N*-(2-Aminoethyl)-*N,N-*dimethylamin miteinander umgesetzt. Es werden 42.5 mg (64.5 % d.Th.) der Titelverbindung erhalten.
Die spektroskopischen Daten stimmen mit denen der enantiomeren Verbindung (Beispiel 159) überein.

### Beispiel 169

### N-[(3S)-1-Azabicyclo[2.2.2]oct-3-yl]-7-(3-{[(3-methoxypropyl)amino]carbonyl}-phenyl)-1-benzofuran-2-carboxamid-Hydrochlorid

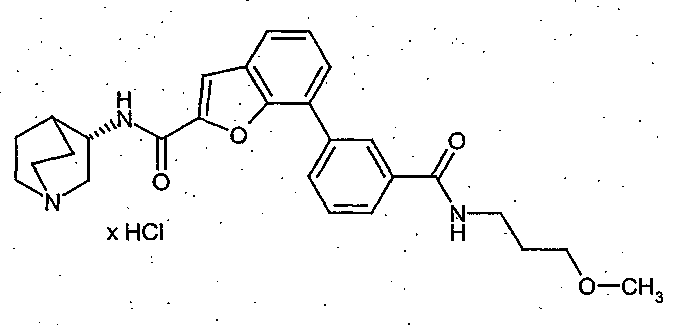

Gemäß der allgemeinen Arbeitsvorschrift E werden 50 mg (0.12 mmol) 3-(2-{[(3S)-1-Azabicyclo[2.2.2]oct-3-yl-amino]carbonyl}-1-benzofuran-7-yl)-benzoesäure-Hydrochlorid (Beispiel 153) und 20.9 mg (0.23 mmol) 3-Methoxypropylamin miteinander umgesetzt. Es werden 29.8 mg (44.5 % d.Th.) der Titelverbindung erhalten.
Die spektroskopischen Daten stimmen mit denen der enantiomeren Verbindung (Beispiel 160) überein.

### Beispiel 170

### N-[(3S)-1-Azabicyclo[2.2.2]oct-3-yl]-7-(3-{[(2-methoxyethyl)(methyl)amino]-carbonyl}phenyl)-1-benzofuran-2-carboxamid-Hydrochlorid

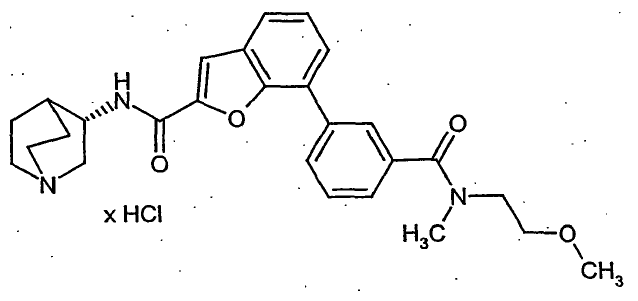

Gemäß der allgemeinen Arbeitsvorschrift E werden 50 mg (0.12 mmol) 3-(2-{[(3S)-1-Azabicyclo[2.2.2]oct-3-yl-amino]carbonyl}-1-benzofuran-7-yl)-benzoesäure-Hydrochlorid (Beispiel 153) und 20.9 mg (0.23 mmol) *N*-(2-Methoxyethyl)-*N-*methylamin miteinander umgesetzt. Es werden 22.1 mg (35.1 % d.Th.) der Titelverbindung erhalten.
Die spektroskopischen Daten stimmen mit denen der enantiomeren Verbindung (Beispiel 161) überein.

### Beispiel 171

### N-[(3S)-1-Azabicyclo[2.2.2]oct-3-yl]-7-(3-{[(3-ethoxypropyl)amino]carbonyl}-phenyl)-1-benzofuran-2-carboxamid-Hydrochlorid

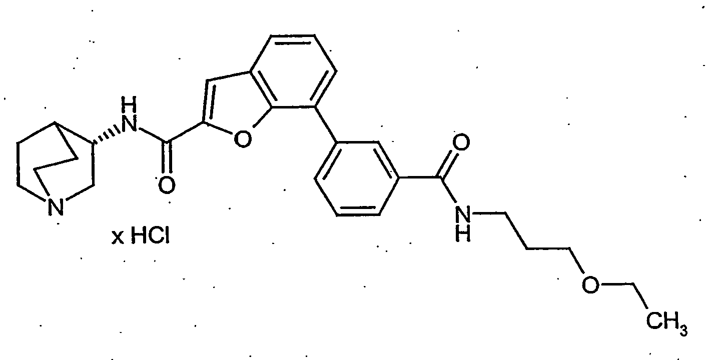

Gemäß der allgemeinen Arbeitsvorschrift E werden 50 mg (0.12 mmol) 3-(2-{[(3S)-1-Azabicyclo[2.2.2]oct-3-yl-amino]carbonyl}-1-benzofuran-7-yl)-benzoesäure-Hydrochlorid (Beispiel 153) und 24.2 mg (0.23 mmol) 3-Ethoxypropylamin miteinander umgesetzt. Es werden 23.6 mg (36.9 % d.Th.) der Titelverbindung erhalten.
Die spektroskopischen Daten stimmen mit denen der enantiomeren Verbindung (Beispiel 162) überein.

### Beispiel 172

### N-[(3S)-1-Azabicyclo[2.2.2]oct-3-yl]-7-{3-[(4-methyl-1-piperazinyl)carbonyl]-phenyl}-1-benzofuran-2-carboxamid-Dihydrochlorid

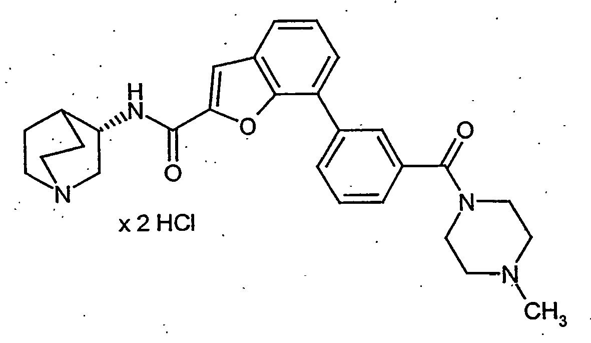

Gemäß der allgemeinen Arbeitsvorschrift E werden 50 mg (0.12 mmol) 3-(2-{[(3S)-1-Azabicyclo[2.2.2]oct-3-yl-amino]carbonyl}-1-benzofuran-7-yl)-benzoesäure-Hydrochlorid (Beispiel 153) und 23.5 mg (0.23 mmol) *N*-Methylpiperazin miteinander umgesetzt. Es werden 9.2 mg (15.4 % d.Th.) der Titelverbindung erhalten.
Die spektroskopischen Daten stimmen mit denen der enantiomeren Verbindung (Beispiel 163) überein.

### Beispiel 173

### N-[(3R)-1-Azabicyclo[2.2.2]oct-3-yl]-7-[3-(1-pyrrolidinyl)phenyl]-1-benzofuran-2-carboxamid-Hydrochlorid

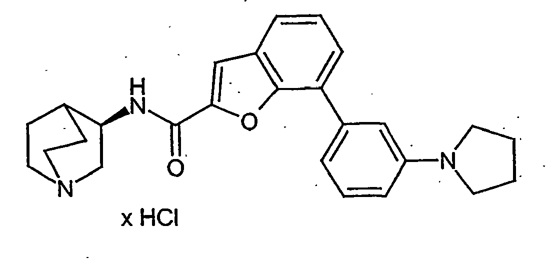

Gemäß der allgemeinen Arbeitsvorschrift D werden 114.3 mg (0.51 mmol) 1-(3-Bromphenyl)pyrrolidin, 148.1 mg (0.58 mmol) Bis(pinacolato)dibor, 124.1 mg (1.26 mmol) Kaliumacetat, 14.2 mg (0.02 mmol) PdCl₂(dppf), 150 mg (0.39 mmol) *N-*[(3R)-1-Azabicyclo[2.2.2]oct-3-yl]-7-brom-1-benzofuran-2-carboxamid-Hydrochlorid (Beispiel 30A), 0.97 mL 2 M Natriumcarbonat-Lösung und weitere 14.2 mg (0.02 mmol) PdCl₂(dppf) in 2.0 mL DMF umgesetzt. Nach Trocknen im Hochvakuum werden 95.6 mg (54.4 % d.Th.) der Titelverbindung erhalten.
HPLC (Methode 1): Rₜ= 3.85 min.
MS (ESIpos): m/z = 416 (M+H)⁺ (freie Base).

### Beispiel 174

### 7-[2-(Aminomethyl)phenyl]-N-[(3S)-1-azabicyclo[2.2.2]oct-3-yl]-1-benzofuran-2-carboxamid-Dihydrochlorid

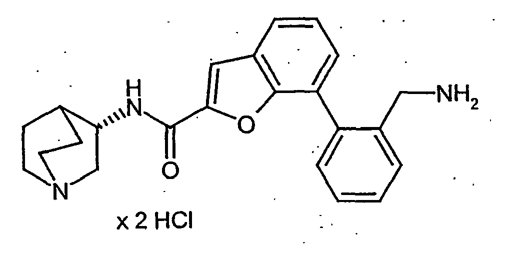

Gemäß der allgemeinen Arbeitsvorschrift D werden 500 mg (1.75 mmol) tert.-Butyl-2-brombenzylcarbamat, 512 mg (2.02 mmol) Bis(pinacolato)dibor, 428.7 mg (4.37 mmol) Kaliumacetat, 49.2 mg (0.07 mmol) PdCl₂(dppf), 518.4 mg (1.34 mmol) *N-*[(3S)-1-Azabicyclo[2.2.2]oct-3-yl]-7-brom-1-benzofuran-2-carboxamid-Hydrochlorid (freie Base: Beispiel 31A), 3.36 mL 2 M Natriumcarbonat-Lösung und weitere 49.2 mg (0.07 mmol) PdCl₂(dppf) in 5 mL DMF umgesetzt. Das im Hochvakuum getrocknete Rohprodukt wird in 4 ml einer 1:1-Mischung aus Methanol und 4 M Chlorwasserstoff in Dioxan 2 h lang bei Raumtemperatur gerührt. Die Reaktionslösung wird eingeengt und das erhaltene Rohprodukt mittels präparativer HPLC aufgereinigt. Die Produktfraktionen werden eingeengt, mit einer 3:1-Mischung aus Methanol und 1 N Salzsäure versetzt, erneut eingeengt und im Hochvakuum getrocknet. Man erhält 121.6 mg (22.4 % d.Th.) der Titelverbindung. Die spektroskopischen Daten stimmen mit denen der enantiomeren Verbindung (Beispiel 152) überein.

### Beispiel 175

### N-[(3R)-1-Azabicyclo[2.2.2]oct-3-yl]-7-[2-({[(methylamino)carbonyl]amino}-methyl)phenyl]-1-benzofuran-2-carboxamid-Hydrochlorid

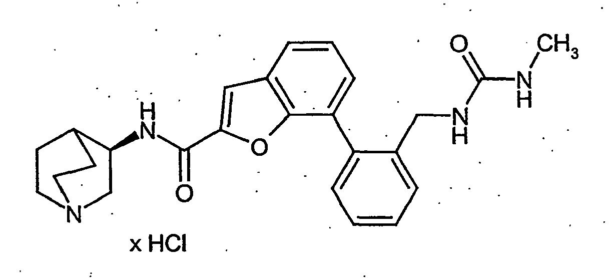

Zu einer Lösung von 100 mg (0.22 mmol) 7-[2-(Aminomethyl)phenyl]-*N*-[(3R)-1-azabicyclo[2.2.2]oct-3-yl]-1-benzofuran-2-carboxamid-Dihydrochlorid (Beispiel 152) in 1 mL einer 5:1-Mischung aus THF und DMF werden 62.2 µL (0.45 mmol) Triethylamin und 53 µL (0.89 mmol) Methylisocyanat hinzugefügt. Nach 18 h bei Raumtemperatur wird die Reaktionsmischung eingeengt und mittels präparativer HPLC gereinigt. Die Produktfraktionen werden eingeengt, mit einer 3:1-Mischung aus Methanol und 1 N Salzsäure versetzt, erneut eingeengt und im Hochvakuum getrocknet. Man erhält 75 mg (66 % d.Th.) der Titelverbindung.
¹H-NMR (400 MHz, Methanol-d₄): δ = 7.78 (m, 1H), 7.68 (s, 1H), 7.52-7.30 (m, 6H), 4.47 (m, 1H), 4.23 (m, 2H), 3.77 (m, 1H), 3.53-3.25 (m, 5H), 2.69 (s, 3H), 2.37 (m, 1H), 2.20 (m, 1H), 2.08 (m, 2H), 1.88 (m, 1H).
HPLC (Methode 1): Rₜ = 3.78 min.
MS (ESIpos): m/z = 433 (M+H)⁺ (freie Base).

### Beispiel 176

### N-[(3S)-1-Azabicyclo[2.2.2]oct-3-yl]-7-[2-({[(methylamino)carbonyl]amino}-methyl)phenyl]-1-benzofuran-2-carboxamid-Hydrochlorid

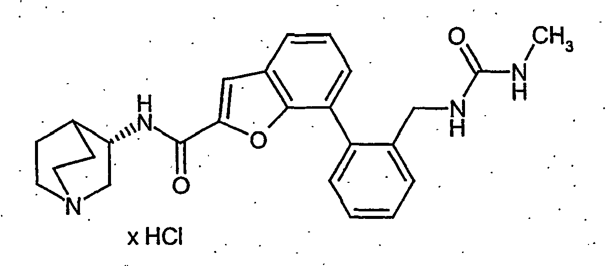

Zu einer Lösung von 57.9 mg (0.13 mmol) 7-[2-(Aminomethyl)phenyl]-*N*-[(3S)-1-azabicyclo[2.2.2]oct-3-yl]-1-benzofuran-2-carboxamid-Dihydrochlorid (Beispiel 174) in 0.7 mL einer 5:1-Mischung aus THF und DMF werden 36 µL (0.26 mmol) Triethylamin und 29.5 µL (0.52 mmol) Methylisocyanat hinzugefügt. Nach 18 h bei Raumtemperatur wird die Reaktionsmischung eingeengt und mittels präparativer HPLC gereinigt. Die Produktfraktionen werden eingeengt, mit einer 3:1-Mischung aus Methanol und 1 N Salzsäure versetzt, erneut eingeengt und im Hochvakuum getrocknet. Man erhält 49.2 mg (81.2 % d.Th.) der Titelverbindung.
Die spektroskopischen Daten stimmen mit denen der enantiomeren Verbindung (Beispiel 175) überein.

### Beispiel 177

### N-[(3R)-1-Azabicyclo[2.2.2]oct-3-yl]-7-(3-[(2,2-dimethylpropanoyl)amino]phenyl}-1-benzofuran-2-carboxamid-Hydrochlorid

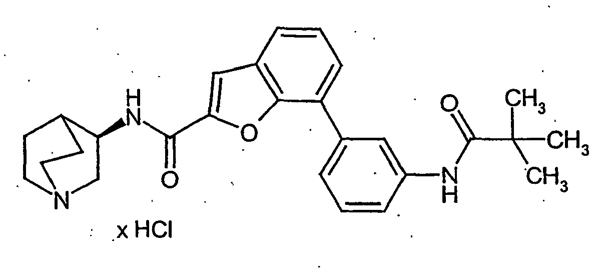

Gemäß der allgemeinen Arbeitsvorschrift F werden 50 mg (0.14 mmol) 7-(3-Aminophenyl)-*N*-[(3R)-1-azabicyclo[2.2.2]oct-3-yl]-1-benzofuran-2-carboxamid (Beispiel 114) und 33.4 mg (0.28 mmol) Pivalinsäurchlorid miteinander umgesetzt. Es werden 15.2 mg (20.7 % d.Th.) der Titelverbindung erhalten.
HPLC (Methode 1): Rₜ = 4.30 min.
MS (ESIpos): m/z = 446 (M+H)⁺ (freie Base).

### Beispiel 178

### N-[3-(2-{[(3R)-1-Azabicyclo[2.2.2]oct-3-ylamino]carbonyl}-1-benzofuran-7-yl)-phenyl]-5-isoxazolcarboxamid-Hydrochlorid

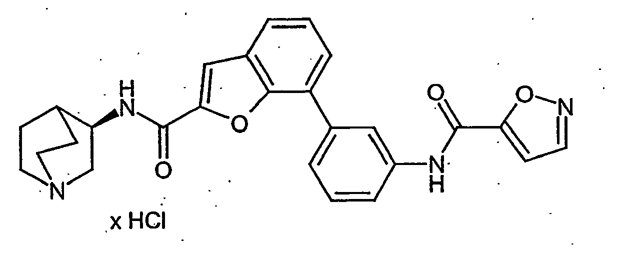

Gemäß der allgemeinen Arbeitsvorschrift F werden 50 mg (0.14 mmol) 7-(3-Aminophenyl)-*N*-[(3R)-1-azabicyclo[2.2.2]oct-3-yl]-1-benzofuran-2-carboxamid (Beispiel 114) und 36.4 mg (0.28 mmol) 5-Isoxazolcarbonsäurechlorid miteinander umgesetzt. Es werden 39.6 mg (53.3 % d.Th.) der Titelverbindung erhalten.
HPLC (Methode 1): Rₜ = 4.18 min.
MS (ESIpos): m/z = 457 (M+H)⁺ (freie Base).

### Beispiel 179

### N-[(3R)-1-Azabicyclo[2.2.2]oct-3-yl]-7-{3-[(cyclopentylcarbonyl)amino]phenyl}-1-benzofuran-2-carboxamid-Hydrochlorid

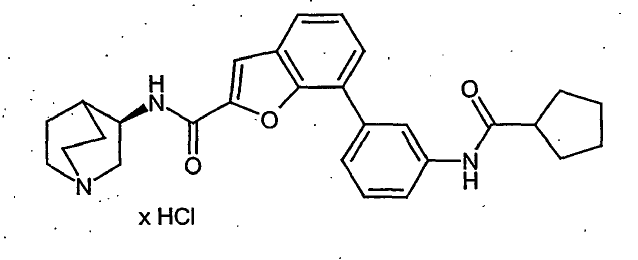

Gemäß der allgemeinen Arbeitsvorschrift F werden 50 mg (0.14 mmol) 7-(3-Aminophenyl)-*N*-[(3R)-1-azabicyclo[2.2.2]oct-3-yl]-1-benzofuran-2-carboxamid (Beispiel 114) und 36.7 mg (0.28 mmol) Cyclopentancarbonsäurechlorid miteinander umgesetzt. Es werden 33.2 mg (45.1 % d.Th.) der Titelverbindung erhalten.
HPLC (Methode 1): Rₜ = 4.38 min.
MS (ESIpos): m/z = 458 (M+H)⁺ (freie Base).

### Beispiel 180

### N-[(3R)-1-Azabicyclo[2.2.2]oct-3-yl]-7-[3-(isobutyrylamino)phenyl]-1-benzofuran-2-carboxamid-Hydrochlorid

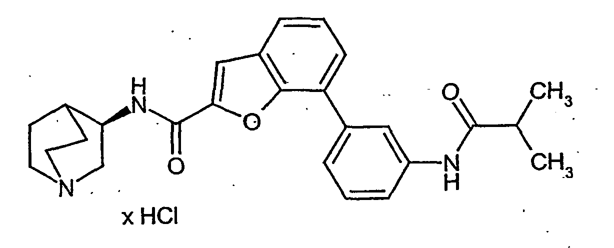

Gemäß der allgemeinen Arbeitsvorschrift F werden 50 mg (0.14 mmol) 7-(3-Aminophenyl)-*N*-[(3R)-1-azabicyclo[2.2.2]oct-3-yl]-1-benzofuran-2-carboxamid (Beispiel 114) und 29.5 mg (0.28 mmol) Isobuttersäurchlorid miteinander umgesetzt. Es werden 12.8 mg (19.5 % d.Th.) der Titelverbindung erhalten.
HPLC (Methode 1): Rₜ = 4.19 min.
MS (ESIpos): m/z = 432 (M+H)⁺ (freie-Base).

### Beispiel 181

### N-[(3R)-1-Azabicyclo[2.2.2]oct-3-yl]-7-[3-(2-furoylamino)phenyl]-1-benzofuran-2-carboxamid-Hydrochlorid

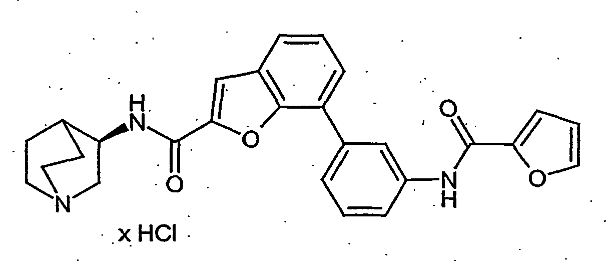

Gemäß der allgemeinen Arbeitsvorschrift F werden 50 mg (0.14 mmol) 7-(3-Aminophenyl)-*N*-[(3R)-1-azabicyclo[2.2.2]oct-3-yl]-1-benzofuran-2-carboxamid (Beispiel 114) und 36.1 mg (0.28 mmol) Furan-2-carbonsäurchlorid miteinander umgesetzt. Es werden 7.4 mg (10.6 % d.Th.) der Titelverbindung erhalten.
HPLC (Methode 1): Rₜ =4.27 min.
MS (ESIpos): m/z = 456 (M+H)⁺ (freie Base).

### Beispiel 182

### N-[(3R)-1-Azabicyclo[2.2.2]oct-3-yl]-7-(2-methoxyphenyl)-1-benzofuran-2-carboxamid-Acetat

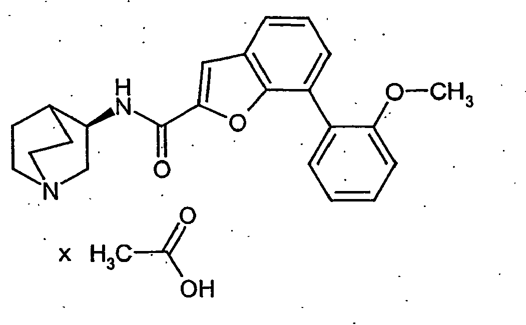

95.9 mg (0.25 mmol) *N*-[(3R)-1-Azabicyclo[2.2.2]oct-3-yl]-7-(2-methoxyphenyl)-1-benzofuran-2-carboxamid (Beispiel 130) werden in 2 mL Methanol gelöst. Nach dem Hinzufügen von 15.3 mg (0.25 mmol) Essigsäure wird das Gemisch eingeengt und der Rückstand im Hochvakuum getrocknet. Man erhält 114.9 mg (99.7 % d.Th.) der Titelverbindung.
¹H-NMR (200 MHz, DMSO-d₆): δ = 8.37 (d, 1H), 7.74 (dd, 1H), 7.71 (s, 1H), 7.50-7.33 (m, 4H), 7.20 (d, 1H), 7.08 (m, 1H), 3.93 (m, 1H), 3.76 (s, 3H), 3.10 (m, 1H), 2.87 (m, 1H), 2.78-2.60 (m, 4H), 1.90 (s, 3H), 1.87 (m, 1H), 1.75 (m, 1H), 1.58 (m, 2H), 1.33 (m, 1H).

### Beispiel 183

### N-[(3R)-1-Azabicyclo[2.2.2]oct-3-yl]-7-(2-methoxyphenyl)-1-benzofuran-2-carboxamid-Tosylat

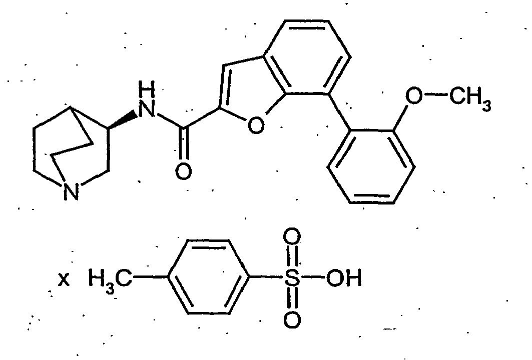

95.9 mg (0.25 mmol) *N*-[(3R)-1-Azabicyclo[2.2.2]oct-3-yl]-7-(2-methoxyphenyl)-1-benzofuran-2-carboxamid (Beispiel 130) werden in 2 mL Methanol gelöst. Nach dem Hinzufügen von 49.2 mg (0.25 mmol) p-Toluolsulfonsäure wird das Gemisch eingeengt und der Rückstand im Hochvakuum getrocknet. Man erhält 143 mg (99.4 % d.Th.) der Titelverbindung.
¹H-NMR (200 MHz, DMSO-d₆): δ = 9.34 (br. s, 1H), 8.70 (d, 1H), 7.77 (dd, 1H), 7.72 (s, 1H), 7.52-7.35 (m, 6H), 7.21 (d, 1H), 7.15-7.04 (m, 3H), 4.30 (m, 1H), 3.76 (s, 3H), 3.68 (m, 1H), 3.33-3.11 (m, 5H), 2.29 (s, 3H), 2.19 (m, 1H), 2.07 (m, 1H), 1.91 (m, 2H), 1.74 (m, 1H).

### Beispiel 184

### N-[(3R)-1-Azabicyclo[2.2.2]oct-3-yl]-7-(2-methoxyphenyl)-1-benzofuran-2-carboxamid-Fumarat

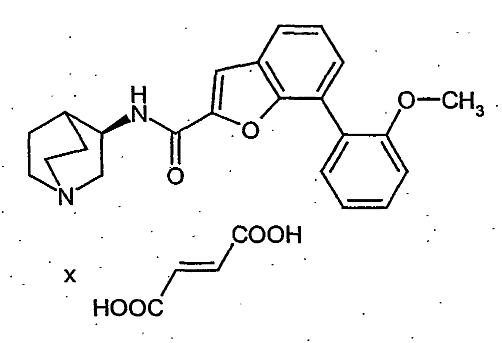

95.9 mg (0.25 mmol) *N*-[(3R)-1-Azabicyclo[2.2.2]oct-3-yl]-7-(2-methoxyphenyl)-1-benzofuran-2-carboxamid (Beispiel 130) werden in 1.5 mL Aceton gelöst. Nach dem Hinzufügen von 29.6 mg (0.25 mmol) Fumarsäure in 1 mL heißem Isopropanol wird das Gemisch 30 min. lang bei 50°C nachgerührt, anschließend eingeengt und der Rückstand im Hochvakuum getrocknet. Man erhält 124.2 mg (99 % d.Th.) der Titelverbindung.
¹H-NMR (200 MHz, DMSO-d₆): δ = 8.62 (d, 1H), 7.76 (dd, 1H), 7.72 (s, 1H), 7.49-7.33 (m, 4H), 7.21 (d, 1H), 7.09 (m, 1H), 6.50 (s, 2H), 4.16 (m, 1H), 3.76 (s, 3H), 3.38 (m, 1H), 3.11 (m, 1H), 3.06-2.85 (m, 4H), 2.03 (m, 1H), 1.92 (m, 1H), 1.76 (m, 2H), 1.56 (m, 1H).

### Beispiel 185

### N-[(3R)-1-Azabicyclo[2.2.2]oct-3-yl]-7-(2-methoxyphenyl)-1-benzofuran-2-carboxamid-Oxalat

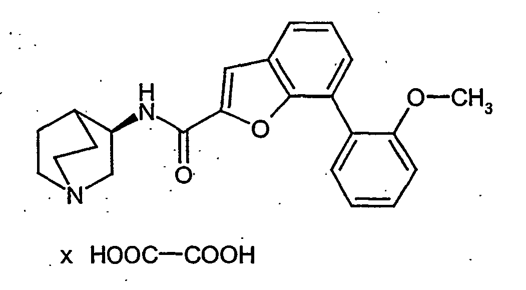

95.9 mg (0.25 mmol) *N*-[(3R)-1-Azabicyclo[2.2.2]oct-3-yl]-7-(2-methoxyphenyl)-1-benzofuran-2-carboxamid (Beispiel 130) werden in 1.5 mL Aceton gelöst. Nach dem Hinzufügen von 22.9 mg (0.25 mmol) Oxalsäure in 1 mL heißem Isopropanol wird das Gemisch 30 min. lang bei 50°C nachgerührt, anschließend eingeengt und der Rückstand im Hochvakuum getrocknet. Man erhält 117.6 mg (99 % d.Th.) der Titelverbindung.
¹H-NMR (200 MHz, DMSO-d₆): δ = 8.75 (d, 1H), 7.77 (dd, 1H), 7.72 (s, 1H), 7.50-7.33 (m, 4H), 7.20 (d, 1H), 7.09 (m, 1H), 4.28 (m, 1H), 3.75 (s, 3H), 3.62 (m, 1H), 3.32-3.08 (m, 5H), 2.17 (m, 1H), 2.04 (m, 1H), 1.89 (m, 2H), 1.71 (m, 1H).

### Beispiel 186

### N-[(3R)-1-Azabicyclo[2.2.2]oct-3-yl]-7-(2-hydroxyphenyl)-1-benzofuran-2-carboxamid

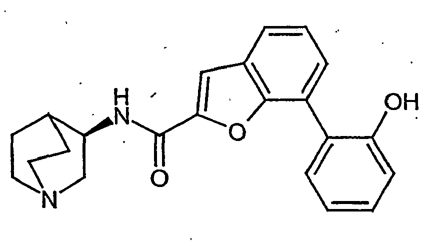

Zu einer auf -20°C gekühlten Suspension von 200 mg (0.48 mmol) *N*-[(3R)-1-Azabicyclo[2.2.2]oct-3-yl]-7-(2-methoxyphenyl)-1-benzofuran-2-carboxamid-Hydrochlorid (Beispiel 102) in 8 mL Dichlormethan werden 2.42 mL einer 1 M Bortribromid-Lösung in Dichlormethan hinzugetropft. Nach 2 h wird die Reaktion durch Zugabe von Diethylether abgebrochen. Es wird 30 min. bei Raumtemperatur gerührt, anschließend mit Wasser versetzt und mit 1 N Natronlauge neutral gestellt. Es wird mit Essigsäureethylester extrahiert; die organischen Phasen werden vereinigt und über Natriumsulfat getrocknet. Nach Einengen und Trocknen im Hochvakuum erhält man 125.9 mg (71.7 % d.Th.) der Titelverbindung.
HPLC (Methode 1): Rₜ = 3.84 min.
MS (ESIpos): m/z = 363 (M+H)⁺.

### Beispiel 187

### (3R)-3-({[7-(2-Methoxyphenyl)-1-benzofuran-2-yl]carbonyl}amino)-1-methyl-1-azoniabicyclo[2.2.2]octan-Chlorid

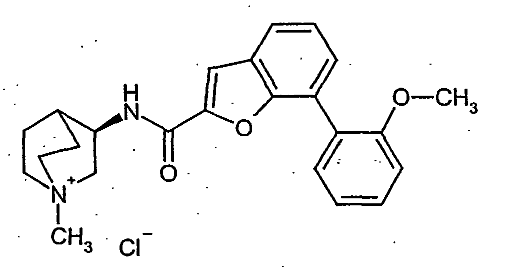

Zu einer auf -20°C gekühlten Lösung von 250 mg (0.61 mmol) *N*-[(3R)-1-Azabicyclo[2.2.2]oct-3-yl]-7-(2-methoxyphenyl)-1-benzofuran-2-carboxamid-Hydrochlorid (Beispiel 102) in 2.5 mL DMF werden 60.5 mg (1.51 mmol) Natriumhydrid (60%-ige Suspension in Mineralöl) hinzugefügt. Nach 30 min. bei Raumtemperatur und erneutem Herunterkühlen auf -20°C werden 33.9 µL (0.54 mmol) Iodmethan addiert. Nach 18 h bei Raumtemperatur wird die Reaktion durch Zugabe von Wasser abgebrochen. Das Reaktionsgemisch wird mittels präparativer HPLC aufgereinigt. Die Produktfraktionen werden eingeengt, mit 1 N Salzsäure co-destilliert, erneut eingeengt und im Hochvakuum getrocknet. Man erhält 206 mg (79.7 % d.Th.) der Titelverbindung.
¹H-NMR (200 MHz, DMSO-d₆): δ = 9.23 (d, 1H), 8.02 (s, 1H), 7.77 (d, 1H), 7.46 (dd, 1H), 7.39 (m, 3H), 7.20 (d, 1H), 7.09 (dd, 1H), 4.32 (m, 1H), 3.83 (m, 1H), 3.75 (s, 3H), 3.63 (m, 1H), 3.49-3.33 (m, 4H), 2.96 (s, 3H), 2.27 (m, 1H); 2.20 (m, 1H), 1.97 (m, 2H), 1.83 (m, 1H).
HPLC (Methode 1): Rₜ = 4.19 min.
MS (ESIpos): m/z = 391 (M+H)⁺.

### Beispiel 188

### (3R)-1-Benzyl-3-({[7-(2-methoxyphenyl)-1-benzofuran-2-yl]carbonyl}amino)-1-azoniabicyclo[2.2.2]octan-Bromid

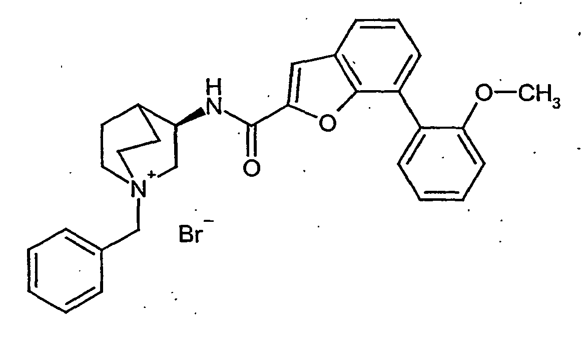

Zu einer Lösung von 500 mg (1.21 mmol) *N*-[(3R)-1-Azabicyclo[2.2.2]oct-3-yl]-7-(2-methoxyphenyl)-1-benzofuran-2-carboxamid-Hydrochlorid (Beispiel 102) in 12.5 mL DMF werden 288 µL (2.42 mmol) Benzylbromid und 502 mg (3.63 mmol) Kaliumcarbonat hinzugefügt. Nach 20 h bei 50°C wird das Reaktionsgemisch mittels präparativer HPLC aufgereinigt. Die Produktfraktionen werden eingeengt, mit 50%-iger Bromwasserstoffsäure versetzt, erneut eingeengt und im Hochvakuum getrocknet. Umkristallisation aus Cyclohexan/Aceton ergibt 537 mg (77 % d.Th.) der Titelverbindung.
HPLC (Methode 1): Rₜ = 4.44 min.
MS (ESIpos): m/z = 467 (M+H)⁺.

### Beispiel 189

### (3R)-3-[{[7-(2-Methoxyphenyl)-1-benzofuran-2-yl]carbonyl}(methyl)amino]-1-methyl-1-azoniabicyclo[2.2.2]octan-Chlorid

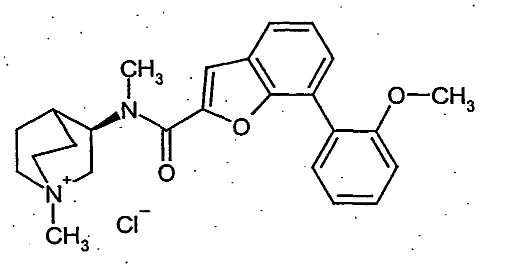

Zu einer auf -20°C gekühlten Lösung von 250 mg (0.61 mmol) *N*-[(3R)-1-Azabicyclo[2.2.2]oct-3-yl]-7-(2-methoxyphenyl)-1-benzofuran-2-carboxamid-Hydrochlorid (Beispiel 102) in 2.5 mL DMF werden 84.8 mg (2.12 mmol) Natriumhydrid (60%-ige Suspension in Mineralöl) hinzugefügt. Nach 30 min. bei Raumtemperatur und erneutem Herunterkühlen auf -20°C werden 94.2 µL (1.51 mmol) Iodmethan addiert. Nach 18 h bei Raumtemperatur wird die Reaktion durch Zugabe von Wasser abgebrochen. Das Reaktionsgemisch wird mittels präparativer HPLC aufgereinigt. Die Produktfraktionen werden eingeengt, mit einem 1:1-Gemisch aus Methanol und 4 M Chlorwasserstoff in Dioxan co-destilliert, erneut eingeengt und im Hochvakuum getrocknet. Man erhält 58 mg (21.7 % d.Th.) der Titelverbindung.
HPLC (Methode 1): Rₜ = 4.17 min.
MS (ESIpos): m/z = 405 (M+H)⁺.

### Beispiel 190

### 7-(2-Methoxyphenyl)-N-[(3R)-1-oxido-1-azabicyclo[2.2.2]oct-3-yl]-1-benzofuran-2-carboxamid

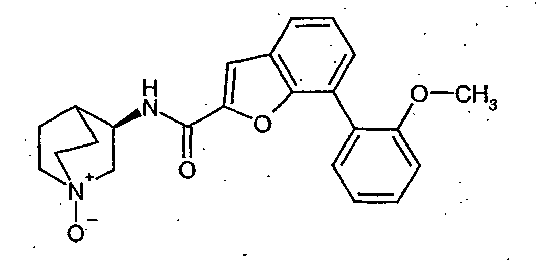

Zu einer auf 0°C gekühlten Lösung von 110 mg (0.29 mmol) *N*-[(3R)-1-Azabicyclo-[2.2.2]oct-3-yl]-7-(2-methoxyphenyl)-1-benzofuran-2-carboxamid (Beispiel 130) in 2 mL Methanol werden 35.8 µL (0.35 mmol) 30%-iges Wasserstoffperoxid addiert. Nach 18 h bei Raumtemperatur werden weitere 35.8 µL (0.35 mmol) 30%-iges Wasserstoffperoxid hinzugefügt. Nach weiteren 18 h bei Raumtemperatur wird die Reaktionslösung eingeengt und der Rückstand im Hochvakuum getrocknet. Man erhält 111.5 mg (97.2 % d.Th.) der Titelverbindung.
¹H-NMR (400 MHz, Methanol-d₄): δ = 7.69 (d, 1H), 7.59 (s, 1H), 7.41 (m, 3H), 7.37 (dd, 1H), 7.16 (d, 1H), 7.08 (dd, 1H), 4.57 (m, 1H), 3.78 (s, 3H), 3.75 (m, 1H), 3.43-3.30 (m, 5H), 2.22 (m, 2H), 2.13 (m, 2H), 1.99 (m, 1H).
HPLC (Methode 1): Rₜ = 4.18 min.
MS (FSIpos): m/z = 393 (M+H)⁺.

### Beispiel 191

### N-[(3R)-1-Azabicyclo[2.2.2]oct-3-yl]-6-[3-(4-morpholinylmethyl)phenyl]-1-benzothiophen-2-carboxamid-Dihydrochlorid

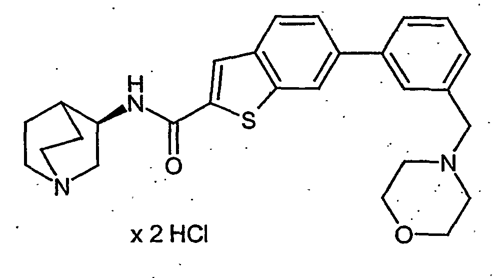

Zu einer Lösung von 80 mg (0.17 mmol) *N*-[(3R)-1-Azabicyclo[2.2.2]oct-3-yl]-6-(3-formylphenyl)-1-benzothiophen-2-carboxamid-Hydrochlorid (Beispiel 33A) in 1.0 mL einer 6:1-Mischung aus Methanol und Essigsäure werden sukzessive 290 mg (3.32 mmol) Morpholin und 31 mg (0.50 mmol) Natriumcyanoborhydrid gegeben. Nach 18 h bei 80°C wird mittels präparativer HPLC aufgereinigt. Die Produktfraktionen werden eingeengt, mit einer 5:1-Mischung aus Methanol und 1 N Salzsäure versetzt und erneut eingeengt. Nach Trocknen im Hochvakuum erhält man 47 mg (49.8 % d.Th.) der Titelverbindung.
HPLC (Methode 1): Rₜ= 3.64 min.
MS (ESIpos): m/z = 462 (M+H)⁺ (freie Base).

### Beispiel 192

### 7-(5-Acetyl-2-thienyl)-N-[(3R)-1-azabicyclo[2.2.2]oct-3-yl]-1-benzofuran-2-carboxamid-Hydrochlorid

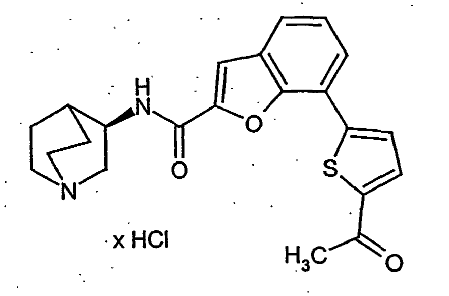

Zu einer Mischung aus 150 mg (0.39 mmol) *N-*[(3R)-1-Azabicylo[2.2.2]oct-3-yl]-7-brom-1-benzofuran-2-carboxamid-Hydrochlorid (Beispiel 30A) und 66.1 mg (0.39 mmol) 5-Acetyl-2-thienylboronsäure in 2 mL DMF werden 0.58 mL 2 M wässrige Natriumcarbonat-Lösung und 15.9 mg (0.02 mmol) PdCl₂(dppf) gegeben. Das Reaktionsgemisch wird 18 h auf 70°C erhitzt, dann über Kieselgur filtriert und zur Trockene eingeengt. Nach Reinigung des Rohprodukts mittels präparativer HPLC, anschließendem Versetzen mit einem 1:1-Gemisch aus Methanol und 1 N Salzsäure, Einengen sowie Trocknen im Hochvakuum erhält man 83.6 mg (49.9 % d.Th.) der Titelverbindung.
¹H-NMR (400 MHz, DMSO-d₆): δ = 10.29 (br. s, 1H), 9.10 (d, 1H), 8.07 (m, 2H), 7.95 (m, 1H), 7.93 (d, 1H), 7.87 (d, 1H), 7.46 (dd, 1H), 4.38 (m, 1H), 3.63 (m, 1H), 3.40 (m, 2H), 3.23 (m, 3H), 2.60 (s, 3H), 2.27 (m, 1H), 2.16 (m, 1H), 1.94 (m, 2H), 1.77 (m, 1H).
HPLC (Methode 1): Rₜ = 3.99 min.
MS (ESIpos): m/z = 495 (M+H)⁺ (freie Base).

## Patentansprüche

1. Verbindungen der Formel 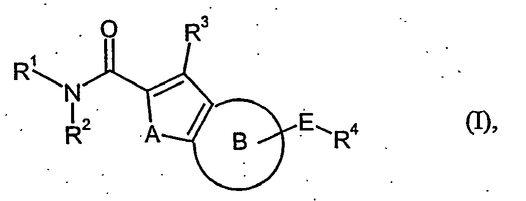 in welcher
R¹ 1-Aza-bicyclo[2.2.2]oct-3-yl, welches gegebenenfalls über das Stickstoffatom mit einem Rest ausgewählt aus der Gruppe C₁-C₄₋Alkyl, Benzyl und Oxy substituiert ist,
R² Wasserstoff oder C₁-C₆-Alkyl,
R³ Wasserstoff, Halogen oder C₁-C₆-Alkyl,
R⁴ Wasserstoff, Halogen, Cyano, Amino, Trifluormethyl, Trifluormethoxy, C₁-C₆-Alkyl, C₁-C₆-Alkylcarbonyl, C₁-C₆-Alkylamino, Formyl, Hydroxycarbonyl, C₁-C₆-Alkoxy, C₁-C₆-Alkoxycarbonyl, C₁-C₆₋Alkylthio, C₁-C₆-Alkylcarbonylamino, C₁-C₆-Alkylaminocarbonyl, C₁-C₄-Alkylsulfonylamino, C₃-C₈-Cycloalkylcarbonylamino, C₃-C₆₋Cycloalkylaminocarbonyl, Pyrrolyl, C₁-C₆-Alkylaminocarbonylamino, Heterocyclylcarbonyl, Heterocyclylcarbonylamino, Heteroarylcarbonylamino, Hydroxy, Phenyl oder Heterocyclyl,
wobei C₁-C₆-Alkyl gegebenenfalls mit Hydroxy, Cyano, Amino, C₁₋C₆-Alkylaminocarbonylamino, C₁-C₆-Alkylaminocarboxyl, Heterocyclyl oder Aryl,
C₁-C₆-Alkylaminocarbonyl gegebenenfalls mit C₁-C₆-Akoxy oder C₁-C₆-Alkylamino,
C₁-C₆-Alkylcarbonylamino gegebenenfalls mit C₁-C₆-Alkoxy, und Heterocyclyl gegebenenfalls mit Oxo substituiert sein können,
A Sauerstoff oder Schwefel,
der Ring B Benzo oder Pyrido, die jeweils gegebenenfalls durch Reste aus der Reihe Halogen, Cyano, Formyl, Trifluormethyl, Trifluormethoxy, Nitro, Amino, C₁-C₆-Alkyl und C₁-C₆-Alkoxy substituiert sind,
und
E C≡C, Aryl und Heteroaryl, wobei Aryl und Heteroaryl durch Reste aus der Reihe Halogen, Cyano, Trifluormethyl, Trifluormethoxy, Nitro, Amino, C₁-C₆-Alkoxy und C₁-C₆-Alkyl substituiert sein können,

2. Verbindungen nach Anspruch 1, der Formel (I), in welcher
R¹ 1-Aza-bicyclo[2.2.2]oct-3-yl,
R² Wasserstoff oder C₁-C₄-Alkyl,
R³ Wasserstoff, Fluor, Chlor, Brom oder C₁-C₄-Alkyl,
R⁴ Wasserstoff, Fluor, Chlor, Brom, Cyano, Amino, Trifluormethyl, Trifluormethoxy, C₁-C₄-Alkyl, C₁-C₄-Alkylcarbonyl, C₁-C₄₋Alkylamino, Formyl, Hydroxycarbonyl, C₁-C₄-Alkoxy, C₁-C₄₋Alkoxycarbonyl, C₁-C₄-Alkylthio, C₁-C₄-Alkylcarbonylamino, C₁-C₄₋Alkylaminocarbonyl, C₁-C₄-Alkylsulfonylamino, C₃-C₆-Cycloalkylcarbonylamino, C₃-C₆-Cycloalkylaminocarbonyl, Pyrrolyl, C₁-C₄₋Alkylaminocarbonylamino, Heterocyclylcarbonyl, Heterocyclylcarbonylamino, Heteroarylcarbonylamino, Hydroxy, Phenyl oder Heterocyclyl,
wobei C₁-C₄-Alkyl gegebenenfalls mit Hydroxy, Cyano, Amino, C₁₋C₄-Alkylaminocarbonylamino, C₁-C₄-Alkylaminocarboxyl, Heterocyclyl oder Aryl,
C₁-C₄-Alkylaminocarbonyl gegebenenfalls mit C₁-C₄-Alkoxy oder C₁-C₄-Alkylamino,
C₁-C₄-Akylcarbonylamino gegebenenfalls mit C₁-C₄-Alkoxy, und Heterocyclyl gegebenenfalls mit Oxo substituiert sein können,
A Sauerstoff oder Schwefel,
der Ring B Benzo oder Pyrido, die jeweils gegebenenfalls durch Reste aus der Reihe Halogen, Cyano, Trifluormethyl, Trifluormethoxy und C₁-C₄-Alkyl substituiert sind,
und
E C≡C, Aryl und Heteroaryl, wobei Aryl und Heteroaryl durch Reste aus der Reihe Halogen, Cyano, Trifluormethyl, Trifluormethoxy, Nitro, Amino, C₁-C₄-Alkoxy und C₁-C₄-Alkyl substituiert sein können,
bedeuten, sowie die Solvate, Salze oder Solvate der Salze dieser Verbindungen.

3. Verbindungen nach Ansprüchen 1 und 2, der Formel (I), in welcher
R¹ 1-Aza-bicyclo[2.2.2]oct-3-yl,
R² und R³ Wasserstoff,
R⁴ Wasserstoff, Fluor, Chlor, Brom, Cyano, Amino, Trifluormethyl, Trifluormethoxy, C₁-C₄-Alkyl, C₁-C₄-Alkylcarbonyl, C₁-C₄-Alkylamino, Formyl, Hydroxycarbonyl, C₁-C₄-Alkoxy, C₁-C₄-Alkoxycarbonyl, C₁-C₆-Alkylthio, C₁-C₄-Alkylcarbonylamino, C₁-C₄-Alkylaminocarbonyl, C₁-C₄-Alkylsulfonylamino, C₃-C₆-Cycloalkylcarbonylamino, C₃-C₆-Cycloalkylaminocarbonyl, Pyrrolyl, C₁-C₄-Alkylaminocarbonylamino, Heterocyclylcarbonyl, Heterocyclylcarbonylamino, Heteroarylcarbonylamino, Hydroxy, Phenyl oder Heterocyclyl,
wobei C₁-C₄-Alkyl gegebenenfalls mit Hydroxy, Cyano, Amino, C₁₋C₄-Alkylaminocarbonylamino, C₁-C₄-Alkylaminocarboxyl, Heterocyclyl oder Aryl,
C₁-C₄-Alkylaminocarbonyl gegebenenfalls mit C₁-C₄-Alkoxy oder C₁-C₄-Alkylamino,
C₁-C₄-Alkylcarbonylamino gegebenenfalls mit C₁-C₄-Alkoxy, und Heterocyclyl gegebenenfalls mit Oxo substituiert sein können,
A Sauerstoff,
der Ring B Benzo oder Pyrido, die jeweils gegebenenfalls durch Reste aus der Reihe Halogen, Cyano, Trifluormethyl, Trifluormethoxy und C₁-C₄-Alkyl substituiert sind,
und
E C≡C, Aryl und Heteroaryl, wobei Aryl und Heteroaryl durch Reste aus der Reihe Halogen, Cyano, Trifluormethyl, Trifluormethoxy, Nitro, Amino, C₁-C₄-Alkoxy und C₁-C₄-Alkyl substituiert sein können,
bedeuten, sowie die Solvate, Salze oder Solvate der Salze dieser Verbindungen.

4. Verbindungen nach Ansprüchen 1 bis 3, der Formel (I), in welcher
R¹ 1-Aza-bicyclo[2.2.2]oct-3-yl,
R² Wasserstoff oder C₁-C₆-Alkyl,
R³ Wasserstoff, Halogen oder C₁-C₆-Alkyl,
R⁴ Wasserstoff, Halogen, Cyano, Amino, Trifluormethyl, Trifluormethoxy, C₁-C₆-Alkyl, C₁-C₆-Alkylcarbonyl, C₁-C₆-Alkylamino, Formyl, Hydroxycarbonyl, C₁-C₆-Alkoxy, C₁-C₆-Alkoxycarbonyl, C₁₋C₆-Alkylthio, C₁-C₆-Akylcarbonylamino, C₁-C₄-Alkylsulfonylamino, C₃-C₈-Cycloalkylcarbonylamino, Pyrrolyl, C₁-C₆-Alkylaminocarbonylamino, Heterocyclylcarbonyl, Phenyl oder Heterocyclyl,
wobei C₁-C₆-Alkyl gegebenenfalls durch Hydroxy, Amino, C₁-C₆₋Alkylaminocarbonylamino, C₁-C₆-Alkylaminocarboxyl, Heterocyclyl oder Aryl,
C₁-C₆-Alkylcarbonylamino gegebenenfalls mit C₁-C₆-Alkoxy, und
Heterocyclyl gegebenenfalls mit Oxo substituiert sind,
A Sauerstoff oder Schwefel,
der Ring B Benzo oder Pyrido, die jeweils gegebenenfalls durch Reste aus der Reihe Halogen, Cyano, Formyl, Trifluormethyl, Trifluormethoxy, Nitro, Amino, C₁-C₆-Alkyl und C₁-C₆-Alkoxy substituiert sind,
und
E C≡C, Aryl und Heteroaryl, wobei Aryl und Heteroaryl gegebenenfalls durch Reste aus der Reihe Halogen, Cyano, Trifluormethyl, Trifluormethoxy, Nitro, Amino, C₁-C₆-Alkoxy und C₁-C₆-Alkyl substituiert sind,
bedeuten, sowie die Solvate, Salze oder Solvate der Salze dieser Verbindungen.

5. Verbindungen der Formel (I) nach Ansprüchen 1 bis 4, in welcher
R¹ 1-Aza-bicyclo[2.2.2]oct-3-yl,
R² Wasserstoff oder C₁-C₆-Alkyl,
R³ Wasserstoff, Halogen oder C₁-C₆-Alkyl,
R⁴ Wasserstoff, Halogen, Cyano, Trifluormethyl, Trifluormethoxy, C₁₋C₆-Alkyl, C₁-C₆-Alkoxy oder Heterocyclyl, wobei Alkyl gegebenenfalls durch einen Rest Hydroxy substituiert ist,
A Sauerstoff oder Schwefel,
der Ring B Benzo oder Pyrido, die jeweils gegebenenfalls durch Reste aus der Reihe Halogen, Cyano, Trifluormethyl, Trifluormethoxy, Nitro, Amino, C₁-C₆-Alkyl und C₁-C₆-Alkoxy substituiert sind,
und
E C≡C, Arylen oder Heteroarylen, wobei Arylen und Heteroarylen gegebenenfalls durch Reste aus der Reihe Halogen, Cyano, Trifluormethyl, Trifluormethoxy, Nitro, Amino, C₁-C₆-Alkyl und C₁-C₆₋Alkoxy substituiert sind,
bedeuten, sowie die Solvate, Salze oder Solvate der Salze dieser Verbindungen.

6. Verbindungen nach Ansprüchen 1 bis 5, der Formel 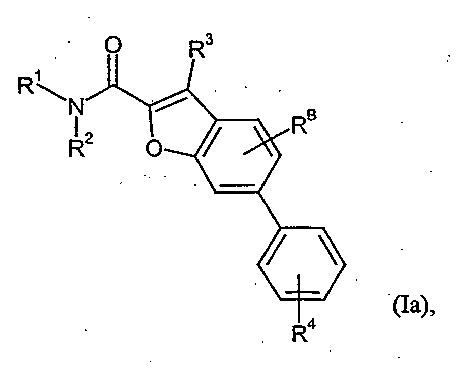 in welcher
R¹ (3*R*)-1-Aza-bicyclo[2.2.2]oct-3-yl,
R² und R³ unabhängig voneinander Wasserstoff oder Methyl,
R⁴ Wasserstoff, Halogen, Cyano, Trifluormethyl, Trifluormethoxy, C₁₋C₆-Alkyl, C₁-C₆-Alkoxy oder Heterocyclyl, wobei Alkyl gegebenenfalls durch einen Hydroxyrest substituiert ist,
und
R^{B} Wasserstoff, Halogen, Cyano, Trifluormethyl, Trifluormethoxy, Nitro, Amino, C₁-C₆-Alkyl oder C₁-C₆-Alkoxy
bedeuten, sowie die Solvate, Salze oder Solvate der Salze dieser Verbindungen.

7. Verbindungen nach Ansprüchen 1 bis 6, der Formel 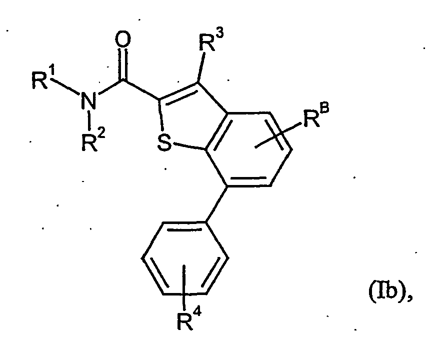 in welcher
R¹ (3*R*)-1-Aza-bicyclo[2.2.2]oct-3-yl,
R² und R³ unabhängig voneinander Wasserstoff oder Methyl,
R⁴ Wasserstoff, Halogen, Cyano, Trifluormethyl, Trifluormethoxy, C₁₋C₆-Alkyl, C₁-C₆-Alkoxy oder Heterocyclyl, wobei Alkyl gegebenenfalls durch einen Hydroxyrest substituiert ist, und
R^{B} Wasserstoff, Halogen, Cyano, Trifluormethyl, Trifluormethoxy, Nitro, Amino, C₁-C₆-Alkyl und C₁-C₆-Alkoxy
bedeuten, sowie die Solvate, Salze oder Solvate der Salze dieser Verbindungen.

8. Verbindungen nach Ansprüchen 1 bis 7, wobei
R¹ (3*R*)-1-Aza-bicylo[2.2.2]oct-3-yl,
R² und R³, Wasserstoff,
R⁴ Wasserstoff, Fluor, Chlor, Brom, Trifluormethoxy, Hydroxymethyl, Methoxy oder 6-gliedriges Heterocyclyl und
R^{B} Wasserstoff, Halogen, Cyano, Trifluormethyl, Trifluormethoxy oder C₁-C₄-Alkyl,
bedeuten, sowie die Solvate, Salze oder Solvate der Salze dieser Verbindungen.

9. Verbindungen nach Ansprüchen 1 bis 8, der Formel 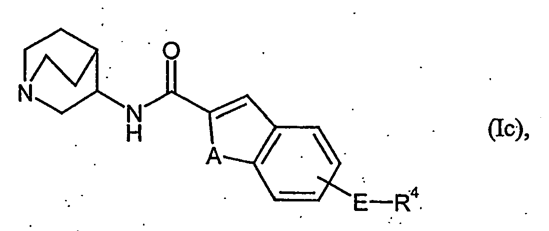 in welcher
E Phenylen,
R⁴ C₁-C₆-Alkoxy, Aminomethylen, Hydroxycarbonyl, C₃-C₈-Cycloalkylcarbonyl eine Gruppe der Formel 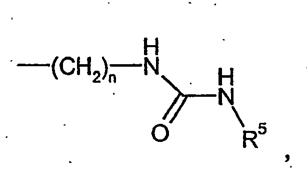 wobei
R⁵ C₁-C₆-Alkyl,
n null; 1, 2, 3 oder 4,
oder
5- bis 6-gliedriges Heterocyclyl, das gegebenenfalls mit Oxo substituiert ist,
A Schwefel oder Sauerstoff,
bedeuten, sowie deren Solvate, Salze oder Solvate der Salze.

10. Verbindungen nach Ansprüchen 1 bis 9, der Formel (Ic), in welcher
E Phenylen,
R⁴ C₁-C₄-Alkoxy, Aminomethylen, Hydroxycarbonyl, C₃-C₆-Cycloalkylcarbonylamino, eine Gruppe der Formel 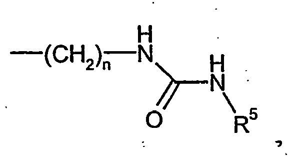 wobei
R⁵ C₁-C₄-Alkyl,
n null, 1 oder 2,
oder
5- bis 6-gliedriges Heterocyclyl, das gegebenenfalls mit Oxo substituiert ist,
A Schwefel oder Sauerstoff,
bedeuten, sowie deren Solvate, Salze oder Solvate der Salze.

11. Verbindungen nach Ansprüchen 1 bis 10, der folgenden Formeln 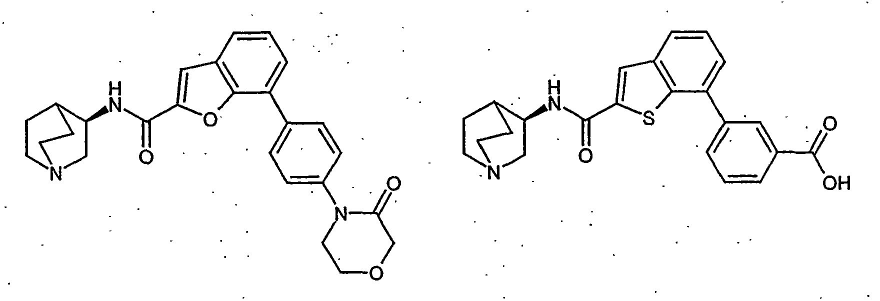 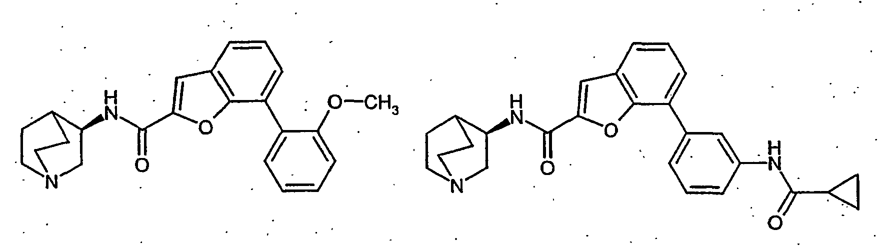 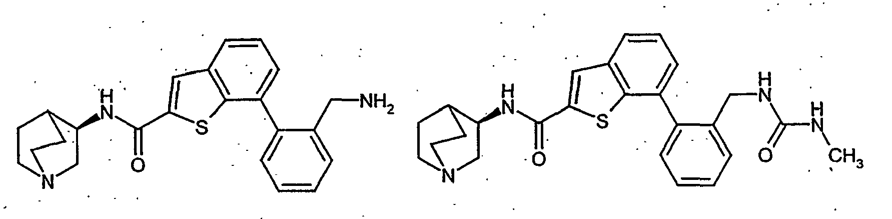 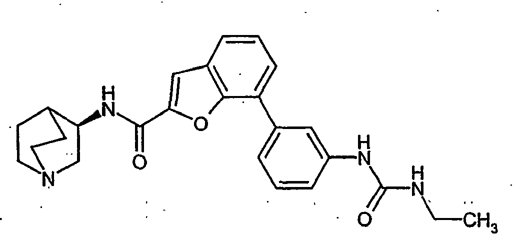 sowie die Solvate, Salze oder Solvate der Salze dieser Verbindungen.

12. Verfahren zur Herstellung der Verbindungen der Formel (I), wonach man Verbindungen der Formel
X¹-E-R⁴ (II),
in welcher
R⁴ die in Anspruch 1 angegebenen Bedeutungen hat und
X¹ im Falle, dass E Arylen oder Heteroarylen bedeutet, für -B(OH)₂ oder
oder 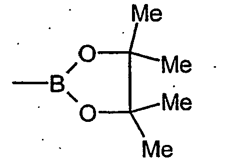 steht,
und im Falle, dass E-C≡C-bedeutet, für Wasserstoff steht,
mit einer Verbindung der Formel 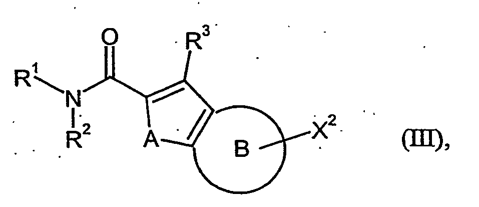 in welcher
R¹, R², R³, A und der Ring B die in Anspruch 1 angegebenen Bedeutungen besitzen und
X² für Triflat oder Halogen, bevorzugt Chlor, Brom oder Iod, steht,
und gegebenenfalls
[A] die resultierenden Verbindungen (I) mit entsprechenden Alkylierungsreagentien am Chinuklidinstickstoffatom alkyliert, oder
[B] die resultierenden Verbindungen (I) mit geeigneten Oxidationsmitteln am Chinuklidinstickstoffatom oxidiert,
und die resultierenden Verbindungen (I) gegebenenfalls mit den entsprechenden (i) Lösungsmitteln und/oder (ii) Basen oder Säuren zu ihren Solvaten, Salzen oder Solvaten der Salze umsetzt.

13. Verfahren zur Herstellung der erfindungsgemäßen Verbindungen, wonach man Verbindungen der Formel
X¹-E-R⁴ (II),
in welcher
R⁴ die in Anspruch 1 angegebenen Bedeutungen hat und
X¹ im Falle, dass E Arylen oder Heteroarylen bedeutet, für -B(OH)₂ oder 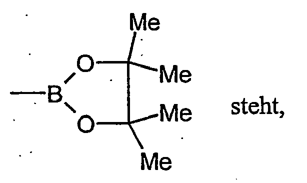 und im Falle, dass E -C≡C- bedeutet, für Wasserstoff steht,
mit einer Verbindung der Formel 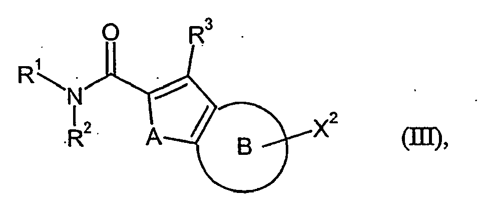 in welcher
R¹, R², R³, A und der Ring B die in Anspruch 1 angegebenen Bedeutungen besitzen und
X² für Triflat oder Halogen, bevorzugt Chlor, Brom oder Iod, steht,
und die resultierenden Verbindungen (I) gegebenenfalls mit den entsprechenden (i) Lösungsmitteln und/oder (ii) Basen oder Säuren zu ihren Solvaten, Salzen oder Solvaten der Salze umsetzt.

14. Verbindungen nach einem der Ansprüche 1 bis 11 zur Behandlung und/oder Prophylaxe von Krankheiten.

15. Arzneimittel enthaltend mindestens eine Verbindung nach einem der Ansprüche 1 bis 11 und mindestens einen pharmazeutisch verträglichen, im wesentlichen nichtgiftigen Träger oder Exzipienten.

16. Verwendung von Verbindungen nach einem der Ansprüche 1 bis 11 zur Herstellung eines Mittels zur Verbesserung der Wahrnehmung, Konzentrationsleistung, Lernleistung und/oder Gedächtnisleistung.

17. Verwendung von Verbindungen nach einem der Ansprüche 1 bis 11 zur Herstellung eines Arzneimittels zur Behandlung und/oder Prophylaxe von Störungen der Wahrnehmung, Konzentrationsleistung, Lernleistung und/oder Gedächtnisleistung.

18. Arzneimittel nach Anspruch 15 zur Behandlung und/oder Prophylaxe von Störungen der Wahrnehmung, Konzentrationsleistung, Lernleistung und/oder Gedächtnisleistung.

## Claims

1. Compounds of the formula 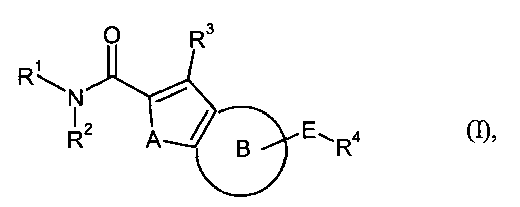 in which
R¹ is 1-azabicyclo[2.2.2]oct-3-yl, which is optionally substituted via the nitrogen atom by a radical selected from the group of C₁-C₄-alkyl, benzyl and oxy,
R² is hydrogen or C₁-C₆-alkyl,
R³ is hydrogen, halogen or C₁-C₆-alkyl,
R⁴ is hydrogen, halogen, cyano, amino, trifluoromethyl, trifluoromethoxy, C₁-C₆-alkyl, C₁-C₆-alkylcarbonyl, C₁-C₆-alkylamino, formyl, hydroxycarbonyl, C₁-C₆-alkoxy, C₁-C₆-alkoxycarbonyl, C₁-C₆-alkylthio, C₁₋C₆-alkylcarbonylamino, C₁-C₆-alkylaminocarbonyl, C₁-C₄₋alkylsulphonylamino, C₃-C₈-cycloalkylcarbonylamino, C₃-C₆₋cycloalkylaminocarbonyl, pyrrolyl, C₁-C₆-akylaminocarbonylamino, heterocyclylcarbonyl, heterocyclylcarbonylamino, heteroarylcarbonylamino, hydroxyl, phenyl or heterocyclyl,
where C₁-C₆-alkyl may optionally be substituted by hydroxyl, cyano, amino, C₁-C₆-alkylaminocarbonylamino, C₁-C₆₋alkylaminocarboxyl, heterocyclyl or aryl,
C₁-C₆-alkylaminocarbonyl may optionally be substituted by C₁-C₆-alkoxy or C₁-C₆-alkylamino,
C₁-C₆-alkylcarbonylamino may optionally be substituted by C₁₋C₆-alkoxy, and heterocyclyl may optionally be substituted by oxo,
A is oxygen or sulphur,
the ring B is benzo or pyrido, each of which are optionally substituted by radicals from the series halogen, cyano, formyl, trifluoromethyl, trifluoromethoxy, nitro, amino, C₁-C₆-alkyl and C₁-C₆-alkoxy,
and
E is C≡C, aryl and heteroaryl, where aryl and heteroaryl may be substituted by radicals from the series halogen, cyano, trifluoromethyl, trifluoromethoxy, nitro, amino, C₁-C₆-alkoxy and C₁-C₆-alkyl,
and the solvates, salts or solvates of the salts of these compounds.

2. Compounds according to Claim 1, of the formula (I), in which
R¹ is 1-azabicyclo[2.2.2]oct-3-yl,
R² is hydrogen or C₁-C₄-alkyl,
R³ is hydrogen, fluorine, chlorine, bromine or C₁-C₄-alkyl,
R⁴ is hydrogen, fluorine, chlorine, bromine, cyano, amino, trifluoromethyl, trifluoromethoxy, C₁-C₄-alkyl, C₁-C₄-alkylcarbonyl, C₁-C₄-alkylamino, formyl, hydroxycarbonyl, C₁-C₄-alkoxy, C₁-C₄-alkoxycarbonyl, C₁-C₄₋alkylthio, C₁-C₄-akylcarbonylamino, C₁-C₄-alkylaminocarbonyl, C₁-C₄₋alkylsulphonylamino, C₃-C₆-cycloalkylcarbonylamino, C₃-C₆-cycloalkylaminocarbonyl, pyrrolyl, C₁-C₄-alkylaminocarbonylamino, heterocyclylcarbonyl, heterocyclylcarbonylamino, heteroarylcarbonylamino, hydroxyl, phenyl or heterocyclyl,
where C₁-C₄-alkyl may optionally be substituted by hydroxyl, cyano, amino, C₁-C₄-alkylaminocarbonylamino, C₁-C₄₋alkylaminocarboxyl, heterocyclyl or aryl,
C₁-C₄-alkylaminocarbonyl may optionally be substituted by C₁₋C₄-alkoxy or C₁-C₄-alkylamino,
C₁-C₄-alkylcarbonylamino may optionally be substituted by C₁₋C₄-alkoxy, and heterocyclyl may optionally be substituted by oxo,
A is oxygen or sulphur,
the ring B is benzo or pyrido, each of which are optionally substituted by radicals from the series halogen, cyano, trifluoromethyl, trifluoromethoxy and C₁-C₄-alkyl,
and
E is C≡C, aryl and heteroaryl, where aryl and heteroaryl may be substituted by radicals from the series halogen, cyano, trifluoromethyl, trifluoromethoxy, nitro, amino, C₁-C₄-akoxy and C₁-C₄-alkyl,
and the solvates, salts or solvates of the salts of these compounds.

3. Compounds according to Claims 1 and 2, of the formula (I), in which
R¹ is 1-azabicyclo[2.2.2]oct-3-yl,
R² and R³ are hydrogen,
R⁴ is hydrogen, fluorine, chlorine, bromine, cyano, amino, trifluoromethyl, trifluoromethoxy, C₁-C₄-alkyl, C₁-C₄-alkylcarbonyl, C₁-C₄-alkylamino, formyl, hydroxycarbonyl, C₁-C₄-alkoxy, C₁-C₄₋alkoxycarbonyl, C₁-C₆-alkylthio, C₁-C₄-alkylcarbonylamino, C₁-C₄₋alkylaminocarbonyl, C₁-C₄-alkylsulphonylamino, C₃-C₆-cycloalkylcarbonylamino, C₃-C₆-cycloalkylaminocarbonyl, pyrrolyl, C₁-C₄₋alkylaminocarbonylamino, heterocyclylcarbonyl, heterocyclylcarbonylamino, heteroarylcarbonylamino, hydroxyl, phenyl or heterocyclyl,
where C₁-C₄-alkyl may optionally be substituted by hydroxyl, cyano, amino, C₁-C₄-alkylaminocarbonylamino, C₁-C₄₋alkylaminocarboxyl, heterocyclyl or aryl,
C₁-C₄-alkylaminocarbonyl may optionally be substituted by C₁₋C₄-alkoxy or C₁-C₄-alkylamino,
C₁-C₄-alkylcarbonylamino may optionally be substituted by C₁₋C₄-alkoxy, and heterocyclyl may optionally be substituted by oxo,
A is oxygen,
the ring B is benzo or pyrido, each of which are optionally substituted by radicals from the series halogen, cyano, trifluoromethyl, trifluoromethoxy and C₁-C₄-alkyl,
and
E is C≡C, aryl and heteroaryl, where aryl and heteroaryl may be substituted by radicals from the series halogen, cyano, trifluoromethyl, trifluoromethoxy, nitro, amino, C₁-C₄-alkoxy and C₁-C₄-alkyl,
and the solvates, salts or solvates of the salts of these compounds.

4. Compounds according to Claims 1 to 3, of the formula (I), in which
R¹ is 1-azabicyclo[2.2.2]oct-3-yl,
R² is hydrogen or C₁-C₆-alkyl,
R³ is hydrogen, halogen or C₁-C₆-alkyl,
R⁴ is hydrogen, halogen, cyano, amino, trifluoromethyl, trifluoromethoxy, C₁-C₆-alkyl, C₁-C₆-alkylcarbonyl, C₁-C₆-alkylamino, formyl, hydroxycarbonyl, C₁-C₆-alkoxy, C₁-C₆-alkoxycarbonyl, C₁-C₆-alkylthio, C₁₋C₆-alkylcarbonylamino, C₁-C₄-alkylsulphonylamino, C₃-C₈-cycloalkylcarbonylamino, pyrrolyl, C₁-C₆-alkylaminocarbonylamino, heterocyclylcarbonyl, phenyl or heterocyclyl,
where C₁-C₆-alkyl may optionally be substituted by hydroxyl, amino, C₁-C₆-alkylaminocarbonylamino, C₁-C₆-alkylaminocarboxyl, heterocyclyl or aryl,
C₁-C₆-alkylcarbonylamino may optionally be substituted by C₁₋C₆-alkoxy, and
heterocyclyl may optionally be substituted by oxo,
A is oxygen or sulphur,
the ring B is benzo or pyrido, each of which are optionally substituted by radicals from the series halogen, cyano, formyl, trifluoromethyl, trifluoromethoxy, nitro, amino, C₁-C₆-alkyl and C₁-C₆-alkoxy,
and
E is C≡C, aryl and heteroaryl, where aryl and heteroaryl are optionally substituted by radicals from the series halogen, cyano, trifluoromethyl, trifluoromethoxy, nitro, amino, C₁-C₆-alkoxy and C₁-C₆-alkyl,
and the solvates, salts or solvates of the salts of these compounds.

5. Compounds of the formula (I) according to Claims 1 to 4, in which
R¹ is 1-azabicyclo[2.2.2]oct-3-yl,
R² is hydrogen or C₁-C₆-alkyl,
R³ is hydrogen, halogen or C₁-C₆-alkyl,
R⁴ is hydrogen, halogen, cyano, trifluoromethyl, trifluoromethoxy, C₁-C₆₋alkyl, C₁-C₆-alkoxy or heterocyclyl, where alkyl is optionally substituted by a hydroxyl radical,
A is oxygen or sulphur,
the ring B is benzo or pyrido, each of which are optionally substituted by radicals from the series halogen, cyano, trifluoromethyl, trifluoromethoxy, nitro, amino, C₁-C₆-alkyl and C₁-C₆-alkoxy,
and
E is C≡C, arylene or heteroarylene, where arylene and heteroarylene are optionally substituted by radicals from the series halogen, cyano, trifluoromethyl, trifluoromethoxy, nitro, amino, C₁-C₆-alkyl and C₁₋C₆-alkoxy,
and the solvates, salts or solvates of the salts of these compounds.

6. Compounds according to Claims 1 to 5, of the formula 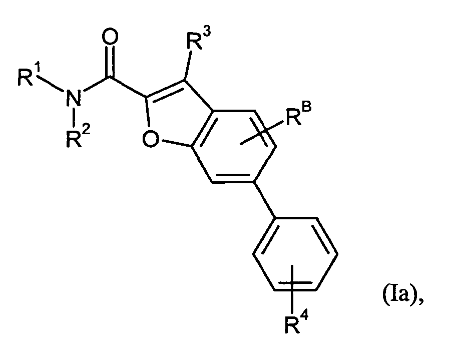 in which
R¹ is (3*R*)-1-azabicyclo[2.2.2]oct-3-yl,
R² and R³ are, independently of one another, hydrogen or methyl,
R⁴ is hydrogen, halogen, cyano, trifluoromethyl, trifluoromethoxy, C₁-C₆₋alkyl, C₁-C₆-alkoxy or heterocyclyl, where alkyl is optionally substituted by a hydroxyl radical,
and
R^{B} is hydrogen, halogen, cyano, trifluoromethyl, trifluoromethoxy, nitro, amino, C₁-C₆-alkyl or C₁-C₆-alkoxy,
and the solvates, salts or solvates of the salts of these compounds.

7. Compounds according to Claims 1 to 6, of the formula 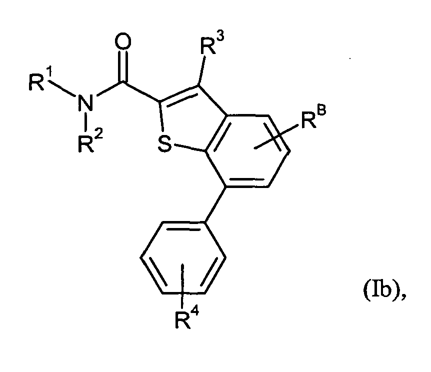 in which
R¹ is (3*R*)-1-azabicyclo[2.2.2]oct-3-yl,
R² and R³ are, independently of one another, hydrogen or methyl,
R⁴ is hydrogen, halogen, cyano, trifluoromethyl, trifluoromethoxy, C₁-C₆₋alkyl, C₁-C₆-alkoxy or heterocyclyl, where alkyl is optionally substituted by a hydroxyl radical, and
R^{B} is hydrogen, halogen, cyano, trifluoromethyl, trifluoromethoxy, nitro, amino, C₁-C₆-alkyl and C₁-C₆-alkoxy,
and the solvates, salts or solvates of the salts of these compounds.

8. Compounds according to Claims 1 to 7, where
R¹ is (3*R*)-1-azabicyclo[2.2.2]oct-3-yl,
R² and R³ are hydrogen,
R⁴ is hydrogen, fluorine, chlorine, bromine, trifluoromethoxy, hydroxymethyl, methoxy or 6-membered heterocyclyl and
R^{B} is hydrogen, halogen, cyano, trifluoromethyl, trifluoromethoxy or C₁₋C₄-alkyl,
and the solvates, salts or solvates of the salts of these compounds.

9. Compounds according to Claims 1 to 8, of the formula 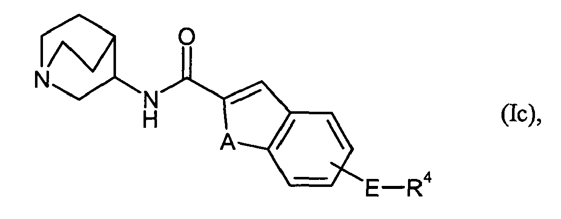 in which
E is phenylene,
R⁴ is C₁-C₆-akoxy, aminomethylene, hydroxycarbonyl, C₃-C₈-cycloalkylcarbonylamino, a group of the formula 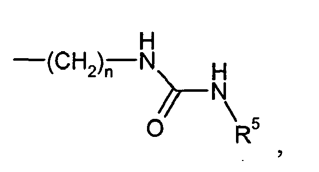 where
R⁵ is C₁-C₆-alkyl,
n is zero, 1, 2, 3 or 4,
or
5- to 6-membered heterocyclyl which is optionally substituted by oxo,
A is sulphur or oxygen,
and the solvates, salts or solvates of the salts thereof.

10. Compounds according to Claims 1 to 9, of the formula (Ic), in which
E is phenylene,
R⁴ is C₁-C₄-alkoxy, aminomethylene, hydroxycarbonyl, C₃-C₆-cycloalkylcarbonylamino, a group of the formula 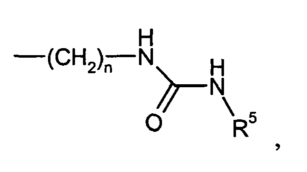 where
R⁵ is C₁-C₄-alkyl,
n is zero, 1 or 2,
or
5- to 6-membered heterocyclyl which is optionally substituted by oxo,
A is sulphur or oxygen,
and the solvates, salts or solvates of the salts thereof.

11. Compounds according to Claims 1 to 10, of the following formulae 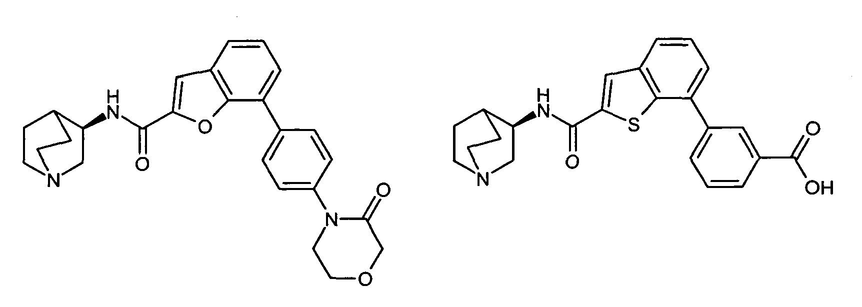 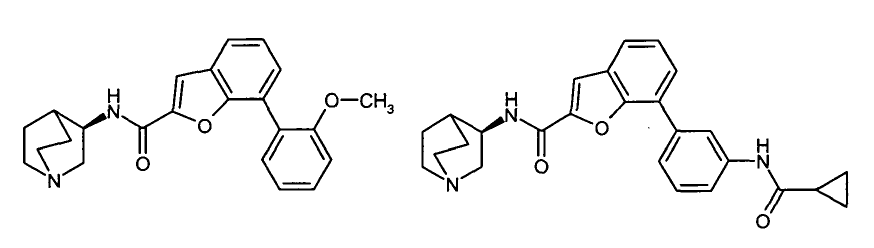 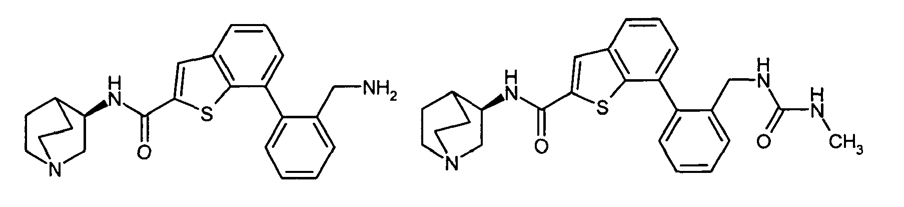 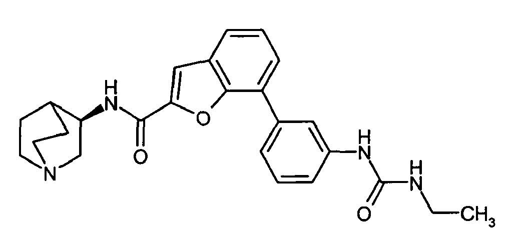 and the solvates, salts or solvates of the salts of these compounds.

12. Process for the preparation of the compounds of the formula (I), in which compounds of the formula
X¹-E-R⁴ (II),
in which
R⁴ has the meanings indicated in Claim 1, and
X¹ is -B(OH)₂ or
or 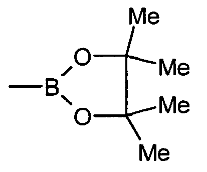 in the case where E is arylene or heteroarylene, and is hydrogen in the
case where E is -C≡C-,
are reacted with a compound of the formula 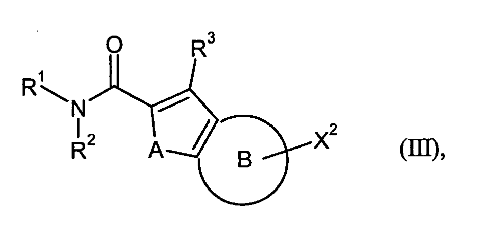 in which
R¹, R², R³, A and the ring B have the meanings indicated in Claim 1, and
X² is triflate or halogen, preferably chlorine, bromine or iodine,
and where appropriate
[A] the resulting compounds (I) are alkylated on the quinuclidine nitrogen atom with appropriate alkylating reagents, or
[B] the resulting compounds (I) are oxidized on the quinuclidine nitrogen atom with suitable oxidizing agents,
and the resulting compounds (I) are converted into their solvates, salts or solvates of the salts where appropriate with the appropriate (i) solvents and/or (ii) bases or acids.

13. Process for the preparation of the compounds of the invention, in which compounds of the formula
X¹-E-R⁴ (II),
in which
R⁴ has the meanings indicated in Claim 1, and
X¹ is -B(OH)₂ or 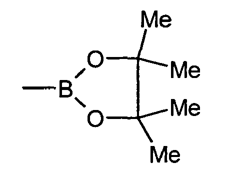 in the case where E is arylene or heteroarylene, and is hydrogen in the case where E is -C≡C-,
are reacted with a compound of the formula 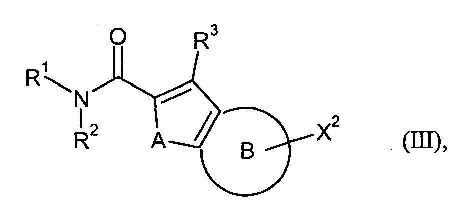 in which
R¹, R², R³, A and the ring B have the meanings indicated in Claim 1, and
X² is triflate or halogen, preferably chlorine, bromine or iodine,
and the resulting compounds (I) are converted into their solvates, salts or solvates of the salts where appropriate with the appropriate (i) solvents and/or (ii) bases or acids.

14. Compounds according to any of Claims 1 to 11 for the treatment and/or prophylaxis of diseases.

15. Medicament comprising at least one compound according to any of Claims 1 to 11 and at least one pharmaceutically acceptable, essentially nontoxic carrier or excipient.

16. Use of compounds according to any of Claims 1 to 11 for producing a composition for improving perception, concentration, learning and/or memory.

17. Use of compounds according to any of Claims 1 to 11 for producing a medicament for the treatment and/or prophylaxis of impairments of perception, concentration, learning and/or memory.

18. Medicament according to Claim 15 for the treatment and/or prophylaxis of impairments of perception, concentration, learning and/or memory.

## Revendications

1. Composés de formule 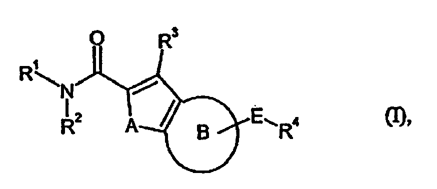 dans laquelle
R¹ est un groupe 1-aza-bicyclo[2.2.2]oct-3-yle qui est éventuellement substitué, par l'intermédiaire de l'atome d'azote, par un reste choisi dans le groupe des restes alkyle en C₁ à C₄, benzyle et oxy,
R² représente l'hydrogène ou un reste alkyle en C₁ à C₆,
R³ représente l'hydrogène, un halogène ou un reste alkyle en C₁ à C₆,
R⁴ représente l'hydrogène, un halogène, un groupe cyano, amino, un reste trifluorométhyle, trifluorométhoxy, alkyle en C₁ à C₆, (alkyle en C₁ à C₆)carbonyle, alkylamino en C₁ à C₆, formyle, hydroxycarbonyle, alkoxy en C₁ à C₆, (alkoxy en C₁ à C₆)carbonyle, alkylthio en C₁ à C₆, (alkyle en C₁ à C₆)carbonylamino, (alkyle en C₁ à C₆)aminocarbonyle, alkylsulfonylamino en C₁ à C₄, (cycloalkyle en C₃ à C₈)carbonylamino, (cycloalkyle en C₃ à C₆)aminocarbonyle, pyrrolyle, (alkyle en C₁ à C₆)aminocarbonylamino, hétérocyclylcarbonyle, hétérocyclylcarbonylamino, hétéroarylcarbonylamino, hydroxy, phényle ou hétérocyclyle,
les restes alkyle en C₁ à C₆ pouvant éventuellement être substitués par un radical hydroxy, cyano, amino, (alkyle en C₁ à C₆)aminocarbonylamino, (alkyle en C₁ à C₆)aminocarboxyle, hétérocyclyle ou aryle,
les restes (alkyle en C₁ à C₆)aminocarbonyle pouvant éventuellement être substitués par un radical alkoxy en C₁ à C₆ ou alkylamino en C₁ à C₆,
les restes (alkyle en C₁ à C₆)carbonylamino pouvant éventuellement être substitués par un radical alkoxy en C₁ à C₆, et les restes hétérocyclyle éventuellement par un radical oxo,
A représente l'oxygène ou le soufre,
le noyau B représente un noyau benzo ou pyrido, chacun étant éventuellement substitué par des restes de la série halogéno, cyano, formyle, trifluorométhyle, trifluorométhoxy, nitro, amino, alkyle en C₁ à C₆ et alkoxy en C₁ à C₆,
et
E représente C≡C, un reste aryle et un reste hétéroaryle, les restes aryle et hétéroaryle pouvant être substitués par des restes de la série halogéno, cyano, trifluorométhyle, trifluorométhoxy, nitro, amino, alkoxy en C₁ à C₆ et alkyle en C₁ à C₆,
ainsi que les produits de solvatation, les sels ou les produits de solvatation des sels de ces composés.

2. Composés suivant la revendication 1, de formule (I) dans laquelle
R¹ est un groupe 1-aza-bicyclo[2.2.2]oct-3-yle,
R² représente l'hydrogène ou un reste alkyle en C₁ à C₄,
R³ représente l'hydrogène, le fluor, le chlore, le brome ou un reste alkyle en C₁ à C₄,
R⁴ représente l'hydrogène, le fluor, le chlore, le brome, un groupe cyano, amino, un reste trifluorométhyle, trifluorométhoxy, alkyle en C₁ à C₄, (alkyle en C₁ à C₄)carbonyle, alkylamino en C₁ à C₄, formyle, hydroxycarbonyle, alkoxy en C₁ à C₄, (alkoxy en C₁ à C₄)carbonyle, alkylthio en C₁ à C₄, (alkyle en C₁ à C₄)carbonylamino, (alkyle en C₁ à C₄)aminocarbonyle, alkylsulfonylamino en C₁ à C₄, (cycloalkyle en C₃ à C₆)carbonylamino, (cycloalkyle en C₃ à C₆)aminocarbonyle, pyrrolyle, (alkyle en C₁ à C₄)aminocarbonylamino, hétérocyclylcarbonyle, hétérocyclylcarbonylamino, hétéroarylcarbonylamino, hydroxy, phényle ou hétérocyclyle,
les restes alkyle en C₁ à C₄ pouvant éventuellement être substitués par un radical hydroxy, cyano, amino, (alkyle en C₁ à C₄)aminocarbonylamino, (alkyle en C₁ à C₄)aminocarboxyle, hétérocyclyle ou aryle,
les restes (alkyle en C₁ à C₄)aminocarbonyle pouvant éventuellement être substitués par un radical alkoxy en C₁ à C₄ ou alkylamino en C₁ à C₄,
les restes (alkyle en C₁ à C₄)carbonylamino pouvant éventuellement être substitués par un reste alkoxy en C₁ à C₄, et les restes hétérocyclyle éventuellement par un radical oxo,
A représente l'oxygène ou le soufre,
le noyau B représente un noyau benzo ou pyrido, chacun étant éventuellement substitué par des restes de la série halogéno, cyano, trifluorométhyle, trifluorométhoxy et alkyle en C₁ à C₄,
et
E représente C=C, un reste aryle et un reste hétéroaryle, les restes aryle et hétéroaryle pouvant être substitués par des restes de la série halogéno, cyano, trifluorométhyle, trifluorométhoxy, nitro, amino, alkoxy en C₁ à C₄ et alkyle en C₁ à C₄,
ainsi que les produits de solvatation, les sels ou les produits de solvatation des sels de ces composés.

3. Composés suivant les revendications 1 et 2, de formule (I) dans laquelle
R¹ est un groupe 1-aza-bicyclo[2.2.2]oct-3-yle,
R² et R³ représentent l'hydrogène,
R⁴ représente l'hydrogène, le fluor, le chlore, le brome, un groupe cyano, amino, un reste trifluorométhyle, trifluorométhoxy, alkyle en C₁ à C₄, (alkyle en C₁ à C₄)carbonyle, alkylamino en C₁ à C₄, formyle, hydroxycarbonyle, alkoxy en C₁ à C₄, (alkoxy en C₁ à C₄)carbonyle, alkylthio en C₁ à C₆, (alkyle en C₁ à C₄)carbonylamino, (alkyle en C₁ à C₄)aminocarbonyle, alkylsulfonylamino en C₁ à C₄, (cycloalkyle en C₃ à C₆)carbonylamino, (cycloalkyle en C₃ à C₆)aminocarbonyle, pyrrolyle, (alkyle en C₁ à C₄)aminocarbonylamino, hétérocyclylcarbonyle, hétérocyclylcarbonylamino, hétéroarylcarbonylamino, hydroxy, phényle ou hétérocyclyle,
les restes alkyle en C₁ à C₄ pouvant éventuellement être substitués par un radical hydroxy, cyano, amino, (alkyle en C₁ à C₄)aminocarbonylamino, (alkyle en C₁ à C₄)aminocarboxyle, hétérocyclyle ou aryle,
les restes (alkyle en C₁ à C₄)aminocarbonyle pouvant éventuellement être substitués par un radical alkoxy en C₁ à C₄ ou alkylamino en C₁ à C₄,
les restes (alkyle en C₁ à C₄)carbonylamino pouvant éventuellement être substitués par un radical alkoxy en C₁ à C₄, et les restes hétérocyclyle éventuellement par un radical oxo,
A représente l'oxygène,
le noyau B représente un noyau benzo ou pyrido, chacun étant éventuellement substitué par des restes de la série halogéno, cyano, trifluorométhyle, trifluorométhoxy et alkyle en C₁ à C₄,
et
E représente C=C, un reste aryle et un reste hétéroaryle, les restes aryle et hétéroaryle pouvant être substitués par des restes de la série halogéno, cyano, trifluorométhyle, trifluorométhoxy, nitro, amino, alkoxy en C₁ à C₄ et alkyle en C₁ à C₄,
ainsi que les produits de solvatation, les sels ou les produits de solvatation des sels de ces composés.

4. Composés de formule (I) suivant les revendications 1 à 3, formule dans laquelle
R¹ est un groupe 1-aza-bicyclo[2.2.2]oct-3-yle,
R² représente l'hydrogène ou un reste alkyle en C₁ à C₆,
R³ représente l'hydrogène, un halogène ou un reste alkyle en C₁ à C₆,
R⁴ représente l'hydrogène, un halogène, un groupe cyano, amino, un reste trifluorométhyle, trifluorométhoxy, alkyle en C₁ à C₆, (alkyle en C₁ à C₆)carbonyle, alkylamino en C₁ à C₆, formyle, hydroxycarbonyle, alkoxy en C₁ à C₆, (alkoxy en C₁ à C₆)carbonyle, alkylthio en C₁ à C₆, (alkyle en C₁ à C₆)carbonylamino, alkylsulfonylamino en C₁ à C₄, (cycloalkyle en C₃ à C₈)carbonylamino, pyrrolyle, (alkyle en C₁ à C₆)aminocarbonylamino, hétérocyclylcarbonyle, phényle ou hétérocyclyle,
les restes alkyle en C₁ à C₆ étant éventuellement substitués par un radical hydroxy, amino, (alkyle en C₁ à C₆)aminocarbonylamino, (alkyle en C₁ à C₆)aminocarboxyle, hétérocyclyle ou aryle,
les restes (alkyle en C₁ à C₆)carbonylamino étant éventuellement substitués par un radical alkoxy en C₁ à C₆,
et
les restes hétérocyclyle éventuellement par un radical oxo,
A représente l'oxygène ou le soufre,
le noyau B représente un noyau benzo ou pyrido, chacun étant éventuellement substitué par des restes de la série halogéno, cyano, formyle, trifluorométhyle, trifluorométhoxy, nitro, amino, alkyle en C₁ à C₆ et alkoxy en C₁ à C₆,
et
E représente C=C, un reste aryle et un reste hétéroaryle, les restes aryle et hétéroaryle étant éventuellement substitués par des restes de la série halogéno, cyano, trifluorométhyle, trifluorométhoxy, nitro, amino, alkoxy en C₁ à C₆ et alkyle en C₁ à C₆,
ainsi que les produits de solvatation, les sels ou les produits de solvatation des sels de ces composés.

5. Composés de formule (I) suivant les revendications 1 à 4, formule dans laquelle
R¹ est un groupe 1-aza-bicyclo[2.2.2]oct-3-yle,
R² représente l'hydrogène ou un reste alkyle en C₁ à C₆,
R³ représente l'hydrogène, un halogène ou un reste alkyle en C₁ à C₆,
R⁴ représente l'hydrogène, un halogène, un groupe cyano, un reste trifluorométhyle, trifluorométhoxy, alkyle en C₁ à C₆, alkoxy en C₁ à C₆ ou hétérocyclyle, le reste alkyle étant éventuellement substitué par un reste hydroxy,
A représente l'oxygène ou le soufre,
le noyau B représentant des noyaux benzo ou pyrido qui sont substitués chacun éventuellement par des restes de la série halogéno, cyano, trifluorométhyle, trifluorométhoxy, nitro, amino, alkyle en C₁ à C₆ ou alkoxy en C₁ à C₆,
et
E représente C=C, un reste arylène ou un reste hétéroarylène, les restes arylène et hétéroarylène étant éventuellement substitués par des restes de la série halogéno, cyano, trifluorométhyle, trifluorométhoxy, nitro, amino, alkyle en C₁ à C₆, et alkoxy en C₁ à C₆,
ainsi que les produits de solvatation, les sels ou les produits de solvatation des sels de ces composés.

6. Composés suivant la revendication 1 à 5, de formule 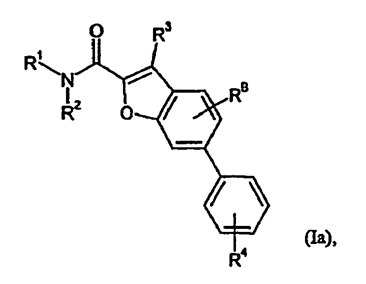 dans laquelle
R¹ est un groupe (3*R*)-1-aza-bicyclo[2.2.2]oct-3-yle,
R² et R³ représentent, indépendamment l'un de l'autre, l'hydrogène ou un reste méthyle,
R⁴ représente l'hydrogène, un halogène, un groupe cyano, un reste trifluorométhyle, trifluorométhoxy, alkyle en C₁ à C₆, alkoxy en C₁ à C₆ ou hétérocyclyle, le reste alkyle étant éventuellement substitué par un reste hydroxy, et
et
R^{B} représente l'hydrogène, un halogène, un groupe cyano, un reste trifluorométhyle, trifluorométhoxy, nitro, amino, alkyle en C₁ à C₆ ou alkoxy en C₁ à C₆,
ainsi que les produits de solvatation, les sels ou les produits de solvatation des sels de ces composés.

7. Composés suivant les revendications 1 à 6, de formule 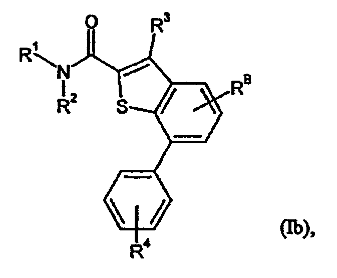 dans laquelle
R¹ est un groupe (3*R*)-1-aza-bicyclo[2.2.2]oct-3-yle,
R² et R³ représentent, indépendamment l'un de l'autre, l'hydrogène ou le reste méthyle,
R⁴ représente l'hydrogène, un halogène, un groupe cyano, un reste trifluorométhyle, trifluorométhoxy, alkyle en C₁ à C₆, alkoxy en C₁ à C₆ ou hétérocyclyle, le reste alkyle étant éventuellement substitué par un reste hydroxy, et
R^{B} représente l'hydrogène, un halogène, un groupe cyano, un reste trifluorométhyle, trifluorométhoxy, un groupe nitro, amino, un reste alkyle en C₁ à C₆ ou alkoxy en C₁ à C₆,
ainsi que les produits de solvatation, les sels ou les produits de solvatation de sels de ces composés.

8. Composés suivant les revendications 1 à 7, dans lesquels
R¹ est un groupe (3*R*)-1-aza-bicyclo[2.2.2]oct-3-yle,
R² et R³ représentent l'hydrogène,
R⁴ représente l'hydrogène, le fluor, le chlore, le brome, un reste trifluorométhoxy, hydroxyméthyle, méthoxy ou un reste hétérocyclyle hexagonal, et
R^{B} représente l'hydrogène, un halogène, un groupe cyano, un reste trifluorométhyle, trifluorométhoxy ou alkyle en C₁ à C₄,
ainsi que les produits de solvatation, les sels ou les produits de solvatation des sels de ces composés.

9. Composés suivant les revendications 1 à 8, de formule 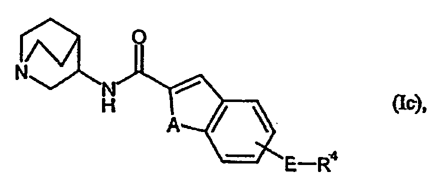 dans laquelle
E est un groupe phénylène,
R⁴ est un reste alkoxy en C₁ à C₆, aminoéthylène, hydroxycarbonyle, (cycloalkyle en C₃ à C₈)carbonylamino, un groupe de formule 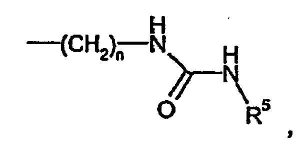 dans laquelle
R⁵ est un reste alkyle en C₁ à C₆,
n a la valeur 0, 1, 2, 3 ou 4,
ou
un reste hétérocyclyle pentagonal ou hexagonal qui est éventuellement substitué avec un groupe oxo,
A représente le soufre ou l'oxygène,
ainsi que leurs produits de solvatation, leurs sels, ou les produits de solvatation de leurs sels.

10. Composés suivant les revendications 1 à 9, de formule (Ic), dans laquelle
E est un groupe phénylène,
R⁴ est un reste alkoxy en C₁ à C₄, aminoéthylène, hydroxycarbonyle, (cycloalkyle en C₃ à C₆)carbonylamino, un groupe de formule 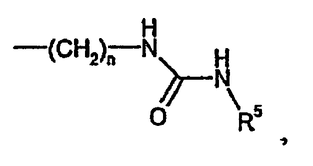 dans laquelle
R⁵ est un reste alkyle en C₁ à C₄,
n a la valeur 0, 1 ou 2,
ou
un reste hétérocyclyle pentagonal ou hexagonal qui est éventuellement substitué par un groupe oxo,
A représente le soufre ou l'oxygène,
ainsi que leurs produits de solvatation, leurs sels, ou les produits de solvatation de leurs sels.

11. Composés suivant les revendications 1 à 10, de formules suivantes 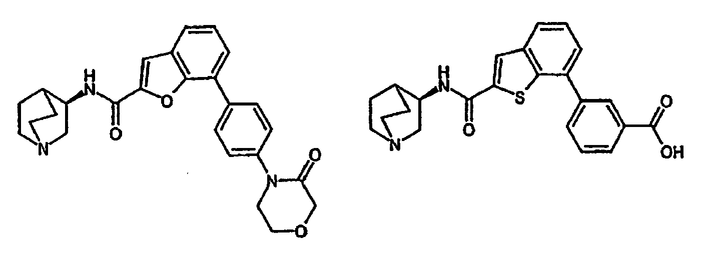 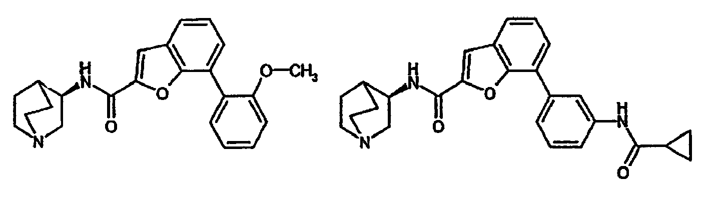 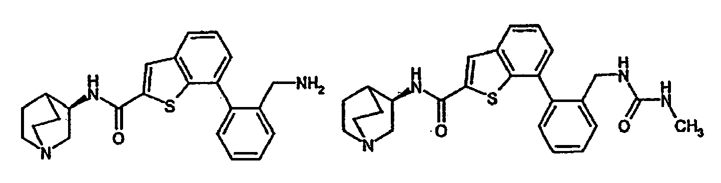 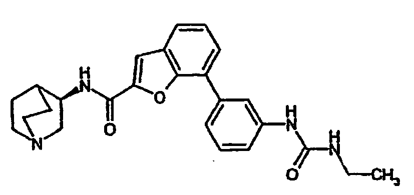 ainsi que les produits de solvatation, les sels ou les produits de solvatation de sels de ces composés.

12. Procédé de production des composés de formule (I), selon lequel on fait réagir des composés de
**X**^{**1**}**-E-R**^{**4**} **(II),**
dans laquelle
R⁴ a les définitions indiquées dans la revendication 1, et
X¹, au cas où E est un reste arylène ou hétéroarylène, représente -B(OH)₂ ou 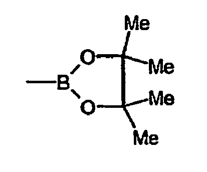 et représente l'hydrogène au cas où E est un groupe -C≡C-, avec un composé de formule 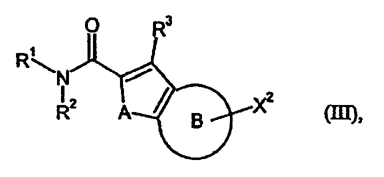
dans laquelle
R¹, R², R³, A et le noyau B ont les définitions indiquées dans la revendication 1, et
X² est un groupe triflate ou un halogène, avantageusement le chlore, le brome ou l'iode,
et, le cas échéant,
[A] on alkyle les composés (I) résultants avec des réactifs d'alkylation correspondants sur l'atome d'azote de la quinuclidine, ou bien
[B] on oxyde les composés (I) résultants avec des agents oxydants convenables sur l'atome d'azote de la quinuclidine,
et, le cas échéant, on fait réagir les composés (I) résultants avec les solvants (i) correspondants et/ou les bases ou les acides (ii) correspondants pour former leurs produits de solvatation, leurs sels ou les produits de solvatation des sels.

13. Procédé de production des composés conformes à l'invention, selon lequel on fait réagir des composés de formule
**X**^{**1**}**-E-R**^{**4**} **(II),**
dans laquelle
R⁴ a les définitions indiquées dans la revendication 1, et
X¹, au cas où E est un reste arylène ou hétéroarylène, représente -B(OH)₂, ou 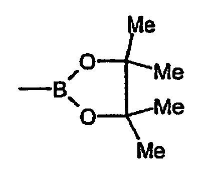 et représente l'hydrogène au cas où E est un groupe -C≡C-, avec un composé de formule 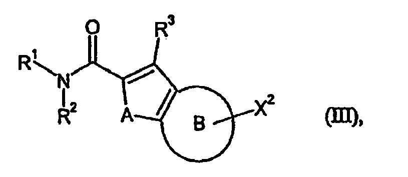
dans laquelle
R¹ , R², R³, A et le noyau B ont les définitions indiquées dans la revendication 1, et
X² est un groupe triflate ou un halogène, avantageusement le chlore, le brome ou l'iode,
et on fait réagir les composés (I) résultants, éventuellement avec les solvants (i) correspondants et/ou les bases ou les acides (ii) correspondants pour former leurs produits de solvatation, leurs sels ou les produits de solvatation des sels.

14. Composés suivant l'une des revendications 1 à 11, destinés au traitement et/ou à la prophylaxie de maladies.

15. Médicaments contenant au moins un composé suivant l'une des revendications 1 à 11, et au moins un support ou excipient pharmaceutiquement acceptable, principalement non toxique.

16. Utilisation de composés suivant l'une des revendications 1 à 11, pour la préparation d'un produit destiné à améliorer la perception, la capacité de concentration, la capacité d'apprentissage et/ou la capacité de mémoire.

17. Utilisation de composés suivant l'une des revendications 1 à 11, pour la préparation d'un médicament destiné au traitement et/ou à la prophylaxie de troubles de la perception, de la capacité de concentration, de la capacité d'apprentissage et/ou de la capacité de mémoire.

18. Médicament suivant la revendication 15, destiné au traitement et/ou à la prophylaxie de troubles de la perception, de la capacité de concentration, de la capacité d'apprentissage et/ou de la capacité de mémoire.
